# EUROPEAN PATENT APPLICATION

(11) **EP 4 389 893 A1**
(43) Date of publication of application: **26.06.2024**
(21) Application number: 22861282.6
(22) Date of filing: 19.08.2022
(51) Int. Cl.: C12N 15/113, A61K 31/7088, A61K 31/712, A61K 31/7125, A61K 31/713, A61K 38/10, A61K 47/64, A61K 48/00, A61P 21/00, C07K 7/08

(54) **HUMAN TRANSFERRIN RECEPTOR BINDING PEPTIDE-DRUG CONJUGATE**

(30) Priority: 21.08.2021 JP 2021135254
(71) Applicant: Takeda Pharmaceutical Company Limited, Osaka-shi, Osaka 541-0045 (JP)
(72) Inventor: YOGO, Takatoshi, Fujisawa-shi, Kanagawa 251-0012 (JP); SUGIYAMA, Hideyuki, Fujisawa-shi, Kanagawa 251-0012 (JP); MIYATA, Kenichi, Fujisawa-shi, Kanagawa 251-0012 (JP); TAKADA, Hiroyuki, Fujisawa-shi, Kanagawa 251-0012 (JP); YOSHIDA, Masato, Fujisawa-shi, Kanagawa 251-0012 (JP); TOKUNOH, Ryosuke, Fujisawa-shi, Kanagawa 251-0012 (JP); NAKAGAWA, Yasuo, Fujisawa-shi, Kanagawa 251-0012 (JP); NIWA, Masato, Fujisawa-shi, Kanagawa 251-0012 (JP); SASAKI, Shigekazu, Fujisawa-shi, Kanagawa 251-0012 (JP); OHUCHI, Masaki, Kawasaki-shi, Kanagawa 210-0821 (JP); SAWAI, Naoki, Kawasaki-shi, Kanagawa 210-0821 (JP); TAKUWA, Masatoshi, Kawasaki-shi, Kanagawa 210-0821 (JP); UCHINO, Yujiro, Fujisawa-shi, Kanagawa 251-0012 (JP); KANEMATSU, Yoko, Fujisawa-shi, Kanagawa 251-0012 (JP); FUKUDA, Koichiro, Fujisawa-shi, Kanagawa 251-0012 (JP); YOKOTA, Takanori, Tokyo 113-8510 (JP); NAGATA, Tetsuya, Tokyo 113-8510 (JP); YAMADA, Hiroki, Tokyo 113-8510 (JP)
(74) Representative: Jones, Nicholas Andrew
(86) International application number: PCT/JP2022/031434
(87) International publication number: WO 2023/026994

(57) **Abstract**

The present invention aims to provide a novel drug delivery system (DDS) technique capable of selectively delivering a drug (compound containing oligonucleotides for producing at least partially functional dystrophin protein) to muscle tissues such as cardiac muscle, skeletal muscle and the like and efficiently introducing the drug into the muscle cells. The present invention relates to a conjugate or a salt thereof including the following: (1) a peptide that binds to a transferrin receptor, and contains the amino acid sequence shown in SEQ ID NO: 1 (Ala-Val-Phe-Val-Trp-Asn-Tyr-Tyr-Ile-Ile-Ser-Cys); or an amino acid sequence resulting from substitution, deletion, addition, and/or insertion of not less than one and not more than 10 amino acid residues in the amino acid sequence shown in SEQ ID NO: 1, and (2) a compound comprising an oligonucleotide for producing an at least partially functional dystrophin protein.

## Description

### [Technical Field]

The present invention relates to a conjugate (hereinafter also to be referred to as a complex) containing a peptide capable of binding to a human transferrin receptor (hTfR) and a drug (compound containing oligonucleotide for producing at least partially functional dystrophin protein). The present invention also relates to a conjugate containing a peptide having directivity to muscle tissue and a drug. The present invention further relates to a medicament containing the conjugate.

### (Background of the Invention)

Duchenne muscular dystrophy is an X-linked recessive inheritance muscular disease in which functional dystrophin protein is no longer produced due to mutations in the dystrophin gene. Dystrophin protein is involved in the membrane stability of muscle cells and makes muscle cells less likely to break down. In patients with Duchenne muscular dystrophy, the muscle cell membrane becomes susceptible to damage caused by muscle contraction due to dystrophin deficiency, which results in muscle cell death, as well as inflammation, muscle fibrosis, and decreased muscle function associated therewith.

The dystrophin gene is composed of 79 exons, and the protein encoded thereby consists of 3685 amino acids and has a molecular weight of 427 kDa. Both ends thereof are essential for the function, but the middle part is a repetition of the basic structure. Thus, even when the middle part is slightly shortened, the function is still exhibited partially as long as the structures of the both ends are maintained. In Duchenne muscular dystrophy, the reading frame of codon is shifted due to deletion or duplication of exon unit, single base insertion or deletion, splice site mutation, and the like in the dystrophin gene (out-of-frame mutation), or a nonsense mutation is caused by a single nucleotide substitution. As a result, a stop codon appears in the middle of translation and the translation is stopped, and a dystrophin protein lacking the C-terminal region is synthesized, but it is rapidly degraded due to its unstable structure.

Exon skipping is used as a treatment method for Duchenne muscular dystrophy. Exon skipping is a therapeutic strategy to alleviate the symptoms of Duchenne muscular dystrophy by using antisense oligonucleotide (ASO) to correct the shift of reading frame caused by out-of-frame mutation by skipping one or more exons, or when an immature stop codon occurs due to a nonsense mutation, by skipping the exon in which the mutation is present, to make the number of deleted bases at the mRNA level by a multiple of 3 (when the number of bases in the exon is not a multiple of 3, adjacent exons are further skipped to make the number of deleted bases a multiple of 3), thereby producing partially functional dystrophin protein.

At present, Eteplirsen and SRP-5051, which target exon 51, Viltolarsen (Patent Literature 1) and Golodirsen, which target exon 53, and Renadirsen and NS-089, which target exon 45, have been approved or are undergoing clinical trials in Japan and overseas.

However, in order to efficiently perform exon skipping therapy for DMD, it is necessary to deliver a drug (compound containing oligonucleotide for producing at least partially functional dystrophin protein) to the cardiac muscle and skeletal muscle tissue and efficiently introduce the drug into the muscle cells.

### [Citation List]

### [Patent Literature]

[Patent Literature 1]
WO2012/029986

### [Summary of Invention]

### [Technical Problem]

The present invention aims to provide a novel drug delivery system (DDS) technique capable of delivering a drug (compound containing oligonucleotide for producing at least partially functional dystrophin protein) to muscle tissues such as cardiac muscle, skeletal muscle and the like and efficiently introducing the drug into the muscle cells. More specifically, the present invention aims to provide a conjugate containing a peptide having directivity to muscle tissue and a drug (compound containing oligonucleotide for producing at least partially functional dystrophin protein).

Furthermore, the present invention aims to provide a medicament containing the above-mentioned conjugate.

### [Solution to Problem]

The present inventors have conducted intensive studies in an attempt to achieve the above-mentioned purposes and found that a conjugate in which a peptide having a specific structure and a compound containing oligonucleotide for producing at least partially functional dystrophin protein are bound directly or via a linker is superior in directivity to muscle cell.

The present inventors have conducted further studies based on these findings and completed the present invention.

Accordingly, the present invention provides the following.
[1] A conjugate or a salt thereof comprising the following:
   (1) a peptide that binds to a transferrin receptor, and comprises
      the amino acid sequence shown in SEQ ID NO: 1 (Ala-Val-Phe-Val-Trp-Asn-Tyr-Tyr-Ile-Ile-Ser-Cys); or
      an amino acid sequence resulting from substitution, deletion, addition, and/or insertion of not less than one and not more than 10 amino acid residues in the amino acid sequence shown in SEQ ID NO: 1, and
   (2) a compound comprising an oligonucleotide for producing an at least partially functional dystrophin protein.
[1-1] The conjugate or a salt thereof of the above-mentioned [1], wherein
   the aforementioned peptide that binds to a transferrin receptor is a peptide comprising
   the amino acid sequence shown in SEQ ID NO: 296 (Ala-Val-MeF-Val-Trp-Asn-Tyr-Tyr-Ile-Ile-Arg-Arg-Tyr-MeY-Cys); or an amino acid sequence resulting from substitution, deletion, addition, and/or insertion of not less than one and not more than 10 amino acid residues in the amino acid sequence shown in SEQ ID NO: 296.
[1-2] The conjugate or a salt thereof of the above-mentioned [1] or [1-2], wherein
   the aforementioned peptide that binds to a transferrin receptor is a peptide comprising
   the amino acid sequence shown in SEQ ID NO: 296; or an amino acid sequence resulting from substitution, deletion, addition, and/or insertion of not less than one and not more than 5 amino acid residues in the amino acid sequence shown in SEQ ID NO: 296.
[1-3] The conjugate or a salt thereof of any one of the above-mentioned [1], [1-1], and [1-2], wherein the aforementioned peptide that binds to a transferrin receptor is a peptide comprising
   the amino acid sequence shown in SEQ ID NO: 296; or an amino acid sequence resulting from substitution of any of the 3rd, 5th, 7th, 8th, 11th, 12th, and 13th amino acid residues of the SEQ ID NO: 296.
[1-4] The conjugate or a salt thereof of any one of the above-mentioned [1] and [1-1] to [1-3], wherein the aforementioned peptide that binds to a transferrin receptor is any of peptides in which
   the 3rd amino acid residue of SEQ ID NO: 296 is an optionally modified phenylalanine (Phe),
   the 5th amino acid residue of SEQ ID NO: 296 is an optionally modified tryptophan (Trp),
   the 7th amino acid residue of SEQ ID NO: 296 is an optionally modified tyrosine (Tyr),
   the 8th amino acid residue of SEQ ID NO: 296 is an optionally modified tyrosine (Tyr),
   the 11th amino acid residue of SEQ ID NO: 296 is an optionally modified arginine (Arg) or an optionally modified lysine (Lys),
   the 12th amino acid residue of SEQ ID NO: 296 is an optionally modified arginine (Arg) or an optionally modified lysine (Lys), or
   the 13th amino acid residue of SEQ ID NO: 296 is an optionally modified tyrosine (Tyr) or an optionally modified phenylalanine (Phe).
[1-5] The conjugate or a salt thereof of any one of the above-mentioned [1] and [1-1] to [1-4], wherein the aforementioned peptide that binds to a transferrin receptor is a peptide in which
   the 3rd amino acid residue of SEQ ID NO: 296 is phenylalanine (Phe), methylated phenylalanine (MeF), or N-methyl-3-chloro-L-phenylalanine (MeF3C),
   the 5th amino acid residue of SEQ ID NO: 296 is tryptophan (Trp) or methylated tryptophan (MeW),
   the 7th amino acid residue of SEQ ID NO: 296 is tyrosine (Tyr),
   the 8th amino acid residue of SEQ ID NO: 296 is tyrosine (Tyr) or (S)-2-amino-3-(4-methoxyphenyl)propanoic acid (F4OMe),
   the 11th amino acid residue of SEQ ID NO: 296 is arginine (Arg) or lysine (Lys),
   the 12th amino acid residue of SEQ ID NO: 296 is arginine (Arg) or D-type arginine (dr), and
   the 13th amino acid residue of SEQ ID NO: 296 is tyrosine (Tyr) or phenylalanine (Phe).
[1-6] The conjugate or a salt thereof of the above-mentioned [1], wherein the aforementioned peptide that binds to a transferrin receptor is the amino acid sequence shown in SEQ ID NO: 296 (Ala-Val-MeF-Val-Trp-Asn-Tyr-Tyr-Ile-Ile-Arg-Arg-Tyr-MeY-Cys) .
[1-7] The conjugate or a salt thereof of the above-mentioned [1], wherein the aforementioned peptide that binds to a transferrin receptor is the amino acid sequence shown in SEQ ID NO: 300 (Ala-Val-MeF-Val-MeW-Asn-Tyr-Tyr-Ile-Ile-Lys-Arg-Tyr-MeY-Cys) .
[1-8] The conjugate or a salt thereof of any one of the above-mentioned [1] and [1-1] to [1-7], wherein the aforementioned peptide that binds to a transferrin receptor is a cyclic peptide.
[1-9] The conjugate or a salt thereof of any one of the above-mentioned [1] and [1-1] to [1-8], wherein the aforementioned peptide that binds to a transferrin receptor consists of 15 amino acid residues.
[1-10] The conjugate or a salt thereof of any one of the above-mentioned [1-1] to [1-9], wherein the aforementioned peptide that binds to a transferrin receptor is a peptide consisting of the 1st to the 15th amino acid sequences, and the amino acid sequence site has a cyclic structure.
[2] The conjugate or a salt thereof of any of the above-mentioned [1], and [1-1] to [1-10], wherein the aforementioned oligonucleotide is an oligonucleotide that induces exon skipping of the dystrophin gene.
[3] The conjugate or a salt thereof of the above-mentioned [2], wherein the aforementioned exon is selected from exon 7, exon 8, exon 9, exon 19, exon 23, exon 44, exon 45, exon 46, exon 50, exon 51, exon 52, exon 53, exon 55, or any combination of these exons.
[3-1] The conjugate or a salt thereof of the above-mentioned [3], wherein the aforementioned exon is exon 44.
[3-2] The conjugate or a salt thereof of the above-mentioned [3], wherein the aforementioned exon is exon 53.
[4] The conjugate or a salt thereof of any one of the above-mentioned [1] to [3], [1-1] to [1-10], [3-1], and [3-2], wherein a sugar moiety and/or a phosphoric acid binding site of at least one nucleotide constituting the aforementioned compound comprising an oligonucleotide is modified.
[5] The conjugate or a salt thereof of any one of the above-mentioned [1] to [4], [1-1] to [1-10], [3-1], and [3-2], wherein the aforementioned compound comprising an oligonucleotide comprises an antisense oligonucleotide of 10 to 35 bases in length.
[5-1] The conjugate or a salt thereof of any one of the above-mentioned [1] to [5], [1-1] to [1-10], [3-1], and [3-2], wherein the aforementioned compound comprising an oligonucleotide comprises an antisense oligonucleotide of 16 to 30 bases in length.
[5-2] The conjugate or a salt thereof of any one of the above-mentioned [1] to [5], [1-1] to [1-10], [3-1], [3-2] and [5-1], wherein the aforementioned compound comprising an oligonucleotide comprises an antisense oligonucleotide of 18 to 25 bases in length.
[6] The conjugate or a salt thereof of any one of the above-mentioned [5], [5-1], and [5-2], wherein the aforementioned antisense oligonucleotide comprises a sequence complementary to 12 or more continuous nucleotides in the target region of dystrophin mRNA.
[6-1] The conjugate or a salt thereof of the above-mentioned [6], wherein the target region of the aforementioned dystrophin mRNA is TACAAGAACACCTTCAGAACCGGAGGCAACAGTTG (SEQ ID NO: 647).
[6-2] The conjugate or a salt thereof of any one of the above-mentioned [5], [5-1], [5-2], [6], and [6-1], wherein the aforementioned antisense oligonucleotide consists of a sequence complementary to GAACACCTTCAGAACCGGAGGCAAC (SEQ ID NO: 648).
[6-3] The conjugate or a salt thereof of any one of the above-mentioned [5], [5-1], [5-2], [6], and [6-1], wherein the aforementioned antisense oligonucleotide consists of a sequence complementary to GAACACCTTCAGAACCGGAGG (SEQ ID NO: 649).
[6-4] The conjugate or a salt thereof of any one of the above-mentioned [6] and [6-1] to [6-3], wherein the complementary sequence is not less than 90% complementary.
[6-5] The conjugate or a salt thereof of any one of the above-mentioned [6] and [6-1] to [6-3], wherein the complementary sequence is 100% complementary.
[7] The conjugate or a salt thereof of any one of the above-mentioned [5], [6], [5-1], [5-2], and [6-1] to [6-5], wherein the aforementioned antisense oligonucleotide is a phosphorodiamidatemorpholino oligomer.
[8] The conjugate or a salt thereof of any one of the above-mentioned [1] to [7], [1-1] to [1-8], [3-1], [3-2], [5-1], [5-2], and [6-1] to [6-5], wherein the aforementioned compound comprising an oligonucleotide is a compound composed of an antisense oligonucleotide.
[9] The conjugate or a salt thereof of any one of the above-mentioned [1] to [7], [1-1] to [1-10], [3-1], [3-2], [5-1], [5-2], and [6-1] to [6-5], wherein the aforementioned compound comprising an oligonucleotide consists of a nucleic acid complex comprising an antisense oligonucleotide and a second nucleic acid strand complementary to all or part of the antisense oligonucleotide, wherein the antisense oligonucleotide anneals to the second nucleic acid strand.
[10] The conjugate or a salt thereof of any one of the above-mentioned [1] to [9], [1-1] to [1-10], [3-1], [3-2], [5-1], [5-2], and [6-1] to [6-5], wherein the aforementioned peptide and the compound comprising an oligonucleotide are bound via a linker.
[11] A composition comprising the conjugate or a salt thereof of any one of the above-mentioned [1] to [10], [1-1] to [1-8], [3-1], [3-2], [5-1], [5-2], and [6-1] to [6-5], for delivering the conjugate or a salt thereof to at least one selected from cardiac muscle and skeletal muscle.
[12] A medicament comprising the conjugate or a salt thereof of any one of the above-mentioned [1] to [10], [1-1] to [1-10], [3-1], [3-2], [5-1], [5-2], and [6-1] to [6-5] as an active ingredient.
[13] The medicament of the above-mentioned [12], which is a prophylactic or therapeutic agent for Duchenne muscular dystrophy.
[14] The conjugate or a salt thereof of any one of the above-mentioned [1] to [10], [1-1] to [1-10], [3-1], [3-2], [5-1], [5-2], and [6-1] to [6-5], for use in the prophylaxis or treatment of Duchenne muscular dystrophy.
[15] A method for the prophylaxis or treatment of Duchenne muscular dystrophy in a mammal, comprising administering the conjugate or a salt thereof of any one of [1] to [10], [1-1] to [1-10], [3-1], [3-2], [5-1], [5-2], and [6-1] to [6-5] to the mammal.
[2A] The conjugate or a salt thereof of the above-mentioned [1], wherein the peptide is a peptide comprising an amino acid sequence having one or more substitutions selected from the following group:
   (I) substitution of alanine residue at position 1 of SEQ ID NO: 1 with aliphatic amino acid or methylated aliphatic amino acid;
   (II) substitution of valine residue at position 2 of SEQ ID NO: 1 with basic amino acid residue or methylated basic amino acid residue;
   (III) substitution of phenylalanine residue at position 3 of SEQ ID NO: 1 with aromatic amino acid residue, methylated aromatic amino acid residue, amino acid residue with aromatic ring added, or amino acid residue with fused ring added;
   (IV) substitution of valine residue at position 4 of SEQ ID NO: 1 with methylated valine residue;
   (V) substitution of tryptophan residue at position 5 of SEQ ID NO: 1 with aromatic amino acid residue, methyltryptophan residue, methylated aromatic amino acid residue, amino acid residue with aromatic ring added, or amino acid residue with fused ring added;
   (VI) substitution of asparagine residue at position 6 of SEQ ID NO: 1 with neutral amino acid or methylated neutral amino acid;
   (VII) substitution of tyrosine residue at positions 7 and 8 of SEQ ID NO: 1 with aromatic amino acid residue, methylated aromatic amino acid residue, amino acid residue with aromatic ring added, or amino acid residue with fused ring added;
   (VIII) substitution of isoleucine residue at position 9 of SEQ ID NO: 1 with aliphatic amino acid residue, methylated aliphatic amino acid residue, or amino acid residue with branched chain structure;
   (IX) substitution of isoleucine residue at position 10 of SEQ ID NO: 1 with any amino acid; and
   (X) substitution of serine residue at position 11 of SEQ ID NO: 1 with neutral amino acid residue.
[3A] The conjugate or a salt thereof of the above-mentioned [1], wherein the peptide is a peptide comprising an amino acid sequence shown in SEQ ID NO: 2 (Ala-Val-Phe-Val-Trp-Asn-Tyr-Tyr-Ile-Ile-Arg-Arg-Tyr-MeTyr-Cys), or a peptide comprising an amino acid sequence having one or more substitutions, in the amino acid sequence shown in SEQ ID NO:2, selected from the following group:
   (I) substitution of alanine residue at position 1 of SEQ ID NO: 2 with aliphatic amino acid or methylated aliphatic amino acid;
   (II) substitution of valine residue at position 2 of SEQ ID NO: 2 with basic amino acid residue or methylated basic amino acid residue;
   (III) substitution of phenylalanine residue at position 3 of SEQ ID NO: 2 with aromatic amino acid residue, methylated aromatic amino acid residue, amino acid residue with aromatic ring added, or amino acid residue with fused ring added;
   (IV) substitution of valine residue at position 4 of SEQ ID NO: 2 with methylated valine residue;
   (V) substitution of tryptophan residue at position 5 of SEQ ID NO: 2 with aromatic amino acid residue, methylated aromatic amino acid residue, amino acid residue with aromatic ring added, or amino acid residue with fused ring added;
   (VI) substitution of asparagine residue at position 6 of SEQ ID NO: 2 with neutral amino acid or methylated neutral amino acid;
   (VII) substitution of tyrosine residue at positions 7 and 8 of SEQ ID NO: 2 with aromatic amino acid residue, methylated aromatic amino acid residue, amino acid residue with aromatic ring added, or amino acid residue with fused ring added;
   (VIII) substitution of isoleucine residue at position 9 of SEQ ID NO: 2 with aliphatic amino acid residue, methylated aliphatic amino acid residue, or amino acid residue with branched chain structure;
   (IX) substitution of isoleucine residue at position 10 of SEQ ID NO: 2 with any amino acid;
   (XI) substitution of arginine residue at positions 11 and 12 of SEQ ID NO: 2 with basic amino acid residue;
   (XII) substitution of tyrosine residue at position 13 of SEQ ID NO: 2 with hydrophilic amino acid residue;
   (XIII) substitution of methyltyrosine residue at position 14 of SEQ ID NO: 2 with tyrosine residue, aromatic amino acid residue, or methylated aromatic amino acid residue; and
   (XIV) substitution of cysteine residue at position 15 of SEQ ID NO: 2 with methylated cysteine residue.
[4A] The conjugate or a salt thereof of the above-mentioned [1], wherein the peptide is any of
   peptide A which is a peptide having a peptide length of not less than 10 and not more than 17 and comprising the 1st to the 10th amino acid sequence of SEQ ID NO: 18 (Ala-Val-Phe-Val-Trp-Asn-Tyr-Tyr-Ile-Val-Ser-Cys),
   a peptide comprising an amino acid sequence resulting from substitution, deletion, and/or insertion of not less than 1 and not more than 6 amino acid residues in peptide A,
   a peptide comprising an amino acid sequence resulting from substitution of any of the 2nd, 3rd, 5th, 8th, and 10th amino acid residues of SEQ ID NO: 18 in peptide A, and
   a peptide in which
   the 2nd amino acid residue of SEQ ID NO: 18 is an optionally modified valine (Val) or optionally modified glutamic acid (Glu),
   the 3rd amino acid residue of SEQ ID NO: 18 is an optionally modified phenylalanine (Phe),
   the 5th amino acid residue of SEQ ID NO: 18 is an optionally modified tryptophan (Trp),
   the 8th amino acid residue of SEQ ID NO: 18 is an optionally modified tyrosine (Tyr), and
   the 10th amino acid residue of SEQ ID NO: 18 is an optionally modified isoleucine (Ile) or optionally modified valine (Val), in peptide A.
[5A] The conjugate or a salt thereof of the above-mentioned [4A], wherein the length of the peptide is not less than 11 and not more than 13.
[6A] The conjugate or a salt thereof of the above-mentioned [1], wherein the peptide is any of
   peptide B which is a peptide having a peptide length of not less than 10 and not more than 19 and comprising the 1st to the 10th amino acid sequence of SEQ ID NO: 15 (Ala-Val-Phe-Val-Trp-Asn-Tyr-Tyr-Ile-Val-Pro-Arg-Asp-Cys),
   a peptide comprising an amino acid sequence resulting from substitution, deletion, and/or insertion of not less than 1 and not more than 5 amino acid residues in peptide B,
   a peptide comprising an amino acid sequence resulting from substitution of any of the 2nd, 3rd, 5th, 8th, and 10th amino acid residues of SEQ ID NO: 15 in peptide B, and
   a peptide in which
   the 2nd amino acid residue of SEQ ID NO: 15 is an optionally modified valine (Val) or optionally modified glutamic acid (Glu),
   the 3rd amino acid residue of SEQ ID NO: 15 is an optionally modified phenylalanine (Phe) or tryptophan (Trp),
   the 5th amino acid residue of SEQ ID NO: 15 is an optionally modified tryptophan (Trp),
   the 8th amino acid residue of SEQ ID NO: 15 is an optionally modified tyrosine (Tyr), and
   the 10th amino acid residue of SEQ ID NO: 15 is an optionally modified isoleucine (Ile) or optionally modified valine (Val), in peptide B.
[7A] The conjugate or a salt thereof of the above-mentioned [6A], wherein the length of the peptide is not less than 13 and not more than 15.
[8A] The conjugate or a salt thereof of the above-mentioned [1], wherein the peptide is any of
   peptide C which is a peptide having a peptide length of not less than 11 and not more than 19 and comprising the 1st to the 10th amino acid sequence of SEQ ID NO: 214 (MeA-Val-MeF3C-Val-MeW-Asn-Tyr-F4OMe-Ile-Ile-Arg-Arg-Phe-MeY-Cys),
   a peptide comprising an amino acid sequence resulting from substitution, deletion, and/or insertion of not less than 1 and not more than 5 amino acid residues in peptide C,
   a peptide comprising an amino acid sequence resulting from substitution of any of the 1st, 3rd, 5th, and 8th amino acid residues of SEQ ID NO: 214 in peptide C, and
   a peptide in which
   the 1st amino acid residue of SEQ ID NO: 214 is an optionally modified alanine (Ala) or optionally modified glutamic acid (Glu),
   the 3rd amino acid residue of SEQ ID NO: 214 is an optionally modified phenylalanine (Phe),
   the 5th amino acid residue of SEQ ID NO: 214 is an optionally modified tryptophan (Trp), and
   the 8th amino acid residue of SEQ ID NO: 214 is an optionally modified phenylalanine (Phe), in peptide C.
[9A] The conjugate or a salt thereof of the above-mentioned [8A], wherein the length of the peptide is not less than 15 and not more than 18.
[10A] The conjugate or a salt thereof of the above-mentioned [1], wherein the peptide is any of
   peptide D which is a peptide having a peptide length of not less than 11 and not more than 19 and comprising the 1st to the 10th amino acid sequence of SEQ ID NO: 219 (Ala-Glu-Phe-Val-Trp-Asn-Tyr-Tyr-Ile-Ile-Arg-Arg-Tyr-MeY-Cys),
   a peptide comprising an amino acid sequence resulting from substitution, deletion, and/or insertion of not less than 1 and not more than 5 amino acid residues in peptide D,
   a peptide comprising an amino acid sequence resulting from substitution of any of the 2nd, 3rd, 5th, 8th, and 10th amino acid residues of SEQ ID NO: 219 in peptide D, and
   a peptide in which
   the 2nd amino acid residue of SEQ ID NO: 219 is an optionally modified valine (Val), optionally modified glutamic acid (Glu), optionally modified arginine (Arg), optionally modified lysine (Lys), optionally modified aspartic acid (Asp), or optionally modified phenylalanine (Phe),
   the 3rd amino acid residue of SEQ ID NO: 219 is an optionally modified phenylalanine (Phe),
   the 5th amino acid residue of SEQ ID NO: 219 is an optionally modified tryptophan (Trp),
   the 8th amino acid residue of SEQ ID NO: 219 is an optionally modified tyrosine (Tyr), and
   the 10th amino acid residue of SEQ ID NO: 219 is an optionally modified isoleucine (Ile), optionally modified glutamic acid (Glu), or optionally modified lysine (Lys), in peptide D.
[11A] The conjugate or a salt thereof of the above-mentioned [10A], wherein the length of the peptide is not less than 15 and not more than 18.
[12A] The conjugate or a salt thereof of the above-mentioned [1], wherein the peptide is
   peptide E which is a peptide having a peptide length of not less than 15 and not more than 18 and comprising the 1st to the 15th amino acid sequence of SEQ ID NO: 296 (Ala-Val-MeF-Val-Trp-Asn-Tyr-Tyr-Ile-Ile-Arg-Arg-Tyr-MeY-Cys), or
   a peptide comprising an amino acid sequence resulting from substitution, deletion, and/or insertion of not less than 1 and not more than 10, or not less than 1 and not more than 5, amino acid residues in peptide E.
[13A] The conjugate or a salt thereof of the above-mentioned [12A], wherein the peptide is a peptide comprising an amino acid sequence resulting from substitution of any of the 3rd, 5th, 7th, 8th, 11th, 12th, and 13th amino acid residues of SEQ ID NO: 296 in peptide E.
[14A] The conjugate or a salt thereof of the above-mentioned [12A], wherein the peptide is any of peptides in which
   the 3rd amino acid residue of SEQ ID NO: 296 is an optionally modified phenylalanine (Phe),
   the 5th amino acid residue of SEQ ID NO: 296 is an optionally modified tryptophan (Trp),
   the 7th amino acid residue of SEQ ID NO: 296 is an optionally modified tyrosine (Tyr),
   the 8th amino acid residue of SEQ ID NO: 296 is an optionally modified tyrosine (Tyr),
   the 11th amino acid residue of SEQ ID NO: 296 is an optionally modified arginine (Arg) or an optionally modified lysine (Lys),
   the 12th amino acid residue of SEQ ID NO: 296 is an optionally modified arginine (Arg) or an optionally modified lysine (Lys), or
   the 13th amino acid residue of SEQ ID NO: 296 is an optionally modified tyrosine (Tyr) or an optionally modified phenylalanine (Phe).
[15A] The conjugate or a salt thereof of the above-mentioned [12A], wherein the peptide is a peptide in which
   the 3rd amino acid residue of SEQ ID NO: 296 is phenylalanine (Phe), methylated phenylalanine (MeF), or N-methyl-3-chloro-L-phenylalanine (MeF3C),
   the 5th amino acid residue of SEQ ID NO: 296 is tryptophan (Trp) or methylated tryptophan (MeW),
   the 7th amino acid residue of SEQ ID NO: 296 is tyrosine (Tyr),
   the 8th amino acid residue of SEQ ID NO: 296 is tyrosine (Tyr) or (S)-2-amino-3-(4-methoxyphenyl)propanoic acid (F4OMe),
   the 11th amino acid residue of SEQ ID NO: 296 is arginine (Arg) or lysine (Lys),
   the 12th amino acid residue of SEQ ID NO: 296 is arginine (Arg) or D-type arginine (dr), and
   the 13th amino acid residue of SEQ ID NO: 296 is tyrosine (Tyr) or phenylalanine (Phe), in peptide E.
[16A] The conjugate or a salt thereof of the above-mentioned [1], wherein the peptide consists of the amino acid sequence of any of SEQ ID NOs: 3 to 200, or an amino acid sequence in which the N-terminal of the amino acid sequence of any of SEQ ID NOs: 3 to 200 is chloroacetyl-Ala.
[16B] The conjugate or a salt thereof of the above-mentioned [1], wherein the peptide consists of the amino acid sequence of SEQ ID NO: 296 or 300, or an amino acid sequence in which the N-terminal of the amino acid sequence of SEQ ID NO: 296 or 300 is chloroacetyl-Ala.
[17A] The conjugate or a salt thereof of the above-mentioned [1], wherein the peptide comprises the 1 to the 10th amino acid sequence of the amino acid sequence of any of SEQ ID NOs: 1 to 200, 203 to 552, 645, and 723 to 725 or a complex of the amino acid sequence and a linker, and the amino acid sequence moiety has a cyclic structure.
[17B] The conjugate or a salt thereof of the above-mentioned [1], wherein the peptide comprises the 1 to the 10th amino acid sequence of the amino acid sequence of SEQ ID NO: 296 or 300 or a complex of the amino acid sequence and a linker, and the amino acid sequence moiety has a cyclic structure.
[18A] The conjugate or a salt thereof of the above-mentioned [17A], wherein the peptide comprises the 1st to the 15th amino acid sequence of the amino acid sequence of any of SEQ ID NOs: 2, 9, 21 to 148, 159 to 200, 213 to 448, 450 to 552, 645 and 723 to 725 or a complex of the amino acid sequence and a linker, and the amino acid sequence moiety has a cyclic structure.
[18B] The conjugate or a salt thereof of the above-mentioned [1], wherein the peptide comprises the 1st to the 15th amino acid sequence of the amino acid sequence of SEQ ID NO: 296 or 300 or a complex of the amino acid sequence and a linker, and the amino acid sequence moiety has a cyclic structure.
[19A] The conjugate or a salt thereof of any of the above-mentioned [1] and the above-mentioned [2A] to [18A], [16B] to [18B], wherein the peptide is a cyclic peptide.
[20A] The conjugate or a salt thereof of the above-mentioned [17A], [18A], [17B], or [18B], wherein the peptide consists of 15 amino acid residues.
[21A] The conjugate or a salt thereof of the above-mentioned [1], wherein the peptide has directivity to muscle tissue.
[22A] The conjugate or a salt thereof of the above-mentioned [1], comprising a linker having an amino acid length of not less than 1 and not more than 15 and comprising one or more selected from glycine (Gly) and serine (Ser).
[23A] The conjugate or a salt thereof of the above-mentioned [22A], wherein the N-terminal of the aforementioned linker is an optionally modified cysteine (Cys) or optionally modified lysine (Lys).
[24A] The conjugate or a salt thereof of the above-mentioned [1], comprising a linker having an amino acid length of not less than 1 and not more than 5 and comprising one or more selected from glutamic acid in a D form (de) and methylated glycine (MeG).
[25A] The conjugate or a salt thereof of the above-mentioned [24A], wherein the N-terminal of the aforementioned linker is an optionally modified Cys or optionally modified lysine (Lys).
[26A] The conjugate or a salt thereof of the above-mentioned [1], comprising a PEG linker comprising polyethylene glycol (PEG) or a polyethylene glycol derivative.
[27A] The conjugate or a salt thereof of the above-mentioned [26A], wherein the PEG linker further comprises one or more selected from glycine (Gly), serine (Ser), glutamic acid (Glu), arginine (Arg), and lysine (Lys).
[28A] The conjugate or a salt thereof of the above-mentioned [26A] or [27A], wherein the N-terminal of the aforementioned linker is an optionally modified cysteine (Cys) or optionally modified lysine (Lys).
[29A] The conjugate or a salt thereof of the above-mentioned [1], comprising a linker having the amino acid sequence shown in any of SEQ ID NOs: 553 to 644.
[30A] The conjugate or a salt thereof of the above-mentioned [1], comprising
   polyethylene glycol (PEG), and
   G linker, GS linker, or a linker having the amino acid sequence shown in any of SEQ ID NOs: 553 to 644.
[31A] The conjugate or a salt thereof of the above-mentioned [1], which has directivity to muscle tissue.
[32A] A prophylactic or therapeutic agent for a neuromuscular disease, comprising the conjugate or a salt thereof of the above-mentioned [1].
[33A] A diagnostic agent for a neuromuscular disease, comprising the conjugate or a salt thereof of the above-mentioned [1].

### [Advantageous Effects of Invention]

According to the invention described in this specification, as demonstrated in the Example, a conjugate of a peptide such as a peptide that binds to a human transferrin receptor (hTfR), a peptide having directivity to muscle tissue, or the like, and a drug and the like can be provided.

### (Detailed Description of the Invention)

The embodiments for practicing the present invention are described below. The present invention is not limited to the embodiments described below, but also includes those that are appropriately modified from the embodiments below within the range obvious to those skilled in the art

### Conjugate (complex)

The conjugate of the present invention contains
(1) a peptide that binds to a transferrin receptor, and includes
   the amino acid sequence shown in SEQ ID NO: 1; or
   an amino acid sequence resulting from substitution, deletion, addition, and/or insertion of not less than one and not more than 10 amino acid residues in the amino acid sequence shown in SEQ ID NO: 1, and
(2) a compound containing an oligonucleotide for producing an at least partially functional dystrophin protein.

As used herein, the peptide and the compound containing the oligonucleotide may be directly bound or bound via a linker, and are preferably bound via a linker.

### Transferrin receptor

The transferrin receptor refers to a receptor having the function of binding to transferrin, which is a protein contained in plasma and binding to iron ion, and bringing them into cells. Transferrin receptors are expressed on various cells such as reticulocytes, placental trophoblast cells, lymphocytes, and the like, and expression thereof particularly on tumor cells is suggested. In addition, transferrin receptors have the property of triggering endocytosis of cells by stimulating the binding of iron ions in plasma. Therefore, research is underway to make a desired substance pass through BBB by using antibodies that bind to transferrin receptors as DDS. In the present specification, unless otherwise specified, human transferrin receptor is described as human TfR, hTfR, or simply TfR.

### Peptide that binds to transferrin receptor

Binding to transferrin receptor (also referred to as having binding activity or having affinity) means specifically binding to transferrin receptor.

Affinity is expressed by the equilibrium constant (KD) for the dissociation between transferrin receptor and binding peptide. This is a scale showing the binding strength between the transferrin receptor and the binding peptide, where as the KD value becomes smaller, the binding strength between the transferrin receptor and the binding peptide becomes stronger (instead, the affinity can also be shown by affinity (KA), which is 1/KD). As is clear to those of ordinary skill in the art (for example, based on further disclosure in the present specification), the affinity can be determined by a formats known per se, according to the kind and properties of the binding substance. The binding activity is a scale of the strength of the binding between the transferrin receptor and the binding peptide. The binding activity is related to both the affinity between the transferrin receptor and the binding site on the binding peptide and the number of relevant binding sites that exist on the binding molecule. The transferrin receptor is known to include type I and type II. As the transferrin receptor in the present invention, type I (GENE ID: 7037) is preferred.

The specific binding between the transferrin receptor and the binding peptide can be determined by, for example, surface plasmon resonance (SPR) assay, Scatchard analysis and/or radioimmunoassay (RIA), enzyme immunoassay (EIA), competitive binding assay such as sandwich competitive assay, and the like described in the present specification, and any appropriate formats known per se, including a different variant thereof known per se in the art.

### Being able to pass through blood-brain barrier (BBB)

Being able to pass through BBB means, for example, that a substance can be passed through the BBB into the brain, and that the substance or its metabolites can be detected at any site in the brain at a certain time after administration, or that findings are obtained that suggest influence of the substance in the brain.

### Brain-related associated disease

A brain-related disease is a disease caused by some abnormality that has occurred in the brain and includes, for example, central nervous system (CNS) diseases. Examples of the brain-related disease include, but are not limited to, diseases affecting the psyche as Alzheimer's disease, Parkinson's disease, prion disease, Huntington's disease, lysosomal disease, central nervous disorders, tumor of the central nervous system including brain tumor, cerebral ischemia, diseases associated with brain disorder, traumatic central nervous system disorder, viral and bacterial central nervous system diseases, schizophrenia, depression, and the like.

### Having directivity to muscle tissue

The muscle tissue may be any of cardiac muscle, skeletal muscle, and smooth muscle. Particularly preferred muscle tissue is cardiac or skeletal muscle. Having directivity to muscle tissue means having the property of specifically and efficiently transferring to muscle tissue.

### Neuromuscular disease

Neuromuscular diseases refer to diseases that cause movement disorders such as muscle weakness due to lesions in nerves such as brain, spinal cord, and peripheral nerves, or muscles and the like. Examples of the neuromuscular disease include, but are not limited to, spinocerebellar degeneration, amyotrophic lateral sclerosis, myasthenia gravis, muscular dystrophy (e.g., Duchenne muscular dystrophy, Becker muscular dystrophy), polymyositis, hereditary myopathy, neuromuscular disease, muscular atrophy, drug-induced myopathy, acute cardiac failure, chronic cardiac failure, myocardial infarction, chronic fatigue syndrome, mitochondrial disease, mitochondrial respiratory chain complex disorder, and Guillain-Barre syndrome.

### Peptides having cell permeability

Peptides having cell permeability are known, for example, as described in JP-B-6478632, JP-B-6708770, WO 2009/147368, WO 2013/030569, WO 2015/022504 (peptide having cell penetrating). Peptides having cell permeability are then taken up into cells. Therefore, the complex of the present invention may further contain a peptide having cell permeability. By using the complex of the present invention further containing a peptide having cell permeability, it becomes possible to deliver a target active ingredient into cells, for example, it becomes possible to deliver a nucleic acid medicament into cells. A peptide having cell permeability is preferably Pip7a, Pip7b, Pip7b2, Pip7c, Pip7c2, Pip8a, Pip8b, Pip8c, Pip8c2, Pip8d, Pip9b, Pip9b2, Pip9c, pip9c2, Pip9c3, pip9d, Pip9d2, or a combination of any of these, which are disclosed in WO 2013/030569 (preferably, Pip7a, Pip7b, Pip7b2, Pip7c, Pip7c2, Pip8a, Pip8b, Pip8c, Pip8c2, Pip8d, Pip9b, Pip9b2, Pip9c, pip9c2, Pip9c3, pip9d, or Pip9d2). More preferably, it is Pip9b, Pip9b2, Pip9c, Pip9d, Pip9d2, or a combination of any of these (preferably, Pip9b, Pip9b2, Pip9c, Pip9d, or Pip9d2). Further preferably, it is Pip9b2. Pip9b2 has the following structure.

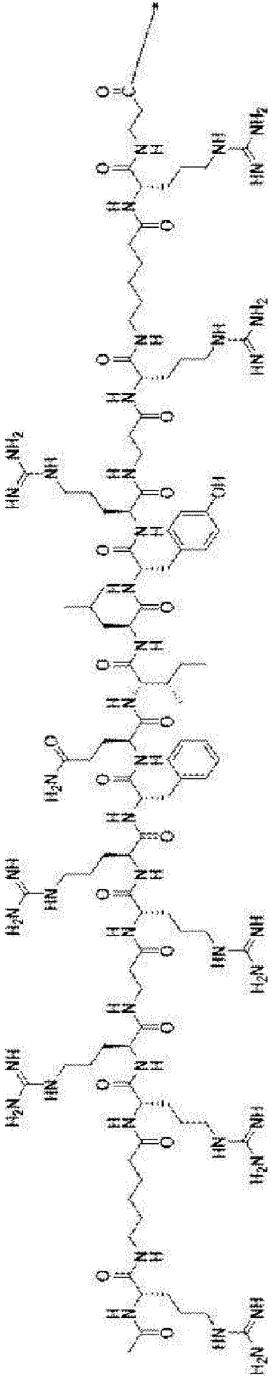

### Peptide

It refers to a structure including multiple consecutive amino acids, and also encompasses polypeptides and proteins. In the present invention, amino acids are not only naturally occurring amino acids (natural amino acids) that are incorporated into peptide chains when mRNA is translated within cells, but also amino acids that do not occur naturally (non-natural amino acids) that can form part of a peptide chain through peptide bonds. The amino acid may be one that is artificially synthesized or one that exists in nature.

In the present invention, the peptides include those in which a cyclic moiety is formed by post-synthesis cyclization (also referred to as cyclic peptides), and those obtained by further chemically modifying the peptides.

In the present specification, the cyclic peptide refers to a peptide in which two amino acids separated by one or more amino acid residues in the amino acid sequence of the peptide are bonded to each other to make all or part of the peptide. The type of bond between the two amino acids is not particularly limited, and the cyclic peptide encompasses an amide bond between the carboxyl group of one amino acid and the amino group of the other amino acid, a thioether bond between the carboxyl group of one amino acid and the thiol group of the other amino acid, a thiol bond between the thiol group of one amino acid and the thiol group of the other amino acid, a cyclic structure formed by lactam ring formation or macrocyclization reaction, one having a lasso peptide-like structure, and the like. When the two amino acids are bonded together by an amide bond, the amide bond is not limited to one formed by the binding of the carboxyl group of one amino acid and the amino group of the other amino acid, and a bond by an amide bond resulting from a synthesis reaction suffices. The same applies to other bond types.

That is, in the present invention, the cyclic peptide may be one in which a moiety thereof forms a cyclic structure, and it optionally has a linear chain moiety.

In the present specification, a part of the amino acids may sometimes be modified for cyclization of the peptide. Amino acids that have been partially modified are also encompassed in the amino acid of the present application. For example, a chloroacetyl group is added to an amino acid located at the N-terminal, and is bonded to a cysteine residue in a peptide to cause cyclization. Various (natural/non-natural) amino acids with a chloroacetyl group added thereto are also encompassed in the amino acid of the present application.

The non-natural amino acid refers to a compound that is other than natural amino acids and that has the properties of amino acid. Examples thereof include, but are not limited to, β-amino acid, γ-amino acid, L-amino acid, D-amino acid (also referred to as D-type amino acid), amino acid variant, chemically-modified amino acids such as amino acid derivative, and the like, amino acids that are not constituent materials for proteins in living organisms, such as norleucine, β-alanine, ornithine, and the like, and the like. N-methylamino acid, N-ethylamino acid, D-amino acid, histidine-like amino acids, amino acids having redundant structures such as methylene, aromatic ring, and the like in the side chain, amino acid derivatives having a structure in which a carboxylic acid functional group in the side chain is substituted with a sulfonic acid group, and the like can be mentioned.

Examples of non-natural amino acids and abbreviations in the present specification are shown below. The CAS reference number or the supplier is shown in parentheses, and the Synthesis Example No. is shown for newly synthesized ones. The special amino acids are not limited to these and, for example, those having a structure in which one or more of the hydrogen atoms in these molecules are substituted by an alkyl group are also special amino acids. When a hydrogen atom is substituted by an alkyl group, the alkyl group is preferably a methyl group or an ethyl group, more preferably a methyl group. In the present specification, an amino acid with Me or N-Me written before the amino acid name indicates an N-methyl amino acid unless otherwise specified. For example, the N-methylated amino acid of alanine (Ala or A) is indicated as MeAla, N-MeAla, MeA, or N-MeA. Furthermore, in the one-letter amino acid notation, an amino acid with d written in front thereof indicates D-amino acid. For example, the D-amino acid of alanine (Ala or A) is designated as da. Those without a CAS NO. or supplier information can be purchased as general reagents. The following amino acids can be used for peptide synthesis by protecting the alpha amino group with Fmoc by a known method.

Yph (S)-2-amino-3-(4-phenoxyphenyl)propanoic acid (CAS NO.:150351-64-7)
W7OMe (S)-2-amino-3-(7-methoxy-1H-indol-3-yl)propanoic acid (CAS NO.:25198-03-2)
W7N (S)-2-amino-3-(1H-pyrrolo[2,3-β]pyridin-3-yl)propanoic acid (CAS NO.:49758-35-2)
W7F (S)-2-amino-3-(7-fluoro-1H-indol-3-yl)propanoic acid (CAS NO.:138514-97-3)
W6N (S)-2-amino-3-(1H-pyrrolo [2,3-c]pyridin-3-yl)propanoic acid (KISHIDA CHEMICAL CO., LTD.) (CAS NO.:149704-63-2)
W6F (S)-2-amino-3-(6-fluoro-1H-indol-3-yl)propanoic acid (CAS NO.:19310-00-0)
W5OMe 5-methoxy-L-tryptophan (CAS NO.:25197-96-0)
W5F (S)-2-amino-3-(5-fluoro-1H-indol-3-yl)propanoic acid (CAS NO.:16626-02-1)
W4OMe 4-methoxy-L-tryptophan (CAS NO.:406938-53-2)
W4N (S)-2-amino-3-(1H-pyrrolo[3,2-β]pyridin-3-yl)propanoic acid (CAS NO.:149818-23-5)
W4F (S)-2-amino-3-(4-fluoro-1H-indol-3-yl)propanoic acid (CAS NO.:106034-22-4)
W4C (S)-2-amino-3-(4-chloro-1H-indol-3-yl)propanoic acid (CAS NO.:52448-14-3)
W2N (S)-2-amino-3-(1H-indol-3-yl)propanoic acid (CAS NO.:53538-54-8)
W1iPr 1-isopropyl-L-tryptophan (CAS NO.:1219485-46-7)
W1EtVCl (S)-2-amino-3-(7-chloro-1-ethyl-1H-indol-3-yl)propanoic acid (N-protected body was synthesized in Synthetic Example 5-1)
W1Et 1-ethyl-L-tryptophan (CAS NO.:158059-28-0)
Tbg (S)-2-amino-3,3-dimethylbutanoic acid (CAS NO.:20859-02-3) pHPeG N-(4-hydroxyphenethyl)glycine (CAS NO.:169836-45-7)
PeG N-(2-phenylethyl)-glycine (CAS NO.:7738-38-7)
Nva L-norvaline (CAS NO.:6600-40-4)
Nle L-norleucine (CAS NO.:327-57-1)
Nal2 β-(2-naphthyl)L-alanine (CAS NO.:58438-03-2)
Nal1 β-(1-naphthyl)L-alanine (CAS NO.:55516-54-6)
MeoBph N-α-methyl-2-phenyl-L-phenylalanine
MeNal2 N-α-methyl-β-(2-naphthyl)-L-alanine (CAS NO.:2137098-18-9)
MeNal1 N-α-methyl-β-(1-naphthyl)-L-alanine (CAS NO.:2137057-01-1)
MemBph N-α-methyl-3-phenyl-L-phenylalanine
Hph L-homophenylalanine (CAS NO.:943-73-7)
Hly (S)-2,7-diaminoheptanoic acid (CAS NO.:37689-89-7)
F4OMe (S)-2-amino-3-(4-methoxyphenyl)propanoic acid (CAS NO.:6230-11-1)
F4G (4-guanidyl)-L-phenylalanine (CAS NO.:59574-11-7)
F4F 4-fluoro-L-phenylalanine (CAS NO.:1132-68-9)
F4C 4-chloro-L-phenylalanine (CAS NO.:14173-39-8)
F3OMe (S)-2-amino-3-(3-methoxyphenyl)propanoic acid (CAS NO.:33879-32-2)
F3F 3-fluoro-L-phenylalanine (CAS NO.:19883-77-3)
F3C 3-chloro-L-phenylalanine (CAS NO.:80126-51-8)
F2OMe (S)-2-amino-3-(2-methoxyphenyl)propanoic acid (CAS NO.:193546-31-5)
F2C (S)-2-amino-3-(2-chlorophenyl)propanoic acid (CAS NO.:103616-89-3)
MeF4OMe (S)-3-(4-methoxyphenyl)-2-(methylamino)propanoic acid (CAS NO.:52939-33-0)
MeF4F N-α-methyl-4-fluoro-L-phenylalanine (CAS NO.:347851-71-2)
MeF3F N-α-methyl-3-fluoro-L-phenylalanine (CAS NO.:955126-85-9)
MeF3C N-methyl-3-chloro-L-phenylalanine (CAS NO.:2255324-91-3)
MeBph N-α-methyl-4-phenyl-L-phenylalanine
Me4Py N-α-methyl-4-pyridyl-L-alanine
Me3Py N-α-methyl-3-pyridyl-L-alanine
dr D-arginine
dp D-proline
dc D-cysteine
dk D-lysine
Dap L-α,β-diaminopropionic acid (CAS NO.:515-94-6)
Dab (S)-2,4-diamino-butanoic acid (CAS NO.:1758-80-1)
Cit 2-amino-5-ureidopentanoic acid (CAS NO.:627-77-0)
Cha β-cyclohexyl-L-alanine (CAS NO.:27527-05-5)
CeG N-(2-carboxyethyl)-glycine (CAS NO.:505-72-6)
Cbg (S)-2-amino-2-cyclobutylacetic acid (CAS NO.:49607-08-1)
Cba L-cyclobutylalanine (CAS NO.:1201593-65-8)
aMeY α-methyl-L-tyrosine (CAS NO.:672-87-7)
aMeW α-methyl-L-tryptophan (CAS NO.:16709-25-4)
aMeK α-methyl-L-lysine (CAS NO.:104112-34-7)
aMeC α-methyl-L-cysteine(CAS NO.:441317-73-3)
Aib α-methylalanine (CAS NO.:62-57-7)
Ahp/Alahp (S)-2-aminoheptanoic acid (CAS NO.:44902-02-5)
Abu L-α-aminobutanoic acid (CAS NO.:1492-24-6)
A4paa (S)-2-amino-3-(1-(carboxymethyl)piperazin-4-yl)propanoic acid (KISHIDA CHEMICAL CO., LTD.)
5Ind (S)-2-amino-3-(1H-indol-5-yl)propanoic acid (CAS NO.:460096-38-2)
4Py2NH2 (S)-2-amino-3-(2-aminopyridin-4-yl)propanoic acid (KISHIDA CHEMICAL CO., LTD.) (N-protected body was synthesized in Synthetic Example 5-2)
4Py 4-pyridyl-L-alanine (CAS NO.:37535-49-2)
3Py6NH2 (S)-2-amino-3-(6-aminopyridin-3-yl)propanoic acid (N-protected body was synthesized in Synthetic Example 5-3)
3Py 3-pyridyl-L-alanine (CAS NO.:64090-98-8)
W1aa 1-(carboxymethyl)-L-tryptophan (CAS NO.:773823-50-0) KCOpipzMe N6-(4-methylpiperazine-1-carbonyl)-L-lysine (KISHIDA CHEMICAL CO., LTD.) (N-protected body was synthesized in Synthetic Example 5-4)
W1mCON 1-(2-amino-2-oxoethyl)-L-tryptophan (N-protected body was synthesized in Synthetic Example 5-5)
W1EtOH 1-(2-hydroxyethyl)-L-tryptophan (N- and O-protected body was synthesized in Synthetic Example 5-9)
3Py6OMe (S)-2-amino-3-(6-methoxypyridin-3-yl)propanoic acid (CAS NO.:1270178-24-9)
Epyrl2RCOO 2-amino-(5-((R)-2-((allyloxy)carbonyl)pyrrolidin-1-yl)-5-oxopentanoic acid (Glu(d-Pro-O-allyl)-OH) (N-protected body was synthesized in Synthetic Example 5-6)
Dpyrl2RCOO 2-amino-(4-((R)-2-((allyloxy)carbonyl)pyrrolidin-1-yl)-4-oxobutanoic acid (Asp(d-Pro-O-allyl)-OH) (N-protected body was synthesized in Synthetic Example 5-7)
MeF3COO 3-carboxy-N-methyl-phenylalanine (CAS NO.:1499826-56-0) 3Imp 2-amino-3-(imidazo[1,2-a]pyridin-3-yl)propanoic acid (CAS NO.:2276942-95-9)
KaAc N6-glycyl-L-lysine (Lys(Gly-O-allyl)-OH) (N-protected body was synthesized in Synthetic Example 5-8)
A1Me4pip 4-amino-1-methylpiperazine-4-carboxylic acid (CAS NO.:15580-66-2)
Har N6-carbamimidoyl-L-lysine (CAS NO.:156-86-5)
Acpr (S)-2-amino-3-cyclopropylpropanoic acid (CAS NO.:102735-53-5)
Atb (S)-2-amino-4,4-dimethylpentanoic acid (CAS NO.:57224-50-7)
MeF35dC (S)-3-(3,5-dichlorophenyl)-2-(methylamino)propanoic acid (L form of CAS NO.:1520729-91-2)
Adod 12-aminododecanoic acid (CAS NO.:693-57-2)
Hly L-homolysine (CAS NO.:37689-89-7)
W5C 5-chloro-L-tryptophan (CAS NO.:52448-15-4)
F3COO L-3-carboxyphenylalanine (CAS NO.:13861-02-4)
F3CON L-3-carbamoylphenylalanine (CAS NO.:1217651-22-3)
Hgl L-2-aminoadipic acid (CAS NO.:1118-90-7)
Ndm N,N-dimethyl-L-asparagine (CAS NO.:62937-43-3)
KN3 or LysN3 6-azido-L-norleucine (CAS NO.:159610-92-1)
KAc N6-acetyl-L-lysine (CAS NO.:692-04-6)
dorn D-ornithine (CAS NO.:348-66-3)
F3H 3-hydroxy-L-phenylalanine (CAS NO.:587-33-7)
Yae O-(2-aminoethyl)-L-tyrosine (CAS NO.:1909283-20-0)
F4aao O-(2-carboxymethyl)-L-tyrosine (CAS NO.:24558-63-2) f4OEt O-ethyl-L-tyrosine (CAS NO.:32795-52-1)
F34dOMe 3,4-dimethoxy-L-phenylalanine (CAS NO.:142995-28-6) alT L-allothreonine (CAS NO.:28954-12-3)
alI L-alloisoleucine (CAS NO.:1509-34-8)
MeK N-methyl-L-lysine (CAS NO.:7431-89-2)
Tbg (S)-2-amino-3,3-dimethylbutyric acid (CAS NO.:20859-02-3) Nva L-norvaline (CAS NO.:6600-40-4)
Abu (S)-(+)-2-aminobutyric acid (CAS NO.:1492-24-6)
da D-alanine
Bph 4-phenyl-L-phenylalanine (CAS NO.:155760-02-4)
de D-glutamic acid
MeA N-methyl-L-alanine (CAS NO.:3913-67-5)
PEG4c or PEG3 1-amino-3,6,9,12-tetraoxapentadecan-15-oic acid (CAS NO.:663921-15-1)
MeR N-methyl-L-arginine (CAS NO.:2480-28-6)
MeW N-methyl-L-tryptophan (CAS NO.:526-31-8)
MeY N-methyl-L-tyrosine (CAS NO.:537-49-5)
PEG8c 1-amino-3,6,9,12,15,18,21,24-octaoxaheptacosan-27-oic acid (CAS NO.:756526-04-2)
PEG12c or PEG11 or PEG12 1-amino-3,6,9,12,15,18,21,24,27,30,33,36-dodecaoxanonatriacontan-39-oic acid (CAS NO.:1415408-69-3)

Note that newly synthesized amino acids are useful because they may add new functions to various peptides when producing various peptide derivatives.

The peptide in the present invention contains the amino acid sequence shown in SEQ ID NO: 1 (Ala-Val-Phe-Val-Trp-Asn-Tyr-Tyr-Ile-Ile-Ser-Cys); or an amino acid sequence resulting from substitution, deletion, addition, and/or insertion of not less than one and not more than 10 amino acid residues in the amino acid sequence shown in SEQ ID NO: 1. The peptide is a peptide that binds to a transferrin receptor.

A preferred example of this peptide is a peptide having directivity to muscle tissue.

### Peptide sequence

The number of amino acid substitutions, deletions, additions, and/or insertions may be not less than one and not more than 10, and the lower limit is 1. The upper limits are 10, 9, 8, 7, 6, 5, 4, 3, and 2, and the minimum is 1. Such amino acid substitutions are preferably conservative amino acid substitutions.

### Conservative amino acid substitution

The "conservative amino acid substitution" means substitution with a functionally equivalent or similar amino acid. Generally, substitution within a group can be considered conservative in the structure and function. However, as is obvious to those of ordinary skill in the art, the role played by a specific amino acid residue can be determined by its meaning in the three-dimensional structure of a molecule containing the amino acid. For example, cysteine residues can be in the oxidized (disulfide) form, which is less polar as compared with the reduced (thiol) form. The long aliphatic moiety of the arginine side chain may constitute a structurally and functionally important feature. In addition, side chains containing aromatic rings (tryptophan, tyrosine, phenylalanine) can contribute to ion-aromatic interaction or cation-pi interaction. In such cases, substitution of amino acids having these side chains with amino acids belonging to acidic or non-polar groups may be structurally and functionally conservative. Residues such as proline, glycine, cysteine (in disulfide form), and the like may have a direct effect on the steric structure of the main chain and often cannot be substituted without structural distortion.

Conservative amino acid substitution includes, as shown below, specific substitutions based on side chain similarity (Lehninger, Biochemistry, 2nd revised edition, published in 1975, pp. 73-75: L. Lehninger, Biochemistry, 2nd edition), pp 73-75, Worth Publisher, New York (1975)) and typical substitutions.

A preferred example of this peptide contains an amino acid sequence having one or more substitutions selected from the following group:
(I) substitution of alanine residue at position 1 of SEQ ID NO: 1 with aliphatic amino acid or methylated aliphatic amino acid;
(II) substitution of valine residue at position 2 of SEQ ID NO: 1 with basic amino acid residue or methylated basic amino acid residue;
(III) substitution of phenylalanine residue at position 3 of SEQ ID NO: 1 with aromatic amino acid residue, methylated aromatic amino acid residue, amino acid residue with aromatic ring added, or amino acid residue with fused ring added;
(IV) substitution of valine residue at position 4 of SEQ ID NO: 1 with methylated valine residue;
(V) substitution of tryptophan residue at position 5 of SEQ ID NO: 1 with aromatic amino acid residue, methyltryptophan residue, methylated aromatic amino acid residue, amino acid residue with aromatic ring added, or amino acid residue with fused ring added;
(VI) substitution of asparagine residue at position 6 of SEQ ID NO: 1 with neutral amino acid or methylated neutral amino acid;
(VII) substitution of tyrosine residue at positions 7 and 8 of SEQ ID NO: 1 with aromatic amino acid residue, methylated aromatic amino acid residue, amino acid residue with aromatic ring added, or amino acid residue with fused ring added;
(VIII) substitution of isoleucine residue at position 9 of SEQ ID NO: 1 with aliphatic amino acid residue, methylated aliphatic amino acid residue, or amino acid residue with branched chain structure;
(IX) substitution of isoleucine residue at position 10 of SEQ ID NO: 1 with any amino acid; and
(X) substitution of serine residue at position 11 of SEQ ID NO: 1 with neutral amino acid residue.

Naturally occurring amino acids can be divided into the following groups based on the properties of the common side chains thereof.
(1) non-polar amino acid group: alanine (hereinafter indicated as "Ala" or simply "A"), valine (hereinafter indicated as "Val" or simply "V"), leucine (hereinafter indicated as "Leu" or simply "L"), isoleucine (hereinafter indicated as "Ile" or simply "I"), proline (hereinafter indicated as "Pro" or simply "P"), phenylalanine (indicated as "Phe" or simply "F"), tryptophan (hereinafter indicated as "Trp" or simply "W"), methionine (hereinafter indicated as "Met" or simply "M")
(2) uncharged polar amino acid group: glycine (hereinafter indicated as "Gly" or simply "G"), serine (hereinafter indicated as "Ser" or simply "S"), threonine (hereinafter indicated as "Thr" or simply "T"), cysteine(hereinafter indicated as "Cys" or simply "C"), tyrosine (hereinafter indicated as "Tyr" or simply "Y"), asparagine (hereinafter indicated as "Asn" or simply "N"), glutamine (hereinafter "Gln" or simply "Q")
(3) acidic amino acid group: aspartic acid (hereinafter indicated as "Asp" or simply "D"), glutamic acid (hereinafter indicated as "Glu" or simply "E")
(4) basic amino acid group: lysine (hereinafter indicated as "Lys" or simply "K"), arginine (hereinafter indicated as "Arg" or simply "R"), histidine (hereinafter indicated as "His" or simply "H")

Also, they can be divided into the following groups.

(1) hydrophobic amino acid group: norleucine, Met, Ala, Val, Leu, Ile
(2) neutral hydrophilic amino acid group: Cys, Ser, Thr, Asn, Gln
(3) acidic amino acid group: Asp, Glu
(4) basic amino acid group: His, Lys, Arg
(5) amino acid group that affects the direction of the main chain: Gly, Pro
(6) aromatic amino acid group: Trp, Tyr, Phe

Each group also encompasses non-natural amino acids such as N-methylated amino acids and the like.

A preferred example of this peptide is a peptide containing an amino acid sequence shown in SEQ ID NO: 2 (Ala-Val-Phe-Val-Trp-Asn-Tyr-Tyr-Ile-Ile-Arg-Arg-Tyr-MeTyr-Cys).

A preferred example of this peptide, in the amino acid sequence shown in SEQ ID NO: 2, contains an amino acid sequence having one or more substitutions selected from the following group:
(I) substitution of alanine residue at position 1 of SEQ ID NO: 2 with aliphatic amino acid or methylated aliphatic amino acid;
(II) substitution of valine residue at position 2 of SEQ ID NO: 2 with basic amino acid residue or methylated basic amino acid residue;
(III) substitution of phenylalanine residue at position 3 of SEQ ID NO: 2 with aromatic amino acid residue, methylated aromatic amino acid residue, amino acid residue with aromatic ring added, or amino acid residue with fused ring added;
(IV) substitution of valine residue at position 4 of SEQ ID NO: 2 with methylated valine residue;
(V) substitution of tryptophan residue at position 5 of SEQ ID NO: 2 with aromatic amino acid residue, methylated aromatic amino acid residue, amino acid residue with aromatic ring added, or amino acid residue with fused ring added;
(VI) substitution of asparagine residue at position 6 of SEQ ID NO: 2 with neutral amino acid or methylated neutral amino acid;
(VII) substitution of tyrosine residue at positions 7 and 8 of SEQ ID NO: 2 with aromatic amino acid residue, methylated aromatic amino acid residue, amino acid residue with aromatic ring added, or amino acid residue with fused ring added;
(VIII) substitution of isoleucine residue at position 9 of SEQ ID NO: 2 with aliphatic amino acid residue, methylated aliphatic amino acid residue, or amino acid residue with branched chain structure;
(IX) substitution of isoleucine residue at position 10 of SEQ ID NO: 2 with any amino acid;
(XI) substitution of arginine residue at positions 11 and 12 of SEQ ID NO: 2 with basic amino acid residue;
(XII) substitution of tyrosine residue at position 13 of SEQ ID NO: 2 with hydrophilic amino acid residue;
(XIII) substitution of methyltyrosine residue at position 14 of SEQ ID NO: 2 with tyrosine residue, aromatic amino acid residue, or methylated aromatic amino acid residue; and
(XIV) substitution of cysteine residue at position 15 of SEQ ID NO: 2 with methylated cysteine residue.

Another preferred example of this peptide is
peptide A which is a peptide having a peptide length of not less than 10 and not more than 17 and containing the 1st to the 10th amino acid sequence of SEQ ID NO: 18 (Ala-Val-Phe-Val-Trp-Asn-Tyr-Tyr-Ile-Val-Ser-Cys), or
a peptide containing an amino acid sequence resulting from substitution, deletion, and/or insertion of not less than 1 and not more than 6 amino acid residues in peptide A.

It may be a peptide containing an amino acid sequence resulting from substitution of any of the 2nd, 3rd, 5th, 8th, and 10th amino acid residues of SEQ ID NO: 18 in peptide A.

That an "amino acid residue is substituted" means that a specific amino acid residue is substituted with other optionally modified amino acid residue.

In peptide A, it may be a peptide wherein
the 2nd amino acid residue of SEQ ID NO: 18 is an optionally modified valine (Val) or optionally modified glutamic acid (Glu),
the 3rd amino acid residue of SEQ ID NO: 18 is an optionally modified phenylalanine (Phe),
the 5th amino acid residue of SEQ ID NO: 18 is an optionally modified tryptophan (Trp),
the 8th amino acid residue of SEQ ID NO: 18 is an optionally modified tyrosine (Tyr), and
the 10th amino acid residue of SEQ ID NO: 18 is an optionally modified isoleucine (Ile) or optionally modified valine (Val), in peptide A.

Being "optionally modified" means that known amino acid modification or alteration may be performed. Examples of the modification include N-methylation, amino acid modification with the below-mentioned abbreviation, modification (conversion) into D form, and known conversion of amino acid into a derivative.

The length of this peptide is preferably not less than 11 and not more than 13.

In addition, in peptide A, it may be a peptide wherein
the 2nd amino acid residue of SEQ ID NO: 18 is Val or Glu,
the 3rd amino acid residue of SEQ ID NO: 18 is Phe or MeF3C,
the 5th amino acid residue of SEQ ID NO: 18 is Trp or MeTrp,
the 8th amino acid residue of SEQ ID NO: 18 is Tyr or F4OMe, and
the 10th amino acid residue of SEQ ID NO: 18 is Ile or Val, in peptide A.

Furthermore, this peptide may be a peptide having the following amino acid sequence at the N-terminal of peptide A in which:
the 11th amino acid residue of SEQ ID NO: 18 is Ser or His, and
the 12th amino acid residue of SEQ ID NO: 18 is Cys or Hgl.

Another preferred example of this peptide is
peptide B which is a peptide having a peptide length of not less than 10 and not more than 19 and containing the 1st to the 10th amino acid sequence of SEQ ID NO: 15 (Ala-Val-Phe-Val-Trp-Asn-Tyr-Tyr-Ile-Val-Pro-Arg-Asp-Cys), or
a peptide containing an amino acid sequence resulting from substitution, deletion, and/or insertion of not less than 1 and not more than 5 amino acid residues in peptide B.

In peptide B, it may be a peptide containing an amino acid sequence resulting from substitution of any of the 2nd, 3rd, 5th, 8th, and 10th amino acid residues of SEQ ID NO: 15 in peptide B.

In peptide B, it may be a peptide wherein
the 2nd amino acid residue of SEQ ID NO: 15 is an optionally modified valine (Val) or optionally modified glutamic acid (Glu),
the 3rd amino acid residue of SEQ ID NO: 15 is an optionally modified phenylalanine (Phe) or tryptophan (Trp),
the 5th amino acid residue of SEQ ID NO: 15 is an optionally modified tryptophan (Trp),
the 8th amino acid residue of SEQ ID NO: 15 is an optionally modified tyrosine (Tyr), and
the 10th amino acid residue of SEQ ID NO: 15 is an optionally modified isoleucine (Ile) or optionally modified valine (Val).

The length of these peptides may be not less than 13 and not more than 15.

In addition, in peptide B, it may be a peptide wherein
the 2nd amino acid residue of SEQ ID NO: 15 is Val or Glu,
the 3rd amino acid residue of SEQ ID NO: 15 is Phe, Trp, or MeF3C,
the 5th amino acid residue of SEQ ID NO: 15 is Trp or MeTrp,
the 8th amino acid residue of SEQ ID NO: 15 is Tyr, Phe, or F4OMe, and
the 10th amino acid residue of SEQ ID NO: 15 is Ile or Val.

Furthermore, this peptide may be a peptide having the following amino acid sequence at the N-terminal of peptide B in which:
the 11th amino acid residue of SEQ ID NO: 15 is Pro,
the 12th amino acid residue of SEQ ID NO: 15 is Arg,
the 13th amino acid residue of SEQ ID NO: 15 is Glu, Asn, Asp, His, Gln, or MeTrp, and
the 14th amino acid residue of SEQ ID NO: 15 is Cys.

Another preferred example of this peptide is
peptide C which is a peptide having a peptide length of not less than 11 and not more than 19 and containing the 1st to the 10th amino acid sequence of SEQ ID NO: 214 (MeA-Val-MeF3C-Val-MeW-Asn-Tyr-F40Me-Ile-Ile-Arg-Arg-Phe-MeY-Cys), or
a peptide containing an amino acid sequence resulting from substitution, deletion, and/or insertion of not less than 1 and not more than 5 amino acid residues in peptide C.

It may be a peptide containing an amino acid sequence resulting from substitution of any of the 1st, 3rd, 5th, and 8th amino acid residues of SEQ ID NO: 214 in peptide C.

In peptide C, it may be a peptide wherein
the 1st amino acid residue of SEQ ID NO: 214 is an optionally modified alanine (Ala) or optionally modified glutamic acid (Glu),
the 3rd amino acid residue of SEQ ID NO: 214 is an optionally modified phenylalanine (Phe),
the 5th amino acid residue of SEQ ID NO: 214 is an optionally modified tryptophan (Trp), and
the 8th amino acid residue of SEQ ID NO: 214 is an optionally modified tyrosine (Tyr).

The length of these peptides is preferably not less than 15 and not more than 18.

In addition, in peptide C, it may be a peptide wherein
the 1st amino acid residue of SEQ ID NO: 214 is Ala, Aib, Abu, Glu, Gly, Ser, Phe, Pro, or MeA, particularly preferably Ala or MeA,
the 3rd amino acid residue of SEQ ID NO: 214 is Phe, F3C, F2C, F2OMe, F4C, Cha, MeF, MeF35dC, MeF4F, MeF4Ome, MeNal1, Me3Py, Me4Py, Me3OMe, MeF3COO, MeF3F, Glu, Epyrl2RCOO, Dpyrl2RCOO, or MeF3C, particularly preferably Phe, MeF, or MeF3C,
the 5th amino acid residue of SEQ ID NO: 214 is Trp, MeW, aMeW, dp, F3C, F3F, F3OMe, F4C, F4F, Hph, MemBph, MeNal1, MeNal2, MeoBph, W4OMe, W1Et, W1Et7Cl, W1iPr, Yph, W1Pr, W5C, W5F, W1aa, W1EtOH, W4OMe, W1mCON, or W6F, particularly preferably Trp or MeW, and
the 8th amino acid residue of SEQ ID NO: 214 is Phe, Tyr, Typ, Ahp, MeY, F4OMe, 3Imp, 4Py, 3Py, 3Py6OMe, F3C, F3CON, F4C, F4aao, F4F, f4OEt, MeF34dOMe, Yae, Lys, Orn, or Nal1, particularly preferably Tyr or F4OMe.

Furthermore, this peptide may be a peptide having the following amino acid sequence at the N-terminal of peptide C in which:
the 11th amino acid residue of SEQ ID NO: 214 is Arg, Ala, Asp, Gly, Glu, Lys, MeK, MeR, Dap, Dap, Abu, Aib, Hly, dorn, aMeK, A1Me4pip, KCOpipzMe, F4G, Nle, Nva, or Orn, particularly preferably Lys or Arg,
the 12th amino acid residue of SEQ ID NO: 214 is Lys, Arg, dr, Tyr, F4G, Orn, Hly, da, Cit, Dap, or Dab, particularly preferably Lys, Arg or dr,
the 13th amino acid residue of SEQ ID NO: 214 is Ala, Phe, Asn, Tyr, or pHPeG, particularly preferably Phe or Tyr,
the 14th amino acid residue of SEQ ID NO: 214 is MeTyr, Tyr, Phe, Ala, aMeY, Glu, Gly, Arg, Val, MeoBphMeBph, MeF, MemBph, MeNal1, MeNal2, MeoBph, MeW, or pHPeG, particularly preferably Phe or MeW, and
the 15th amino acid residue of SEQ ID NO: 214 is Cys or Hgl.

Another preferred example of this peptide is
peptide D which is a peptide having a peptide length of not less than 11 and not more than 19 and containing the 1st to the 10th amino acid sequence of SEQ ID NO: 219 (Ala-Glu-Phe-Val-Trp-Asn-Tyr-Tyr-Ile-Ile-Arg-Arg-Tyr-MeY-Cys), or
a peptide containing an amino acid sequence resulting from substitution, deletion, and/or insertion of not less than 1 and not more than 5 amino acid residues in peptide D.

It may be a peptide containing an amino acid sequence resulting from substitution of any of the 2nd, 3rd, 5th, 8th, and 10th amino acid residues of SEQ ID NO: 219 in peptide D.

In peptide D, it may be a peptide wherein
the 2nd amino acid residue of SEQ ID NO: 219 is an optionally modified valine (Val), optionally modified glutamic acid (Glu), optionally modified arginine (Arg), optionally modified lysine (Lys), optionally modified aspartic acid (Asp), or optionally modified phenylalanine (Phe),
the 3rd amino acid residue of SEQ ID NO: 219 is an optionally modified phenylalanine (Phe),
the 5th amino acid residue of SEQ ID NO: 219 is an optionally modified tryptophan (Trp),
the 8th amino acid residue of SEQ ID NO: 219 is an optionally modified tyrosine (Tyr), and
the 10th amino acid residue of SEQ ID NO: 219 is an optionally modified isoleucine (Ile), optionally modified glutamic acid (Glu), or optionally modified lysine (Lys).

The length of these peptides is preferably not less than 15 and not more than 18.

In addition, in peptide D, it may be a peptide wherein
the 2nd amino acid residue of SEQ ID NO: 219 is Val, Glu, Ala, Arg, Lys, Asp, Phe, Dap, Har, Abu, Nva, AcPr, AtbAhp, or Hgl, particularly preferably Glu or Val,
the 3rd amino acid residue of SEQ ID NO: 219 is Phe, F3C, F2C, F2OMe, F4C, Cha, MeF, MeF35dC, MeF4F, MeF4Ome, MeNal1, Me3Py, Me4Py, Me3OMe, MeF3COO, MeF3F, Glu, Epyrl2RCOO, Dpyrl2RCOO, or MeF3C, particularly preferably Phe, MeF, or MeF3C,
the 5th amino acid residue of SEQ ID NO: 219 is Trp, MeW, aMeW, dp, F3C, F3F, F3OMe, F4C, F4F, Hph, MemBph, MeNal1, MeNal2, MeoBph, W4OMe, W1Et, W1Et7Cl, W1iPr, Yph, W1Pr, W5C, W5F, W1aa, W1EtOH, W4OMe, W1mCON, or W6F, particularly preferably Trp or MeW,
the 8th amino acid residue of SEQ ID NO: 219 is Phe, Tyr, Typ, Ahp, MeY, F4OMe, 3Imp, 4Py, 3Py, 3Py6OMe, F3C, F3CON, F4C, F4aao, F4F, f4OEt, MeF34dOMe, Yae, Lys, Orn, or Nal1, particularly preferably Tyr or F4OMe, and
the 10th amino acid residue of SEQ ID NO: 219 is Ala, Abu, Acpr, Ahp, Aib, alI, alT, Atb, Dab, Dap, dorn, Gln, Hly, Ile, Lys, KCOpipzMe, Leu, Nle, Nva, Pro, Arg, Ser, Thr, Tbg, Val, or Tyr, particularly preferably Ile or alI.

Furthermore, this peptide may be a peptide having the following amino acid sequence at the N-terminal of peptide D in which:
the 11th amino acid residue of SEQ ID NO: 219 is Arg, Ala, Asp, Gly, Glu, Lys, MeK, MeR, Dap, Dap, Abu, Aib, Hly, dorn, aMeK, A1Me4pip, KCOpipzMe, F4G, Nle, Nva, or Orn, particularly preferably Lys or Arg,
the 12th amino acid residue of SEQ ID NO: 219 is Lys, Arg, dr, Tyr, F4G, Orn, Hly, da, Cit, Dap, or Dab, particularly preferably Lys, Arg, or dr,
the 13th amino acid residue of SEQ ID NO: 219 is Ala, Phe, Asn, Tyr, or pHPeG, particularly preferably Phe or Tyr,
the 14th amino acid residue of SEQ ID NO: 219 is MeTyr, Tyr, Phe, Ala, aMeY, Glu, Gly, Arg, Val, MeoBphMeBph, MeF, MemBph, MeNal1, MeNal2, MeoBph, MeW, or pHPeG, particularly preferably Phe or MeW, and
the 15th amino acid residue of SEQ ID NO: 219 is Cys or Hgl.

Another preferred example of this peptide is
peptide E which is a peptide having a peptide length of not less than 15 and not more than 18 and containing the 1st to the 15th amino acid sequence of SEQ ID NO: 296 (Ala-Val-MeF-Val-Trp-Asn-Tyr-Tyr-Ile-Ile-Arg-Arg-Tyr-MeY-Cys), or
a peptide containing an amino acid sequence resulting from substitution, deletion, and/or insertion of not less than 1 and not more than 10, or not less than 1 and not more than 5, amino acid residues in peptide E.

It may be a peptide containing an amino acid sequence resulting from substitution of any of the 3rd, 5th, 7th, 8th, 11th, 12th, and 13th amino acid residues of SEQ ID NO: 296 in peptide E.

In peptide E, it may be a peptide wherein
the 3rd amino acid residue of SEQ ID NO: 296 is an optionally modified phenylalanine (Phe),
the 5th amino acid residue of SEQ ID NO: 296 is an optionally modified tryptophan (Trp),
the 7th amino acid residue of SEQ ID NO: 296 is an optionally modified tyrosine (Tyr),
the 8th amino acid residue of SEQ ID NO: 296 is an optionally modified tyrosine (Tyr),
the 11th amino acid residue of SEQ ID NO: 296 is an optionally modified arginine (Arg) or an optionally modified lysine (Lys),
the 12th amino acid residue of SEQ ID NO: 296 is an optionally modified arginine (Arg) or an optionally modified lysine (Lys), or
the 13th amino acid residue of SEQ ID NO: 296 is an optionally modified tyrosine (Tyr) or an optionally modified phenylalanine (Phe).

In addition, in peptide E, it may be a peptide wherein
the 3rd amino acid residue of SEQ ID NO: 296 is Phe, F3C, F2C, F2OMe, F4C, Cha, MeF, MeF35dC, MeF4F, MeF4Ome, MeNal1, Me3Py, Me4Py, Me3OMe, MeF3COO, MeF3F, Glu, Epyrl2RCOO, Dpyrl2RCOO, or MeF3C, particularly preferably Phe, MeF, or MeF3C,
the 5th amino acid residue of SEQ ID NO: 296 is Trp, MeW, aMeW, dp, F3C, F3F, F3OMe, F4C, F4F, Hph, MemBph, MeNal1, MeNal2, MeoBph, W4OMe, W1Et, W1Et7Cl, W1iPr, Yph, W1Pr, W5C, W5F, W1aa, W1EtOH, W4OMe, W1mCON, or W6F, particularly preferably Trp or MeW,
the 7th amino acid residue of SEQ ID NO: 296 is Tyr, 3Py6OMe, Ala, Ahp, Phe, F3H, F4C, Nal1, Arg, or Trp, particularly preferably Tyr,
the 8th amino acid residue of SEQ ID NO: 296 is Phe, Tyr, Typ, Ahp, MeY, F4OMe, 3Imp, 4Py, 3Py, 3Py6OMe, F3C, F3CON, F4C, F4aao, F4F, f4OEt, MeF34dOMe, Yae, Lys, Orn, or Nal1, particularly preferably Tyr or F4OMe,
the 11th amino acid residue of SEQ ID NO: 296 is Arg, Ala, Asp, Gly, Glu, Lys, MeK, MeR, Dap, Dap, Abu, Aib, Hly, dorn, aMeK, A1Me4pip, KCOpipzMe, F4G, Nle, Nva, or Orn, particularly preferably Lys or Arg,
the 12th amino acid residue of SEQ ID NO: 296 is Lys, Arg, dr, Tyr, F4G, Orn, Hly, da, Cit, Dap, or Dab, particularly preferably Lys, Arg, or dr, and
the 13th amino acid residue of SEQ ID NO: 296 is Ala, Phe, Asn, Tyr, or pHPeG, particularly preferably Phe or Tyr.

Furthermore, this peptide may be a peptide having the following amino acid sequence at the N-terminal of peptide E:
the 14th amino acid residue of SEQ ID NO: 296 is MeTyr, Tyr, Phe, Ala, aMeY, Glu, Gly, Arg, Val, MeoBphMeBph, MeF, MemBph, MeNal1, MeNal2, MeoBph, MeW, or pHPeG, particularly preferably Phe or MeW, and
the 15th amino acid residue of SEQ ID NO: 296 is Cys or Hgl.

Furthermore, in peptide E, it may be a peptide wherein
the 3rd amino acid residue of SEQ ID NO: 296 is phenylalanine (Phe), methylated phenylalanine (MeF), or N-methyl-3-chloro-L-phenylalanine (MeF3C),
the 5th amino acid residue of SEQ ID NO: 296 is tryptophan (Trp) or methylated tryptophan (MeW),
the 7th amino acid residue of SEQ ID NO: 296 is tyrosine (Tyr),
the 8th amino acid residue of SEQ ID NO: 296 is tyrosine (Tyr) or (S)-2-amino-3-(4-methoxyphenyl)propanoic acid (F4OMe),
the 11th amino acid residue of SEQ ID NO: 296 is arginine (Arg) or lysine (Lys),
the 12th amino acid residue of SEQ ID NO: 296 is arginine (Arg) or D-type arginine (dr), and
the 13th amino acid residue of SEQ ID NO: 296 is tyrosine (Tyr) or phenylalanine (Phe).

A preferred example of this peptide is a peptide consisting of the amino acid sequence of any of SEQ ID NOs: 3 to 200, or an amino acid sequence in which the N-terminal of the amino acid sequence of any of SEQ ID NOs: 3 to 200 is chloroacetyl-Ala. A preferred example of this peptide is a peptide consisting of the amino acid sequence of any of SEQ ID NOs: 296, 300, 724, and 725, or an amino acid sequence in which the N-terminal of the amino acid sequence of any of SEQ ID NOs: 296, 300, 724, and 725 is chloroacetyl-Ala. A more preferred example of this peptide is a peptide consisting of the amino acid sequence of SEQ ID NO: 296 or 300, or an amino acid sequence in which the N-terminal of the amino acid sequence of SEQ ID NO: 296 or 300 is chloroacetyl-Ala.

In another embodiment, a preferred example of this peptide is a peptide consisting of the amino acid sequence of any of SEQ ID NOs: 3 to 200 described in the below-mentioned Tables 14 to 16, or the amino acid sequence in which the N-terminal of the amino acid sequence of any of SEQ ID NOs: 3 to 200, described in the below-mentioned Tables 14 to 16, is chloroacetyl-Ala. A preferred example of this peptide is a peptide consisting of the amino acid sequence of any of SEQ ID NOs: 296, 300, 724, and 725, or the amino acid sequence in which the N-terminal of the amino acid sequence of any of SEQ ID NOs: 296, 300, 724, and 725 is chloroacetyl-Ala. A more preferred example of this peptide is a peptide consisting of the amino acid sequence of SEQ ID NO: 296 or 300, or an amino acid sequence in which the N-terminal of the amino acid sequence of SEQ ID NO: 296 or 300is chloroacetyl-Ala.

A preferred example of this peptide is any of the above-mentioned peptides and is a cyclic peptide.

A preferred example of this peptide is a peptide containing the 1st to the 10th amino acid sequence of amino acid sequence of any of SEQ ID NOs: 1 to 200, 203 to 552, 645, and 723 to 725, or a complex (the below-mentioned linker-added peptide) of the amino acid sequence and a linker, wherein the amino acid sequence moiety has a cyclic structure. A more preferred example of this peptide is a peptide containing the 1st to the 10th amino acid sequence of the amino acid sequence of any of SEQ ID NOs: 296, 297, 300, 499, 501, 547, 548, 645, 723, 724, and 725, or a complex (the below-mentioned linker-added peptide) of the amino acid sequence and a linker, wherein the amino acid sequence moiety has a cyclic structure. A further more preferred example of this peptide is a peptide containing the 1st to the 10th amino acid sequence of the amino acid sequence of SEQ ID NO: 296 or 300, or a complex (the below-mentioned linker-added peptide) of the amino acid sequence and a linker, wherein the amino acid sequence moiety has a cyclic structure.

A particularly preferred example of this peptide is a peptide containing the 1st to the 15th amino acid sequence of the amino acid sequence of any of SEQ ID NOs: 2, 9, 21 to 148, 159 to 200, 213 to 448, 450 to 552, 645, and 723 to 725, or a complex (the below-mentioned linker-added peptide) of the amino acid sequence and a linker, wherein the amino acid sequence moiety has a cyclic structure. A particularly preferred example of this peptide is a peptide containing the 1st to the 15th amino acid sequence of the amino acid sequence of any of SEQ ID NOs: 296, 297, 300, 499, 501, 547, 548, 645, 723, 724, and 725, or a complex (the below-mentioned linker-added peptide) of the amino acid sequence and a linker, wherein the amino acid sequence moiety has a cyclic structure. A more particularly preferred example of this peptide is a peptide containing the 1st to the 15th amino acid sequence of the amino acid sequence of SEQ ID NO: 296 or 300, or a complex (the below-mentioned linker-added peptide) of the amino acid sequence and a linker, wherein the amino acid sequence moiety has a cyclic structure.

In another embodiment, a preferred example of this peptide is a peptide containing the 1st to the 10th amino acid sequence of the amino acid sequence of any of SEQ ID NO: 1 and SEQ ID NOs: 2 to 200, 203 to 552, 645, and 723 to 725 described in the below-mentioned Tables 14 to 19, or a complex (the below-mentioned linker-added peptide) of the amino acid sequence and a linker, wherein the amino acid sequence moiety has a cyclic structure. A more preferred example of this peptide is a peptide containing the 1st to the 10th amino acid sequence of the amino acid sequence of any of SEQ ID NOs: 296, 297, 300, 499, 501, 547, 548, 645, 723, 724, and 725 described in the below-mentioned Tables 17 to 19, or a complex (the below-mentioned linker-added peptide) of the amino acid sequence and a linker, wherein the amino acid sequence moiety has a cyclic structure. A further more preferred example of this peptide is a peptide containing the 1st to the 10th amino acid sequence of the amino acid sequence of SEQ ID NO: 296 or 300, or a complex (the below-mentioned linker-added peptide) of the amino acid sequence of any of SEQ ID NOs: 297, 499, 501, and 547 described in the below-mentioned Tables 17 to 19 and a linker, wherein the amino acid sequence moiety has a cyclic structure.

A particularly preferred example of this peptide is a peptide containing the 1st to the 15th amino acid sequence of the amino acid sequence of any of SEQ ID NOs: 2, 9, 21 to 148, 159 to 200, 213 to 448, 450 to 552, 645, and 723 to 725 described in the below-mentioned Tables 14 to 19, or a complex (the below-mentioned linker-added peptide) of the amino acid sequence and a linker, wherein the amino acid sequence moiety has a cyclic structure. A particularly preferred example of this peptide is a peptide containing the 1st to the 15th amino acid sequence of the amino acid sequence of any of SEQ ID NOs: 296, 297, 300, 499, 501, 547, 548, 645, 723, 724, and 725 described in the below-mentioned Tables 17 to 19, or a complex (the below-mentioned linker-added peptide) of the amino acid sequence and a linker, wherein the amino acid sequence moiety has a cyclic structure.

A further particularly preferred example of this peptide is a peptide with a cyclic structure consisting of the amino acid sequence of any of SEQ ID NOs: 296, 300, 724, and 725, or a complex (the below-mentioned linker-added peptide) of the amino acid sequence and a linker. The most preferred example of this peptide is a peptide with a cyclic structure consisting of the amino acid sequence of SEQ ID NO: 296 or 300, or a complex (the below-mentioned linker-added peptide) of the amino acid sequence and a linker.

In another embodiment, a further particularly preferred example of this peptide is a peptide with a cyclic structure consisting of the amino acid sequence of any of SEQ ID NOs: 296, 300, 724, and 725, or a complex (the below-mentioned linker-added peptide) of the amino acid sequence of any of SEQ ID NOs: 297, 499, 501, 547, 548, 645, and 723 described in the below-mentioned Tables 17 to 19 and a linker.

In another embodiment, a most preferred example of this peptide is a peptide with a cyclic structure consisting of the amino acid sequence of SEQ ID NO: 296 or 300, or a complex (the below-mentioned linker-added peptide) of the amino acid sequence of any of SEQ ID NOs: 297, 499, 501, and 547 described in the below-mentioned Tables 17 to 19 and a linker.

### Cyclic peptide

It refers to a peptide in which two amino acids are bonded and all or part thereof is cyclic. In the present application, peptides in which amino acids therein form a crosslinking structure, peptides in which a cyclic structure is formed by lactam ring formation or macrocyclization reaction, peptides having a lasso peptide-like structure, and the like are also included. That is, in the present application, the cyclic peptide may be one in which a moiety thereof forms a cyclic structure, and may have a linear chain moiety.

Peptides generally have poor metabolic stability in vivo, and have large sizes, which causes a problem of difficult penetration through cell membranes. In response to such problem, a method has been taken to cyclize peptides. Cyclization of peptides improves protease resistance and metabolic stability, and also limits conformational changes, which suggests that rigidity increases and membrane permeability and affinity for target proteins are improved.

### Cyclization method

Cyclization of the peptide can be performed according to known methods.

Although not limited to this, for example, by designing a peptide to include two or more cysteine residues, a cyclic structure can be formed by disulfide bonds after translation. In addition, cyclization can also be achieved by synthesizing a peptide with a chloroacetyl group at the N-terminal using genetic code reprogramming technology according to the method of Goto et al. (Y. Goto, et al. Acss Chem. Biol. 3, 120-129 (2008)), and placing a cysteine residue in the peptide. As a result, the mercapto group spontaneously makes a nucleophilic attack on the chloroacetyl group after translation, and the peptide is cyclized by a thioether bond. Through genetic code reprogramming techniques, other combinations of amino acids that are bound to form a ring may be placed within the peptide to allow for a cyclization. Alternatively, cyclization can be achieved by synthesizing a peptide having cycloamide at the N-terminal and placing an Hgl residue in the peptide. As described above, any known cyclization method can be performed without any particular limitation.

A preferred example of this peptide is any of the above-mentioned peptides, which is a peptide consisting of 15 amino acid residues.

### Peptide length

The number of amide bonds (number and length of amino acids) of the peptide and peptide site is not particularly limited, but the total amino acid residues (when the substance bound to the peptide or the linker that binds the substance and the peptide contains amino acid(s), the amino acid(s) are excluded) are preferably within 20 residues. Preferably, the number of amino acids is not less than 6, not less than 7, not less than 8, not less than 9, not less than 10, not less than 11, and preferably not more than 19, not more than 18, not more than 17, not more than 16, or not more than 15.

### Linker

The conjugate (complex) of the present invention contains a peptide and a drug, and the peptide and drug are preferably bonded via a linker. The peptide-drug linker may be formed by chemical binding of a linker of the linker-containing peptide (hereinafter also referred to as a linker-added peptide) and a linker bonded to an oligonucleotide for producing an at least partially functional dystrophin protein, which is a drug, by chemical binding of any preferable reactive group.

The linker-containing peptide may be one having a connection to a linker bound to the 5'-terminal, 3'-terminal, or both terminals of the antisense strand and/or the sense strand. In one embodiment, it is a peptide having a connection to a linker bound to the 5'-terminal, the 3'-terminal, or both terminals of an antisense strand, preferably one having a connection to a linker bound to the 5'-terminal. In one embodiment, it is a peptide having a connection to a linker bound to the 5'-terminal, the 3'-terminal, or both terminals of a sense strand, preferably one having a connection to a linker bound to the 5'-terminal. Alternatively, the linker-containing peptide is one having a connection to a linker bound to internal nucleotide of the antisense strand and/or the sense strand. In one embodiment, the linker-containing peptide is one having a connection to a linker bound to an internal nucleotide of the antisense strand. In one embodiment, the linker-containing peptide is one having a connection to a linker bound to an internal nucleotide of the sense strand. The antisense strand and/or sense strand contain two or more linker-containing peptides, which (1) may be linked at multiple positions on the antisense strand, and (2) may be linked at multiple positions on the sense strand, and/or (3) may be linked as one group at one position of the antisense strand and/or the sense strand. One linker-containing peptide may be linked to the 5'-terminal and 3'-terminal of the antisense strand and/or the sense strand, respectively.

In antisense strand and/or sense strand, the linker(s) bound to the 5'-terminal, the 3'-terminal, or both terminals thereof, a linker-containing peptide, and additionally, a peptide having cell permeability may be linked. In one embodiment, linker(s) bound to the 5'-terminal, the 3'-terminal, or both terminals of an antisense strand, and a peptide having cell permeability may be further linked, preferably, a linker bound to the 5'-terminal, a linker-containing peptide, and additionally, a peptide having cell permeability may be further linked. In one embodiment, linker(s) bound to the 5'-terminal, the 3'-terminal, or both terminals of a sense strand, and a peptide having cell permeability are further linked, preferably, a linker bound to the 5'-terminal and a peptide having cell permeability are further linked. Alternatively, a linker bound to an internal nucleotide of antisense strand and/or sense strand and a peptide having cell permeability are further linked. In one embodiment, a linker bound to an internal nucleotide of an antisense strand and a peptide having cell permeability are further linked. In one embodiment, a linker bound to an internal nucleotide of a sense strand and a peptide having cell permeability are further linked. The antisense strand and/or sense strand contain two or more peptides having cell permeability, which (1) may be linked at multiple positions on the antisense strand, and (2) may be linked at multiple positions on the sense strand, and/or (3) may be linked as one group at one position of the antisense strand and/or the sense strand. One peptide having cell permeability may be linked to the 5'-terminal and 3'-terminal of the antisense strand and/or the sense strand, respectively.

The peptides having cell permeability are preferably Pip7a, Pip7b, Pip7b2, Pip7c, Pip7c2, Pip8a, Pip8b, Pip8c, Pip8c2, Pip8d, Pip9b, Pip9b2, Pip9c, pip9c2, Pip9c3, pip9d, and Pip9d2 disclosed in WO 2013030569, or a combination of any of these. More preferably, they are Pip9b, Pip9b2, Pip9c, Pip9d, and Pip9d2, or a combination of any of these. Further preferably, the peptide is Pip9b2. Pip9b2 has the following structure.

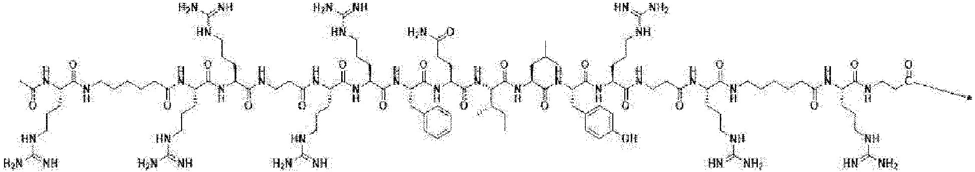

When the linker(s) bound to the 5'-terminal, the 3'-terminal, or both terminals, or internal nucleotides of antisense strand and/or sense strand and a peptide having cell permeability are linked, the peptide or linker-containing peptide of the present invention may be linked to the peptide having cell permeability. Preferably, the peptide or linker-containing peptide of the present invention is linked to the N-terminal or the inside of the peptide having cell permeability, more preferably to the N-terminal. In another embodiment, the peptide or linker-containing peptide of the present invention is linked to the inside of the peptide having cell permeability.

The linker-added peptide is described below.

Examples of the linker in the linker-added peptide include a linker having an amino acid length of not less than 1 and not more than 15 and containing one or more selected from glycine (Gly) and serine (Ser).

In a preferred example of this linker, the N-terminal is an optionally modified cysteine (Cys) or optionally modified lysine (Lys).

Another example of the linker in the linker-added peptide is a linker having an amino acid length of not less than 1 and not more than 5 and containing one or more selected from glutamic acid in a D form (de) and methylated glycine (MeG).

In a preferable example of this linker, the N-terminal is an optionally modified cysteine (Cys) or optionally modified lysine (Lys).

Another example of the linker in the linker-added peptide is a PEG linker containing polyethylene glycol (PEG) or a polyethylene glycol derivative. The derivative of the polyethylene glycol includes all those known as PEG linkers.

The PEG linker preferably further contains one or more selected from glycine (Gly), serine (Ser), glutamic acid (Glu), arginine (Arg), and lysine (Lys).

In a preferable example of this linker, the N-terminal is an optionally modified cysteine (Cys) or optionally modified lysine (Lys).

Another example of the linker in the linker-added peptide is a linker having the amino acid sequence shown in any of SEQ ID NOs: 553 to 644.

A preferred example of the linker in the linker-added peptide is
polyethylene glycol (PEG),
G linker which is a peptide linker consisting of Gly or MeG, GS linker which is a peptide linker consisting of Gly or MeG and Ser, or
a linker having the amino acid sequence shown in any of SEQ ID NOs: 553 to 644 shown in the below-mentioned Table 20.

Specific examples of the linker-added peptide include linker-added peptides shown in the below-mentioned Tables 15 to 19, linkers shown in the below-mentioned Table 20, and linker-added peptides derived from peptide shown in the below-mentioned Tables 14, 17, and 18. The amino acid sequence of the peptide moiety in the linker-added peptide of each SEQ ID NO is shown in the attached Sequence Listing.

Specific preferred examples of the linker-added peptide include the following.

(SEQ ID NO: 297) 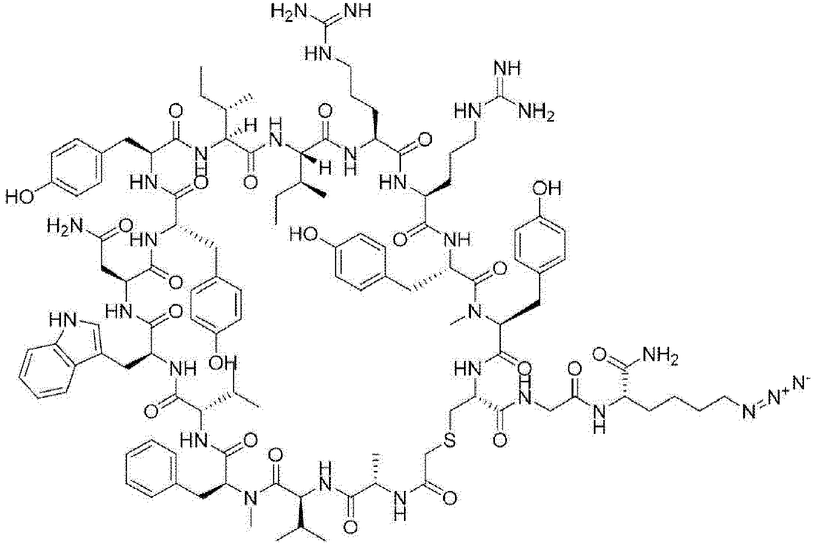
(SEQ ID NO: 499) 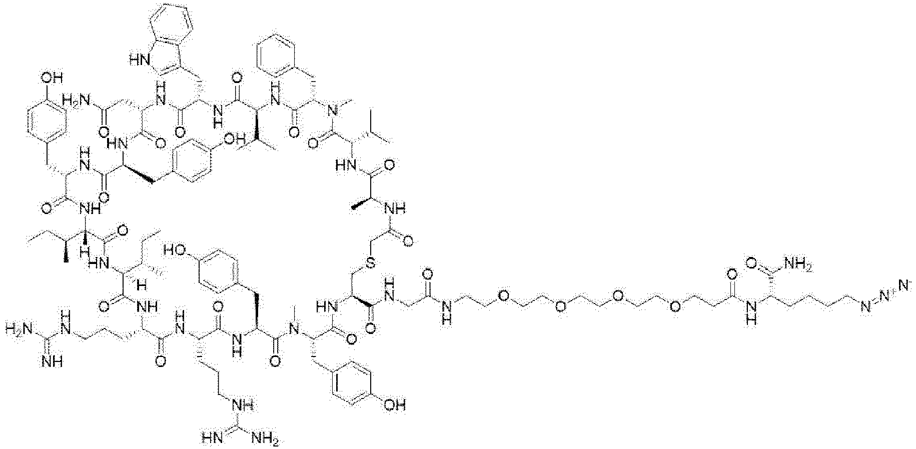
(SEQ ID NO: 501) 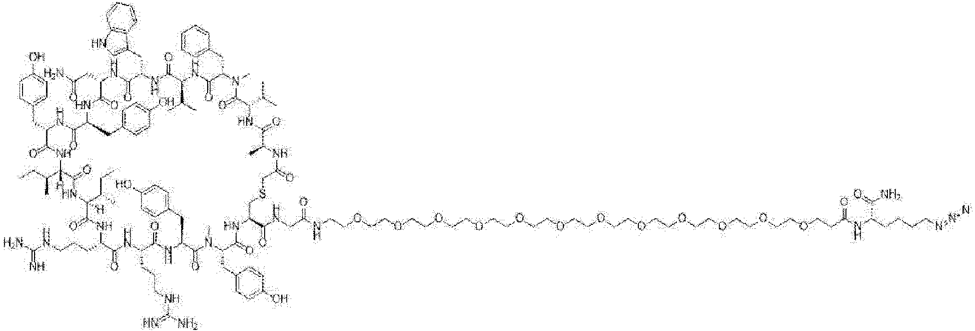
(SEQ ID NO: 547) 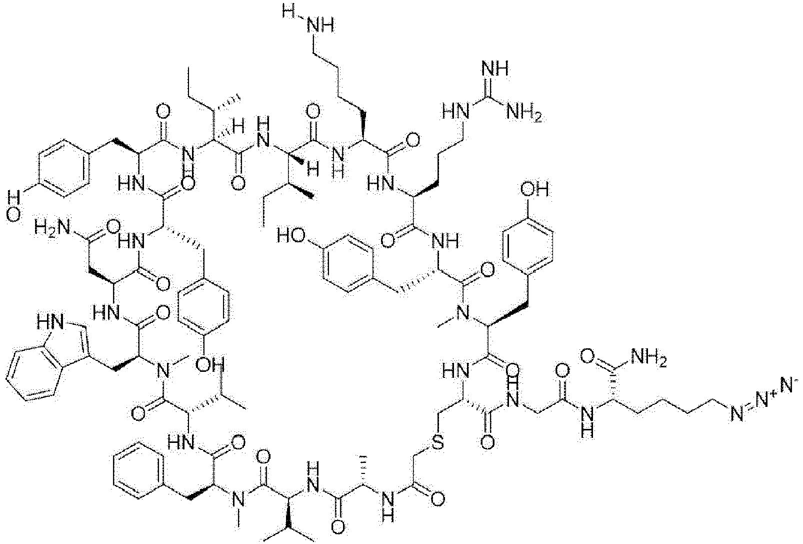
(SEQ ID NO: 645) 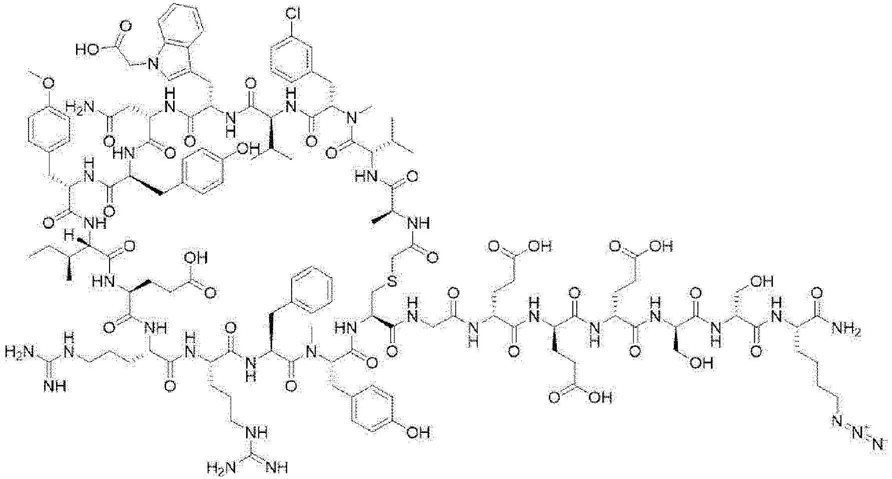
(SEQ ID NO: 723)

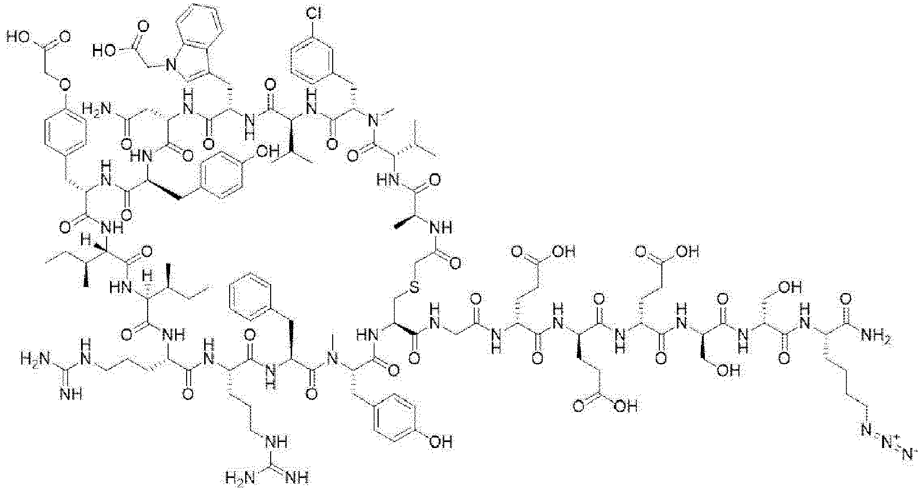

The azide group (-N₃) at the terminal of the linker-added peptide in the above-mentioned specific examples is chemically bound to reaction groups of the above-mentioned linker bound to oligonucleotide, the 5'-terminal, the 3'-terminal, or both terminals, or internal nucleotides of an antisense strand and/or sense strand to form a linker between peptide-drug. In one embodiment, one having a connection to a linker bound to the 5'-terminal, the 3'-terminal, or both terminals, preferably one having a connection to a linker bound to the 5'-terminal. In one embodiment, one having a connection to a linker bound to the 5'-terminal, the 3'-terminal, or both terminals of the sense strand are, preferably one having a linker bound to the 5'-terminal.

A linker (to be also referred to as crosslinker) in the present specification shows an intermolecular linkage between the peptide that binds to the transferrin receptor and the drug desired to be delivered to muscle tissue, and may be any linker known or described in the present specification. In a specific embodiment, the aforementioned linker is, for example, a chemical linker, a fatty acid linker, or a peptide linker (polypeptide linker). Also, it may be, for example, a complex of a chemical linker and a peptide linker or the like. For example, it may be a linker structure having both PEG and an amino acid residue or peptide moiety shown in SEQ ID NO: 616, SEQ ID NO: 627, and the like in the below-mentioned Table 20.

The linker may be, for example, one that is dissociated or separated depending on the environment and conditions, or one that maintains a stable structure.

Chemical linker: In some embodiments, the linker may be a chemical linker. Examples of the chemical linker include, but are not limited to, substituted or unsubstituted alkylene, substituted or unsubstituted heteroalkylene, substituted or unsubstituted cycloalkylene, substituted or unsubstituted hetero cycloalkylene, substituted or unsubstituted arylene and/or substituted or unsubstituted heteroarylene. In addition, the peptide and linker can be conjugated via sulfhydryl group, amino group (amine), and/or carbohydrate or any preferred reactive group. Homobifunctional and heterobifunctional cross reacting linkers (conjugation agents) are available from many commercial sources. The cross reacting linker may contain flexible arms, for example, 2, 3, 4, 5, 6, 7, 8, 9, 10, 11, 12, 13, 14, or 15 carbon atoms. Examples of the cross reacting linker include BSS3(bis (sulfosuccinimidyl)suberate), NHSS/EDC(N-hydroxysuccinimide-N-ethyl-(dimethylaminopropyl)carbodiimide), sulfo-EMCSS([N-e-maleimide caproic acid]hydrazide), hydrazide, SSATA(N-succinimidyl-SS-acetylthioacetic acid), and the like.

A preferred example of the chemical linker is a PEG (polyethylene glycol) linker. For example, the PEG linker may be a PEG linker consisting of 1 to 24 ethylene glycol units.

Fatty acid linker: The linker may be a fatty acid linker that includes a divalent chemical moiety derived from a fatty acid. For example, the fatty acid linker may be a linker with 12-aminododecanoic acid.

Peptide linker: The peptide linker contains at least one amino acid (e.g., a peptide of at least 2, 3, 4, 5, 6, 7, 10, 15, 20, 25, 40, or 50 amino acids). In a specific embodiment, the linker is a single amino acid (e.g., any naturally occurring amino acid such as Cys). In other embodiments, a glycine-rich peptide such as a peptide having the sequence [Gly-Gly-Gly-Gly-Ser (SEQ ID NO: 726)]n wherein n is 1, 2, 3, 4, 5, or 6), as described in US Patent No. 7,271,149, or the like is used. In other embodiments, serine-rich peptide linkers are used, such as those described in US Patent No. 5,525,491. As the serine-rich peptide linker, one having the formula [X-X-X-X-Gly]y wherein up to two of X's are Thr, the remaining X's are Ser, and y is from 1 to 5 (e.g., [Ser-Ser-Ser-Ser-Gly (SEQ ID NO: 727)]y wherein y is two or more)) can be mentioned. In some cases, the linker is a single amino acid (e.g., any amino acid such as Gly, Ala, or the like).

Linkers that can be cleaved under specific environments and conditions include N-acylhydrazone, disulfide, Val-Cit (valine-citrulline-p-aminobenzyl alcohol) described in a document (Chem. Soc. Rev., 2019, 48, 4361., etc.), and the like.

Alternatively, other known linkers, such as linkers described in, for example, WO 2021/054370, WO 2020/209285, WO 2020/028832, WO 2017/221883, WO 2012/150960, J. Am. Chem. Soc., 2021, 143, 3416-3429, Antibodies 2020, 9, 2, and the like can be mentioned.

Preferred examples in which the linker of the above-mentioned linker-added peptide is bound to the linker bound to oligonucleotide, the 5'-terminal, the 3'-terminal, or both terminals, or internal nucleotides of an antisense strand and/or sense strand include the following. In one embodiment, one having a connection to a linker bound to the 5'-terminal, or 3'-terminal, or both terminals of an antisense strand, preferably one having a connection to a linker bound to the 5'-terminal. In one embodiment, one having a connection to a linker bound to the 5'-terminal, the 3'-terminal, or both terminals of a sense strand, preferably one having a connection to a linker bound to the 5'-terminal. 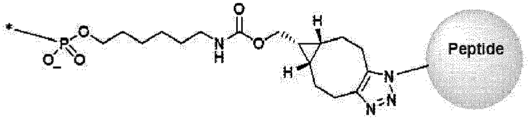 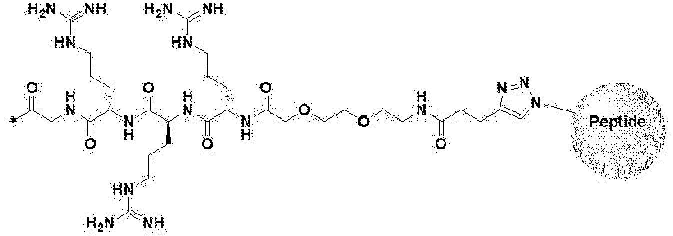 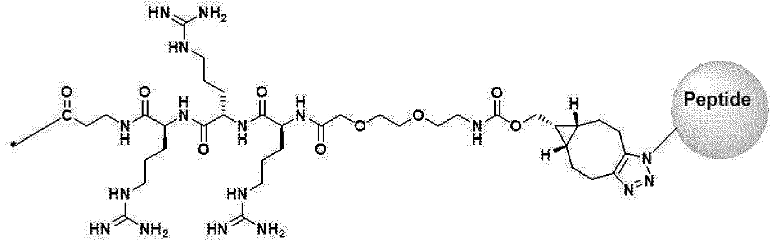 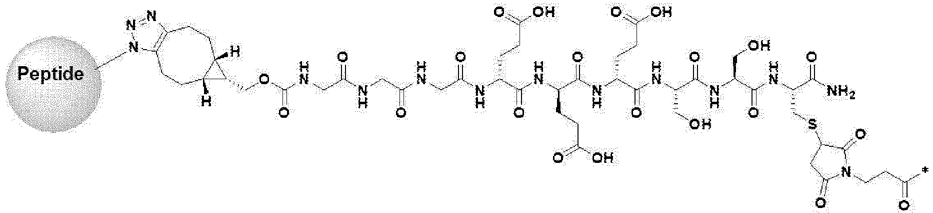 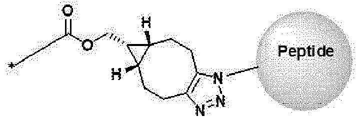 wherein Peptide is a linker-added peptide.

The peptide in the present invention can be prepared by a known peptide synthesis process, for example, chemical synthesis processes such as liquid phase method, solid phase method, and hybrid method using liquid phase synthesis and solid phase synthesis in combination; genetic recombination, and the like.

In solid phase method, for example, esterification is performed between the hydroxyl group of a hydroxyl-containing resin and the carboxyl group of a first amino acid (usually, the C-terminal amino acid of an intended peptide) having an α-amino group protected with a protecting group. As an esterifying catalyst, a known dehydration condensation agent such as 1-mesitylenesulfonyl-3-nitro-1,2,4-triazole (MSNT), dicyclohexylcarbodiimide (DCC), or diisopropylcarbodiimide (DIPCDI) can be used.

Next, the protecting group is removed from the α-amino group of the first amino acid and at the same time, a second amino acid having functional groups, other than the carboxyl group of the main chain, all protected is added to activate the carboxyl group and bind the second amino acid to the first amino acid. Then, the α-amino group of the second amino acid is deprotected and a third amino acid having functional groups, other than the carboxyl group of the main chain, all protected is added to activate the carboxyl group and bind the third amino acid to the second amino acid. After a peptide having a desired length is synthesized by repeating the above-mentioned steps, all the functional groups are deprotected.

Examples of the resin to be used in solid phase synthesis include Merrifield resin, MBHA resin, Cl-Trt resin, SASRIN resin, Wang resin, Rink amide resin, HMFS resin, Amino-PEGA resin (Merck), and HMPA-PEGA resin (Merck). These resins can be used after washing with a solvent (dimethylformamide (DMF), 2-propanol, or methylene chloride, etc.).

Examples of the protecting group of the α-amino group include a benzyloxycarbonyl (Cbz or Z) group, a tert-butoxycarbonyl (Boc) group, fluorenylmethoxycarbonyl (Fmoc) group, a benzyl group, an allyl group, and an allyloxycarbonyl (Alloc) group. The Cbz group can be removed using hydrofluoric acid, hydrogenation, or the like; the Boc group can be removed using trifluoroacetic acid (TFA); and the Fmoc group can be removed by the treatment with piperidine.

The α-carboxyl group can be protected with a methyl ester, an ethyl ester, a benzyl ester, a tert-butyl ester, a cyclohexyl ester, or the like.

As other functional groups of amino acids, the hydroxyl group of serine or threonine can be protected with a benzyl group or tert-butyl group; and the hydroxyl group of tyrosine can be protected with a 2-bromobenzyloxycarbonyl or tert-butyl group. The amino group of the lysine side chain or the carboxyl group of glutamic acid or aspartic acid can be protected in a manner similar to that used for protecting the α-amino group or α-carboxyl group.

The carboxyl group can be activated using a condensing agent. Examples of the condensing agent include dicyclohexylcarbodiimide (DCC), diisopropylcarbodiimide (DIPCDI), 1-ethyl-3-(3-dimethylaminopropyl)carbodiimide (EDC or WSC), (1H-benzotriazol-1-yloxy)tris(dimethylamino)phosphonium hexafluorophosphate (BOP), 1-[bis(dimethylamino)methyl]-1 H-benzotriazolium-3-oxide hexafluorophosphate (HBTU).

A peptide chain can be cleaved from the resin by treating it with an acid such as TFA or hydrogen fluoride (HF).

Preparation of a peptide by genetic recombination method (translation synthesis system) can be performed using a nucleic acid encoding the peptide of the present invention. The nucleic acid encoding the peptide in the present invention may be DNA or RNA.

The nucleic acid encoding the peptide of the present invention can be prepared by a known method or a method analogous thereto. For example, it can be synthesized by an automatic synthesizer. A restriction enzyme recognition site may be added in order to insert the resulting DNA into a vector, or a base sequence encoding an amino acid sequence for cleaving the resulting peptide chain by an enzyme may be incorporated.

As described above, when a peptide in the present invention is fused with a peptide having cell permeability or the like, the above-mentioned nucleic acid may also include a nucleic acid encoding peptides having cell permeability.

In order to suppress degradation by host-derived proteases, a chimeric protein expression method can also be used in which the target peptide is expressed as a chimeric peptide with another peptide. In this case, a nucleic acid encoding a peptide of interest and a peptide that binds to the peptide is used as the above-mentioned nucleic acid.

Next, an expression vector is prepared using the nucleic acid encoding the peptide in the present invention. The nucleic acid as it is, or after digestion with a restriction enzyme or adding a linker, can be inserted downstream of the promoter of the expression vector. Examples of the vector include Escherichia coli-derived plasmids (pBR322, pBR325, pUC12, pUC13, pUC18, pUC19, pUC118, pBluescript II etc.), Bacillus subtilis-derived plasmids (pUB110, pTP5, pC1912, pTP4, pE194, pC194 etc.), yeast-derived plasmids (pSH19, pSH15, YEp, YRp, YIp, YAC etc.), bacteriophages (e phage, M13 phage etc.), viruses (retrovirus, vaccinia virus, adenovirus, adeno-associated virus (AAV), cauliflower mosaic virus, tobacco mosaic virus, baculovirus etc.), and cosmids.

The promoter can be appropriately selected according to the kind of a host. When the host is an animal cell, for example, SV40 (simian virus 40)-derived promoter or CMV (cytomegalovirus)-derived promoter can be used. When the host is Escherichia coli, a trp promoter, a T7 promoter, lac promoter, or the like can be used.

The expression vector may contain a DNA replication origin (ori), a selection marker (antibiotic resistance, nutrition requirements, or the like), an enhancer, a splicing signal, a poly-A additional signal, a nucleic acid encoding a tag (FLAG, HA, GST, GFP, or the like), and the like.

Next, a suitable host cell is transformed with the above-mentioned expression vector. The host can be appropriately selected based on the relation with the vector and for example, Escherichia coli, Bacillus subtilis, Bacillus bacteria), yeasts, insects or insect cells, and animal cells can be used. Examples of the animal cells include HEK293T cells, CHO cells, COS cells, myeloma cells, HeLa cells, and Vero cells. Transformation can be performed in a known method such as lipofection method, calcium phosphate method, electroporation method, microinjection method, or particle gun method, depending on the kind of the host. The transformant is then cultured by a conventional method to express an intended peptide.

Purification of the peptide from the culture of the transformant can be performed in the following: cultured cells are collected and then suspended in an appropriate buffer, the resulting suspension is subjected to ultrasonic treatment, freezing and thawing, or the like to destruct the cells, then centrifugation or filtration is performed to obtain a crude product. When the peptide is secreted in the culture medium, the supernatant is collected.

Purification of the crude extract or culture supernatant can also be performed by a known method or a method analogous thereto (for example, salting-out, dialysis, ultrafiltration, gel filtration, SDS-PAGE, ion exchange chromatography, affinity chromatography, or reverse-phase high-performance liquid chromatography).

The peptide thus obtained may be converted from a free form to a salt or from a salt to a free form by a known method or a method analogous thereto.

The translation synthesis system may be a cell-free translation system. The cell-free translation system includes, for example, ribosomal protein, aminoacyl tRNA synthase (ARS), ribosomal RNA, amino acid, rRNA, GTP, ATP, translation initiation factor (IF) elongation factor (EF), release factor (RF), and ribosome recycling factor (RRF), as well as other factors necessary for translation. Escherichia coli extract and wheat germ extract may be added to increase the expression efficiency. Furthermore, rabbit erythrocyte extracts and insect cell extracts may be added.

By continuously supplying energy by using dialysis to a system containing these, it is possible to produce proteins of several hundred µg to several mg/mL. A system containing RNA polymerase may also be used to perform transcription from gene DNA. As commercially available cell-free translation systems, RTS-100 (registered trademark) of Roche Diagnostics, PURESYSTEM of GeneFrontier Corporation, PURExpress In Vitro Protein Synthesis Kit of NEW ENGLAND Biolabs, and the like can be used as systems derived from Escherichia coli, and those available from ZOEGENE Corporation and CellFree Sciences Co., Ltd. can be used as systems using wheat germ extracts.

According to the cell-free translation system, the expression product can be obtained in a highly pure form without purification.

In the cell-free translation system, an artificial aminoacyl tRNA obtained by linking (acylating) a desired amino acid, or hydroxy acid to a tRNA may be used instead of an aminoacyl tRNA synthesized with a natural aminoacyl tRNA synthetase. Such an aminoacyl tRNA can be synthesized using an artificial ribozyme.

Examples of such a ribozyme include flexizyme (H. Murakami, H. Saito, and H. Suga, (2003), Chemistry & Biology, Vol. 10, 655-662; H. Murakami, D. Kourouklis, and H. Suga, (2003), Chemistry & Biology, Vol. 10, 1077-1084; H. Murakami, A. Ohta, H. Ashigai, H. Suga (2006) Nature Methods 3, 357-359 "The flexizyme system: a highly flexible tRNA aminoacylation tool for the synthesis of nonnatural peptides"; N. Niwa, Y. Yamagishi, H. Murakami, H. Suga (2009) Bioorganic & Medicinal Chemistry Letters 19, 3892-3894 "A flexizyme that selectively charges amino acids activated by a water-friendly leaving group"; and WO2007/066627 etc.). Flexizyme is also known under the name of flexizyme (Fx) in original form and also under the name of a modified one such as dinitrobenzyl flexizyme (dFx), enhanced flexizyme (eFx), or aminoflexizyme (aFx).

By using tRNA to which the desired amino acid or hydroxy acid has been linked and which is produced by flexizyme, the desired codon can be translated in association with the desired amino acid or hydroxyl acid. A special amino acid may be used as the desired amino acid. For example, non-natural amino acids necessary for the aforementioned cyclization can also be introduced into the binding peptide by this method.

The cyclic peptides and analogs thereof in the present invention can be chemically synthesized using methods widely used in the pertinent technique field such as stepwise solid-phase synthesis, semi-synthesis of peptide fragments via conformationally-assisted religation, and chemical ligation. The synthesis of the cyclic peptides and analogs thereof described in present specification is, for example, chemical synthesis using various solid-phase techniques, such as those described in K. J. Jensen, P. T. Shelton, S. L. Pedersen, Peptide Synthesis and Applications, 2nd Edition, Springer, 2013 and the like. A preferred strategy is based on the combination of an Fmoc group that temporarily protects the α-amino group and allows selective removal by base, and a protecting group that temporarily protects the side chain functional group and is stable to de-Fmoc conditions. Such general selection of peptide side chains is known from the aforementioned Peptide Synthesis and Applications, 2nd edition, G. B. Fields, R. L. Noble, Solid Phase Peptide Synthesis Utilizing 9-Fluorenylmethoxycarbonyl Amino Acids, Int. J. Peptide Protein Res. 35, 1990, 161-214, and the like. Preferred peptide side chain protecting group includes Boc group and Mtt group for amino groups such as lysine, tert-butyl group for the carboxyl group of glutamic acid and aspartic acid, and Trt and Mmt groups for the thiol group of cysteine.

The peptides and analogs thereof described in the present invention can be synthesized in a stepwise manner on the aforementioned solid phase resin. The α-amino protecting group of the C-terminal amino acid to be used and all amino acids and peptides used in the synthesis must be selectively removed during the synthesis process. Preferably, using the aforementioned solid-phase resin, the C-terminal carboxyl group of a peptide whose N-terminus is appropriately protected with Fmoc or the C-terminal carboxyl group of an amino acid protected with Fmoc is converted into an activated ester with an appropriate reagent, and then added to the amino groups on the solid phase resin. Subsequent elongation of the peptide chain can be achieved by sequentially repeating removal of the N-terminal protecting group (Fmoc group) and then condensation of the protected amino acid derivative according to the amino acid sequence of the target peptide. These can release the target peptide in the final step. For example, as the conditions for release, it can be released using a TFA solution containing water/silyl hydride/thiol as a scavenger in TFA, such as those listed in Teixeira, W. E. Benckhuijsen, P. E. de Koning, A. R. P. M. Valentijn, J. W. Drijfhout, Protein Pept. Lett., 2002, 9, 379-385 and the like. As a typical example, TFA/Water/TIS/DODT (volume ratio 92.5: 2.5: 2.5: 2.5) can be mentioned.

The peptide analogue described in the present specification can be synthesized using a single or multichannel peptide synthesizer, such as the Liberty Blue Synthesizer of CEM or the Syro I Synthesizer of Biotage.

The carboxyl group can be activated using a condensing agent. Examples of the condensing agent include dicyclohexylcarbodiimide (DCC), diisopropylcarbodiimide (DIPCDI), 1-ethyl-3-(3-dimethylaminopropyl)carbodiimide (EDC or WSC), (1H-benzotriazol-1-yloxy)tris(dimethylamino)phosphonium hexafluorophosphate (BOP), 1-[bis(dimethylamino)methyl]-1H-benzotriazolium-3-oxide hexafluorophosphate (HBTU), and the like.

### Drug

The drug in the present invention is a compound containing oligonucleotide for producing at least partially functional dystrophin protein (hereinafter also to be referred to as "the compound containing an oligonucleotide of the present invention").

The dystrophin gene, which is responsible for Duchenne muscular dystrophy, Becker muscular dystrophy, and X-linked dilated cardiomyopathy, is a large gene located in Xp21.1 and consists of 79 exons. As abnormalities in the dystrophin gene, for example, according to Muscle & Nerve 2006; 135-144, out of 4700 cases, 72% had deletions of one or more exons, 7% had duplications of one or more exons, 20% had minute mutation such as single base substitution·insertion·deletion, and the like, and 1% had other mutations such as splice site abnormalities (e.g., natural exon skipping). As a result of these mutations, the reading frame of the codon shifts (out-of-frame mutation) or nonsense mutation causes the appearance of immature stop codon to stop the translation prematurely, resulting in no production of functional dystrophin protein and Duchenne muscular dystrophy is developed.

Dystrophin protein contains an N-terminal actin-binding domain (encoded by exons 2 to 8), a central rod domain (exons 9 to 61) containing 24 spectrin-like repeat units (SLRs), a cysteine rich (CR) domain (exon 62 to 70) containing a dystroglycan-binding site, and a C-terminal (CT) domain containing α-syntrophin binding site, actin binding domain, CR and CT domain are essential for the function of dystrophin, whereas the central rod domain partially retains its dystrophin function even when some of the 24 SLRs are deleted. Therefore, an at least partially functional dystrophin protein can be produced by correcting the reading frame shifts caused by out-of-frame mutation by skipping one or more exons, preferably the exon encoding the central rod domain (exon skipping), or by inducing cryptic splicing (exon inclusion) to make the total number of deleted or duplicated bases to a multiple of 3, thereby producing partially functional dystrophin protein. Alternatively, even when an immature stop codon occurs due to a nonsense mutation, an at least partially functional dystrophin protein can be produced by removing immature stop codons by skipping the exon containing the mutation (when the number of bases in the exon is not a multiple of 3, adjacent exons are further skipped to set the number of deleted bases to a multiple of 3) .

The "compound containing an oligonucleotide for producing at least partially functional dystrophin protein" in the conjugate of the present invention includes an oligonucleotide that hybridizes with a specific nucleotide sequence in pre-mRNA encoding dystrophin to change the splicing of the target exon, resulting in the production of an mRNA encoding an at least partially functional dystrophin protein.

Such oligonucleotides include nucleotide sequences complementary to sequences at the 5' or 3' splice site of the target exon in the pre-mRNA, or to cis sequences that promote or suppress splicing within the target exon, and oligonucleotides that can alter the splicing of the target exon can be mentioned.

The term "nucleic acid" or "nucleic acid molecule" used in the present specification may refer to a monomeric nucleotide or nucleoside, or may refer to an oligonucleotide consisting of multiple monomers. The term "nucleic acid strand" or "strand" is also used to refer to an oligonucleotide in the present specification. Nucleic acid strand can be made into full-length or partial strands by chemical synthesis methods (e.g., using an automated synthesizer) or by enzymatic processes (e.g., polymerase, ligase, or restriction reaction, though not limited thereto).

In the present specification, the "nucleoside" is a combination of a base and a sugar. The nucleobase (also known as base) moiety of a nucleoside is typically a heterocyclic base moiety. In the present specification, the "nucleotide" further includes a phosphate group covalently bonded to the sugar moiety of the nucleoside. For nucleosides containing pentofuranosyl sugar, the phosphate group can be linked to the 2', 3', or 5' hydroxyl moiety of the sugar.

In the present specification, the "oligonucleotide" refers to a linear oligomer formed by covalently linking several to several dozen hydroxyl groups of sugar moieties and phosphate groups between adjacent nucleotides. The phosphate group moieties are generally considered to form an internucleoside bond of the oligonucleotide within the oligonucleotide structure.

The "modified oligonucleotide" means an oligonucleotide in which the sugar moiety and/or base moiety and/or phosphate moiety (internucleoside bond) of at least one nucleotide residue constituting the oligonucleotide has been modified. Specific embodiments of modifications of these are described later.

Oligonucleotides may include natural nucleotides and/or non-natural nucleotides. The "natural nucleotide" include deoxyribonucleotides found in DNA and ribonucleotides found in RNA. The "deoxyribonucleotide" and "ribonucleotide" are also sometimes referred to as "DNA nucleotide" and "RNA nucleotide", respectively.

Similarly, the "natural nucleoside" includes deoxyribonucleosides found in DNA and ribonucleosides found in RNA. The "deoxyribonucleoside" and "ribonucleoside" are also sometimes referred to as "DNA nucleoside" and "RNA nucleoside", respectively. The natural nucleoside may include modified bases. In one embodiment, the natural nucleoside does not include modified bases.

The "non-natural nucleotide" refers to any nucleotide other than natural nucleotides and includes modified nucleotides. Similarly, the "non-natural nucleoside" refers to any nucleoside other than naturally occurring nucleosides, and includes modified nucleosides. The "modified nucleotide" means a nucleotide having one or more of a modified sugar moiety, a modified internucleoside linkage, and a modified nucleobase. The "modified nucleoside" refers to a nucleoside having a modified sugar moiety and/or a modified nucleobase. The nucleic acid strand containing non-natural oligonucleotide often has desirable properties such as, for example, enhanced cellular uptake, enhanced affinity for nucleic acid targets, increased stability in the presence of nucleases, or increased inhibitory activity and is preferred over the natural form.

In the present specification, the "nucleobase" or "base" includes both modified and unmodified nucleobases (bases), unless otherwise specified. Therefore, unless otherwise specified, the purine base may be either modified or non-modified purine base. Furthermore, unless otherwise specified, the pyrimidine base may be either a modified or non-modified pyrimidine base.

In the present specification, the "modified nucleobase" or "modified base" means any nucleobase other than adenine, cytosine, guanine, thymine, or uracil. The "non-modified nucleobase" or "non-modified base" ("natural nucleobase") means adenine (A) and guanine (G) as purine bases, as well as thymine (T), cytosine (C), and uracil (U) as pyrimidine bases. Examples of the modified nucleobase include 5-methylcytosine, 5-fluorocytosine, 5-bromocytosine, 5-iodocytosine or N4-methylcytosine; N6-methyladenine or 8-bromoadenine; 2-thio-thymine; and N2-methylguanine or 8-bromoguanine; inosine, thymine, xanthine, hypoxanthine, nubularine, isoguanisine, tubercidine, and the like. Examples of the aforementioned base include, but are not limited to, 2-aminoadenine, alkyl derivatives such as 6-methylated purine; alkyl derivatives such as 2-propylated purine; 5-halouracil and 5-halocytosine; 5-propynyluracil and 5-propynylcytosine; 6-azouracil, 6-azocytosine and 6-azothymine; 5-uracil (pseudouracil), 4-thiouracil, 5-halouracil, 5-(2-aminopropyl)uracil, 5-aminoallyluracil; 8-halogenated, aminated, thiolated, thioalkylated, hydroxylated, and other 8-substituted purines; 5-trifluoromethylated, and other 5-substituted pyrimidines; 7-methylguanine; 5-substituted pyrimidine; 6-azapyrimidine; N-2, N-6, and 0-6 substituted purines (including 2-aminopropyladenine); 5-propynyluracil and 5-propynylcytosine; dihydrouracil; 3-deaza-5-azacytosine; 2-aminopurine; 5-alkyluracil; 7-alkylguanine; 5-alkylcytosine; 7-deazaadenine; N6,N6-dimethyladenine; 2,6-diamino purine; 5-amino-allyl-uracil; N3-methyluracil; -substituted 1,2,4-triazole; 2-pyridinone; 5-nitroindole; 3-nitropyrrole; 5-methoxyuracil; uracil-5-oxyacetic acid; 5-methoxycarbonylmethyluracil; 5-methyl-2-thiouracil; 5-methoxycarbonylmethyl-2-thiouracil; 5-methylaminomethyl-2-thiouracil; 3-(3-amino-3-carboxypropyl)uracil; 3-methylcytosine; N4-acetylcytosine; 2-thiocytosine; N6-methyladenine; N6-isopentyladenine; 2-methylthio-N6-isopentenyladenine; N-methylguanine; O-alkylated base; 5-hydroxycytosine; -acetylcytosine; 3-C1-6 alkylcytosine; 5-C1-6 alkylcytosine; 2-thiothymine; dihydrothymine; pseudothymine; 2-thiouracil; 5-(carboxyhydroxymethyl)uracil; 5-carboxymethylaminomethyl-2-thiouracil; 5-carboxymethylaminomethyluracil; 5-methylaminomethyluracil; 8-aminoguanine; 8-halogeno guanine; 7-deaza-7-(2-phenylethynyl)guanine; 7-deaza-7-(2-pyridylethynyl)guanine; 7-deaza-7-[2-(C1-7 alkanoyl oxy-C1-6 alkyl)ethynyl]guanine; 7-deaza-7-[2-(hydroxy-C1-6 alkyl)ethynyl]guanine; 1-C1-6 alkylguanine; 2,2-di(C1-6 alkyl)guanine, 2-C1-6 alkylguanine; 7-deaza-7-C1-6 alkylguanine; 7-deaza-7-C2-6 alkenylguanine; 7-deaza-7-C2-6 alkynylguanine; 7-deaza-7-halogeno guanine; 1-C1-6 alkylinosine; queuosine; β,D-galactosyl-queuosine; β,D-mannosyl-queuosine; N6-C2-6 alkenyladenine; 1-C1-6 alkyladenine; 2-C1-6 alkyladenine; N6-C1-6 alkyladenine; 2-C1-6 alkylthio-N6-C2-6 alkenyladenine, and the like. The modified nucleobase is preferably 5-methylcytosine or 2-thio-thymine, more preferably 5-methylcytosine.

In one preferred embodiment, the "compound containing an oligonucleotide for producing at least partially functional dystrophin protein" contains an oligonucleotide that induces exon skipping of dystrophin gene.

In the present specification, the "dystrophin gene" or "DMD" means a gene that encodes the dystrophin protein, which is a major constituent element of the dystrophin-glycoprotein complex that bridges the internal cytoskeleton of muscle cells, particularly myofibers, and extracellular matrix. The deletion, duplication, and point mutation in DMD cause dystrophin abnormalities such as Duchenne muscular dystrophy, Becker muscular dystrophy, cardiomyopathy, and the like. In addition, many different transcriptional variants and protein isoforms of this gene exist due to alternative use of promoters and alternative splicing. In some embodiments, the dystrophin gene may be a human (Gene ID: 1756), non-human primate (e.g., Gene ID: 465559), or rodent gene (e.g., Gene ID: 13405; Gene ID: 24907). Furthermore, multiple human transcript variants (e.g., annotated with GenBank RefSeq Accession Numbers. NM_000109.3, NM_004006.2, NM_004009.3, NM_004010.3 and NM_00401 1.3) have been confirmed to encode different protein isoforms. The dystrophin gene is preferably a human dystrophin gene. The dystrophin is preferably human dystrophin.

The target exon to be skipped in the present invention is not particularly limited as long as an mRNA encoding an at least partially functional dystrophin protein can be produced as a result of skipping the target exon, and can be selected as appropriate depending on the abnormality of the dystrophin gene in the subject of administration. For example, mRNA encoding at least partially functionally dystrophin protein can be generated by (1) skipping exon 51 for DMD that lacks exons 29 to 50, 45 to 50, 48 to 50, 49 to 50, 50, 52, or 52 to 63, (2) skipping exon 53 for DMD that lacks exons 43 to 52, 45 to 52, 47 to 52, 48 to 52, 49 to 52, 50 to 52, or 52, (3) skipping exon 45 for DMD that lacks exons 18 to 44, 44, 46 to 47, 46 to 48, 46 to 49, 46 to 51, or 46 to 53, (4) skipping exon 44 for DMD that lacks exons 35 to 43, 45, or 45 to 54, (5) skipping exon 46 for DMD that lacks exon 45, or 47 to 54, (6) skipping exon 50 for DMD that lacks exon 51, 51 to 53, or 51 to 55, and (7) skipping exon 52 for DMD that lacks exon 51, 53, or 53 to 55. Further examples of DMD mutation are disclosed in, for example, Flanigan KM, et ah, Mutational spectrum of DMD mutations in dystrophinopathy patients: Application of modern diagnostic techniques to a large cohort, and Hum Mutat. 2009 Dec; 30 (12): 1657-66, the contents of which are incorporated in full herein by reference.

As the target exons to be skipped in the present invention, exon 7, exon 8, exon 9, exon 19, exon 23, exon 44, exon 45, exon 46, exon 50, exon 51, exon 52, exon 53, exon 55, and any combination of these exons can be preferably mentioned. The target exon may be more preferably exon 44, exon 45, exon 46, exon 50, exon 51, exon 53, or exon 55, further preferably exon 44, exon 45, or exon 53, particularly preferably exon 53. In another embodiment, it may be exon 44. In another embodiment, it may be exon 45. In another embodiment, it may be exon 46.

Examples of the oligonucleotide in which the target exon is skipped include oligonucleotides containing a nucleotide sequence complementary to sequence at 5' or 3' splice site of the target exon in pre-mRNA, or a splicing promoting sequence (exon splicing enhancer; ESE) within a target exon. Preferably, the oligonucleotide includes a nucleotide sequence complementary to the sequence of all or part of the sequence of ESE of the target exon. The nucleotide sequence of each exon of the human dystrophin gene is known (GenBank accession No. NM_004006), and those of ordinary skill in the art can easily predict the ESE in each target exon based on the sequence information using, for example, the ESE search software "ESE finder" provided by Cold Spring Harbor Laboratory.

For example, even when the target exons are human exon 7, exon 8, exon 9, exon 19, exon 23, exon 44, exon 45, exon 46, exon 50, exon 51, exon 52, exon 55, the preferred target sequences are described in various documents (e.g., Wilton S.D. et al., Mol. Ther., 15(7): 1288-1296 (2007) describes antisense oligonucleotides targeting ESE located at positions 45 to 67 of exon 7, junction moiety (positions -6 to 18; negative number indicates nucleotide in intron, same below), of intron 7 and exson 8, junction moiety (positions -6 to 23) of intron 8 and exon 9, ESE located at positions 35 to 65 of exon 19, ESE located at positions 69 to 98 of exon 23, ESE located at positions 61 to 84 of exon 44, junction moiety (positions -6 to 20) of intron 44 and exon 45, ESE located at positions 107 to 137 of exon 46, ESE located at positions 2 to 30 of exon 50, ESE located at positions 66 to 90 of exon 51, ESE located at positions 12 to 41 of exon 52, ESE located at positions 39 to 69 of exon 53, ESE located at positions 141 to 160 of exon 55, and the like, each predicted using ESE finder, and those skilled in the art can easily design oligonucleotides containing nucleotide sequences complementary to all or part of those target sequences.

Alternatively, in one preferred embodiment, when the target exon is human exon 53 (the nucleotide sequence of exon 53 of the wild-type human dystrophin gene is shown in SEQ ID NO: 646) : 
(SEQ ID NO: 646)
ttgaaagaat tcagaatcag tgggatgaag tacaagaaca ccttcagaac cggaggcaac
agttgaatga aatgttaaag gattcaacac aatggctgga agctaaggaa gaagctgagc
aggtcttagg acaggccaga gccaagcttg agtcatggaa ggagggtccc tatacagtag
atgcaatcca aaagaaaatc acagaaaccaag,
the whole or a partial sequence of 10 or more consecutive nucleotides of the sequence consisting of 31st to 65th nucleotides from the 5'-terminal:
   TACAAGAACACCTTCAGAACCGGAGGCAACAGTTG (SEQ ID NO: 647)
(Unless otherwise specified, nucleotide sequences are written in the 5' to 3' direction in the present specification) can be the target sequence (e.g., WO 2012/029986, US8084601). Preferably, the following sequences:
   GAACACCTTCAGAACCGGAGGCAAC (SEQ ID NO: 648; nucleotide sequence from 36th to 60th of exon 53 (target sequence of Golodirsen)),
   GAACACCTTCAGAACCGGAGG (SEQ ID NO: 649; nucleotide sequence from 36th to 56th of exon 53 (target sequence of Viltolarsen))
can be mentioned.

In another embodiment, for example, when the target exon is human exon 44 (the nucleotide sequence of exon 44 of the wild-type human dystrophin gene is shown in SEQ ID NO: 650): 
(SEQ ID NO: 650)
gcgatttgac agatctgttg agaaatggcg gcgttttcat tatgatataa
agatatttaa
tcagtggcta acagaagctg aacagtttct cagaaagaca caaattcctg
agaattggg,
 a partial sequence of 10 to 35 consecutive nucleotides of the sequence consisting of -1st to 44th nucleotides from the 5'-terminal:
GGCGATTTGACAGATCTGTTGAGAAATGGCGGCGTTTTCATTATG (SEQ ID NO: 651); or the sequence consisting of 58th to 115th nucleotides from the 5'-terminal: 
TAATCAGTGGCTAACAGAAGCTGAACAGTTTCTCAGAAAGACACAAATTCCTGAGAA
T (SEQ ID NO: 652)
 can be the target sequence (e.g., WO 2015/194520). Preferably, TGAGAAATGGCGGC (SEQ ID NO: 653; 19th to 32nd of exon 44), AAATGGCGGCGT (SEQ ID NO: 654; 23rd to 34th of exon 44), GAAATGGCGGCG (SEQ ID NO: 655; 22nd to 33rd of exon 44), CTCAGAAAGACAC (SEQ ID NO: 656; 89th to 101st of exon 44), GTGGCTAACAGA (SEQ ID NO: 657; 64th to 75th of exon 44), AGTGGCTAAC AG (SEQ ID NO: 658; 63rd to 74th of exon 44), GTGGCTAACAGAAG (SEQ ID NO: 659; 64th to 77th of exon 44), and AGAAAGACAC AA (SEQ ID NO: 660; 92nd to 103rd of exon 44) can be mentioned. More preferably, two non-consecutive, nonoverlapping sequences such as SEQ ID NOs: 653 and 657, SEQ ID NOs: 654 and 658, SEQ ID NOs: 655 and 659, and SEQ ID NOs: 656 and 60 can be the target regions.

Alternatively, the whole or a partial sequence of 10 or more consecutive nucleotides of the sequence consisting of 9th to 34th nucleotides from the 5'-terminal of exon 44:
ACAGATCTGTTGAGAAATGGCGGCGT (SEQ ID NO: 661); or the sequence consisting of 24th to 49th nucleotides from the 5'-terminal of exon 44:
AATGGCGGCGTTTTCATTATGATATA (SEQ ID NO: 662) can be the target sequence (e.g., WO 2013/100190).

Alternatively, when the target exon is human exon 45 (the nucleotide sequence of exon 45 of the wild-type human dystrophin gene is shown in SEQ ID NO: 663):
the whole or a partial sequence of 10 or more consecutive nucleotides of the sequence consisting of 1st to 34th nucleotides from the 5'-terminal:
CAAGTCCAGGATGGCATTGGGCAGCGGCAAACTG (SEQ ID NO: 664)
can be the target sequence (e.g., US 8,324,371). Preferably, the sequence consisting of 5th to 34th nucleotides from the 5'-terminal:
   TCCAGGATGGCATTGGGCAGCGGCAAACTG (SEQ ID NO: 665); or
the sequence consisting of 1st to 30th nucleotides from the 5'-terminal:
   CAAGTCCAGGATGG CATTGGGCAG CGGCAA (SEQ ID NO: 666)
can be mentioned.

Alternatively, the whole or a partial sequence of 10 or more consecutive nucleotides of the sequence consisting of 9th to 34th nucleotides from the 5'-terminal of exon 45:
GGATGGC ATTGGGCAGC GGCAAACTG (SEQ ID NO: 667);
can be the target sequence (e.g., US 7,902,160). Preferably, the sequence consisting of 14th to 28th nucleotides from the 5'-terminal:
   GCATTGGGCAGCGGC (SEQ ID NO: 668);
the sequence consisting of 9th to 26th nucleotides from the 5'-terminal:
   GGATGGCATTGGGCAGCG (SEQ ID NO: 669; target sequence of Renadirsentarget); or
the sequence consisting of 9th to 26th nucleotides from the 5'-terminal:
   TTGGGCAGC GGCAAACTG (SEQ ID NO: 670)
can be mentioned.

Alternatively, the whole or a partial sequence of 10 or more consecutive nucleotides of the sequence consisting of -6th to 20th nucleotides from the 5'-terminal of exon 45:
TTACAGGAACTCCAGGATGGCATTGG (SEQ ID NO: 671)
can be the target sequence (e.g., US8,524,880). Preferably, the sequence consisting of -3rd to 19th nucleotides from the 5'-terminal:
   CAGGAACTCCAGGATGGCATTG (SEQ ID NO: 672; target sequence of Casimersentarget)
can be mentioned.

Alternatively, the whole or a partial sequence of 10 or more consecutive nucleotides of the sequence consisting of -3rd to 32nd nucleotides from the 5'-terminal of exon 45:
CAGGAACTCCAGGATGGCATTGGGCAGCGGCAAAC (SEQ ID NO: 673) can be the target sequence (e.g., WO 2013/100190).

Alternatively, when the target exon is human exon 46 (the nucleotide sequence of exon 46 of the wild-type human dystrophin gene is shown in SEQ ID NO: 674): 
(SEQ ID NO: 674)
gctagaagaa caaaagaata tcttgtcaga atttcaaaga gatttaaatg aatttgtttt
atggttggag gaagcagata acattgctag tatcccactt gaacctggaa aagagcagca
actaaaagaa aagcttgagc aagtcaag,
 a partial sequence of 10 to 35 consecutive nucleotides of the sequence consisting of 91st to 133rd nucleotides from the 5'-terminal:
TATCCCACTTGAACCTGGAAAAGAGCAGCAACTAAAAGAAAAG (SEQ ID NO: 675)
can be the target sequence (e.g., US 8,552,172). Preferably, as the target sequence, any of the following sequences can be mentioned.
TATCCCACTTGAACCTGGAAAAGAGCAGCA (SEQ ID NO: 676;
   nucleotide sequence from 91st to 120th of exon 46)
TTGAACCTGGAAAAGAGCAGCAACTAAAAG (SEQ ID NO: 677; nucleotide sequence from 99th to 128th of exon 46)
CCTGGAAAAGAGCAGCAACTAAAAGAAAAG (SEQ ID NO: 678; nucleotide sequence from 104th to 133rd of exon 46)
CACTTGAACCTGGAAAAGAGCAGCAACTAA (SEQ ID NO: 679; nucleotide sequence from 96th to 125th of exon 46)

Alternatively, when the target exon is human exon 50 (the nucleotide sequence of exon 50 of the wild-type human dystrophin gene is shown in SEQ ID NO: 680): the whole or a partial sequence of 10 or more consecutive nucleotides of the sequence consisting of 105th to 129th nucleotides (110th to 129th are the sequence of intron 50) from 5'-terminal: agcctg taagtatact ggatcccat (SEQ ID NO: 681) can be the target sequence (e.g., WO 2013/100190).

For example, when the target exon is human exon 51 (the nucleotide sequence of exon 51 of the wild-type human dystrophin gene is shown in SEQ ID NO: 682): 
(SEQ ID NO: 682)
ctcctactca gactgttact ctggtgacac aacctgtggt tactaaggaa actgccatct
ccaaactaga aatgccatct tccttgatgt tggaggtacc tgctctggca gatttcaacc
gggcttggac agaacttacc gactggcttt ctctgcttga tcaagttata aaatcacaga
gggtgatggt gggtgacctt gaggatatca acgagatgat catcaagcag aag,
 the whole or a partial sequence of 10 or more consecutive nucleotides of the sequence consisting of 66th to 95th nucleotides from the 5'-terminal:
CTAGAAATGCCATCTTCCTTGATGTTGGAG (SEQ ID NO: 683)
can be the target sequence (e.g., US9,018,368). Preferably, CTAGAAATGCCATCTTCCTTGATGTTGGAG (SEQ ID NO: 683; target sequence of Eteplirsen) can be mentioned.

Alternatively, the whole or a partial sequence of 10 or more consecutive nucleotides of the sequence consisting of 68th to 87th nucleotides from the 5'-terminal of exon 51:
AGAAATGCCATCTTCCTTGA (SEQ ID NO: 684)
can be the target sequence. Preferably, AGAAATGCCATCTTCCTTGA (SEQ ID NO: 684; target sequence of Drisapersen) can be mentioned.

Alternatively, when the target exon is human exon 55 (the nucleotide sequence of exon 55 of the wild-type human dystrophin gene is shown in SEQ ID NO: 685): 
(SEQ ID NO: 685)
ggtgagtgag cgagaggctg ctttggaaga aactcataga ttactgcaac agttccccct
ggacctggaa aagtttcttg cctggcttac agaagctgaa acaactgcca atgtcctaca
ggatgctacc cgtaaggaaa ggctcctaga agactccaag ggagtaaaag agctgatgaa
acaatggcaa,
 a partial sequence of 10 to 35 consecutive nucleotides of the sequence consisting of -2th to 36th nucleotides from the 5'-terminal: AGGGTGAGTGAGCGAGAGGCTGCTTTGGAAGAAACTCA (SEQ ID NO: 686)
can be the target sequence (e.g., WO 2013/100190).

Preferred embodiments of oligonucleotides capable of skipping exon 44, exon 45, exon 46, exon 50, exon 51, exon 53, or exon 55 as the exemplary target exon are described in detail in the following. Those of ordinary skill in the art can similarly design and synthesize, according to suitable target sequences within other target exons, preferred oligonucleotides that target them.

In one embodiment, the oligonucleotide that causes the target exon to be skipped includes a nucleotide sequence complementary to
(a) in exon 53, for example, the whole or a partial sequence of 10 or more consecutive nucleotides of the nucleotide sequence shown in SEQ ID NO: 647, preferably the nucleotide sequence shown in SEQ ID NO: 648 or 649;
(b) in exon 44, (i) for example, a partial sequence of 10 to 35 consecutive nucleotides in the nucleotide sequence shown in SEQ ID NO: 651 or 652, preferably, two non-consecutive and nonoverlapping sequences selected from the nucleotide sequences shown in SEQ ID NOs: 653, 654, 655, SEQ ID NOs: 656, 657, 658, 659 and 660, or (ii) the whole or a partial sequence of 10 or more consecutive nucleotides of the nucleotide sequence shown in SEQ ID NO: 661 or 662;
(c) in exon 45, (i) the whole or a partial sequence of 10 or more consecutive nucleotides of the nucleotide sequence shown in SEQ ID NO: 664, the nucleotide sequence shown in SEQ ID NO: 665 or 666, (ii) the whole or a partial sequence of 10 or more consecutive nucleotides of the nucleotide sequence shown in SEQ ID NO: 667, preferably, the nucleotide sequence shown in SEQ ID NO: 668, 669, or 670, or (iii) the whole or a partial sequence of 10 or more consecutive nucleotides of the nucleotide sequence shown in SEQ ID NO: 671, preferably, the nucleotide sequence shown in SEQ ID NO: 672, (iv) the whole or a partial sequence of 10 or more consecutive nucleotides of the nucleotide sequence shown in SEQ ID NO: 673;
(d) in exon 46, a partial sequence of 10 to 35 consecutive nucleotides of the nucleotide sequence shown in SEQ ID NO: 675, preferably, the nucleotide sequence shown in SEQ ID NO: 676, 677, 678 or 679;
(e) in exon 50, the whole or a partial sequence of 10 or more consecutive nucleotides of the nucleotide sequence shown in SEQ ID NO: 681;
(f) in exon 51, (i) the whole or a partial sequence of 10 or more consecutive nucleotides of the nucleotide sequence shown in SEQ ID NO: 683, preferably, the nucleotide sequence shown in SEQ ID NO: 683, or (ii) the whole or a partial sequence of 10 or more consecutive nucleotides of the nucleotide sequence shown in SEQ ID NO: 684, preferably, the nucleotide sequence shown in SEQ ID NO: 684;
(g) in exon 55, a partial sequence of 10 to 35 consecutive nucleotides of the nucleotide sequence shown in SEQ ID NO: 686 (in the present specification, oligonucleotide chain containing the target sequence (not limited to exon 44, 45, 46, 50, 51, 53, 55) and the complementary nucleotide sequence is also to be referred to as "the antisense strand of the present invention").

In one embodiment, the oligonucleotide that causes the target exon to be skipped includes a nucleotide sequence complementary to
(a) in exon 53, for example, the whole or a partial sequence of 10 or more consecutive nucleotides of the nucleotide sequence shown in SEQ ID NO: 647, preferably the nucleotide sequence shown in SEQ ID NO: 648 or 649;
(b) in exon 44, (i) for example, a partial sequence of 10 to 35 consecutive nucleotides in the nucleotide sequence shown in SEQ ID NO: 651 or 652, preferably, two non-consecutive and nonoverlapping sequences selected from the nucleotide sequences shown in SEQ ID NOs: 653, 654, 655, SEQ ID NOs: 656, 657, 658, 659 and 660, or (ii) the whole or a partial sequence of 10 or more consecutive nucleotides of the nucleotide sequence shown in SEQ ID NO: 661 or 662;
(c) in exon 45, (i) the whole or a partial sequence of 10 or more consecutive nucleotides of the nucleotide sequence shown in SEQ ID NO: 664, the nucleotide sequence shown in SEQ ID NO: 665 or 666, (ii) the whole or a partial sequence of 10 or more consecutive nucleotides of the nucleotide sequence shown in SEQ ID NO: 667, preferably, the nucleotide sequence shown in SEQ ID NO: 668, 669, or 670, or (iii) the whole or a partial sequence of 10 or more consecutive nucleotides of the nucleotide sequence shown in SEQ ID NO: 671, preferably, the nucleotide sequence shown in SEQ ID NO: 672, (iv) the whole or a partial sequence of 10 or more consecutive nucleotides of the nucleotide sequence shown in SEQ ID NO: 673;
(d) in exon 46, a partial sequence of 10 to 35 consecutive nucleotides of the nucleotide sequence shown in SEQ ID NO: 675, preferably, the nucleotide sequence shown in SEQ ID NO: 676, 677, 678 or 679;
(e) in exon 50, the whole or a partial sequence of 10 or more consecutive nucleotides of the nucleotide sequence shown in SEQ ID NO: 681;
(f) in exon 51, (i) the whole or a partial sequence of 10 or more consecutive nucleotides of the nucleotide sequence shown in SEQ ID NO: 683, preferably, the nucleotide sequence shown in SEQ ID NO: 683, or (ii) the whole or a partial sequence of 10 or more consecutive nucleotides of the nucleotide sequence shown in SEQ ID NO: 684, preferably, the nucleotide sequence shown in SEQ ID NO: 684.

In one embodiment, the oligonucleotide that causes the target exon to be skipped includes a nucleotide sequence complementary to
(a) in exon 53, for example, the whole or a partial sequence of 10 or more consecutive nucleotides of the nucleotide sequence shown in SEQ ID NO: 647, preferably the nucleotide sequence shown in SEQ ID NO: 648 or 649;
(b) in exon 44, (i) for example, a partial sequence of 10 to 35 consecutive nucleotides in the nucleotide sequence shown in SEQ ID NO: 651 or 652, preferably, two non-consecutive and nonoverlapping sequences selected from the nucleotide sequences shown in SEQ ID NOs: 653, 654, 655, SEQ ID NOs: 656, 657, 658, 659 and 660, or (ii) the whole or a partial sequence of 10 or more consecutive nucleotides of the nucleotide sequence shown in SEQ ID NO: 661 or 662;
(c) in exon 45, (i) the whole or a partial sequence of 10 or more consecutive nucleotides of the nucleotide sequence shown in SEQ ID NO: 664, the nucleotide sequence shown in SEQ ID NO: 665 or 666, (ii) the whole or a partial sequence of 10 or more consecutive nucleotides of the nucleotide sequence shown in SEQ ID NO: 667, preferably, the nucleotide sequence shown in SEQ ID NO: 668, 669, or 670, or (iii) the whole or a partial sequence of 10 or more consecutive nucleotides of the nucleotide sequence shown in SEQ ID NO: 671, preferably, the nucleotide sequence shown in SEQ ID NO: 672, (iv) the whole or a partial sequence of 10 or more consecutive nucleotides of the nucleotide sequence shown in SEQ ID NO: 673;
(d) in exon 46, a partial sequence of 10 to 35 consecutive nucleotides of the nucleotide sequence shown in SEQ ID NO: 675, preferably, the nucleotide sequence shown in SEQ ID NO: 676, 677, 678 or 679;
(e) in exon 51, (i) the whole or a partial sequence of 10 or more consecutive nucleotides of the nucleotide sequence shown in SEQ ID NO: 683, preferably, the nucleotide sequence shown in SEQ ID NO: 683, or (ii) the whole or a partial sequence of 10 or more consecutive nucleotides of the nucleotide sequence shown in SEQ ID NO: 684, preferably, the nucleotide sequence shown in SEQ ID NO: 684.

In one embodiment, the oligonucleotide that causes the target exon to be skipped includes a nucleotide sequence complementary to
(a) in exon 53, for example, the whole or a partial sequence of 10 or more consecutive nucleotides of the nucleotide sequence shown in SEQ ID NO: 647, preferably the nucleotide sequence shown in SEQ ID NO: 648 or 649;
(b) in exon 44, (i) for example, a partial sequence of 10 to 35 consecutive nucleotides in the nucleotide sequence shown in SEQ ID NO: 651 or 652, preferably, two non-consecutive and nonoverlapping sequences selected from the nucleotide sequences shown in SEQ ID NOs: 653, 654, 655, SEQ ID NOs: 656, 657, 658, 659 and 660, or (ii) the whole or a partial sequence of 10 or more consecutive nucleotides of the nucleotide sequence shown in SEQ ID NO: 661 or 662;
(c) in exon 45, (i) the whole or a partial sequence of 10 or more consecutive nucleotides of the nucleotide sequence shown in SEQ ID NO: 664, the nucleotide sequence shown in SEQ ID NO: 665 or 666, (ii) the whole or a partial sequence of 10 or more consecutive nucleotides of the nucleotide sequence shown in SEQ ID NO: 667, preferably, the nucleotide sequence shown in SEQ ID NO: 668, 669, or 670, or (iii) the whole or a partial sequence of 10 or more consecutive nucleotides of the nucleotide sequence shown in SEQ ID NO: 671, preferably, the nucleotide sequence shown in SEQ ID NO: 672, (iv) the whole or a partial sequence of 10 or more consecutive nucleotides of the nucleotide sequence shown in SEQ ID NO: 673;
(d) in exon 46, a partial sequence of 10 to 35 consecutive nucleotides of the nucleotide sequence shown in SEQ ID NO: 675, preferably, the nucleotide sequence shown in SEQ ID NO: 676, 677, 678 or 679.

In one embodiment, the oligonucleotide that causes the target exon to be skipped includes a nucleotide sequence complementary to
(a) in exon 53, for example, the whole or a partial sequence of 10 or more consecutive nucleotides of the nucleotide sequence shown in SEQ ID NO: 647, preferably the nucleotide sequence shown in SEQ ID NO: 648 or 649;
(b) in exon 44, (i) for example, a partial sequence of 10 to 35 consecutive nucleotides in the nucleotide sequence shown in SEQ ID NO: 651 or 652, preferably, two non-consecutive and nonoverlapping sequences selected from the nucleotide sequences shown in SEQ ID NOs: 653, 654, 655, SEQ ID NOs: 656, 657, 658, 659 and 660, or (ii) the whole or a partial sequence of 10 or more consecutive nucleotides of the nucleotide sequence shown in SEQ ID NO: 661 or 662;
(c) in exon 45, (i) the whole or a partial sequence of 10 or more consecutive nucleotides of the nucleotide sequence shown in SEQ ID NO: 664, the nucleotide sequence shown in SEQ ID NO: 665 or 666, (ii) the whole or a partial sequence of 10 or more consecutive nucleotides of the nucleotide sequence shown in SEQ ID NO: 667, preferably, the nucleotide sequence shown in SEQ ID NO: 668, 669, or 670, or (iii) the whole or a partial sequence of 10 or more consecutive nucleotides of the nucleotide sequence shown in SEQ ID NO: 671, preferably, the nucleotide sequence shown in SEQ ID NO: 672, (iv) the whole or a partial sequence of 10 or more consecutive nucleotides of the nucleotide sequence shown in SEQ ID NO: 673.

In one embodiment, the oligonucleotide that causes the target exon to be skipped includes a nucleotide sequence complementary to
(a) in exon 53, for example, the whole or a partial sequence of 10 or more consecutive nucleotides of the nucleotide sequence shown in SEQ ID NO: 647, preferably the nucleotide sequence shown in SEQ ID NO: 648 or 649;
(b) in exon 44, (i) for example, a partial sequence of 10 to 35 consecutive nucleotides in the nucleotide sequence shown in SEQ ID NO: 651 or 652, preferably, two non-consecutive and nonoverlapping sequences selected from the nucleotide sequences shown in SEQ ID NOs: 653, 654, 655, SEQ ID NOs: 656, 657, 658, 659 and 660, or (ii) the whole or a partial sequence of 10 or more consecutive nucleotides of the nucleotide sequence shown in SEQ ID NO: 661 or 662.

In one embodiment, the oligonucleotide that causes the target exon to be skipped includes a nucleotide sequence complementary to
(a) in exon 53, for example, the whole or a partial sequence of 10 or more consecutive nucleotides of the nucleotide sequence shown in SEQ ID NO: 647, preferably the nucleotide sequence shown in SEQ ID NO: 648 or 649;
(b) in exon 45, (i) the whole or a partial sequence of 10 or more consecutive nucleotides of the nucleotide sequence shown in SEQ ID NO: 664, the nucleotide sequence shown in SEQ ID NO: 665 or 666, (ii) the whole or a partial sequence of 10 or more consecutive nucleotides of the nucleotide sequence shown in SEQ ID NO: 667, preferably, the nucleotide sequence shown in SEQ ID NO: 668, 669, or 670, or (iii) the whole or a partial sequence of 10 or more consecutive nucleotides of the nucleotide sequence shown in SEQ ID NO: 671, preferably, the nucleotide sequence shown in SEQ ID NO: 672, (iv) the whole or a partial sequence of 10 or more consecutive nucleotides of the nucleotide sequence shown in SEQ ID NO: 673.

In one embodiment, the oligonucleotide that causes the target exon to be skipped includes a nucleotide sequence complementary to
(a) in exon 44, (i) for example, a partial sequence of 10 to 35 consecutive nucleotides in the nucleotide sequence shown in SEQ ID NO: 651 or 652, preferably, two non-consecutive and nonoverlapping sequences selected from the nucleotide sequences shown in SEQ ID NOs: 653, 654, 655, SEQ ID NOs: 656, 657, 658, 659 and 660, or (ii) the whole or a partial sequence of 10 or more consecutive nucleotides of the nucleotide sequence shown in SEQ ID NO: 661 or 662;
(b) in exon 45, (i) the whole or a partial sequence of 10 or more consecutive nucleotides of the nucleotide sequence shown in SEQ ID NO: 664, the nucleotide sequence shown in SEQ ID NO: 665 or 666, (ii) the whole or a partial sequence of 10 or more consecutive nucleotides of the nucleotide sequence shown in SEQ ID NO: 667, preferably, the nucleotide sequence shown in SEQ ID NO: 668, 669, or 670, or (iii) the whole or a partial sequence of 10 or more consecutive nucleotides of the nucleotide sequence shown in SEQ ID NO: 671, preferably, the nucleotide sequence shown in SEQ ID NO: 672, (iv) the whole or a partial sequence of 10 or more consecutive nucleotides of the nucleotide sequence shown in SEQ ID NO: 673.

The compound containing an oligonucleotide constituting the conjugate of the present invention include not only single-stranded oligonucleotides consisting of the antisense strand, but also a double-stranded oligonucleotide formed by an oligonucleotide chain containing a nucleotide sequence complementary to all or part of the antisense strand sequence (in the present specification, an oligonucleotide chain containing a nucleotide sequence complementary to an antisense strand sequence is also referred to as "the sense strand of the present invention") and the aforementioned single-stranded oligonucleotide.

As the length of the whole antisense strand, for example 10 to 35 nucleotides long, preferably 12 to 35 nucleotides long, more preferably 15 to 30 nucleotides long, further preferably 20 to 30 nucleotides long, and particularly preferably 20 to 25 nucleotides long can be mentioned. In another embodiment, as the length of the whole antisense strand containing a nucleotide sequence complementary to the target sequence, 15 to 35 nucleotides long, more preferably 20 to 35 nucleotides long, and further preferably 25 to 35 nucleotides long can be mentioned. In another embodiment, as the length of the whole antisense strand containing a nucleotide sequence complementary to the target sequence, 12 to 20 nucleotides long, and more preferably 15 to 20 nucleotides long can be mentioned. The length of the whole antisense strand may be, for example, 9 to 50 nucleotides long, 10 to 40 nucleotides long, 11 to 35 nucleotides long, 12 to 35 nucleotides long, or 12 to 30 nucleotides long, 14 to 30 nucleotides long, 16 to 30 nucleotides long, or 18 to 25 nucleotides long, or 4 to 25 nucleotides long, 8 to 20 nucleotides long, 7 to 20 nucleotides long, 8 to 16 nucleotides long, 8 to 12 nucleotides long, 8 to 10 nucleotides long. In one embodiment, the length of the whole antisense strand is 9 to 35 nucleotides long, 10 to 35 nucleotides long, 11 to 35 nucleotides long, 12 to 35 nucleotides long, 12 to 30 nucleotides long, 12 to 25 nucleotides long or 12 to 22 nucleotides long. In one embodiment, the length of the whole antisense strand is 13 to 35 nucleotides long, 16 to 30 nucleotides long, 18 to 30 nucleotides long, 20 to 30 nucleotides long or 20 to 25 nucleotides long.

The length of the "continuous nucleotides" is not particularly limited, and it may be at least 8 nucleotides long, at least 10 nucleotides long, at least 11 nucleotides long, at least 12 nucleotides long, at least 13 nucleotides long, at least 14 nucleotides long, at least 15 nucleotides long, at least 16 nucleotides long, at least 17 nucleotides long, at least 18 nucleotides long, at least 19 nucleotides long, or at least 20 nucleotides long. The length of the "continuous nucleotides" may be not more than 50 nucleotides long, not more than 45 nucleotides long, not more than 40 nucleotides long, not more than 35 nucleotides long, not more than 30 nucleotides long, not more than 28 nucleotides long, not more than 26 nucleotides long, not more than 24 nucleotides long, not more than 22 nucleotides long, or not more than 20 nucleotides long. The length of the "continuous nucleotides" may be for example 9 to 50 nucleotides long, 10 to 40 nucleotides long, 11 to 35 nucleotides long, 12 to 35 nucleotides long, or 12 to 30 nucleotides long, 14 to 30 nucleotides long, 16 to 30 nucleotides long, or 18 to 25 nucleotides long, or 4 to 25 nucleotides long, 8 to 20 nucleotides long, 7 to 20 nucleotides long, 8 to 16 nucleotides long, 8 to 12 nucleotides long, or 8 to 10 nucleotides long. In one embodiment, the length of the "continuous nucleotides" is 9 to 35 nucleotides long, 10 to 35 nucleotides long, 11 to 35 nucleotides long, 12 to 35 nucleotides long, 12 to 30 nucleotides long, 12 to 25 nucleotides long, or 12 to 22 nucleotides long. In one embodiment, the length of the "continuous nucleotides" is 13 to 35 nucleotides long, 16 to 30 nucleotides long, 18 to 30 nucleotides long, 20 to 30 nucleotides long, or 20 to 25 nucleotides long. In another embodiment, the length of the "continuous nucleotides" is 10 to 35 nucleotides long, preferably 12 to 30 nucleotides long, more preferably 15 to 25 nucleotides long, or further preferably 20 to 25 nucleotides long, but it is not limited thereto. In another embodiment, the length of the "continuous nucleotides" is 15 to 35 nucleotides long, more preferably 20 to 35 nucleotides long, further preferably 20 to 30 nucleotides long. In still another embodiment, the length of the "continuous nucleotides" is 12 to 20 nucleotides long, more preferably 15 to 20 nucleotides long.

In one embodiment, when the antisense strand of the present invention contains a nucleotide (sequence) other than the nucleotide sequence complementary to the target sequence, the nucleotide (sequence) may be complementary to exon 7, exon 8, exon 9, exon 19, exon 23, exon 44, exon 45, exon 46, exon 50, exon 51, exon 52, exon 53, or exon 55 nucleotide (sequence) outside the aforementioned target sequence. The target exon may be more preferably exon 44, exon 45, exon 46, exon 50, exon 51, exon 53, or exon 55, further preferably, exon 44, exon 45, or exon 53, particularly preferably, exon 53. In another embodiment, it may be exon 44. In another embodiment, it may be exon 45. In another embodiment, it may be exon 46.

When the compound containing an oligonucleotide of the present invention contains, as antisense strand, two partial nucleotide sequences targeting two non-continuous, nonoverlapping nucleotide sequences in the same exon (i.e., respectively complementary to the nucleotide sequences), the length of each partial nucleotide sequence may be less than 10 nucleotides long, for example 7, 8, or 9 nucleotides long, as long as the total length of the sequences complementary to the two target sequences is not less than 15 nucleotides long. The upper limit of the length of each partial nucleotide sequence is not particularly limited, but may be, for example, 20 nucleotides or less, preferably 15 nucleotides or less.

The complementarity of the "complementary nucleotide sequence" in the antisense strand of the present invention to the target sequence (identity to a sequence that is completely complementary to the target sequence) is not particularly limited as long as it is not less than 80%, and is preferably not less than 90%, more preferably not less than 95% (e.g., 95%, 96%, 97% or above), particularly preferably 100%. The "identity of the nucleotide sequence" in the present invention can be calculated using homology calculation algorithm NCBI BLAST (National Center for Biotechnology Information Basic Local Alignment Search Tool), and under the following conditions (expectancy=10; accept gap; filtering=ON; matching score=1; mismatch score=-3). Furthermore, the complementarity of individual bases is not limited to forming Watson-Crick type base pairs with the target base, but also includes forming Hoogsteen type base pairs and Wobble base pairs.

The complementarity of the "complementary nucleotide sequence" in the sense strand of the present invention to an antisense strand sequence is not particularly limited as long as it is 80% or more, preferably 90% or more, more preferably 95% or more (e.g., 95%, 96%, 97% or more), particularly preferably 100%, to the whole or part of the antisense strand sequence.

Alternatively, the aforementioned "complementary nucleotide sequence" is not only a sequence that is completely complementary (i.e., hybridizes without mismatch) to the target sequence or antisense strand sequence, but also a sequence containing mismatch of one to several (e.g., 1, 2, 3, 4, 5, 6) nucleotides, preferably 1 to 3 nucleotides, more preferably 1 or 2 nucleotides, as long as it can induce exon skipping under physiological conditions in mammalian cells.

Alternatively, the aforementioned "complementary nucleotide sequence" is a nucleotide sequence that hybridizes to the target sequence or antisense strand sequence under stringent conditions. As used herein, the "stringent conditions" refers to, for example, the conditions described in Current Protocols in Molecular Biology, John Wiley & Sons,6.3.1-6.3.6, 1999, for example, hybridization in 6×SSC (sodium chloride/sodium citrate)/45°C, followed by washing in 0.2×SSC/0.1% SDS/50 to 65°C once or more, and the like can be mentioned. Those skilled in the art can appropriately select hybridization conditions that afford stringency equivalent thereto.

In a preferred embodiment, the compound containing an oligonucleotide of the present invention includes any of the following nucleotide sequences as a sequence complementary to the target sequence in exon 53 of the human dystrophin gene (sequence in antisense strand).
GTTGCCTCCG GTTCTGAAGG TGTTC (SEQ ID NO: 687: sequence completely complementary to the 36th to 60th nucleotide sequence of exon 53)
CCTCCG GTTCTGAAGG TGTTC (SEQ ID NO: 688: sequence completely complementary to the 36th to 56th nucleotide sequence of exon 53)

In another embodiment, the compound containing an oligonucleotide of the present invention includes any of the following nucleotide sequences as a sequence complementary to the target sequence in exon 44 of the human dystrophin gene (sequence in antisense strand) (e.g., WO 2015/194520).
GTGTCTTTCTGAGCCGCCATTTCTCA (SEQ ID NO: 689)
TCTGTTAGCCACGCCGCCATTT (SEQ ID NO: 690)
CTGTTAGCCACTCGCCGCCATTTC (SEQ ID NO: 691)
TTGTGTCTTTCTTCTGTTAGCCAC (SEQ ID NO: 692)

In another embodiment, the compound containing an oligonucleotide of the present invention includes any of the following nucleotide sequences as a sequence complementary to the target sequence in exon 45 of the human dystrophin gene (sequence in antisense strand) (e.g., US 8,324,371).
CAGTTTGCCGCTGCCCAATGCCATCCTGGA (SEQ ID NO: 693)
TTGCCGCTGCCCAATGCCATCCTGGAGTTC (SEQ ID NO: 694)

In another embodiment, the compound containing an oligonucleotide of the present invention includes any of the following nucleotide sequences as a sequence complementary to the target sequence in exon 45 of the human dystrophin gene (sequence in antisense strand) (e.g., US 7,902,160).
GCCGCTGCCCAATGC (SEQ ID NO: 695)
CGCTGCCCAATGCCATCC (SEQ ID NO: 696; sequence of Renadirsen)
CAGTTTGCCGCTGCCCAA (SEQ ID NO: 697)

In another embodiment, the compound containing an oligonucleotide of the present invention includes any of the following nucleotide sequences as a sequence complementary to the target sequence in exon 45 of the human dystrophin gene (sequence in antisense strand) (e.g., US 8,524,880).
CCAATGCCATCCTGGAGTTCCTGTAA (SEQ ID NO: 698)
CAATGCCATCCTGGAGTTCCTG (SEQ ID NO: 699; sequence of Casimersen)

In another embodiment, the compound containing an oligonucleotide of the present invention includes any of the following nucleotide sequences as a sequence complementary to the target sequence in exon 46 of the human dystrophin gene (sequence in antisense strand) (e.g., US 8,552,172).
TGCTGCTCTTTTCCAGGTTCAAGTGGGATA (SEQ ID NO: 700)
CTTTTAGTTGCTGCTCTTTTCCAGGTTCAA (SEQ ID NO: 701)
CTTTTCTTTTAGTTGCTGCTCTTTTCCAGG (SEQ ID NO: 702)
TTAGTTGCTGCTCTTTTCCAGGTTCAAGTG (SEQ ID NO: 703)

Examples of the sequence complementary to the target sequence in exon 50 of the human dystrophin gene (sequence in antisense strand) are disclosed in WO 2013/100190.

In another embodiment, the compound containing an oligonucleotide of the present invention includes the following nucleotide sequence as a sequence complementary to the target sequence in exon 51 of the human dystrophin gene (sequence in antisense strand) (e.g., US9,018,368). CTCCAACATCAAGGAAGATGGCATTTCTAG (SEQ ID NO: 704; sequence of Eteplirsen)

Alternatively, the compound containing an oligonucleotide of the present invention includes the following nucleotide sequence as a sequence complementary to the target sequence in exon 51 of the human dystrophin gene (sequence in antisense strand).
TCAAGGAAGATGGCATTTCT (SEQ ID NO: 705; sequence of Drisapersen)

Examples of the sequence complementary to the target sequence in exon 55 of the human dystrophin gene (sequence in antisense strand) are disclosed in WO 2013/100190.

In another embodiment, the compound containing an oligonucleotide of the present invention includes the following nucleotide sequence as a sequence complementary to the target sequence in exon 23 of the mouse dystrophin gene (sequence in antisense strand).
GGCCAAACCTCGGCTTACCTGAAAT (SEQ ID NO: 706)

In sequences complementary to the target sequences in each of the above-mentioned exons, base thymine (T) may be uracil (U). In addition, base cytosine (C) may be 5-methylcytosine.

Examples of the constitutional unit of the antisense strand and sense strand of the present invention include ribonucleotides and deoxyribonucleotides. These nucleotides may be modified (modified nucleotide residues are sometimes referred to as "modified nucleotide residue"), or non-modified (non-modified nucleotide residue is sometimes referred to as "non-modified nucleotide residue"). Alternatively, the antisense strand and sense strand of the present invention may be composed of those having the below-mentioned non-ribose phosphate skeleton. In the present specification, substitution to a chain having such a non-ribose phosphate skeleton is also included in the nucleotide modification.

The aforementioned nucleotide residues include sugar, base, and phosphoric acid as constituent elements. Ribonucleotide has ribose residue as sugar and adenine (A), guanine (G), cytosine (C), 5-methylcytosine (mC), and uracil

(U) (optionally replaced by thymine (T)) as bases. Deoxyribonucleotide residue has deoxyribose residue as sugar and adenine (dA), guanine (dG), cytosine (dC), 5-methylcytosine (dmC), and thymine (dT) (optionally replaced by uracil (dU)) as bases. In the following, nucleotides having adenine, guanine, (5-methyl)cytosine, uracil, thymine may be referred to as adenine nucleotide, guanine nucleotide, (5-methyl)cytosine nucleotide, uracil nucleotide, and thymine nucleotide, respectively.

In the aforementioned non-modified nucleotide residues, each of the aforementioned constituent elements is, for example, the same or substantially the same as that occurring naturally, preferably the same or substantially the same as that naturally occurring in the human body.

In the aforementioned modified nucleotide residues, for example, any constituent element of the aforementioned non-modified nucleotide residues may be modified. In the present invention, examples of the "modification" include substitution, addition, and/or deletion of the aforementioned constituent elements, and substitution, addition, and/or deletion of an atom and/or a functional group in the aforementioned constituent elements. Examples of the aforementioned modified nucleotide residue include naturally occurring nucleotide residue, artificially modified nucleotide residue, and the like. For the aforementioned naturally-derived modified nucleotide residue, for example, Limbach et al. (Limbach et al., 1994, Summary:the modified nucleosides of RNA, Nucleic Acids Res.22:2183-2196) can be referred to. In addition, examples of the aforementioned modified nucleotide residue include residues of the alternatives of the aforementioned nucleotides.

The "modified sugar" refers to a sugar that has substitutions and/or any changes from the natural sugar moiety (i.e., the sugar moiety found in DNA(2'-H) or RNA(2'-OH)), and the "sugar modification" refers to substitutions and/or any changes from the naturally occurring sugar moiety. The nucleic acid strand optionally includes one or more modified nucleosides, including modified sugars. The "sugur modified nucleoside" refers to a nucleoside that has a modified sugar moiety. Such sugur modified nucleoside may enhance nuclease stability, increase binding affinity, or confer some other beneficial biological property to the nucleic acid strand. In a specific embodiment, the nucleoside includes a chemically-modified ribofuranose ring moiety. Examples of the chemically-modified ribofuranose ring include, but are not limited to, those subjected to addition of substituent (including 5' and 2' substituents), formation of bicyclic nucleic acids (bridged nucleic acids, BNA) by crosslinking formation of nongeminal cyclic atoms, substitution of ribosyl ring oxygen atom with S, N(R), or C(R1)(R2) (R, R1 and R2 are each independently H, C1-C12 alkyl, or protecting group), and combinations thereof.

The "bicyclic nucleoside" refers to a modified nucleoside that includes a bicyclic sugar moiety. Nucleic acids including a bicyclic sugar moiety are generally referred to as bridged nucleic acids (BNA). A nucleoside containing a bicyclic sugar moiety is sometimes referred to as a "bridged nucleoside" or a "BNA nucleoside". Examples of the "bridged nucleoside" or the "BNA nucleoside" include nucleic acids containing methyleneoxy(4'-CH₂-O-2')BNA nucleoside (LNA nucleoside, also known as 2',4'-BNA nucleoside) (e.g., α-L-methyleneoxy(4'-CH₂-O-2')BNA nucleoside, β-D-methyleneoxy(4'-CH₂-O-2')BNA nucleoside), ethyleneoxy(4'-(CH₂)₂-O-2')BNA nucleoside (also known as ENA nucleoside), β-D-thio(4'-CH₂-S-2')BNA nucleoside, aminooxy(4'-CH₂-O-N(R₃)-2')BNA nucleoside, oxyamino(4'-CH₂-N(R₃)-O-2')BNA nucleoside (also known as 2',4'-BNA^{NC} nucleoside; 2',4'-BNA^{NC}[N-H]nucleoside, 2',4'-BNA^{NC}[N-MeJnucleoside), 2',4'-BNA^{COC} nucleoside, 3'-amino-2',4'-BNA nucleoside, 5'-methylBNA nucleoside, (4'-CH(CH₃)-O-2')BNA nucleoside (also known as cEt nucleoside), (4'-CH(CH₂OCH₃)-O-2')BNA nucleoside (also known as cMOE nucleoside), amide BNA nucleoside, (4'-C(O)-N(R)-2')BNA nucleoside (R=H, Me) (also known as AmNA nucleoside; when R=H, it is AmNA[N-H] nucleoside, when R=Me, it is AmNA[N-Me] nucleoside)), guanidine BNA nucleoside (also known as GuNA nucleoside (e.g., when R=H, it is GuNA[N-H] nucleoside, when R=Me, it is GuNA[N-Me] nucleoside, when R=t-Bu, it is GuNA[t-Bu] nucleoside)), amine BNA nucleoside (also known as 2'-Amino-LNA nucleoside) (e.g., 3-(Bis(3-aminopropyl)amino)propanoyl substitutes nucleoside), 2'-O,4'-C-spirocyclopropylene bridged nucleoside (also known as scpBNA nucleoside), S-cEt nucleoside, or triycyclo-DNA nucleoside, and the like. The base moiety of the "bridged nucleoside" or "BNA nucleoside" may be a modified base. It is preferably 5-methylcytosine.

In the present specification, the "cationic nucleoside" is a modified nucleoside that exists as a cationic form as compared to a neutral form (e.g., neutral form of ribonucleoside, etc.) at a certain pH (e.g., human physiological pH (about 7.4), the pH of the delivery site (e.g., organelle, cell, tissue, organ, organism, etc.)). The cationic nucleoside may include one or more cationic modifying groups at any position on the nucleoside. In one embodiment, the cationic nucleoside is 2'-Amino-LNA nucleoside (e.g., 3-(Bis(3-aminopropyl)amino)propanoyl nucleoside), aminoalkyl modified nucleoside (e.g., 2'-O-methylnucleoside and 4'-CH2CH2CH2NH2 nucleoside), GuNA nucleoside (e.g., GuNA[N-H] nucleoside, GuNA[N-Me] nucleoside, when R=t-Bu, it is GuNA[t-Bu] nucleoside), or the like.

Bicyclic nucleosides having a methyleneoxy (4'-CH2-O-2') bridge are sometimes referred to as LNA nucleosides.

Examples of the sugur modified nucleoside, but are not limited to, 5'-vinyl, 5'-methyl (R or S), 5'-allyl (R or S), 4'-S, 2'-F (2'-fluoro group), 2'-OCH3 (2'-OMe group or 2'-O-methyl group), 2'-O(CH2)2OCH3 - nucleoside, bicyclic nucleoside, cationic nucleoside, and peptide nucleic acid nucleoside. Substituents at the 2'-position can also be selected from allyl, amino, azide, thio, -O-allyl, -O-C1-C10 alkyl, -OCF3, - O(CH2)2SCH3, -O(CH2)2-O-N(Rm)(Rn), and O-CH2-C(=O)-N(Rm) (Rn), and Rm and Rn are each independently H or substituted or unsubstituted C1-C10 alkyl. The "2'-modified sugar" means a furanosyl sugar modified at the 2'-position. A nucleoside containing 2'-modified sugar is sometimes referred to as "2'-sugur modified nucleoside". The base moiety of the sugar-modified nucleoside may be a modified base. It is preferably 5-methylcytosine.

The preparation methods of modified sugars are well known to those of ordinary skill in the art. In nucleotides with modified sugar moieties, the nucleobase moiety (natural, modified, or combination thereof) may be maintained for hybridization with an appropriate nucleic acid target.

Generally, modifications can be performed such that nucleotides in the same strand can independently undergo different modifications. Due to the resistance to enzymatical cleavage, the same nucleotides may also have modified internucleoside bonds (e.g., phosphorothioate bonds) and further have modified sugars (e.g., 2'-O-methyl modified sugar or bicyclic sugar). The same nucleotide also has a modified nucleobase (e.g., 5-methylcytosine) and can further have a modified sugar (e.g., 2'-O-methylmodified sugar or bicyclic sugar).

The number, type, and position of non-natural nucleotides in a nucleic acid strand can affect the antisense effect and the like provided by the nucleic acid complex. The choice of modification may vary depending on the sequence of the target gene, and the like, but those of ordinary skill in the art can determine preferred embodiments by referring to the descriptions in the documents (e.g., WO 2007/143315, WO 2008/043753, and WO 2008/049085) related to antisense methods. Furthermore, when the antisense effect of the modified nucleic acid complex is measured and the measured value thus obtained is not significantly lower than that of the nucleic acid complex before modification (for example, when the measured value obtained after modification is 70% or more, 80% or more, or 90% or more, of the measured value of the nucleic acid complex before modification), then the associated modification can be evaluated.

Examples of the aforementioned modification of the nucleotide residue include modification of sugar-phosphate backbone (the backbone also contains a base) (hereinafter sugar phosphate backbone).

In the aforementioned sugar phosphate backbone, when the sugar is ribose, for example, the ribose residue can be modified. The aforementioned ribose residue can modify, for example, the 2'-position carbon (hereinafter also referred to as 2'-substitution modification). Specifically, for example, a hydroxyl group bonded to the 2'-position carbon can be modified with a methyl group, or the hydroxyl group can be substituted with hydrogen or halogen such as fluoro. Furthermore, by replacing the hydroxyl group at the 2'-position carbon with hydrogen, the ribose residue can be replaced with deoxyribose. The aforementioned ribose residue can be substituted, for example, with a stereoisomer and may be substituted, for example, with an arabinose residue. Hereinafter, a nucleic acid in which the hydroxyl group bonded to the 2'-position carbon of the above-mentioned sugar is modified with a methoxy group may be referred to as a 2'-OMe modified nucleic acid. Furthermore, as a substituent at the 2'-position, -O(CH₂)₂CH₃ can be preferably mentioned. Nucleic acids in which the hydroxyl group bonded to the 2'-position carbon is substituted with -O(CH₂)₂CH₃ are sometimes referred to as 2'-MOE-modified nucleic acids. Other substituents at the 2'-position include allyl, amino, azide, thio, -O-allyl, -O-C₁-C₁₀ alkyl, -OCF₃, -O(CH₂)₂SCH₃, -O(CH₂)₂-ON(Rm)(Rn), -O-CH₂-C(=O)-N(Rm)(Rn), -O(CH₂)₂-C(=O)-N(Rm)(Rn), and the like. Rm and Rn are each independently H or substituted or unsubstituted C₁-C₁₀ alkyl. Preferred examples of -O(CH₂)₂-C(=O)-N(Rm) (Rn) include -O(CH₂)₂-C(=O)-NHCH₃, which is sometimes referred to as 2'-MCE-modified nucleic acid. 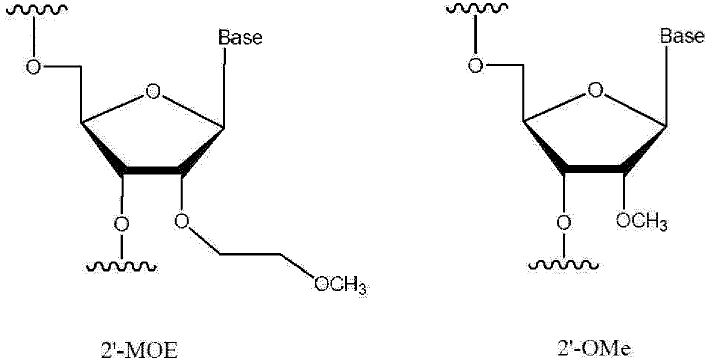 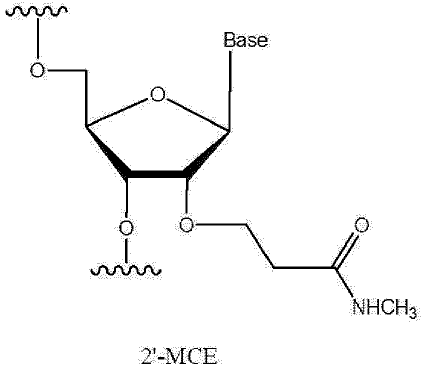 (In each formula, Base is a base.)

The aforementioned sugar phosphate backbone may be substituted, for example, with a non-ribose residue (including a non-deoxyribose residue) and/or a non-ribose phosphate backbone having non-phosphoric acid. Such substitution is also included in the modification of the sugar phosphate backbone. Examples of the aforementioned non-ribose phosphate backbone include uncharged bodies of the aforementioned sugar phosphate backbone. Examples of the alternative wherein the aforementioned nucleotide is substituted with the aforementioned non-ribose phosphate backbone include morpholino alternative, cyclobutyl alternative, pyrrolidine alternative, and the like, and it is preferably a morpholino alternative.

The morpholino alternative is a non-ribose phosphate backbone in which a group represented by the following formula is the constitutional unit. 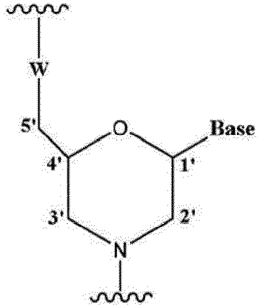 wherein Base is as defined above; and
W is a group represented by any of the following formulas. 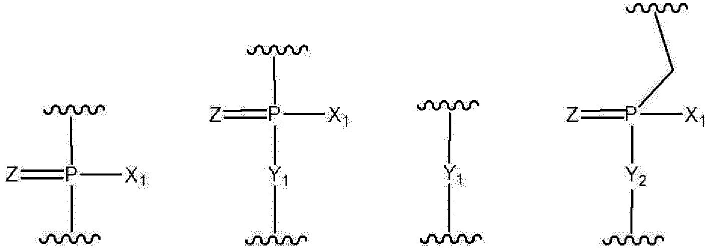 wherein X₁ is -CH₂R¹, -O-CH₂R¹, -S-CH₂R¹, -NR²R³ or F;
R¹ is H, an alkyl (e.g., C₁-C₁₀ alkyl);
R² and R³ are the same or different, and each is H, alkyl (e.g., C₁-C₁₀ alkyl), cycloalkyl (e.g., C₃-C₁₀ cycloalkyl), or aryl (e.g., C₆-C₁₄ aryl);
Y₁ is O, S, CH₂, or NR¹;
Y₂ is O, S, or NR¹;
Z is O or S.)

A morpholino alternative is preferably phosphorodiamidatemorpholino in which a group represented by the following formula is the constitutional unit: 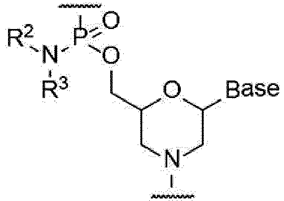
wherein Base, R², and R³ are as defined above,
further preferably, phosphorodimethylamidate morpholino in which a group represented by the following formula is the constitutional unit: 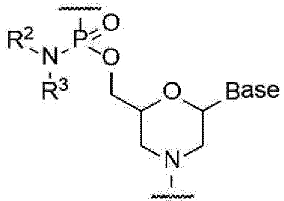
wherein R² and R³ are each alkyl (e.g., C₁-C₁₀ alkyl), and Base is as defined above. The phosphorodimethylamidate morpholino is sometimes referred to as morpholinonucleic acid.

The morpholino alternative can be produced according to, for example, WO 1991/009033, or WO 2009/064471. Particularly, phosphorodiamidatemorpholino can be produced according to the method described in WO 2009/064471.

Other examples of the aforementioned alternative include artificial nucleic acids. Specific examples include PNA (peptide nucleic acid), a nucleic acid containing a bicyclic sugar moiety (also referred to as Bridged Nucleic Acid (BNA)), tricyclo-DNA, and the like. Bicyclic sugars may be sugars in which the 2'-position carbon atom and the 4'-position carbon atom are bridged by two or more atoms. Examples of the bicyclic sugars are known to those skilled in the art. One subgroup of nucleic acid (BNA) containing a bicyclic sugar has 2'-position carbon atom and 4'-position carbon atom bridged by 4'-(CH₂)ₚ-0-2', 4-' (CH₂)ₚ-CH₂-2', 4' - (CH₂)ₚ-S-2¹ , 4' -(CH₂)ₚ-OCH₂0-2', 4'-(CH₂)ₙ-N(R₃)-0-(CH₂)ₘ-2' wherein p, m, and n are each an integer of 1 to 4, an integer of 0 to 2, or an integer of 1 to 3; R₃ is a hydrogen atom, an alkyl group, an alkenyl group, a cycloalkyl group, an aryl group, an aralkyl group, an acyl group, a sulfonyl group, and a unit substituent (fluorescence or chemiluminescence labeling molecule, functional group having nucleic acid cleavage activity, intracellular or intranuclear localization signal peptide, etc.)]. Furthermore, with respect to BNA in a specific embodiment, in the OR₂ substituent on the 3'-position carbon atom and the OR₁ substituent on the 5'-position carbon atom, R₁ and R₂ are typically hydrogen atoms which may be the same or different, further may be hydroxyl-protecting group for nucleic acid synthesis, alkyl group, alkenyl group, cycloalkyl group, aryl group, aralkyl group, acyl group, sulfonyl group, silyl group, phosphate group, phosphate group protected by a protecting group for nucleic acid synthesis, or P(R₄)R₅ [wherein R₄ and R₅ may be the same or different, and each is hydroxyl group, hydroxyl group protected by a protecting group for nucleic acid synthesis, mercapto group, mercapto group protected by a protecting group for nucleic acid synthesis, amino group, alkoxy group having 1 to 5 carbon atoms, alkylthio group having 1 to 5 carbon atoms, cyanoalkoxy group having 1 to 6 carbon atoms, or an amino group substituted by alkyl group having 1 to 5 carbon atoms]. Examples of the BNA include nucleic acids containing methyleneoxy(4'-CH₂-O-2')BNA (also known as LNA, 2',4'-BNA) (e.g., α-L-methyleneoxy(4'-CH₂-O-2')BNA, β-D-methyleneoxy(4'-CH₂-O-2')BNA), ethyleneoxy(4'-(CH₂)₂-O-2')BNA (also known as ENA), β-D-thio(4'-CH₂-S-2')BNA, aminooxy(4'-CH₂-O-N(R₃)-2')BNA, oxyamino (4'-CH₂-N(R₃)-O-2') BNA (also known as 2',4'-BNA^{NC}; when R=H, it is 2' , 4'-BNA^{NC}[N-H], when R=Me, it is 2',4'-BNA^{NC}[N-Me]), 2',4'-BNA^{coc}, 3'-amino-2',4'-BNA, 5'-methyl BNA, (4'-CH(CH₃)-O-2')BNA (also known as cEt), (4'-CH(CH₂OCH₃)-O-2')BNA (also known as cMOE), amide BNA, (4'-C(O)-N(R)-2')BNA (R=H, Me) (also known as AmNA; when R=H, it is AmNA[N-H], when R=Me, it is AmNA[N-Me])), guanidine BNA (also known as GuNA (e.g., when R=H, it is GuNA[N-H], when R=Me, GuNA[N-Me], when R=t-Bu, it is GuNA[t-Bu])), amine BNA (also known as 2'-Amino-LNA) (e.g., 3-(Bis(3-aminopropyl)amino)propanoyl substitute LNA), 2'-O,4'-C-spirocyclopropylene bridged BNA (also known as scpBN), or S-cEt, tricyclo-DNA, and the like. Specific structures (sugar moiety) of BNA that can be used in the present invention are shown below.

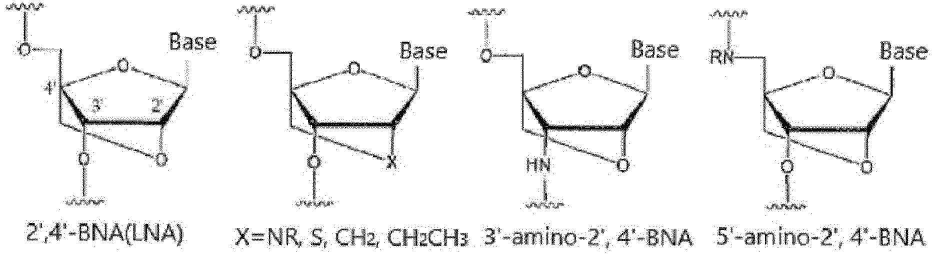

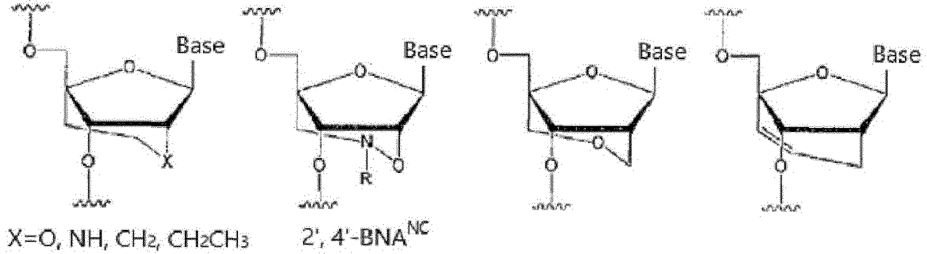

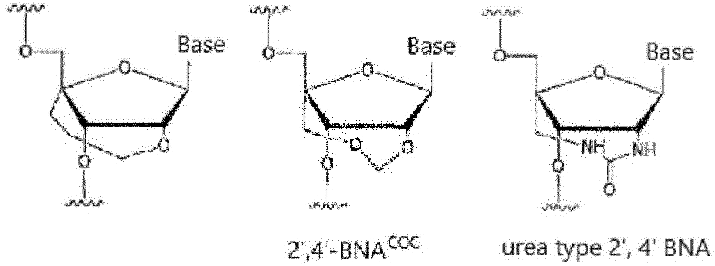

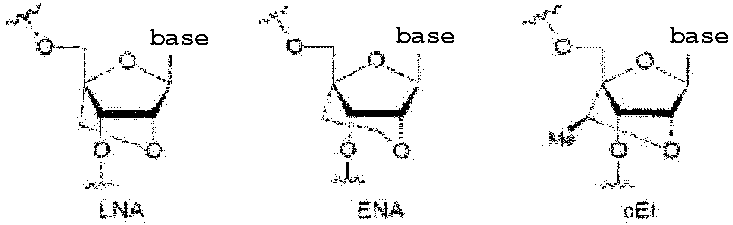

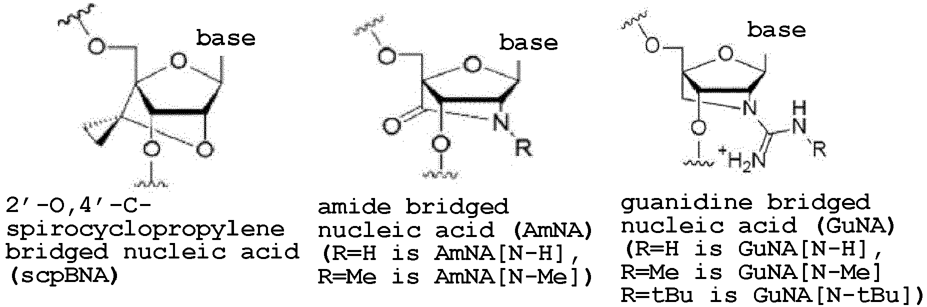

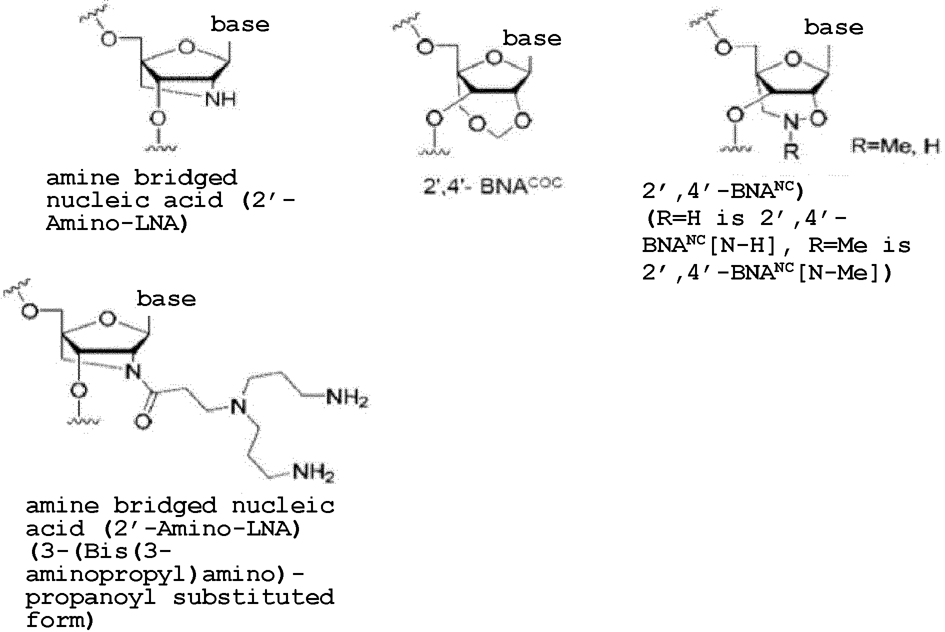

In the formula (I), R is a hydrogen atom, an alkyl group having 1 to 7 carbon atoms that optionally forms a branch or ring, an alkenyl group having 2 to 7 carbon atoms that optionally forms a branch or ring, an aryl group having 3 to 12 carbon atoms that may contain a hetero atom, an aralkyl group having an aryl moiety having 3 to 12 carbon atoms that may contain a hetero atom, or an amino-protecting group for nucleic acid synthesis. R is preferably a hydrogen atom, a methyl group, an ethyl group, an n-propyl group, an isopropyl group, a phenyl group, or a benzyl group, and R is more preferably a hydrogen atom or a methyl group. Furthermore, in each of the above-mentioned formulas, B or Base is a base.

These artificial nucleic acids can be synthesized, for example, by reference to JP-A-2002-241393, JP-A-2000-297097, WO 2011/052436, WO 2014/046212, WO 2015/125783, and the like.

Tricyclo-DNA is an artificial nucleic acid in which a group represented by the following formula is the constitutional unit.

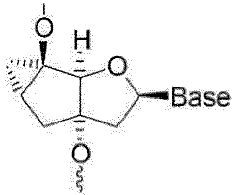

Tricyclo-DNA can be synthesized, for example, by reference to J. Am. Chem. Soc. 1997, 119, 47, 11548-11549, J. Am. Chem. Soc. 2002, 124, 21, 5993-6002, Nucleic Acids Res. 2002 Jul 1; 30(13):2751-7, Steffens, R.; Leumann, C. HelV. Chim. Acta 1997, 80, 2426-2439 and the like.

Peptide nucleic acid is a non-ribose phosphate backbone in which a group represented by the following formula is the constitutional unit. 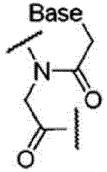 wherein Base is as defined above.

Peptide nucleic acid can be produced, for example, according to the following documents.
1) P.E. Nielsen, M. Egholm, R.H. Berg, O. Buchardt, Science, 254, 1497(1991)
2) M. Egholm, O. Buchardt, P.E. Nielsen, R.H. Berg, Jacs., 114, 1895(1992)
3) K.L. Dueholm, M. Egholm, C. Behrens, L. Christensen, H.F. Hansen, T. Vulpius, K.H. Petersen, R.H. Berg, P.E. Nielsen, O. Buchardt, J. Org. Chem., 59, 5767(1994)
4) L. Christensen, R. Fitzpatrick, B. Gildea, K.H. Petersen, H.F. Hansen, T. Koch, M. Egholm, O. Buchardt, P.E. Nielsen, J. Coull, R.H. Berg, J. Pept. Sci., 1, 175(1995)
5) T. Koch, H.F. Hansen, P. Andersen, T. Larsen, H.G. Batz, K. Otteson, H. Orum, J. Pept. Res., 49, 80(1997)

In the present specification, the "modified internucleoside bond" refers to an internucleoside bond that has a substitution or any change from a naturally occurring internucleoside bond (that is, phosphodiester bond). The modified internucleoside bond includes internucleoside bonds that include a phosphorus atom and internucleoside bonds that do not include a phosphorus atom. In the aforementioned sugar phosphate backbone, the internucleoside bond may be a modified internucleoside bond. For example, the internucleoside bond may include a modified internucleoside bond in which the phosphate group is modified. In the aforementioned sugar phosphate backbone, a phosphate group closest to the sugar residue is called an α phosphate group. The aforementioned α phosphate group is negatively charged, and the charge is evenly distributed over the two oxygen atoms not bonded to the sugar residue. Among the four oxygen atoms in the aforementioned α phosphate group, the two oxygen atoms that are not bonded to sugar residues in the phosphodiester bond between nucleotide residues hereinafter also to be referred to as "non-bonded (non-linking) oxygen". On the other hand, the two oxygen atoms bonded to the sugar residue in the phosphodiester bond between the aforementioned nucleotide residues are hereinafter to be referred to as "bonded (linking) oxygen". The aforementioned α phosphate group is, for example, preferably modified to become uncharged, or modified to have an asymmetric charge distribution in the aforementioned non-bonded oxygen.

The aforementioned modified internucleoside bond may, for example, substitute the aforementioned non-bonded oxygen. The aforementioned oxygen may be, for example, substituted by any of the atoms S (sulfur), Se (selenium), B (boron), C (carbon), H (hydrogen), N (nitrogen), and OR (R is alkyl group or aryl group), and preferably substituted by S. One or both of the aforementioned non-bonded oxygens may be substituted and preferably one or both thereof is/are substituted by S. More specific examples of the aforementioned modified internucleoside bond include, but are not limited to, phosphodiester bond, phosphorothioate bond, phosphorodithioate bond, phosphoroselenate bond, phosphotriester bond (methylphosphotriester bond, ethylphosphotriester bond described in US-B-5,955,599), alkyl phosphonate bond (e.g., methyl phosphonate bond described in US-B-5,264,423 and US-B-5,286,717, methoxypropyl phosphonate bond described in WO 2015/168172), alkyl thiophosphonate bond, methyl thiophosphonate bond, boranophosphate bond, boranophosphate ester bond, modified internucleoside bond having a cyclic guanidine moiety (e.g., the following modified internucleoside bond represented by the formula (II): 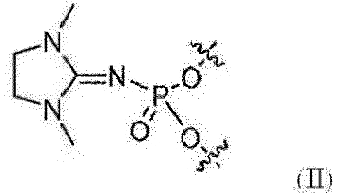
), modified internucleoside bond containing a guanidine moiety substituted with 1 to 4 C₁₋₆ alkyl groups (e.g., tetramethylguanidine (TMG) moiety (e.g., the following modified internucleoside bond represented by the formula (III): 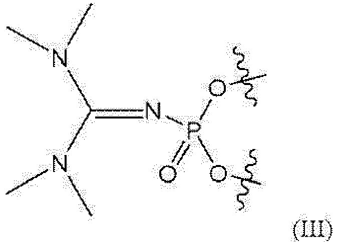
), modified internucleoside bond and phosphoramidate bond used in self-neutralizing nucleic acid (ZON) described in WO 2016/081600. A phosphorothioate bond refers to a modified internucleoside bond in which the non-crosslinked oxygen atom is substituted by a sulfur atom. Methods for preparing phosphorus-containing and non-phosphorus-containing bonds are well known. In one embodiment, the modified internucleoside bond is preferably a bond that is higher nuclease resistant than naturally occurring phosphodiester bonds. In one embodiment, a phosphorothioate bond, a phosphorodithioate bond, or a modified internucleoside bond represented by the aforementioned formula (III) is preferred, a phosphorothioate bond or a modified internucleoside bond represented by the aforementioned formula (III) is more preferred, and a phosphorothioate bond is further preferred.

When the aforementioned modified internucleoside bond has a chiral center, the modified internucleoside bond may be chirally controlled (hereinafter also to be referred to as chirally controlled phosphate backbone or chirally controlled modified phosphate backbone). "Chirally controlled" is intended to mean that it exists in a single diastereomer with respect to a chiral center, such as a chiral bonding phosphorus. The chirally controlled and modified internucleoside bond may be completely chirally pure or of high chiral purity, such as 90%de, 95%de, 98%de, 99%de, 99.5%de, 99.8%de, 99.9%de, or higher. In the present specification, the "chiral purity" refers to the proportion of one diastereomer in a mixture of diastereomers, expressed as diastereomeric excess (%de), and defined as (diastereomer of interest - other diastereomers)/(total diastereomer)×100(%).

For example, chirally controlled modified internucleoside bond may be a chirally controlled phosphorothioate bond in the Rp or Sp configuration, a modified internucleoside bond (e.g., modified internucleoside bond represented by the aforementioned formula (III)) containing a guanidine moiety substituted by 1 to 4 C1-6 alkyl groups (e.g., tetramethylguanidine (TMG) moiety; see, for example, Alexander A. Lomzov et al., Biochem Biophys Res Commun., 2019, 513(4), 807-811), and/or modified internucleoside bond (e.g., modified internucleoside bond represented by the aforementioned formula (II)) containing cyclic guanidine moiety. Methods for preparing chirally controlled modified internucleoside bond are known, for example, chirally controlled phosphorothioate bond in the Rp configuration or Sp configuration can be synthesized, for example, according to the method described in Naoki Iwamoto et al., Angew. Chem. Int. Ed. Engl. 2009, 48(3), 496-9, Natsuhisa Oka et al., J. Am. Chem. Soc. 2003, 125, 8307-8317, Natsuhisa Oka et al., J. Am. Chem. Soc. 2008, 130, 16031-16037, Yohei Nukaga et al., J. Org. Chem. 2016, 81, 2753-2762, Yohei Nukaga et al., J. Org. Chem. 2012, 77, 7913-7922. Chirally controlled phosphorothioate bond in the Rp configuration or Sp configuration is also known and known to afford the effects described in, for example, Naoki Iwamoto et al., Nat. Biotechnol. 2017, 35(9), 845-851, Anastasia Khvorova et al., Nat. Biotechnol., 2017, 35(3), 238-248. For example, in one embodiment, chirally controlled phosphorothioate bond in the Sp configuration is more stable than one in the Rp configuration, and/or a chirally controlled ASO in the Sp configuration facilitates target RNA cleavage by RNase H1, and affords a more sustained response in vivo. Methods for preparing modified internucleoside bond containing guanidine moiety (e.g., TMG moiety) substituted by 1 to 4 C1-6 alkyl groups are known, and it can be synthesized, for example, according to the method described in Alexander A. Lomzov et al., Biochem Biophys Res Commun., 2019, 513(4), 807-811.

In addition, the aforementioned phosphate group may be substituted by a linker free of phosphorus. Examples of the linker include siloxane, carbonate, carboxymethyl, carbamate, amide, thioether, ethylene oxide linker, sulfonate, sulfonamide, thioformacetal, formacetal, oxime, methyleneimino, methylenemethylimino, methylenehydrazo, methylenedimethylhydrazo, and methyleneoxymethylimino, and the like. Preferable examples thereof include methylenecarbonylamino group and methylenemethylimino group. Alternatively, the aforementioned phosphate group may be substituted by another linker free of phosphoric acid. Examples of such linker include those described in "Med. Chem. Commun., 2014, 5, 1454-1471", and the like.

In a preferred embodiment, 1/2 or more, more preferably 2/3 or more of the internucleoside bond contained in the compound containing an oligonucleotide of the present invention is modified with one or more of the above-mentioned phosphate groups, further preferably, all internucleoside bonds are modified internucleoside bonds. For example, when it is a 25-mer oligonucleotide, 13 or more, preferably 18 or more, more preferably all the internucleoside bonds are preferably modified internucleoside bonds, for example, phosphorothioate bonds, phosphorodithioate bonds, or modified internucleoside bonds represented by the aforementioned formula (III), more preferably phosphorothioate bonds or modified internucleside bonds represented by the aforementioned formula (III), further preferably phosphorothioate bonds. In another embodiment, the modified internucleoside bonds are modified internucleoside bonds represented by the aforementioned formula (III). Substitution of the unbounded oxygen in the phosphoric acid diester bond moiety by a sulfur atom is important for the improvement of nuclease resistance and the distribution of the compound containing an oligonucleotide in the tissue.

In the compound containing an oligonucleotide of the present invention, for example, at least one of 3'-terminal and 5'-terminal nucleotide residues of the oligonucleotide is optionally modified. The aforementioned modification may be, for example, at either the 3'-terminal and 5'-terminal, or both. The aforementioned modification is, for example, as described above, and preferably performed on the terminal phosphate group. The aforementioned phosphate group may be, for example, entirely modified, or one or more atoms in the aforementioned phosphate group may be modified. In the former case, for example, the entire phosphate group may be substituted or deleted.

Examples of the modification of the aforementioned terminal nucleotide residue include addition of other molecule. Examples of the aforementioned other molecule include functional molecules such as the below-mentioned labeling substance, protecting group, and the like. Examples of the aforementioned protecting group include groups containing S (sulfur), Si (silicon), B (boron), or ester, and the like. For example, the functional molecules such as the aforementioned labeling substance and the like can be utilized for detection of the compound containing an oligonucleotide of the present invention, and the like.

The aforementioned other molecule may be added to, for example, the phosphate group of the aforementioned nucleotide residue, or the aforementioned phosphate group or the aforementioned sugar residue via spacer. The terminal atom of the aforementioned spacer can be added to or substitute, for example, the aforementioned bounded oxygen of the aforementioned phosphate group, or O, N, S, or C of the sugar residue. The binding site of the aforementioned sugar residue is preferably, for example, C at the 3'-position or C at 5'-position, or an atom binding thereto. The aforementioned spacer can be added to or substitute, for example, the terminal atom of a nucleotide alternative such as the aforementioned PNA or the like.

The aforementioned spacer is not particularly limited, and it may contain, for example, -(CH₂)ₙ-, -(CH₂)ₙN-, -(CH₂)ₙO-, -(CH₂)ₙS-, O(CH₂CH₂O)ₙCH₂CH₂OH, abasic sugar, amide, carboxy, amine, oxyamine, oxyimine, thioether, disulfide, thiourea, sulfonamide, morpholino, and the like, and biotin reagent, fluorescein reagent, and the like. In the aforementioned formula, n is a positive integer (e.g., integer of 1 to 10), preferably n=3 or 6.

Examples of the molecule to be added to the aforementioned terminal include, in addition to these, dye, intercalating agent (e.g., acridine), crosslinking agent (e.g., psoralen, mitomycin C), porphyrin (TPPC4, texaphyrin, sapphyrin), polycyclic aromatic hydrocarbon (e.g., phenazine, dihydrophenazine), artificial endonuclease (e.g., EDTA), lipophilic carrier (e.g., cholesterol, cholic acid, adamantaneacetic acid, 1-pyrenebutyric acid, dihydrotestosterone, 1,3-bis-O(hexadecyl)glycerol, geranyl oxyhexyl group, hexadecylglycerol, borneol, menthol, 1,3-propanediol, heptadecyl group, palmitic acid, myristic acid, O3-(oleoyl)lithocholic acid, O3-(oleoyl)cholic acid, dimethoxytrityl, or phenoxathiine) and peptide complex (e.g., antennapedia peptide, Tat peptide), alkylating agent, phosphoric acid, amino, mercapto, PEG (e.g., PEG-40K), MPEG, [MPEG]₂, polyamino, alkyl, substituted alkyl, radiolabeled marker, enzyme, hapten (e.g., biotin), transport/absorption promoter (e.g., aspirin, vitamin E, folic acid), synthesis ribonuclease (e.g., imidazole, bis imidazole, histamine, imidazole cluster, acridine-imidazole complex, Eu³⁺ complex of tetraaza macrocycle), and the like.

In the compound containing an oligonucleotide of the present invention, the aforementioned 5'-terminal of the oligonucleotide is optionally modified with, for example, a phosphate group or a phosphate group analogue. Examples of the aforementioned phosphate group include 5'-monophosphate ((HO)₂(O)P-O-5'), 5' -diphosphate ((HO)₂(O)P-O-P(HO)(O)-O-5'), 5' -triphosphate ((HO)₂(O)P-O-(HO)(O)P-O-P(HO)(O)-O-5'), 5'-guanosine cap (7-methylated or unmethylated, 7m-G-O-5'-(HO)(O)P-O-(HO)(O)P-O-P(HO)(O)-O-5'), 5'-adenosine cap (Appp), optionally modified or unmodified nucleotide cap structure (NO-5'-(HO)(O)P-O-(HO)(O)P-O-P(HO)(O)-O-5'), 5'-monothiophosphate (phosphorothioate: (HO)₂(S)P-O-5'), 5'-monodithiophosphate (phosphorodithioate: (HO)(HS)(S)P-O-5'), 5'-phosphorthiolate ((HO)₂(O)P-S-5'), sulfur-substituted monophosphate, diphosphoate, and triphosphate (e.g., 5'-α-thiotriphosphate, 5'-γ-thiotriphosphate, etc.), 5'-phosphoramidate ((HO)₂(O)P-NH-5', (HO) (NH₂) (O)P-O-5'), 5'-alkylphosphonic acid (e.g., R⁴P(OH)(O)-O-5', (OH)₂(O)P-5'-CH₂, R⁴ is an alkyl (e.g., methyl, ethyl, isopropyl, propyl, etc.)), 5'-alkyletherphosphate (e.g., R⁵P(OH)(O)-O-5', R⁵ is an alkylether (e.g., methoxymethyl, ethoxymethyl, etc.)), and the like.

In the aforementioned nucleotide residue, the aforementioned base is not particularly limited, and it may be, for example, natural base, non-natural base, unmodified base, or modified base. The aforementioned base may be, for example, a naturally derived product or a synthesis product. As the aforementioned base, for example, general base, modified analogue thereof, universal base, and the like can be used.

Examples of the aforementioned base include purine bases such as adenine, guanine, and the like, and pyrimidine bases such as cytosine, 5-methylcytosine, uracil, thymine, and the like. In addition to these, as the aforementioned base, inosine, thymine, xanthine, hypoxanthine, nubularine, isoguanisine, tubercidine, and the like can be mentioned. Examples of the aforementioned base include 2-aminoadenine, alkyl derivatives such as 6-methylated purine and the like; alkyl derivatives such as 2-propylated purine and the like; 5-halo uracil and 5-halo cytosine; 5-propynyluracil and 5-propynylcytosine; 6-azouracil, 6-azocytosine and 6-azothymine; 5-uracil (pseudouracil), 4-thiouracil, 5-halo uracil, 5-(2-aminopropyl)uracil, 5-aminoallyluracil; 8-halogenated, aminated, thiolated, thioalkylated, hydroxylated and other 8-substituted purines; 5-trifluoromethylated and other 5-substituted pyrimidines; 7-methylguanine; 5-substituted pyrimidine; 6-azapyrimidine; N-2, N-6, and O-6 substituted purines (including 2-aminopropyladenine); 5-propynyluracil and 5-propynylcytosine; dihydrouracil; 3-deaza-5-azacytosine; 2-aminopurine; 5-alkyluracil; 7-alkylguanine; 5-alkylcytosine; 7-deazaadenine; N6,N6-dimethyladenine; 2,6-diamino purine; 5-amino-allyl-uracil; N3-methyluracil; substituted 1,2,4-triazole; 2-pyridinone; 5-nitroindole; 3-nitropyrrole; 5-methoxyuracil; uracil-5-oxyacetic acid; 5-methoxycarbonylmethyluracil; 5-methyl-2-thiouracil; 5-methoxycarbonylmethyl-2-thiouracil; 5-methylaminomethyl-2-thiouracil; 3-(3-amino-3-carboxypropyl)uracil; 3-methylcytosine; N4-acetylcytosine; 2-thiocytosine; N6-methyladenine; N6-isopentyladenine; 2-methylthio-N6-isopentenyladenine; N-methylguanine; O-alkylated base; 5-hydroxycytosine; -acetylcytosine; 3-C₁₋₆ alkylcytosine; 5-C₁₋₆ alkylcytosine; 2-thiothymine; dihydrothymine; pseudothymine; 2-thiouracil; 5-(carboxyhydroxymethyl)uracil; 5-carboxymethylaminomethyl-2-thiouracil; 5-carboxymethylaminomethyluracil; 5-methylaminomethyluracil; 8-aminoguanine; 8-halogeno guanine; 7-deaza-7-(2-phenylethynyl)guanine; 7-deaza-7-(2-pyridylethynyl)guanine; 7-deaza-7-[2-(C₁₋₇ alkanoyloxy-C₁₋₆ alkyl) ethynyl] guanine; 7-deaza-7-[2-(hydroxy-C₁₋₆ alkyl) ethynyl] guanine; 1-C₁₋₆ alkylguanine; 2,2-di (C₁₋₆ alkyl) guanine, 2-C₁₋₆ alkylguanine; 7-deaza-7-C₁₋₆ alkylguanine; 7-deaza-7-C₂₋₆ alkenylguanine; 7-deaza-7-C₂₋₆ alkynylguanine; 7-deaza-7-halogeno guanine; 1-C₁₋₆ alkylinosine; queuosine; β,D-galactosyl queuosine; β,D-mannosyl queuosine; N⁶-C₂₋₆ alkenyladenine; 1-C₁₋₆ alkyladenine; 2-C₁₋₆ alkyladenine; N⁶-C₁₋₆ alkyladenine; 2-C₁₋₆ alkylthio-N⁶-C₂₋₆ alkenyladenine, and the like. In addition, examples of the purine and the pyrimidine include those disclosed in US Patent No. 3,687,808, "Concise Encyclopedia Of Polymer Science And Engineering", pages 858 to 859, Kroschwitz J.I. ed., John Wiley & Sons, 1990, and Englisch, et al.), and Angewandte Chemie, International Edition, 1991, vol. 30, p.613.

In addition to these, the aforementioned modified nucleotide residues may also include, for example, a residue lacking a base, that is, an abasic sugar phosphate backbone. Furthermore, the aforementioned modified nucleotide residues can be, for example, the residues described in WO 2004/080406, and the present invention can make use of such document.

The conjugate of the present invention may form a salt with an inorganic base, an organic base, an inorganic acid, an organic acid, or the like. Examples of the above-mentioned salts with inorganic base include alkali metal salts such as sodium salt, potassium salt; alkaline earth metal salts such as calcium salt, magnesium salt and the like; aluminum salt, ammonium salt and the like. Examples of the above-mentioned salts with organic base include salts with trimethylamine, triethylamine, pyridine, picoline, ethanolamine, diethanolamine, triethanolamine, dicyclohexylamine, N,N'-dibenzylethylenediamine, tris(hydroxymethyl)aminomethane, or meglumine. Examples of the above-mentioned salts with inorganic acid include salts with hydrochloric acid, hydrobromic acid, nitric acid, sulfuric acid, phosphoric acid. Examples of the above-mentioned salts with organic acid include salts with formic acid, acetic acid, trifluoroacetic acid, fumaric acid, oxalic acid, tartaric acid, maleic acid, citric acid, succinic acid, malic acid, methanesulfonic acid, benzenesulfonic acid, or p-toluenesulfonic acid. Of these salts, a pharmacologically acceptable salt is preferred, and a sodium salt is more preferred.

The conjugate of the present invention may be labeled with, for example, a labeling substance. Examples of the aforementioned labeling substance include, but are not limited to, fluorescent substance, dye, isotope, and the like. Examples of the aforementioned fluorescent substance include fluorophores such as pyrene, TAMRA, fluorescein, Cy3 dye, Cy5 dye, and the like. Examples of the aforementioned dye include Alexa dyes such as Alexa488 and the like, and the like. Examples of the aforementioned isotope include stable isotope and radioisotope, and it is preferably a stable isotope. The aforementioned stable isotope has, for example, low risk of exposure and does not require dedicated facilities. Thus, it is easy to handle and can also reduce costs. In addition, the aforementioned stable isotope does not, for example, change the physical properties of the labeled compound, and has superior properties as a tracer. Examples of the aforementioned stable isotope include, but are not limited to, ²H, ¹³C, ¹⁵N, ¹⁷O, ¹⁸O, ³³S, ³⁴S , and ³⁶S.

The compound containing an oligonucleotide of the present invention can be produced by a chemical synthetic method known per se. For example, phosphoramidite method and H-phosphonate method and the like can be mentioned. The chemical synthetic method can be performed using, for example, a commercially available automatic nucleic acid synthesizer (e.g., FOCUS (Aapptec), AKTA oligopilot plus 10/100 (GE Healthcare), nS-8II (GeneDesign, Inc.) etc.). When amidite is used, as a commercially available amidite, for example, RNA Phosphoramidites(2'-O-TBDMSi, trade name, Samchully Pharmaceutical Co.), ACE amidite and TOM amidite, CEE amidite, CEM amidite, TEM amidite, and the like can be used. When the compound containing an oligonucleotide of the present invention is in the form of a double-stranded oligonucleotide, the compound can be produced by separately synthesizing an antisense strand (guide strand) and a sense strand (passanger strand), mixing the both in, for example, an annealing buffer (e.g., 10 mM Tris-HCl buffer (pH 7.5), 20 mM NaCl), heat-denaturing them at 90°C and then annealing them by gradual cooling. Alternatively, the production can be outsourced to a third-party organization (e.g., GeneDesign, Inc., Hokkaido System Science Co., Ltd., Nitto Denko Corporation, Promega KK, Takara, Japan Bio Services Co., LTD., Wuxi, Wuxi STA, or Gene Tools) and the like.

In one embodiment of the compound containing an oligonucleotide of the present invention, the antisense strand containing a nucleotide sequence complementary to the target sequence may be a totalmer, a mixmer, or a gapmer. A totalmer or a mixmer is preferred. A totalmer is more preferred.

### Totalmer

The totalmer means a nucleoside-modified oligonucleotide wherein all the nucleosides in the oligonucleotide are modified. The totalmer may contain only one type of nucleoside modification. It may contain, for example, fully 2'-MOE-modified oligonucleotide, fully 2'-MCE-modified oligonucleotide, fully 2'-OMe-modified oligonucleotide, fully LNA-modified oligonucleotide, fully morpholino-modified oligonucleotide, or fully peptide nucleic acid-modified oligonucleotide, or a mixture of different nucleoside-modified products, for example, a mixture of LNA nucleoside and 2'-MOE nucleoside. In some embodiments, the totalmer may contain one or two 3'terminal LNA nucleosides. In some embodiments, the totalmer may contain any number of the types of modified nucleoside.

### Mixmer

The mixmer means an oligonucleotide containing both of a nucleoside with the sugar moiety of the nucleoside modified (hereinafter sugar-modified nucleoside) and a nucleoside with the sugar moiety which is DNA (hereinafter DNA nucleoside), wherein the oligonucleotide does not contain many consecutive DNA nucleosides in the number of more than 4. The mixmer may be designed, for example, to include a sugar-modified nucleoside that enhances affinity, such as 2'-O-alkyl, 2'-O-methyl(2'-OMe), 2'-alkoxy, 2'-O-methoxyethyl(2'-MOE), 2'-MCE, 2'-amino, 2'-fluoro, and 2'-F-ANA nucleoside, LNA, and the like. In the present specification, a mixmer constituted of DNA nucleoside and 2'-substituted modified nucleoside is referred to as "2'-substituted modified nucleic acid/DNA mixmer". A mixmer constituted of DNA nucleoside and 2'-MOE-modified nucleoside is referred to as"2'-MOE/DNA mixmer". A mixmer constituted of DNA nucleoside and 2'-MCE-modified nucleoside is referred to as "2'-MCE/DNA mixmer". A mixmer constituted of DNA nucleoside and bridged nucleoside is referred to as "BNA/DNA mixmer". The bridged nucleoside may include modified nucleobase (e.g., 5-methylcytosine). In the present specification, a mixmer constituted of natural deoxyribonucleoside and LNA nucleoside is referred to as "LNA/DNA mixmer". The mixmer is not necessarily limited to include two types of nucleosides alone. The mixmer may include any number of the types of nucleoside, irrespective of whether it is an unmodified or modified nucleoside.

In some embodiments, the mixmer does not contain more than 3 consecutive DNA nucleosides. In some embodiments, the mixmer does not contain more than 2 consecutive DNA nucleosides. In some embodiments, the mixmer does not contain a region composed of a sugar phosphate backbone in which two or more (preferably three or more, more preferably four or more, particularly preferably five or more) consecutive sugar moieties are modified, such as LNA.

### Gapmer

A gapmer-type nucleic acid containing
(1) 5' wing region located at the 5'-terminal;
(2) 3' wing region located at the 3'-terminal; and
(3) a deoxy gap region located between region (1) and region (2).

The 5' and 3' wing regions of the gapmer-type antisense strand are each independently 2 to 5 nucleotides long. Furthermore, the length of the deoxy gap region of the gapmer-type antisense strand is 7 to 10 nucleotides. More specifically, for example, "3-9-3" type gapmer, "3-10-2" type gapmer, "2-10-3" type gapmer, "4-9-2" type gapmer having a length of 15 nucleotides; "3-10-3" type gapmer, "4-9-3" type gapmer having a length of 16 nucleotides; "4-10-3" type gapmer, "4-9-4" type gapmer having a length of 17 nucleotides; "4-10-4" type gapmer, "5-9-4" type gapmer having a length of 18 nucleotides; "5-10-4" type gapmer, "5-9-5" type gapmer having a length of 19 nucleotides; or "5-10-5" type gapmer having a length of 20 nucleotides can be mentioned.

In the gapmer-type antisense strand, the sugar moiety of at least one nucleoside constituting the 5' and 3' wing regions is preferably modified. Examples of the sugar moiety modification include modifications by 2'-OMe, 2'-MOE, 2'-MCE, the aforementioned bridged nucleic acid (e.g., LNA, AmNA, GuNA, scpBNA). Preferably, two or more (e.g., 2, 3, 4, 5) nucleotide residues constituting each of the 5' and 3' wing regions are modified.

### LNA gapmer

The term LNA gapmer refers to a gapmer oligonucleotide in which at least one of the affinity-enhancing modified nucleosides in the wing is a nucleoside modified with LNA (hereinafter also referred to as LNA nucleoside). In some embodiments, the LNA gapmer is an LNA gapmer in which the 3'-terminal nucleoside of the oligonucleotide is an LNA nucleoside. In some embodiments, the two nucleosides located closest to 3' of the oligonucleotide are LNA nucleosides. In some embodiments, the 5' wing and 3' wing of the LNA gapmer both include LNA nucleosides, and in some embodiments, nucleoside modified oligonucleotide is an LNA oligonucleotide, for example, gapmer oligonucleotide. Here, all nucleosides of the oligonucleotide are either LNA nucleosides or DNA nucleosides.

### Mixed wing gapmer

The term of mixed wing gapmer or mixed wing gapmer refers to an LNA gapmer in which at least one wing region includes at least one LNA nucleoside, and at least one non-LNA modified nucleoside, for example, at least one 2'-substituted modified nucleoside, for example, 2'-O-alkyl, 2'-O-methyl (2'-OMe), 2'-alkoxy, 2'-O-methoxyethyl (2'-MOE), 2'-MCE, 2'-amino, 2'-fluoro, and 2'-F-ANA nucleoside, and the like. In some embodiments, the mixed wing gapmer has one wing including only 2'-substituted modified nucleoside (e.g., 5' or 3'), and the other wing (e.g., 3' or 5') including 2'-substituted modified nucleoside and optionally, an LNA nucleoside. In some embodiments, the mixed wing gapmer includes LNA nucleoside and 2'-MOE nucleoside in the wing. In some embodiments, the mixed wing gapmer includes LNA nucleoside and 2'-MCE nucleoside in the wing.

In one embodiment, when the antisense strand of the present invention is a totalmer, fully 2'-MOE-modified oligonucleotide, fully 2'-MCE-modified oligonucleotide, fully 2'-O-methyl-modified oligonucleotide, fully LNA-modified oligonucleotide, fully morpholino-modified oligonucleotide, or fully peptide nucleic acid-modified oligonucleotide is preferred. Fully 2'-MOE-modified oligonucleotide, fully 2'-MCE-modified oligonucleotide, or fully morpholino-modified oligonucleotide is more preferred (preferably PMO, fully phosphorodimethylamidate morpholino-modified oligonucleotide is further preferred). Fully morpholino-modified oligonucleotide is particularly preferred. PMO is more preferred. Fully phosphorodimethylamidate morpholino-modified oligonucleotide is further preferred. In another embodiment, fully 2'-MOE-modified oligonucleotide or fully 2'-MCE-modified oligonucleotide can be preferably used.

In one embodiment, when the antisense strand of the present invention is a totalmer or a mixmer such as fully 2'-MOE-modified oligonucleotide, fully 2'-MCE-modified oligonucleotide, fully 2'-OMe-modified oligonucleotide, fully LNA-modified oligonucleotide, or the like, the sugar phosphate backbone preferably contains at least one (e.g., 2 or more, 4 or more, 6 or more, 8 or more, 10 or more, 12 or more, 16 or more) modified internucleoside bond. All of the internucleoside bonds of the sugar phosphate backbone are more preferably modified internucleoside bonds. The modified internucleoside bond is preferably a phosphorothioate bond, a phosphorodithioate bond, or a modified internucleoside bond represented by the aforementioned formula (III), more preferably a phosphorothioate bond or a modified internucleoside bond represented by the aforementioned formula (III), further preferably a phosphorothioate bond. In another embodiment, the modified internucleoside bond is a modified internucleoside bond represented by the aforementioned formula (III) .

In another embodiment, the antisense strand of the present invention contains or consists of 2'-MOE-modified nucleoside connected via a phosphorothionate backbone (in the present specification, also to be referred to as 2'-MOE phosphorothionate nucleotide). One embodiment may be an oligonucleotide further containing modified nucleoside, preferably 2'-MOE-modified nucleoside, more preferably 2'-MOE phosphorothionate nucleotide, particularly preferably an oligonucleotide in which the basic skeleton is a 2'-MOE phosphorothionate nucleotide (hereinafter also to be referred to as fully 2'-MOE phosphorothionate-modified oligonucleotide).

In further another embodiment, the antisense strand of the present invention contains or consists of 2'-MCE-modified nucleoside connected via a phosphorothionate backbone (in the present specification, also to be referred to as 2'-MCE phosphorothionate nucleotide). One embodiment may be an oligonucleotide further containing modified nucleoside, preferably 2'-MCE-modified nucleoside, more preferably 2'-MCE phosphorothionate nucleotide, particularly preferably an oligonucleotide in which the basic skeleton is a 2'-MCE phosphorothionate nucleotide (hereinafter also to be referred to as fully 2'-MCE phosphorothionate-modified oligonucleotide).

In one embodiment, the antisense strand of the present invention is a hybrid oligonucleotide containing 2'-MOE phosphorothionate nucleotide and LNA. This specific oligonucleotide includes better sequence specificity as compared with equivalents where the sugar moiety consists only of LNA modification, and may improve efficacy as compared with fully 2'-MOE phosphorothionate-modified oligonucleotides.

In one embodiment, the antisense strand of the present invention is a hybrid oligonucleotide containing 2'-MCE phosphorothionate nucleotide and LNA. This specific oligonucleotide includes better sequence specificity as compared with equivalents where the sugar moiety consists only of LNA modification, and may improve efficacy as compared with fully 2'-MCE phosphorothionate-modified oligonucleotides.

In another embodiment, the antisense strand of the present invention is a 2'-substituted modified nucleic acid/DNA mixmer or a BNA/DNA mixmer, preferably a 2'-MOE/DNA mixmer, a 2'-MCE/DNA mixmer, or an LNA/DNA mixmer, more preferably a 2'-MOE/DNA mixmer or a 2'-MCE/DNA mixmer, particularly preferably a 2'-MOE/DNA mixmer. In another embodiment, 2'-MCE/DNA mixmer is particularly preferred.

### Fully morpholino-modified oligonucleotide

A fully morpholino-modified oligonucleotide which is the particularly preferred embodiment of the antisense strand of the present invention is an oligonucleotide whose constitutional unit is a group represented by the following formula. 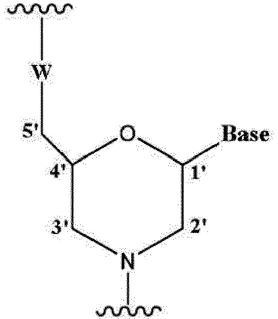 wherein Base and W are as defined above.

The fully morpholino-modified oligonucleotide is preferably a fully phosphorodiamidatemorpholino-modified oligonucleotide (in the present specification, also to be referred to as PMO)) whose constitutional unit is a group represented by the following formula: 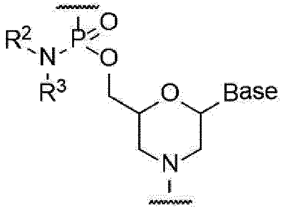 wherein R² and R³ are each alkyl (e.g., C₁-C₁₀ alkyl), and Base is as defined above.

A fully phosphorodimethylamidatemorpholino-modified oligonucleotide whose constitutional unit is a group represented by the following formula (phosphorodimethylamidatemorpholino) is further preferred: 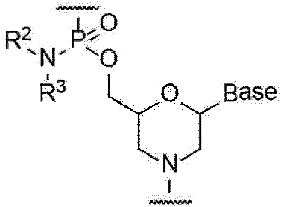 wherein R² and R³ are each methyl, and Base is as defined above.

### Fully peptide nucleic acid-modified oligonucleotide

The fully peptide nucleic acid-modified oligonucleotide is an oligonucleotide whose constitutional unit is a group represented by the following formula: 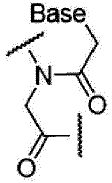 wherein Base is as defined above.

The compound containing an oligonucleotide of the present invention may be a single-stranded oligonucleotide including a sequence complementary to the target sequence, namely, a single-stranded oligonucleotide composed of an antisense oligonucleotide (ASO), or a double-stranded oligonucleotide (nucleic acid complex) composed of an antisense strand (also to be referred to as "the first nucleic acid strand") and a sense strand entirely or partially complementary thereto (also to be referred to as "the second nucleic acid strand"). The complementarity of the antisense strand sequence to the sense strand sequence as defined above for the complementarity of the antisense strand sequence to the target sequence. The antisense strand in the double-stranded oligonucleotide (nucleic acid complex) preferably includes an antisense oligonucleotide region, further preferably antisense oligonucleotide.

In the present invention, the antisense oligonucleotide is a single-stranded oligonucleotide that has an antisense effect. The antisense oligonucleotide may be an antisense strand that forms a nucleic acid complex. In the present invention, an antisense oligonucleotide region is a region of an oligonucleotide (antisense strand) that has an antisense effect. The "antisense effect" means control of the expression of a target transcription product resulting from hybridization of a target transcription product (RNA sense strand) and a strand (e.g., DNA strand) that is complementary to a partial sequence of the transcription product etc. and designed to afford an antisense effect. Control of the expression of a target transcription product may include suppressing or reducing the expression of the target gene or the level (expression level) of the target transcription product or, in a specific example, inhibition of translation or splicing function-altering effects, such as exon skipping or decomposition of transcription product. For example, in the inhibition of translation, when an oligonucleotide containing RNA is introduced into a cell as an antisense oligonucleotide (ASO), the ASO binds to a transcript (mRNA) of the target gene to form a partial double strand.
This partial double strand acts as a cover to prevent translation by the ribosome, therefore inhibiting the expression of the protein encoded by the target gene at the translation level. On the other hand, when an oligonucleotide containing DNA is introduced into a cell as ASO, a partial DNA-RNA heteroduplex is formed. As a result of recognition of this heteroduplex structure by RNase H, the mRNA of the target gene is degraded, and the expression of the protein encoded by the target gene is inhibited at the expression level. This is referred to as the "RNase H-dependent pathway". Moreover, in certain cases, the antisense effect can be achieved by targeting an intron of mRNA precursor. An antisense effect may also be brought about by targeting a miRNA, in which case the function of the miRNA may be inhibited and the expression of the gene whose expression is normally regulated by the miRNA may be increased. In one embodiment, the expression regulation of target transcription product may be a reduction in the amount of target transcription product.

The length of the antisense oligonucleotide region in the first nucleic acid strand may be generally at least 4-nucleotides long, at least 5-nucleotides long, at least 6-nucleotides long, at least 7-nucleotides long, at least 8-nucleotides long, at least 9-nucleotides long, at least 10-nucleotides long, at least 11-nucleotides long, at least 12-nucleotides long, or at least 13-nucleotides long, and is not particularly limited. The length of the antisense oligonucleotide region may be the same as the full-length of the first nucleic acid strand.

The antisense strand includes a base sequence that can hybridize to at least a portion (e.g., any target region) of a target transcription product. The target region may include 3'UTR, 5'UTR, exon, intron, coding region, translation initiation region, translation termination region, or other nucleic acid region.

The antisense effect can be measured, for example, by administering a test nucleic acid compound to a subject (e.g., mouse) and, for example, after several days (e.g., 2 to 7 days), and measuring the expression level of the target gene whose expression is regulated by the antisense effect provided by the test nucleic acid compound or the level (amount) (e.g., mRNA amount or RNA amount such as micro RNA and the like, cDNA amount, protein amount, and the like) of the target transcription product.

For example, when the measured target gene expression or target transcription product level is at least 10%, at least 20%, at least 25%, at least 30%, or at least 40%, as compared with the negative control (e.g., vehicle administration), it is indicated that the test nucleic acid compound can produce an antisense effect (e.g., decrease in the amount of target transcription product).

When the compound containing an oligonucleotide of the present invention is a single-stranded oligonucleotide, for example, single-stranded oligonucleotide consisting of antisense oligonucleotide, a functional molecule other than the peptide of the present invention that can be efficiently delivered to cells, tissues, organs, etc. to be introduced can be bonded to the 5'-terminal, 3'-terminal, or both terminals of the antisense oligonucleotide. Alternatively, the functional molecule may be linked to an internal nucleotide of the antisense oligonucleotide. The structure of the "functional molecule" is not specifically limited according to a specific embodiment. As desired functions, a labeling function and a purification function can be mentioned. Examples of the part imparting the labeling function include compounds such as fluorescent protein, luciferase, and the like. Examples of the part imparting the purification function include compounds such as biotin, avidin, His tag peptide, GST tag peptide, FLAG tag peptide, and the like. The binding position and type of binding in the second nucleic acid strand of the functional molecule are as described above for the binding to the compound containing an oligonucleotide.

The 5'-terminal of the antisense strand or the antisense oligonucleotide may be a group of any of the following chemical formulas (1) to (3).

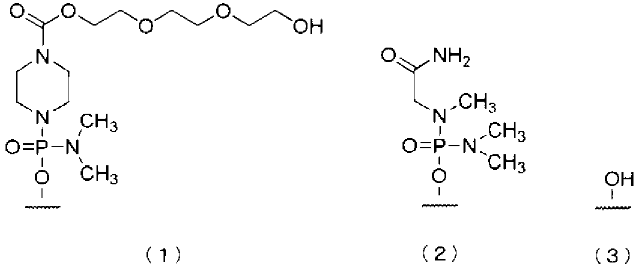

Hereinafter, the groups represented by the above-mentioned (1), (2), and (3) are respectively referred to as "group (1)", "group (2)", and "group (3)". In addition, "group (2)" is preferred.

In one preferred embodiment, the compound containing an oligonucleotide of the present invention may be a single stranded antisense oligonucleotide (ASO). In more preferred embodiment, as the ASO consisting of the nucleotide sequence shown in SEQ ID NO: 687 completely complementary to the 36th to 60th nucleotides sequence of human exon 53, 
[MoG] [MoT] [MoT] [MoG] [MoC] [MoC] [MoT] [MoC] [MoC] [MoG] [MoG] [MoT] [Mo
T] [MoC] [MoT] [MoG] [MoA] [MoA] [MoG] [MoG] [MoT] [MoG] [MoT] [MoT] [MoC]
(SEQ ID NO: 687-1(SEQ ID NO: 728))
can be mentioned, and
as the ASO consisting of the nucleotide sequence shown in SEQ ID NO: 688 completely complementary to the 36th to 56th nucleotides sequence, 
[MoC] [MoC] [MoT] [MoC] [MoC] [MoG] [MoG] [MoT] [MoT] [MoC] [MoT] [MoG] [Mo
A] [MoA] [MoG] [MoG] [MoT] [MoG] [MoT] [MoT] [MoC] (SEQ ID NO: 688-1 (SEQ
ID NO: 729))
can be mentioned.
(in the above-mentioned sequence, "Mo" is PMO (preferably morpholinonucleic acid).)

Alternatively, as the ASO consisting of the nucleotide sequence shown in SEQ ID NO: 706 completely complementary to the sequences of mouse exon 23, 
[MoG] [MoG] [MoC] [MoC] [MoA] [MoA] [MoA] [MoC] [MoC] [MoT] [MoC] [MoG] [Mo
G] [MoC] [MoT] [MoT] [MoA] [MoC] [MoC] [MoT] [MoG] [MoA] [MoA] [MoA] [MoT]
(SEQ ID NO: 706-1(SEQ ID NO: 730))
can be mentioned.
(in the above-mentioned sequence, "Mo" is PMO (preferably morpholinonucleic acid).)

In each ASO sequence mentioned above, base thymine (T) may be uracil (U). In addition, base cytosine (C) may be 5-methylcytosine.

In each ASO sequence mentioned above, the 5'-terminal may be a group of any of the following chemical formulas (1) to (3).

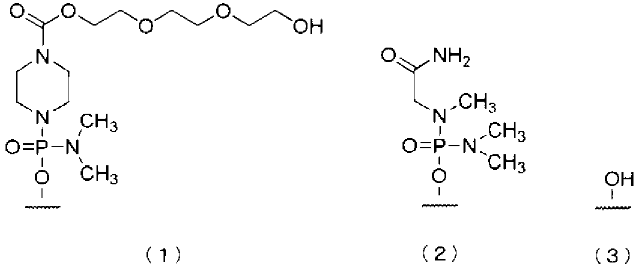

Hereinafter, the groups represented by the above-mentioned (1), (2), and (3) are respectively referred to as "group (1)", "group (2)", and "group (3)". Furthermore, "group (2)" is preferred.

More preferably, as the ASO consisting of the nucleotide sequence shown in SEQ ID NO: 687 completely complementary to the 36th to 60th nucleotides sequence of human exon 53,
can be mentioned, and
as the ASO consisting of the nucleotide sequence shown in SEQ ID NO: 688 completely complementary to the 36th to 56th nucleotides sequence, can be mentioned.
(in the above-mentioned sequence, "Mo" is PMO (preferably morpholinonucleic acid).)

Particularly preferably, as the ASO consisting of the nucleotide sequence shown in SEQ ID NO: 687 completely complementary to the 36th to 60th nucleotides sequence of human exon 53, 
[MoG] [MoT] [MoT] [MoG] [MoC] [MoC] [MoT] [MoC] [MoC] [MoG] [MoG] [MoT] [Mo
T] [MoC] [MoT] [MoG] [MoA] [MoA] [MoG] [MoG] [MoT] [MoG] [MoT] [MoT] [MoC]
(SEQ ID NO: 687-1)
 can be mentioned.

In another embodiment, particularly preferably, as the ASO consisting of the nucleotide sequence shown in SEQ ID NO: 688 completely complementary to the 36th to 56th nucleotides sequence, can be mentioned. In the above-mentioned sequence, "Mo" is PMO (preferably morpholinonucleic acid).

In one preferred embodiment, as the compound containing an oligonucleotide of the present invention, a compound containing the following antisense oligonucleotide (antisense strand) can be mentioned.

As the antisense oligonucleotide consisting of the nucleotide sequence shown in SEQ ID NO: 687 completely complementary to the 36th to 60th nucleotides sequence of human exon 53,

As the antisense oligonucleotide consisting of the nucleotide sequence shown in SEQ ID NO: 688 completely complementary to the 36th to 56th nucleotides sequence, In the above-mentioned sequences, "Mo" is PMO (preferably morpholinonucleic acid).

Alternatively, as the antisense oligonucleotide consisting of the nucleotide sequence shown in SEQ ID NO: 706 completely complementary to the sequences of mouse exon 23, can be mentioned. In the above-mentioned sequence, "Mo" is PMO (preferably morpholinonucleic acid).

More preferably, as the antisense oligonucleotide consisting of the nucleotide sequence shown in SEQ ID NO: 687 completely complementary to the 36th to 60th nucleotides sequence of human exon 53,
can be mentioned, and
as the ASO consisting of the nucleotide sequence shown in SEQ ID NO: 688 completely complementary to the 36th to 56th nucleotides sequence, can be mentioned.
In the above-mentioned sequences, "Mo" is PMO (preferably morpholinonucleic acid).

Particularly preferably, as the antisense oligonucleotide consisting of the nucleotide sequence shown in SEQ ID NO: 687 completely complementary to the 36th to 60th nucleotides sequence of human exon 53, (SEQ ID NO: 687-1) can be mentioned.

In another embodiment, particularly preferably, as the antisense oligonucleotide consisting of the nucleotide sequence shown in SEQ ID NO: 688 completely complementary to the 36th to 56th nucleotides sequence, can be mentioned. In the above-mentioned sequences, "Mo" is PMO (preferably morpholinonucleic acid).

In each ASO sequence mentioned above, base thymine (T) may be uracil (U). In addition, base cytosine (C) may be 5-methylcytosine.

In each ASO sequence mentioned above, the 5'-terminal may be a group of any of the following chemical formulas (1) to (3).

Hereinafter, the groups represented by the above-mentioned (1), (2), and (3) are respectively referred to as "group (1)", "group (2)", and "group (3)". Furthermore, "group (2)" is preferred.

### Nucleic acid complex

In another preferred embodiment, the compound containing an oligonucleotide of the present invention includes an antisense oligonucleotide region in the aforementioned antisense strand (first nucleic acid strand) and a sense strand (second nucleic acid strand) complementary to all or part of the antisense strand, and the first nucleic acid strand may be a nucleic acid complex that is annealed to the second nucleic acid strand. In this case, the aforementioned peptide is bound to the second nucleic acid strand directly or via a linker in the conjugate of the present invention.

When the compound containing an oligonucleotide of the present invention is a double-stranded oligonucleotide composed of an antisense oligonucleotide region in the antisense strand (first nucleic acid strand) and a sense strand complementary thereto (second nucleic acid strand), the second nucleic acid strand may include a nucleotide sequence complementary to the full length of a sequence complementary to the target sequence in the first nucleic acid strand or may include a nucleotide sequence complementary to a part of a sequence complementary to the target sequence.

The second nucleic acid strand contains or consists of a complementary region complementary to all or a part of the first nucleic acid strand.

The length of the nucleotide sequence complementary to the first nucleic acid strand in the second nucleic acid strand may be generally at least 2-nucleotides long, at least 3-nucleotides long, at least 4-nucleotides long, at least 5-nucleotides long, at least 6-nucleotides long, at least 7-nucleotides long, at least 8-nucleotides long, at least 9-nucleotides long, at least 10-nucleotides long, at least 11-nucleotides long, at least 12-nucleotides long, or at least 13-nucleotides long, but it is not particularly limited. The complementary region in the second nucleic acid strand may have not more than 35-nucleotides long, not more than 30-nucleotides long, not more than 25-nucleotides long, not more than 24-nucleotides long, not more than 23-nucleotides long, not more than 22-nucleotides long, not more than 21-nucleotides long, not more than 20-nucleotides long, not more than 19-nucleotides long, not more than 18-nucleotides long, not more than 17-nucleotides long, or not more than 16-nucleotides long. In one embodiment, the complementary region in the second nucleic acid strand has 9- to 35-nucleotides long, 10- to 35-nucleotides long, 11- to 35-nucleotides long, 12- to 35-nucleotides long, 12- to 30-nucleotides long, 12- to 25-nucleotides long, or 12-to 22-nucleotides long. In one embodiment, the complementary region in the second nucleic acid strand has 13- to 35-nucleotides long, 16- to 30-nucleotides long, 18- to 30-nucleotides long, 20- to 30-nucleotides long, or 20- to 25-nucleotides long. In one embodiment, the complementary region in the second nucleic acid strand has 4- to 35-nucleotides long, 4- to 30-nucleotides long, 4- to 25-nucleotides long, 8- to 20-nucleotides long, 8- to 16-nucleotides long, 8- to 12-nucleotides long, or 8- to 10-nucleotides long.

While the second nucleic acid strand is not particularly limited, it may have at least 5-nucleotides long, at least 6-nucleotides long, at least 7-nucleotides long, at least 8-nucleotides long, at least 9-nucleotides long, at least 10-nucleotides long, at least 11-nucleotides long, at least 12-nucleotides long, or at least 13-nucleotides long. The second nucleic acid strand may have not more than 50-nucleotides long, not more than 45-nucleotides long, not more than 40-nucleotides long, not more than 35-nucleotides long, not more than 30-nucleotides long, not more than 28-nucleotides long, not more than 26-nucleotides long, not more than 24-nucleotides long, not more than 22-nucleotides long, not more than 20-nucleotides long, not more than 18-nucleotides long, not more than 16-nucleotides long, not more than 14-nucleotides long, not more than 12-nucleotides long, not more than 10-nucleotides long, or not more than 9-nucleotides long. The second nucleic acid strand may have for example 9- to 50-nucleotides long, 10- to 40-nucleotides long, 11- to 35-nucleotides long, 12- to 35-nucleotides long, or 12- to 30-nucleotides long, 14- to 30-nucleotides long, 16- to 30-nucleotides long, or 18- to 25-nucleotides long, or 4- to 25-nucleotids long, 8- to 20-nucleotides long, 7- to 20-nucleotides long, 8- to 16-nucleotides long, 8- to 12-nucleotides long, or 8- to 10-nucleotides long. In one embodiment, the second nucleic acid strand has 9- to 35-nucleotides long, 10- to 35-nucleotides long, 11- to 35-nucleotides long, 12- to 35-nucleotides long, 12- to 30-nucleotides long, 12- to 25-nucleotides long, or 12-to 22-nucleotides long. In one embodiment, the second nucleic acid strand has 13- to 35-nucleotides long, 16- to 30-nucleotides long, 18- to 30-nucleotides long, 20- to 30-nucleotides long, or 20- to 25-nucleotides long.

In one embodiment, the complementary region in the second nucleic acid strand may be complementary to at least a part of the antisense oligonucleotide region in the first nucleic acid strand. The complementary region in the second nucleic acid strand may be complementary to the entire antisense oligonucleotide region in the first nucleic acid strand. The complementary region in the second nucleic acid strand may be complementary to other parts, in addition to the antisense oligonucleotide region in the first nucleic acid strand. As an example of this embodiment, a heteroduplex oligonucleotide (HDO) disclosed in WO 2013/089283, Nishina K, et. al., Nature Communication, 2015, 6:7969, or Asami Y, et al., Drug Discoveries &Therapeutics. 2016; 10(5):256-262 can be mentioned.

In one embodiment, the second nucleic acid strand may include at least 4 consecutive ribonucleosides complementary to the antisense oligonucleotide region in the first nucleic acid strand.

In one embodiment, the second nucleic acid strand may include at least 4 consecutive ribonucleosides, deoxyribonucleosides and/or modified nucleosides, that are complementary to the antisense oligonucleotide region in the first nucleic acid strand. Ribonucleosides and/or deoxyribonucleosides are preferred. Ribonucleoside is more preferred. At least 4 consecutive ribonucleosides and/or deoxyribonucleosides in the second nucleic acid strand are preferably linked by naturally occurring internucleoside bonds, namely, phosphodiester bonds.

In the second nucleic acid strand, all nucleosides may be composed of ribonucleosides, deoxyribonucleosides and/or modified nucleosides. All nucleosides in the second nucleic acid strand may be composed of deoxyribonucleosides and/or modified nucleosides, and they may be free of ribonucleoside. In one embodiment, all nucleosides in the second nucleic acid strand may be composed of deoxyribonucleosides and/or modified nucleosides.

At least 1, at least 2, at least 3, or at least 4 nucleosides from the terminal (5'-terminal, 3'-terminal, or both terminals) of the second nucleic acid strand may be modified nucleosides. Modified nucleoside may be 2'-modified nucleoside (e.g., 2'-O-methylnucleoside) and/or bridged nucleoside (e.g., 2',4'-BNA^{NC} nucleoside (e.g., 2',4'-BNA^{NC}[N-H]nucleoside, 2',4'-BNA^{NC}[N-Me]nucleoside)), preferably 2'-modified nucleoside (e.g., 2'-O-methylnucleoside) and/or 2',4'-BNA^{NC} nucleoside (e.g., 2',4'-BNA^{NC}[N-H]nucleoside, 2',4'-BNA^{NC}[N-Me]nucleoside, further preferably 2'-O-methylnucleoside and/or 2',4'-BNA^{NC}[N-Me]nucleoside. Modified nucleoside may contain a modified base, and is preferably 5-methylcytosine.

The second nucleic acid strand may be composed of modified nucleosides of 2 to 7 bases in length or 3 to 5 bases in length from the 5'-terminal (e.g., 2'-modified nucleoside (e.g., 2'-O-methylnucleoside), bridged nucleoside (e.g., 2',4'-BNA^{NC} nucleoside (e.g., 2',4'-BNA^{NC}[N-H] nucleoside, 2',4'-BNA^{NC}[N-Me] nucleoside))), ribonucleosides or deoxyribonucleosides of 4 to 15 bases in length or 8 to 12 bases in length (optionally linked by a modified internucleoside bond), and modified nucleosides of 2 to 7 bases in length or 3 to 5 bases in length (e.g., 2'-modified nucleoside (e.g., 2'-O-methylnucleoside), bridged nucleoside (e.g., 2',4'-BNA^{NC} nucleoside (e.g., 2',4'-BNA^{NC}[N-H] nucleoside, 2',4'-BNA^{NC}[N-Me] nucleoside))).

In a preferred embodiment, the compound containing an oligonucleotide which consisting of a double-stranded oligonucleotide of the present invention includes the following nucleotide sequence as a sequence complementary to the antisense oligonucleotide region of the double-stranded oligonucleotide targeting exon 53 of human dystrophin gene (sequence in the 2nd nucleic acid strand).
GAACACCTTCAGAACCGGAGGCAAC (SEQ ID NO: 707)
GAACACCTTCAGAACC (SEQ ID NO: 708)
GAACACCTTCAGAACCGGAGG (SEQ ID NO: 709)
CCTTCAGAACCGGAGG (SEQ ID NO: 710)
CAGAACCGGAGGCAAC (SEQ ID NO: 711)
GAACACCTAAAUCAGAACC (SEQ ID NO: 712)
GAACACAAATTCAGAAAAACGGAGG (SEQ ID NO: 713)
TCAGAACACCTTCAGAACC (SEQ ID NO: 714)
GAACACCUUCAGAACCGGAGG (SEQ ID NO: 715)

In each of the above-mentioned sequence complementary to the antisense oligonucleotide regions, base thymine (T) may be uracil (U). In addition, base cytosine (C) may be 5-methylcytosine.

In another embodiment, the compound containing an oligonucleotide which consisting of a double-stranded oligonucleotide of the present invention includes the following nucleotide sequence as a sequence complementary to the antisense oligonucleotide region of the double-stranded oligonucleotide targeting exon 23 of mouse dystrophin gene (sequence in the 2nd nucleic acid strand).
AUUUCAGGUAAGCCGAGGUUUGGCC (SEQ ID NO: 716)
AUUUCAGGUAAGCCGA (SEQ ID NO: 717)
AUUTCAGGTAAGCCGAGGTTTGGCC (SEQ ID NO: 718)
AUUUCAAAAGUAAGCCAAAAGGUUU (SEQ ID NO: 719)
AUUTCAGGTAAGCCGA (SEQ ID NO: 720)
ATTTCAGGTAAGCCGA (SEQ ID NO: 721)
ATTTCAGGTAAGCCGAGGTTTGGCC (SEQ ID NO: 722)

In each of the above-mentioned sequence complementary to the antisense oligonucleotide regions, base thymine (T) may be uracil (U). In addition, base cytosine (C) may be 5-methylcytosine.

When the compound containing an oligonucleotide of the present invention is a double-stranded oligonucleotide, the antisense strand of the present invention containing any of the aforementioned modified nucleoside residues can be used as the antisense strand (the first nucleic acid strand). It is preferably a totalmer, particularly preferably fully morpholino-modified oligonucleotide (preferably PMO, further preferably fully phosphorodimethylamidatemorpholino-modified oligonucleotide). The antisense strand (the first nucleic acid strand) preferably has antisense effects. In another embodiment, the antisense strand (the first nucleic acid strand) contains an antisense oligonucleotide region. In another embodiment, the antisense strand (the first nucleic acid strand) is an antisense oligonucleotide. When the compound containing an oligonucleotide of the present invention is a double-stranded oligonucleotide, it is preferably HDO.

In one embodiment, as the antisense strand of the present invention an antisense strand consisting of the following sequence can be mentioned.

As the ASO consisting of the nucleotide sequence shown in SEQ ID NO: 687 completely complementary to the 36th to 60th nucleotides sequence of human exon 53,
can be mentioned, and
as the ASO consisting of the nucleotide sequence shown in SEQ ID NO: 688 completely complementary to the 36th to 56th nucleotides sequence, 
[MoC] [MoC] [MoT] [MoC] [MoC] [MoG] [MoG] [MoT] [MoT] [MoC] [MoT] [MoG] [Mo
A] [MoA] [MoG] [MoG] [MoT] [MoG] [MoT] [MoT] [MoC] (SEQ ID NO: 688-1)
 can be mentioned.
In the above-mentioned sequences, "Mo" is PMO (preferably morpholinonucleic acid).

Alternatively, as the ASO consisting of the nucleotide sequence shown in SEQ ID NO: 706 completely complementary to the sequences of mouse exon 23,
can be mentioned.
In the above-mentioned sequence, "Mo" is PMO (preferably morpholinonucleic acid).

More preferably, as the ASO consisting of the nucleotide sequence shown in SEQ ID NO: 687 completely complementary to the 36th to 60th nucleotides sequence of human exon 53,
can be mentioned, and
as the ASO consisting of the nucleotide sequence shown in SEQ ID NO: 688 completely complementary to the 36th to 56th nucleotides sequence, can be mentioned.
In the above-mentioned sequences, "Mo" is PMO (preferably morpholinonucleic acid).

Particularly preferably, as the ASO consisting of the nucleotide sequence shown in SEQ ID NO: 687 completely complementary to the 36th to 60th nucleotides sequence of human exon 53, can be mentioned.

In another embodiment, particularly preferably, as the ASO consisting of the nucleotide sequence shown in SEQ ID NO: 688 completely complementary to the 36th to 56th nucleotides sequence, can be mentioned. In the above-mentioned sequences, "Mo" is PMO (preferably morpholinonucleic acid).

The sense strand (the second nucleic acid strand) is not particularly limited as long as it maintains the stability of double-stranded oligonucleotide until delivery to the target tissue, is cleaved by RNase H in a target cell (RNase H-dependent pathway), or is released in a target cell in an RNase H-independent manner (RNase H-independent pathway), and is suitable for releasing antisense strands.

In one preferred embodiment of the compound containing an oligonucleotide of the present invention, which consisting of a double-stranded oligonucleotide, the second nucleic acid strand includes at least one first exposed region and at least one protected region. Here, "the first exposed region" means a region consisting of one or 2 to 3 consecutive sugar-unmodified ribonucleosides linked by an internucleoside bond, which is complementary to a part of the first nucleic acid strand. In addition, the "protected region" means a region consisting of one of (a) deoxyribonucleoside, (b) sugar-modified nucleoside and/or (c) nucleoside having modified internucleoside bond on the 3'-side, or two or more of said (a) to (c) linked by an internucleoside bond. In another embodiment, "the first exposed region" is a region consisting of one sugar-unmodified ribonucleoside and/or deoxyribonucleoside or 2 to 3 consecutive sugar-unmodified ribonucleosides and/or deoxyribonucleosides linked by an internucleoside bond, which is or are complementary to a part of said first nucleic acid strand.

In one embodiment, the complementary region in the second nucleic acid strand may be complementary to at least a part of the antisense oligonucleotide region in the first nucleic acid strand. the complementary region in the second nucleic acid strand may be complementary to all antisense oligonucleotide regions in the first nucleic acid strand. The complementary region in the second nucleic acid strand may be complementary to other parts in addition to the antisense oligonucleotide region in the first nucleic acid strand.

In one embodiment, the second nucleic acid strand contains at least one, for example, 1 to 10, 1 to 5, 1 to 4, 1 to 3, 1 to 2, or one, at least two, for example, 2 to 10, 2 to 5, 3 to 4, 2 to 3, or two, or at least three, for example, 3 to 10, 3 to 5, 3 to 4, or 3 of the first exposed regions.

The first exposed region may be completely complementary to a part of the first nucleic acid strand, or may have one or more mismatch bases. For example, the first exposed region may have 1 to 3, 1 to 2, or one mismatch base (non-complementary base) with respect to a part of the first nucleic acid strand.

The first exposed region consists of one sugar-unmodified ribonucleosides or 2 to 3 consecutive sugar-unmodified ribonucleosides linked by an internucleoside bond. In the present specification, the "sugar-unmodified ribonucleoside" means a ribonucleoside in which the sugar moiety of natural ribonucleoside does not include sugar-modification, for example, substitution at 2'-position and/or optional change. The first exposed region may contain at least one modified base and/or modified internucleoside bond. In another embodiment, the first exposed region consists of one sugar-unmodified ribonucleoside and/or deoxyribonucleoside or 2 to 3 consecutive sugar-unmodified ribonucleosides and/or deoxyribonucleosides linked by an internucleoside bond.

In one embodiment, the sugar-unmodified ribonucleoside in the first exposed region contain natural ribonucleoside, for example, all sugar-unmodified ribonucleosides are natural ribonucleosides, or a part thereof, for example, one or two sugar-unmodified ribonucleosides are natural ribonucleosides.

In one embodiment, the first exposed region consists of two or three consecutive sugar-unmodified ribonucleosides linked by an internucleoside bond. In one embodiment, the first exposed region consists of three consecutive sugar-unmodified ribonucleosides linked by an internucleoside bond. This embodiment can have an effect that the cleavage activity by RNase H is maintained high, as compared with the first exposed region consisting of 1 or 2 consecutive sugar-unmodified ribonucleosides. This embodiment can afford an effect of maintaining and improving the activity in RNase H-dependent and/or independent pathways, as compared with the center region consisting of sugar-unmodified ribonucleosides.

In one embodiment, the first exposed region contains a nucleoside containing a modified or unmodified purine base. This embodiment can have an effect of low cleavage activity by RNase A while maintaining cleavage activity by RNase H, as compared with the first exposed region fee of a nucleoside containing a purine base (e.g., composed of nucleoside containing pyrimidine base). This embodiment can have an effect of maintaining and improving the activity in RNase H-dependent and/or independent pathways, as compared with the first exposed region fee of a nucleoside containing a purine base (e.g., composed of nucleoside containing pyrimidine base).

In one embodiment, the second nucleic acid strand contains at least one, for example, 1 to 10, 1 to 5, 1 to 4, 1 to 3, 1 to 2, or one, at least two, for example, 2 to 10, 2 to 5, 3 to 4, 2 to 3, or two, or at least three, for example, 3 to 10, 3 to 5, 3 to 4, or three protected regions.

In the present specification, the "protected region" refers to a region that is resistant to cleavage by a nuclease. As used herein, nuclease is not limited. As one embodiment of the nuclease, RNase A, RNase H, and the like can be mentioned. The protected region may be, for example, a region resistant to RNase A, a region resistant to RNase H, or a region resistant to both RNase A and RNase H. Furthermore, resistance to cleavage by nucleases does not need to be complete, but only needs to be increased as compared with a region that has the same base sequence and contains unmodified ribonucleoside.

The protected region consists of one of (a) deoxyribonucleoside, (b) sugar-modified nucleoside, and/or (c) nucleoside having a modified internucleoside bond on the 3'-side, or two or more of said (a) to (c) linked by an internucleoside bond. The protected region may contain at least one modified base and/or modified internucleoside bond. The deoxyribonucleoside and/or sugar-modified nucleoside of the protected region are/is preferably deoxyribonucleoside, 2'-sugar-modified nucleoside (e.g., nucleoside containing 2'-O-methyl group), bridged nucleoside (e.g., LNA nucleoside), and/or cationic nucleoside, more preferably deoxyribonucleoside, 2'-sugar-modified nucleoside (e.g., nucleoside containing 2'-O-methyl group), and/or bridged nucleoside (e.g., LNA nucleoside), further preferably deoxyribonucleoside and/or 2'-sugar-modified nucleoside (e.g., nucleoside containing 2'-O-methyl group), particularly preferably deoxyribonucleoside and/or nucleoside containing 2'-O-methyl group. The nucleoside having a modified internucleoside bond on the 3'-side of the protected region are/is preferably deoxyribonucleoside, ribonucleoside, 2'-sugar-modified nucleoside (e.g., nucleoside containing 2'-O-methyl group), bridged nucleoside (e.g., LNA nucleoside), and/or cationic nucleoside, more preferably, deoxyribonucleoside, ribonucleoside, 2'-sugar-modified nucleoside (e.g., nucleoside containing 2'-O-methyl group) and/or bridged nucleoside (e.g., LNA nucleoside), further preferably, deoxyribonucleoside, ribonucleoside and/or 2'-sugar-modified nucleoside (e.g., nucleoside containing 2'-O-methyl group), further more preferably, deoxyribonucleoside, ribonucleoside and/or nucleoside containing 2'-O-methyl group, particularly preferably, ribonucleoside. The nucleoside having a modified internucleoside bond on the 3'-side of the protected region is preferably a nucleoside having, on the 3'-side, a phosphorothioate bond, a phosphorothioate bond, an internucleoside bond containing a guanidine moiety (preferably, TMG moiety) substituted by 1 to 4 C₁₋₆ alkyl groups (preferably, partial structure represented by the above-mentioned formula (III)), and/or an internucleoside bond containing a cyclic guanidine moiety (preferably, partial structure represented by the above-mentioned formula (II)), further preferably a nucleoside having a phosphorothioate bond on the 3'-side. The modified internucleoside bond on the 3'-side may be chiral-controlled in the Rp configuration or Sp configuration. In the present specification, the nucleoside having a modified internucleoside bond on the 3'-side means a nucleoside having a modified internucleoside bond at least on the 3'-side and optionally has a modified internucleoside bond also on the 5'-side.

The protected region may be completely complementary to a part of the first nucleic acid strand, or may have one or more mismatch bases. For example, the protected region may have 1 to 3, 1 to 2, or 1 mismatch base (non-complementary base) with respect to a part of the first nucleic acid strand.

In one embodiment, the protected region includes (a) deoxyribonucleoside, (b) sugar-modified nucleoside, and/or (c) nucleoside having a modified internucleoside bond on the 3'-side, each having a pyrimidine base. As used herein, the pyrimidine base may be modified or unmodified. For example, at least one protected region, or all protected regions may include (a) deoxyribonucleoside, (b) sugar-modified nucleoside, and/or (c) nucleoside having a modified internucleoside bond on the 3'-side, each having a modified or unmodified pyrimidine base. This embodiment may achieve an effect that the cleavage activity of RNase A against the second nucleic acid strand is suppressed to a lower level than when a nucleoside containing a pyrimidine base is included in a sugar-unmodified region (e.g., the first exposed region or the second exposed region). This embodiment may achieve an effect that the activity in the RNase H dependent and/or independent pathway is maintained and improved, compared to when a nucleoside containing a pyrimidine base is included in a sugar-unmodified region (e.g., the first exposed region or the second exposed region).

In one embodiment, the sugar-modified nucleoside in the protected region is a sugar-modified ribonucleoside.

In one embodiment, the second nucleic acid strand consists of the first exposed region and a protected region. In a further embodiment, the second nucleic acid strand includes the first exposed region and a protected region that are alternately disposed. In a further embodiment, at least one protected region or all protected regions include (a) deoxyribonucleoside, (b) sugar-modified nucleoside, and/or (c) nucleoside having a modified internucleoside bond on the 3'-side, each having a modified or unmodified pyrimidine base.

In one embodiment, the second nucleic acid strand contains at least one, for example, 1 to 5, 1 to 4, 1 to 3, 1 to 2, or 1, or at least two second exposed regions complementary to a part of the first nucleic acid strand.

The second exposed region consists of 4 or more consecutive sugar-unmodified ribonucleosides linked by an internucleoside bond, wherein the base of the sugar-unmodified ribonucleoside in the second exposed region contains a purine base. Here, the purine base may be modified or unmodified. The number of the sugar-unmodified ribonucleosides constituting the second exposed region may be, for example, 4 to 10, 4 to 9, 4 to 8, 4 to 7, 4 to 6, 4 to 5, or 4. The second exposed region may contain at least one modified base and/or modified internucleoside bond. In another embodiment, the second exposed region consists of 4 or more consecutive sugar-unmodified ribonucleosides and/or deoxyribonucleosides linked by an internucleoside bond, wherein the base of the sugar-unmodified ribonucleoside and/or deoxyribonucleoside in the second exposed region contains a purine base.

The second exposed region may be completely complementary to a part of the first nucleic acid strand, or may have one or more mismatch bases. For example, the second exposed region may have 1 to 3, 1 to 2, or one mismatch base (non-complementary base) with respect to a part of the first nucleic acid strand.

In one embodiment, the sugar-unmodified ribonucleoside in the second exposed region contains natural ribonucleoside. For example, all sugar-unmodified ribonucleosides are natural ribonucleosides, or a part thereof, for example, one or two sugar-unmodified ribonucleosides, are natural ribonucleosides.

In one embodiment, the second nucleic acid strand contains at least one, for example, 1 to 10, 1 to 5, 1 to 4, 1 to 3, 1 to 2, or 1, at least two, for example, 2 to 10, 2 to 5, 3 to 4, 2 to 3, or 2, or at least three, for example, 3 to 10, 3 to 5, 3 to 4, or 3, of the second exposed regions.

In one embodiment, the second nucleic acid strand contains, in total, at least one, for example, 1 to 10, 1 to 6, 1 to 5, 1 to 4, 1 to 3, 1 to 2, or 1, at least two, for example, 2 to 10, 2 to 5, 3 to 4, 2 to 3, or 2, or at least three, for example, 3 to 10, 3 to 5, 3 to 4, or 3, of the first exposed regions and/or the second exposed regions.

In one embodiment, the second nucleic acid strand consists of the first exposed region, the second exposed region and a protected region. In a further embodiment, the second nucleic acid strand includes the first exposed region or the second exposed region, and a protected region that are alternately disposed. In a further embodiment, at least one protected region or all protected regions include (a) deoxyribonucleoside, (b) sugar-modified nucleoside, and/or (c) nucleoside having a modified internucleoside bond on the 3'-side, each having a modified or unmodified pyrimidine base.

In one embodiment, the second nucleic acid strand consists of the second exposed region and a protected region. In a further embodiment, the second nucleic acid strand includes the second exposed region and a protected region that are alternately disposed. In a further embodiment, at least one protected region or all protected regions include (a) deoxyribonucleoside, (b) sugar-modified nucleoside, and/or (c) nucleoside having a modified internucleoside bond on the 3'-side, each having a modified or unmodified pyrimidine base.

In one embodiment, at least one protected region of the second nucleic acid strand is linked to the first exposed region and/or the second exposed region at the 5'-terminal and the 3'-terminal by an internucleoside bond.

In one embodiment, the first exposed region and/or the second exposed region include(s) at least one, for example, 1 or 2, modified internucleoside bond, for example, a phosphorothioate bond, for example, a phosphorothioate bond chiral-controlled in the Rp configuration or Sp configuration, an internucleoside bond containing a guanidine moiety (e.g., TMG moiety) substituted by 1 to 4 C₁₋₆ alkyl groups (e.g., partial structure represented by the formula (III)), and/or an internucleoside bond containing a cyclic guanidine moiety (e.g., partial structure represented by the formula (II)).

In one embodiment, the second nucleic acid strand contains or consists of (1) the aforementioned first exposed region, and/or (2) the aforementioned second exposed region, and (3) (a) a deoxyribonucleoside and/or (b) a sugar-modified nucleoside, for example, a sugar-modified ribonucleoside, linked by a modified or unmodified internucleoside bond.

In one embodiment, the second nucleic acid strand further contains the following sugar-unmodified terminal region at the 5'-terminal and/or the 3'-terminal. In one embodiment, the length of the 5'-terminal and/or the 3'-terminal sugar-unmodified terminal region is independently generally 1 to 10 bases in length, 1 to 7 bases in length, 2 to 5 bases in length, or 2 to 3 bases in length.

In one embodiment, the second nucleic acid strand contains or consists of the aforementioned first exposed region and/or the second exposed region, and a sugar-modified ribonucleoside (and optionally a sugar-unmodified terminal region), linked by a modified or unmodified internucleoside bond.

In one embodiment, the second nucleic acid strand contains sugar-unmodified terminal region containing at least one, for example, 1 to 6, 1 to 5, 1 to 4, 1 to 3, 1 to 2, or 1, or at least two sugar-unmodified ribonucleosides at the 5'terminal and/or the 3'-terminal. The toxicity of the nucleic acid complex tends to increase as the number of sugar modifications increases. Therefore, by making the terminal region sugar-unmodified, the number of sugar-modified nucleic acids in the second nucleic acid strand decreases and the toxicity may decrease. The sugar-unmodified terminal region may contain a modified base and/or a modified internucleoside bond. In one embodiment, the sugar-unmodified terminal region is complementary to a part of the first nucleic acid strand. The sugar-unmodified terminal region may be completely complementary to the first nucleic acid strand, or may have one or more mismatch bases. For example, the second exposed region may have 1 to 3, 1 to 2, or 1, mismatch base (non-complementary base) with respect to a part of the first nucleic acid strand. In one embodiment, the sugar-unmodified ribonucleoside in the sugar-unmodified terminal region contains natural ribonucleoside. For example, all sugar-unmodified ribonucleosides are natural ribonucleosides, or a part thereof, for example, 1 to 5, 1 to 4, 1 to 3, or 1, or two sugar-unmodified ribonucleosides are natural ribonucleosides. In one embodiment, the sugar-unmodified terminal region contains a nucleoside containing a modified or unmodified purine base and/or a modified or unmodified pyrimidine base. In one embodiment, the sugar-unmodified terminal region contains a nucleoside containing a modified or unmodified purine base.

In one embodiment, the second nucleic acid strand consists of (1) at least one first exposed region and/or at least one second exposed region, (2) at least one protected region, and (3) sugar-unmodified terminal regions at the 5'-terminal and the 3'-terminal, linked by a modified or unmodified internucleoside bond. In one embodiment, the second nucleic acid strand consists of (1) 1 to 10 first exposed regions and/or 1 to 10 second exposed regions, (2) 1 to 10 protected regions, and (3) sugar-unmodified terminal regions at the 5'-terminal and the 3'-terminal, linked by a modified or unmodified internucleoside bond. In one embodiment, the second nucleic acid strand consists of (1) 1 to 5 first exposed regions and/or 1 to 5 second exposed regions, (2) 1 to 5 protected regions, and (3) sugar-unmodified terminal regions at the 5'-terminal and the 3'-terminal, linked by a modified or unmodified internucleoside bond. In one embodiment, the second nucleic acid strand consists of (1) 1 to 3 first exposed regions and/or 1 to 3 second exposed regions, (2) 1 to 3 protected regions, and (3) sugar-unmodified terminal regions at the 5'-terminal and the 3'-terminal, linked by a modified or unmodified internucleoside bond. In one embodiment, the second nucleic acid strand consists of (1) 2 to 10 first exposed regions and/or the second exposed region, (2) 2 to 10 protected regions, and (3) sugar-unmodified terminal regions at the 5'-terminal and the 3'-terminal, linked by a modified or unmodified internucleoside bond. In one embodiment, the second nucleic acid strand consists of (1) 2 to 6 first exposed regions and/or the second exposed region, (2) 2 to 6 protected regions, and (3) sugar-unmodified terminal regions at the 5'-terminal and the 3'-terminal, linked by a modified or unmodified internucleoside bond. In one embodiment, the second nucleic acid strand consists of (1) 2 to 5 first exposed regions and/or the second exposed region, (2) 2 to 5 protected regions, and (3) sugar-unmodified terminal regions at the 5'-terminal and the 3'-terminal, linked by a modified or unmodified internucleoside bond. In one embodiment, the second nucleic acid strand consists of (1) 2 to 4 first exposed regions and/or the second exposed region, (2) 2 to 4 protected regions, and (3) sugar-unmodified terminal regions at the 5'-terminal and the 3'-terminal, linked by a modified or unmodified internucleoside bond. In one embodiment, the second nucleic acid strand consists of (1) 2 to 3 first exposed regions and/or the second exposed region, (2) 2 to 3 protected regions, and (3) sugar-unmodified terminal regions at the 5'-terminal and the 3'-terminal, linked by a modified or unmodified internucleoside bond. In one embodiment, the second nucleic acid strand consists of (1) 3 to 10 first exposed regions and/or the second exposed region, (2) 3 to 10 protected regions, and (3) sugar-unmodified terminal regions at the 5'-terminal and the 3'-terminal, linked by a modified or unmodified internucleoside bond. In one embodiment, the second nucleic acid strand consists of (1) 3 to 6 first exposed regions and/or the second exposed region, (2) 3 to 6 protected regions, and (3) sugar-unmodified terminal regions at the 5'-terminal and the 3'-terminal, linked by a modified or unmodified internucleoside bond. In one embodiment, the second nucleic acid strand consists of (1) 3 to 5 first exposed region and/or the second exposed region, (2) 3 to 5 protected regions, and (3) sugar-unmodified terminal regions at the 5'-terminal and the 3'-terminal, linked by a modified or unmodified internucleoside bond. In one embodiment, the second nucleic acid strand consists of (1) 3 to 4 first exposed regions and/or the second exposed region, (2) 3 to 4 protected regions, and (3) sugar-unmodified terminal regions at the 5'-terminal and the 3'-terminal, linked by a modified or unmodified internucleoside bond. In this embodiment, the second nucleic acid strand has (a) the first exposed region or the second exposed region, and (b) a protected region that are alternately disposed.

In one embodiment, the second nucleic acid strand contains, at the 5'-terminal and/or the 3'-terminal, a sugar-modified terminal region containing at least one deoxyribonucleoside, sugar-modified nucleoside, and/or a nucleoside having a modified internucleoside bond on the 3'-side. In one embodiment, the second nucleic acid strand contains, at the 5'-terminal and/or the 3'-terminal, a sugar-modified terminal region containing at least one deoxyribonucleoside and/or a sugar-modified nucleoside.

In one embodiment, the sugar-modified terminal region is complementary to a part of the first nucleic acid strand. The sugar-modified terminal region may be completely complementary to a part of the first nucleic acid strand, or may have one or more mismatch bases. For example, the second exposed region may have 1 to 3, 1 to 2, or 1, mismatch base (non-complementary base) with respect to a part of the first nucleic acid strand.

In one embodiment, the sugar-modified terminal region contains deoxyribonucleoside containing a pyrimidine base, sugar-modified nucleoside, and/or a nucleoside having a modified internucleoside bond on the 3'-side. Here, the pyrimidine base may be modified or unmodified. In one embodiment, the sugar-modified terminal region contains deoxyribonucleoside containing a purine base, sugar-modified nucleoside, and/or a nucleoside having a modified internucleoside bond on the 3'-side. In one embodiment, the sugar-modified terminal region contains deoxyribonucleoside containing a purine base and/or sugar-modified nucleoside. Here, the purine base may be modified or unmodified.

In one embodiment, the second nucleic acid strand contains or consists of the aforementioned first exposed region, the second exposed region, a protected region, and/or a sugar-modified terminal region, linked by a modified or unmodified internucleoside bond. In one embodiment, the length of the 5'-terminal and/or the 3'-terminal sugar-modified terminal region is independently generally 1 to 10 in length, 1 to 7 in length, 2 to 5 in length, or 2 to 3 in length.

In one embodiment of the sugar-modified terminal region, all nucleosides are deoxyribonucleosides, sugar-modified nucleosides, and/or nucleosides having a modified internucleoside bond on the 3'-side (preferably, all nucleosides are deoxyribonucleosides and/or sugar-modified nucleosides), or part thereof, for example, one, 1 to 2, 1 to 3, 1 to 4, 1 to 5, 1 to 6, or 1 to 7, are deoxyribonucleosides, sugar-modified nucleosides, and/or nucleosides having a modified internucleoside bond on the 3'-side (preferably, all nucleosides are deoxyribonucleosides and/or sugar-modified nucleosides). In one embodiment, the nucleosides of the sugar-modified terminal region contain, at any proportion, deoxyribonucleoside, sugar-modified nucleoside, and/or nucleoside having a modified internucleoside bond on the 3'-side (preferably, all nucleosides are deoxyribonucleoside and/or sugar-modified nucleoside). For example, not less than 10%, not less than 30%, not less than 50%, not less than 70%, not less than 80%, or not less than 90% are deoxyribonucleosides, sugar-modified nucleosides, and/or nucleosides having a modified internucleoside bond on the 3'-side (preferably, all nucleosides are deoxyribonucleosides and/or sugar-modified nucleosides). The deoxyribonucleoside and/or sugar-modified nucleoside are/is preferably deoxyribonucleoside, 2'-sugar-modified nucleoside (e.g., 2'-O-methylnucleoside), bridged nucleoside (e.g., LNA nucleoside) and/or cationic nucleoside, more preferably deoxyribonucleoside, 2'-sugar-modified nucleoside (e.g., 2'-O-methylnucleoside), and/or bridged nucleoside (e.g., LNA nucleoside), further preferably deoxyribonucleoside, 2'-O-methylnucleoside, and/or LNA nucleoside, further more preferably deoxyribonucleoside and/or LNA nucleoside, particularly preferably LNA nucleoside. The nucleoside having a modified internucleoside bond on the 3'-side is preferably a nucleoside having a phosphorothioate bond on the 3'-side. In another embodiment, the deoxyribonucleoside and/or sugar-modified nucleoside are/is preferably deoxyribonucleoside, 2'-sugar-modified nucleoside (e.g., 2'-O-methyl nucleoside), bridged nucleoside (e.g., 2',4'-BNA^{NC} nucleoside (e.g., 2',4'-BNA^{NC}[N-H] nucleoside, 2',4'-BNA^{NC}[N-Me] nucleoside)) and/or cationic nucleoside, more preferably, deoxyribonucleoside, 2'-sugar-modified nucleoside (e.g., 2'-O-methylnucleoside), and/or bridged nucleoside (e.g., 2',4'-BNA^{NC} nucleoside (e.g., 2',4'-BNA^{NC}[N-H] nucleoside, 2',4'-BNA^{NC}[N-Me] nucleoside)), more preferably, deoxyribonucleoside, 2'-sugar-modified nucleoside (e.g., 2'-O-methylnucleoside), and/or 2',4'-BNA^{NC} nucleoside (e.g., 2',4'-BNA^{NC}[N-H] nucleoside, 2',4'-BNA^{NC}[N-Me] nucleoside), further preferably deoxyribonucleoside, 2'-O-methylnucleoside, and/or 2',4'-BNA^{NC}[N-Me] nucleoside, further more preferably deoxyribonucleoside and/or 2'-O-methyl nucleoside, particularly preferably deoxyribonucleoside. In another embodiment, 2'-O-methylnucleoside is particularly preferred. In another embodiment, 2',4'-BNA^{NC}[N-Me] nucleoside is particularly preferred.

In one embodiment, the second nucleic acid strand consists of (1) at least one first exposed region and/or at least one second exposed region, (2) at least one protected region, and (3) sugar-modified terminal regions at the 5'-terminal and the 3'-terminal, linked by a modified or unmodified internucleoside bond. In one embodiment, the second nucleic acid strand consists of (1) 1 to 10 first exposed regions and/or 1 to 10 second exposed regions, (2) 1 to 10 protected regions, and (3) sugar-modified terminal regions at the 5'-terminal and the 3'-terminal, linked by a modified or unmodified internucleoside bond. In one embodiment, the second nucleic acid strand consists of (1) 1 to 5 first exposed regions and/or 1 to 5 second exposed regions, (2) 1 to 5 protected regions, and (3) sugar-modified terminal regions at the 5'-terminal and the 3'-terminal, linked by a modified or unmodified internucleoside bond. In one embodiment, the second nucleic acid strand consists of (1) 1 to 4 first exposed regions and/or 1 to 4 second exposed regions, (2) 1 to 4 protected regions, and (3) sugar-modified terminal regions at the 5'-terminal and the 3'-terminal, linked by a modified or unmodified internucleoside bond. In one embodiment, the second nucleic acid strand consists of (1) 1 to 3 the first exposed regions and/or 1 to 3 second exposed regions, (2) 1 to 3 protected regions, and (3) sugar-modified terminal regions at the 5'-terminal and the 3'-terminal, linked by a modified or unmodified internucleoside bond. In one embodiment, the second nucleic acid strand consists of (1) 2 to 5 first exposed regions and/or 1 to 5 second exposed regions, (2) 2 to 5 protected regions, and (3) sugar-modified terminal regions at the 5'-terminal and the 3'-terminal, linked by a modified or unmodified internucleoside bond. In one embodiment, the second nucleic acid strand consists of (1) 2 to 4 first exposed regions and/or 1 to 4 the second exposed regions, (2) 2 to 4 protected regions, and (3) sugar-modified terminal regions at the 5'-terminal and the 3'-terminal, linked by a modified or unmodified internucleoside bond. In one embodiment, the second nucleic acid strand consists of (1) 2 to 3 first exposed regions and/or 1 to 3 second exposed regions, (2) 2 to 3 protected regions, and (3) sugar-modified terminal regions at the 5'-terminal and the 3'-terminal, linked by a modified or unmodified internucleoside bond. In one embodiment, the second nucleic acid strand consists of (1) 3 to 5 first exposed regions and/or 1 to 5 second exposed regions, (2) 3 to 5 protected regions, and (3) sugar-modified terminal regions at the 5'-terminal and the 3'-terminal, linked by a modified or unmodified internucleoside bond. In one embodiment, the second nucleic acid strand consists of (1) 3 to 4 first exposed regions and/or 1 to 4 second exposed regions, (2) 3 to 4 protected regions, and (3) sugar-modified terminal regions at the 5'-terminal and the 3'-terminal, linked by a modified or unmodified internucleoside bond. In one embodiment, the second nucleic acid strand consists of, (1) 2 to 10 first exposed regions and/or the second exposed region, (2) 2 to 10 protected regions, and (3) sugar-unmodified terminal regions at the 5'-terminal and the 3'-terminal, linked by a modified or unmodified internucleoside bond. In one embodiment, the second nucleic acid strand consists of (1) 2 to 6 first exposed regions and/or the second exposed region, (2) 2 to 6 protected regions, and (3) sugar-unmodified terminal regions at the 5'-terminal and the 3'-terminal, linked by a modified or unmodified internucleoside bond. In one embodiment, the second nucleic acid strand consists of (1) 2 to 5 first exposed regions and/or the second exposed region, (2) 2 to 5 protected regions, and (3) sugar-unmodified terminal regions at the 5'-terminal and the 3'-terminal, linked by a modified or unmodified internucleoside bond. In one embodiment, the second nucleic acid strand consists of, (1) 2 to 4 first exposed regions and/or the second exposed region, (2) 2 to 4 protected regions, and (3) sugar-unmodified terminal regions at the 5'-terminal and the 3'-terminal, linked by a modified or unmodified internucleoside bond. In one embodiment, the second nucleic acid strand consists of (1) 2 to 3 first exposed regions and/or the second exposed region, (2) 2 to 3 protected regions, and (3) sugar-unmodified terminal regions at the 5'-terminal and the 3'-terminal, linked by a modified or unmodified internucleoside bond. In one embodiment, the second nucleic acid strand consists of (1) 3 to 10 first exposed regions and/or the second exposed region, (2) 3 to 10 protected regions, and (3) sugar-unmodified terminal regions at the 5'-terminal and the 3'-terminal, linked by a modified or unmodified internucleoside bond. In one embodiment, the second nucleic acid strand consists of, (1) 3 to 6 first exposed regions and/or the second exposed region, (2) 3 to 6 protected regions, and (3) sugar-unmodified terminal regions at the 5'-terminal and the 3'-terminal, linked by a modified or unmodified internucleoside bond. In one embodiment, the second nucleic acid strand consists of (1) 3 to 5 first exposed regions and/or the second exposed region, (2) 3 to 5 protected regions, and (3) sugar-unmodified terminal regions at the 5'-terminal and the 3'-terminal, linked by a modified or unmodified internucleoside bond. In one embodiment, the second nucleic acid strand consists of (1) 3 to 4 first exposed regions and/or the second exposed region, (2) 3 to 4 protected regions, and (3) sugar-unmodified terminal regions at the 5'-terminal and the 3'-terminal, linked by a modified or unmodified internucleoside bond. In this embodiment, the second nucleic acid strand has (a) the first exposed region or the second exposed region, and (b) the protected region that are alternately disposed.

In one embodiment, a modified internucleoside bond of the second nucleic acid comprised a non-negatively charged (neutral or cationic) internucleoside bond existing in the neutral form or the cationic form at a certain pH (e.g., human physiological pH (about 7.4) or pH of delivery site (e.g., organella, cell, tissue, organism) as compared to when the modified internucleoside bond is in the anionic form (e.g., -O-P(O)(O⁻)-O- (anionic form of natural phosphate bond) or -O-P(O)(S⁻)-O-((anionic form of phosphorothioate bond)). In one embodiment, a modified internucleoside bond in the second nucleic acid strand comprises a neutral internucleoside bond. In one embodiment, a modified internucleoside bond in the second nucleic acid strand comprises a cationic internucleoside bond. In one embodiment, a non-negatively charged internucleoside bond (e.g., a neutral internucleoside bond) in the neutral form does not have a moiety with pKa less than 8, less than 9, less than 10, less than 11, less than 12, less than 13, or less than 14. In one embodiment, a non-negatively charged internucleoside bond is, for example, a methylphosphonate bond described in US-B-5,264,423 and US-B-5,286,717, a methylphosphotriester bond and an ethylphosphotriester bond disclosed in US-B-5,955,599, a methoxypropylphosphonate bond described in WO 2015/168172 , an internucleoside bond used for a self-neutralizing nucleic acid (ZON) described in WO 2016/081600 , or the like. In one embodiment, a non-negatively charged internucleoside bond comprises a triazole moiety or an alkyne moiety. In one embodiment, a non-negatively charged internucleoside bond comprises a cyclic guanidine moiety and/or a guanidine moiety substituted with 1 to 4 C₁₋₆ alkyl groups (preferably, a TMG moiety). In one embodiment, a modified internucleoside bond comprising a cyclic guanidine moiety has a partial structure represented by formula (II). In one embodiment, the guanidine moiety substituted with 1 to 4 C₁₋₆ alkyl groups has a partial structure represented by formula (III). In one embodiment, a neutral internucleoside bond comprising a cyclic guanidine moiety and/or a guanidine moiety substituted with 1 to 4 C₁₋₆ alkyl groups is chirally controlled. In one embodiment, the present disclosure relates to a composition comprising an oligonucleotide comprising at least one neutral internucleoside bond and at least one phosphorothioate internucleoside bond. Without wishing to be bound by any particular theory, at least in some cases, a neutral internucleotide bond can improve properties and/or activity as compared to comparable nucleic acids that do not comprise a neutral internucleotide bond; for example, it can improve delivery, resistance to exonuclease and endonuclease, cellular uptake, endosome escape, and/or nuclear uptake.

In one embodiment, the second nucleic acid strand may contain 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 35, 40, 45, 50, or more number of modified internucleoside bonds. In one embodiment, the first nucleic acid strand and/or the second nucleic acid strand may each contain the modified internucleoside bond by at least 5%, at least 10%, at least 20%, at least 30%, at least 40%, at least 50%, at least 60%, at least 70%, at least 80%, at least 90%, or more.

In one embodiment, the first exposed region, the second exposed region, the protected region, the sugar-unmodified terminal region, and/or the sugar-modified terminal region of the second nucleic acid strand may each contain 1, 1 to 2, 1 to 3, 1 to 4, 1 to 5, 1 to 6, 1 to 7, 1 to 8, 1 to 9, 1 to 10, 1 to 11, 1 to 12, 1 to 13, 1 to 14, 1 to 15, 1 to 16, 1 to 17, 1 to 18, 1 to 19, 1 to 20, 1 to 21, 1 to 22, modified internucleoside bonds. In one embodiment, the first exposed region, the second exposed region, the protected region, the sugar-unmodified terminal region, and/or the sugar-modified terminal region of the second nucleic acid strand may each contain modified internucleoside bonds by at least 5%, at least 10%, at least 20%, at least 30%, at least 40%, at least 50%, at least 60%, at least 70%, at least 80%, at least 90%, or more.

In one embodiment, the second nucleic acid strand may contain 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 35, 40, 45, 50, or more number of chirally controlled internucleoside bonds. In one embodiment, the second nucleic acid strand may each contain chirally controlled internucleoside bonds by at least 5%, at least 10%, at least 20%, at least 30%, at least 40%, at least 50%, at least 60%, at least 70%, at least 80%, at least 90%, or more.

In one embodiment, the first exposed region, the second exposed region, the protected region, the sugar-unmodified terminal region, and/or the sugar-modified terminal region of the second nucleic acid strand may contain each 1, 1 to 2, 1 to 3, 1 to 4, 1 to 5, 1 to 6, 1 to 7, 1 to 8, 1 to 9, 1 to 10, 1 to 11, 1 to 12, 1 to 13, 1 to 14, 1 to 15, 1 to 16, 1 to 17, 1 to 18, 1 to 19, 1 to 20, 1 to 21, 1 to 22, chirally controlled internucleoside bonds. In one embodiment, the first exposed region, the second exposed region, the protected region, the sugar-unmodified terminal region, and/or the sugar-modified terminal region of the second nucleic acid strand may each contain chirally controlled internucleoside bonds by at least 5%, at least 10%, at least 20%, at least 30%, at least 40%, at least 50%, at least 60%, at least 70%, at least 80%, at least 90%, or more.

In one embodiment, the second nucleic acid strand may contain 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 35, 40, 45, 50, or more number of non-negatively charged internucleoside bonds (preferably, neutral internucleoside bonds). In one embodiment, the first nucleic acid strand and/or the second nucleic acid strand may each contain non-negatively charged internucleoside bonds by at least 5%, at least 10%, at least 20%, at least 30%, at least 40%, at least 50%, at least 60%, at least 70%, at least 80%, at least 90%, or more.

In one embodiment, the first exposed region, the second exposed region, the protected region, the sugar-unmodified terminal region and/or the sugar-modified terminal region of the second nucleic acid strand may each contain 1, 1 to 2, 1 to 3, 1 to 4, 1 to 5, 1 to 6, 1 to 7, 1 to 8, 1 to 9, 1 to 10, 1 to 11, 1 to 12, 1 to 13, 1 to 14, 1 to 15, 1 to 16, 1 to 17, 1 to 18, 1 to 19, 1 to 20, 1 to 21, 1 to 22, non-negatively charged internucleoside bonds (preferably, neutral internucleoside bonds). In one embodiment, the first exposed region, the second exposed region, the protected region, the sugar-unmodified terminal region and/or the sugar-modified terminal region of the second nucleic acid strand may each contain non-negatively charged internucleoside bonds by at least 5%, at least 10%, at least 20%, at least 30%, at least 40%, at least 50%, at least 60%, at least 70%, at least 80%, at least 90%, or more.

In one embodiment, any of the above-mentioned regions (the first exposed region, the protected region, the second exposed region, the sugar-unmodified terminal region, and/or the sugar-modified terminal region) in the second nucleic acid strand include, in the inside region thereof or between two adjacent regions, at least one, for example, 1 or 2, modified internucleoside bond, for example, a phosphorothioate bond, for example, a phosphorothioate bond chirally controlled in the Rp configuration or Sp configuration, an internucleoside bond containing a guanidine moiety (e.g., TMG moiety) substituted by 1 to 4 C₁₋₆ alkyl groups (e.g., partial structure represented by the above-mentioned formula (III)), an internucleoside bond containing a guanidine moiety (e.g., TMG moiety) substituted by 1 to 4 C₁₋₆ alkyl groups (e.g., partial structure represented by the above-mentioned formula (III)) chirally controlled in the Rp configuration or Sp configuration and, an internucleoside bond including a cyclic guanidine moiety (e.g., partial structure represented by the above-mentioned formula (II)), and/or an internucleoside bond including a cyclic guanidine moiety (e.g., partial structure represented by the above-mentioned formula (II)) chirally controlled in the Rp configuration or Sp configuration.

At least one, at least 2, or at least 3, internucleoside bonds from the 5'-terminal of the second nucleic acid strand may be modified internucleoside bonds. At least one, at least 2, or at least 3, internucleoside bonds from the 3'-terminal of the second nucleic acid strand may be modified internucleoside bonds, for example, a phosphorothioate bond, an internucleoside bond containing a guanidine moiety (e.g., TMG moiety) substituted by 1 to 4 C₁₋₆ alkyl groups (e.g., partial structure represented by the above-mentioned formula (III)), and/or an internucleoside bond containing a cyclic guanidine moiety (e.g., partial structure represented by the above-mentioned formula (II)). The modified internucleoside bond may be chirally controlled in the Rp configuration or Sp configuration.

The second nucleic acid strand may not include a sugar-unmodified region other than the first exposed region and/or the second exposed region (and if present, sugar-unmodified terminal region). That is, a nucleic acid sugar in the region other than the first exposed region and/or the second exposed region (and if present, sugar-unmodified terminal region) is a modified sugar, for example, 2'-modified sugar.

In one embodiment, a base in a region other than the first exposed region and/or the second exposed region (and if present, sugar-unmodified terminal region) contains a modified and/or unmodified pyrimidine base. For example, all bases are modified and/or unmodified pyrimidine bases, a part thereof, for example, 1 to 20, 1 to 15, 1 to 10, 1 to 5, 1 to 2, or 1, thereof is a modified and/or unmodified pyrimidine base. The modified and/or unmodified pyrimidine bases are preferably cytosine, uracil, thymine, 5-methylcytosine, 5-fluorocytosine, 5-bromocytosine, 5-iodocytosine, N4-methylcytosine, and/or 2-thio-thymine, further preferably cytosine, uracil, thymine, and/or 5-methylcytosine, particularly preferably cytosine.

In one embodiment, bases in regions other than the first exposed region and/or the second exposed region (and if present, sugar-unmodified terminal region) contain, at any proportion, modified and/or unmodified pyrimidine bases. For example, not less than 10%, not less than 30%, not less than 50%, not less than 70%, not less than 80%, or not less than 90% are modified and/or unmodified pyrimidine bases. The modified and/or unmodified pyrimidine bases are/is preferably cytosine, uracil, thymine, 5-methylcytosine, 5-fluorocytosine, 5-bromocytosine, 5-iodocytosine, N4-methylcytosine, and/or 2-thio-thymine, further preferably cytosine, uracil, thymine, and/or 5-methylcytosine, particularly preferably cytosine.

In one embodiment, the base in the first exposed region includes a purine base, for example, all bases are modified and/or unmodified purine bases, or a part thereof, for example, 1 or 2 bases are modified and/or unmodified purine bases. RNase A cleaves the phosphodiester bond on the 3' side of modified and/or unmodified pyrimidine bases. Therefore, if the base in the first exposed region is a modified and/or unmodified pyrimidine base, this portion can be cleaved by RNase A. Accordingly, by including modified and/or unmodified purine bases in the first exposed region, undesirable degradation by RNase A can be reduced. The bases in the first exposed region do not include, for example, any of the modified and/or unmodified pyrimidine bases C, UC, and UU, for example, any of the three sequences.

In one embodiment, the base in the first exposed region includes a modified and/or unmodified purine base. For example, all bases are modified and/or unmodified purine bases, or a part thereof, for example, 1 to 3, 1 to 2, or 1, is a modified and/or unmodified purine base. In one embodiment, the base in the second exposed region is a modified and/or unmodified purine base. For example, all bases are modified and/or unmodified purine bases, or a part thereof, for example, 1 to 7, 1 to 6, 1 to 5, 1 to 4, 1 to 3, 1 to 2, or 1, is a modified and/or unmodified purine base. In one embodiment, the base in the first exposed region and/or the second exposed region includes, in any proportion, modified and/or unmodified purine bases. For example, not less than 10%, not less than 30%, not less than 50%, not less than 70%, not less than 80%, or not less than 90%, are modified and/or unmodified pyrimidine bases. The modified and/or unmodified purine base are/is preferably adenine, guanine, N6-methyladenine, 8-bromoadenine, N2-methylguanine, and/or 8-bromoguanine, further preferably adenine and/or guanine.

In one embodiment, the second nucleic acid strand includes a sugar-modified nucleoside and/or deoxyribonucleoside containing a modified and/or unmodified pyrimidine base. For example, all bases are modified and/or unmodified pyrimidine bases, or a part thereof, for example, 1 to 20, 1 to 15, 1 to 10, 1 to 5, 1 to 2, or 1, is a modified and/or unmodified pyrimidine base. In one embodiment, the second nucleic acid strand includes, in any proportion, a sugar-modified nucleoside and/or deoxyribonucleoside containing a modified and/or unmodified pyrimidine base. For example, not less than 10%, not less than 30%, not less than 50%, not less than 70%, not less than 80%, or not less than 90%, of the second nucleic acid strands are pyrimidine bases. As the sugar-modified pyrimidine base, cytosine, uracil, thymine, 5-methylcytosine, 5-fluorocytosine, 5-bromocytosine, 5-iodocytosine, N4-methylcytosine, and/or 2-thio-thymine are/is preferred, cytosine, uracil, thymine, and/or 5-methylcytosine are/is further preferred, and cytosine is particularly preferred.

In one embodiment, the protected region of the second nucleic acid strand includes a modified and/or unmodified pyrimidine base. For example, all bases are modified and/or unmodified pyrimidine bases, or a part thereof, for example, 1 to 20, 1 to 15, 1 to 10, 1 to 5, 1 to 2, or 1, is a modified and/or unmodified pyrimidine base. In one embodiment, the protected region of the second nucleic acid strand includes a modified and/or unmodified pyrimidine base. For example, not less than 10%, not less than 30%, not less than 50%, not less than 70%, not less than 80%, not less than 90%, of the second nucleic acid strands are pyrimidine bases. As the sugar-modified pyrimidine base, cytosine, uracil, thymine, 5-methylcytosine, 5-fluorocytosine, 5-bromocytosine, 5-iodo cytosine, N4-methylcytosine, and/or 2-thio-thymine are/is preferred, cytosine, uracil, thymine, and/or 5-methylcytosine are/is further preferred, and cytosine is particularly preferred.

The position of the first exposed region and/or the second exposed region in the second nucleic acid strand is not limited. In one embodiment, at least one, for example, all first exposed regions and/or the second exposed regions, is/are located on the 3' side from the center of the second nucleic acid strand. As used herein, "the first exposed region and/or the second exposed region is/are located on the 3' side from the center of the second nucleic acid strand" means that the center, the 5'-terminal, or the 3'-terminal of the first exposed region and/or the second exposed region is (are) located on the 3' side from the center of the second nucleic acid strand.

In one embodiment, the first exposed region and/or the second exposed region (and if present, sugar-unmodified terminal region) can be cleaved by RNase H. Whether it is cleaved by RNase H can be determined, for example, by reacting nucleic acid strand with RNase H, performing electrophoresis, and measuring the concentration of the undegraded bands, the detail of which is described in Example. That the first exposed region consisting of one, or two or three consecutive sugar-unmodified ribonucleosides linked by internucleoside bonds can be cleaved by RNase H was surprising considering the conventional technique knowledge that the cleavage activity by RNase H requires at least 4 consecutive natural ribonucleosides (e.g., FEES J 2008; 276(6): 1494-505, Fig. 3).

In one embodiment, the first exposed region and/or the second exposed region (and if present, sugar-unmodified terminal region) may have activity in RNase H-dependent and/or independent pathways.

In one embodiment, the second nucleic acid strand may include cationic nucleosides in whole or in part. In one embodiment, the cationic nucleoside is a nucleoside containing 2'-Amino-LNA nucleoside (e.g., 3-(Bis(3-aminopropyl)amino)propanoyl-substituted nucleoside), aminoalkyl-modified nucleoside (e.g., 2'-O-methyl and 4'-CH₂CH₂CH₂NH₂-substituted nucleoside), GuNA nucleoside, or the like.

In one preferred embodiment, the compound containing an oligonucleotide of the present invention may contain oligonucleotides shown in Table 1 as the second nucleic acid strand of double-stranded oligonucleotides targeting human exon 53.

**[Table 1-1]**

| |
|---|
| [mGs] [mAs] [mAs] [de] [A] [de] [de] [dt] [dt] [de] [A] [G] [A] [A] [de] [de] [G] [G] [A] [G] [G] [des] [mAs] [mAs] [mC] (SEQ ID NO: 731) |
| [mGs] [mAs] [mAs] [C] [A] [C] [C] [U] [U] [C] [A] [G] [A] [A] [C] [C] [G] [G] [A] [G] [G] [Cs] [mAs] [mAs] [mC] (SEQ ID NO: 732) |
| [dgs] [das] [das] [de] [da] [de] [de] [dt] [dt] [de] [da] [dg] [da] [da] [de] [d5] [dg ] [dg] [da] [dg] [dg] [des] [das] [das] [de] (SEQ ID NO: 733) |
| [mGs] [mAs] [mAs] [C] [A] [C] [C] [U] [U] [C] [A] [G] [As] [mAs] [mCs] [mC] (SEQ ID NO: 734) |
| [mGs] [mAs] [mAs] [de] [da] [de] [de] [dt] [dt] [de] [da] [dg] [da] [da] [de] [d5] [dg ] [dg] [da] [dg] [dg] [des] [mAs] [mAs] [mC] (SEQ ID NO: 735) |
| [mGs] [mAs] [mAs] [mC] [A] [mC] [mC] [mU] [mU] [mC] [A] [G] [A] [A] [mC] [mC] [G] [G] [A ] [G] [G] [mCs] [mAs] [mAs] [mC] (SEQ ID NO: 736) |
| [bGs] [bAs] [bAs] [de] [A] [de] [de] [dt] [dt] [de] [A] [G] [A] [A] [de] [de] [G] [G] [A] [G] [G] [des] [bAs] [bAs] [b5] (SEQ ID NO: 737) |
| [bG] [bA] [bA] [de] [A] [de] [de] [dt] [dt] [de] [A] [G] [A] [A] [de] [de] [G] [G] [A] [G ] [G] [dc] [bA] [bA] [b5] (SEQ ID NO: 738) |
| [bGs] [bAs] [bAs] [C] [A] [C] [C] [U] [U] [C] [A] [G] [A] [A] [C] [C] [G] [G] [A] [G] [G] [Cs] [bAs] [bAs] [b5] (SEQ ID NO: 739) |
| [mGs] [mAs] [mAs] [de] [da] [de] [de] [dt] [dt] [de] [da] [dg] [da] [da] [de] [d5] [dg] [dgs] [mAs] [mGs] [mG] (SEQ ID NO: 740) |
| [mGs] [mAs] mAs] [de] [A] [de] [de] [dt] [dt] [de] [A] [G] [A] [A] [de] [de] [G] [Gs] [mAs] [mGs] [mG] (SEQ ID NO: 741) |
| [mGs] [mAs] [mAs] [mC] [A] [mC] [mC] [mU] [mU] [mC] [A] [G] [A] [A] [mC] [mC] [G] [Gs] [mAs] [mGs] [mG] (SEQ ID NO: 742) |
| [mGs] [mAs] [mAs] [mC] [da] [mC] [mC] [mU] [mU] [mC] [da] [dg] [da] [da] [mC] [mC] [dg] [dgs] [mAs] [mGs] [mG] (SEQ ID NO: 743) |
| [bGs] [bAs] [bAs] [C] [A] [C] [C] [U] [U] [C] [A] [G] [A] [A] [C] [C] [G] [Gs] [bAs] [bGs][bG] (SEQ ID NO: 744) |
| [bGs] [bAs] [bAs] [de] [da] [de] [de] [dt] [dt] [de] [da] [dg] [da] [da] [de] [d5] [dg ][dgs][bAs][bGs][bG] (SEQ ID NO: 745) |
| [mGs] [mAs] [mAs] [de] [A] [de] [de] [dt] [dt] [de] [A] [G] [As] [mAs] [mCs] [mC] (SEQ ID NO: 746) |
| [mCs] [mCs] [mUs] [dt] [de] [A] [G] [A] [A] [de] [de] [G] [Gs] [mAs] [mGs] [mG] (SEQ ID NO: 747) |
| [mGs] [mAs] [mAs] [mC] [A] [mC] [mC] [mU] [mU] [mC] [A] [G] [As] [mAs] [mCs] [mC] (SEQ ID NO: 748) |
| [mCs] [mCs] [mUs] [mU] [mC] [A] [G] [A] [A] [mC] [mC] [G] [Gs] [mAs] [mGs] [mG] (SEQ ID NO: 749) |
| [mCs] [mAs] [mGs] [A] [A] [mC] [mC] [G] [G] [A] [G] [G] [mCs] [mAs] [mAs] [mC] (SEQ ID NO: 750) |
| [mGs] [mAs] [mAs] [mC] [da] [mC] [mC] [mU] [mU] [mC] [da] [dg] [das] [mAs] [mCs] [mC] (SEQ ID NO: 751) |

**[Table 1-2]**

| |
|---|
| [mGs] [mAs] [mAs] [de] [A] [de] [C] [dt] [U] [de] [da] [dg] [das] [mAs] [mCs] [mC] (SEQ ID NO: 752) |
| [mGs] [mAs] [mAs] [de] [da] [C] [mC] [dt] [U] [de] [da] [dg] [das] [mAs] [mCs] [mC] (SEQ ID NO: 753) |
| [bGs] [bAs] [bAs] [C] [A] [C] [C] [U] [U] [C] [A] [G] [As] [bAs] [b5s] [b5] (SEQ ID NO: 754) |
| [bGs] [bAs] [bAs] [de] [A] [de] [de] [dt] [dt] [de] [A] [G] [As] [bAs] [b5s] [b5] (SEQ ID NO: 755) |
| [bGs] [bAs] [bAs] [de] [da] [C] [mC] [dt] [U] [de] [da] [dg] [das] [bAs] [b5s] [b5] (SEQ ID NO: 756) |
| [mGs] [mAs] [mAs] [mC] [mA] [mC] [mC] [mU] [da] [da] [da] [mU] [mC] [mA] [mG] [mAs] [m As] [mCs] [mC] (SEQ ID NO: 757) |
| [mGs] [mAs] [mAs] [mC] [mA] [mC] [da] [da] [da] [mU] [mU] [mC] [mA] [mG] [mA] [mA] [da ] [da] [da] [mC] [mG] [mGs] [mAs] [mGs] [mG] (SEQ ID NO: 758) |
| [mGs] [mAs] [mAs] [de] [A] [de] [de] [dt] [dt] [de] [A] [G] [As] [mAs] [mCs] [mC] (SEQ ID NO: 759) |
| [dt] [de] [da] [G] [A] [A] [de] [A] [de] [de] [dt] [dt] [de] [A] [G] [As] [mAs] [mCs] [m C] (SEQ ID NO: 760) |
| [mGs] [A] [A] [de] [A] [de] [de] [dt] [dt] [de] [A] [G] [A] [A] [de] [de] [G] [G] [A] [G] [G][dc][A][As][mC] (SEQ ID NO: 761) |
| [dg] [da] [da] [de] [da] [de] [de] [dt] [dt] [de] [da] [dg] [da] [da] [de] [de] (SEQ ID NO: 762) |
| [dg] [da] [da] [de] [da] [de] [de] [dt] [dt] [de] [da] [dg] [da] [da] [de] [d5] [dg] [d g] [da] [dg] [dg] [de] [da] [da] [de] (SEQ ID NO: 763) |
| [dg] [da] [da] [de] [da] [de] [de] [dt] [dt] [de] [da] [dg] [da] [da] [de] [d5] [dg] [d g] [da] [dg] [dg] (SEQ ID NO: 764) |
| [mGs] [mAs] [mAs] [C] [A] [C] [C] [U] [U] [C] [A] [G] [A] [A] [C] [C] [G] [Gs] [mAs] [mGs] [mG] (SEQ ID NO: 765) |

In another embodiment, the compound containing an oligonucleotide of the present invention may contain oligonucleotides shown in Table 2 as the second nucleic acid strand of double-stranded oligonucleotides targeting mouse exon 23.

**[Table 2]**

| |
|---|
| [mAs] [mUs] [mUs] [U] [C] [A] [G] [G] [U] [A] [A] [G] [C] [C] [G] [A] [G] [G] [U] [U] [U] [Gs] [mGs] [mCs] [mC] (SEQ ID NO: 766) |
| [mAs] [mUs] [mUs] [U] [C] [A] [G] [G] [U] [A] [A] [G] [Cs] [mCs] [mGs] [mA] (SEQ ID NO: 767) |
| [mAs] [mUs] [mUs] [dt] [de] [A] [G] [G] [dt] [A] [A] [G] [de] [de] [G] [A] [G] [G] [dt] [ dt] [dt] [Gs] [mGs] [mCs] [mC] (SEQ ID NO: 768) |
| [mAs] [mUs] [mUs] [dt] [de] [A] [G] [G] [dt] [A] [A] [G] [des] [mCs] [mGs] [mA] (SEQ ID NO: 769) |
| [ [mAs] [mUs] [mUs] [mU] [mC] [mA] [da] [da] [da] [mG] [mU] [mA] [mA] [mG] [mC] [mC] [d a] [da] [da] [mA] [mG] [mGs] [mUs] [mUs] [mU] (SEQ ID NO: 770) |
| [mAs] [mUs] [mUs] [dt] [de] [A] [G] [G] [dt] [A] [A] [G] [des] [mCs] [mGs] [mA] [Y3] (SEQ ID NO: 771) |
| [bAs] [bTs] [bTs] [dt] [de] [A] [G] [G] [dt] [A] [A] [G] [des] [b5s] [bGs] [bA] (SEQ ID NO: 772) |
| [da] [dt] [dt] [dt] [de] [da] [dg] [dg] [dt] [da] [da] [dg] [de] [d5] [dg] [da] [dg] [d g] [dt] [dt] [dt] [dg] [dg] [de] [dc] (SEQ ID NO: 773) |

In Tables 1 -1, 1-2, and 2, A, U, G, and C are adenine, uracil, guanine, and cytosine of ribonucleoside, da, dt, dg, dc, and d5 are adenine, thymine, guanine, cytosine, and 5-methylcytosine of deoxyribonucleoside. In the above, base uracil (U) and base thymine (T) are substitutable with each other.
MoA, MoT, MoG, and MoC are each morpholinonucleic acid.
mA, mU, mG, and mC are each 2'-O-methylnucleoside.
bA, bT, bG, and b5 are each 2',4'-BNA^{NC}[N-Me]nucleoside.

The bond between each base is a phosphorodiamidate bond having a dimethylamino group in the case of a morpholinonucleic acid, and a phosphodiester bond in the case of other oligonucleic acid strands. However, when s is added after the base, it indicates a phosphorothioate bond. When the 3'-terminal is not conjugated, it is an unmodified form that is not provided with a phosphate group.

In a preferred embodiment, the compound containing an oligonucleotide of the present invention includes combinations of the first nucleic acid strand and the second nucleic acid strand constituting the conjugates of Examples 1 to 54, 65 to 75, 86, 88, and 89, as the first and second nucleic acid strand of a double-stranded oligonucleotide targeting human exon 53 (see Tables 9 and 11 and the like).

In a preferred embodiment, the compound containing an oligonucleotide of the present invention includes combinations of the first nucleic acid strand and the second nucleic acid strand constituting the conjugates of Examples 55, 56, 77 to 82, 84 and 87, as the first and second nucleic acid strand of a double-stranded oligonucleotide targeting mouse exon 23 (see Tables 10 and 12 and the like).

When the compound containing an oligonucleotide of the present invention is a nucleic acid complex, a functional molecule other than peptide in the present invention that can efficiently deliver to the cells, tissues, organs, and the like into which the introduction is desired can be bound to the 5'-terminal, the 3'-terminal, or both terminals of the antisense strand and/or the sense strand. Alternatively, the functional molecule may be linked to an internal nucleotide of the antisense oligonucleotide. The functional molecules include those described above.

In a preferred embodiment, the conjugate of the present invention includes the compounds of Examples 1 to 54, 57 to 59, 65 to 75, 86, 88 and 89, as conjugates targeting human exon 53.

In another preferred embodiment, the conjugate of the present invention includes the compounds of Examples 55, 56, 60, 61, 77 to 82, 84, 85 and 87, as conjugates targeting mouse exon 23.

That is, in a preferred embodiment, the peptide, linker, and nucleic acid (single-stranded oligonucleotide, first nucleic acid strand, the second nucleic acid strand) constituting the conjugate of the present invention are combinations of those constituting the Example compounds described above.

### Use of conjugate (complex)

Since the conjugate of the present invention include peptides that are directed to muscle tissue, the conjugate of the present invention is directed to muscle tissue and is at least capable of delivering a drug (compound containing oligonucleotide for producing at least partially functional dystrophin protein) to muscle tissue.

Therefore, the conjugate of the present invention may be useful for the prophylaxis or treatment of neuromuscular diseases, particularly dystrophin abnormality (e.g., asymptomatic hyperCKemia, muscle spasm associated with myoglobulinuria, quadriceps myopathy, Becker muscular dystrophy, Duchenne muscular dystrophy and DMD gene-related dilated cardiomyopathy (DCM), female dystrophin abnormality), preferably Duchenne muscular dystrophy.

The conjugate of the present invention is superior in pharmacokinetics (e.g., plasma drug half-life, intracerebral transferability, metabolism stability), has low toxicity (for example, more superior as a medicament, in terms of acute toxicity, chronic toxicity, genetic toxicity, reproductive toxicity, cardiotoxicity, drug interaction, carcinogenicity, and the like), and can be safely administered orally or parenterally to mammals (e.g., human, monkey, bovine, horse, swine, mouse, rat, hamster, rabbit, cat, dog, sheep, goat, etc.) as a medicament as it is, or as a pharmaceutical composition mixed with a pharmaceutically acceptable carrier and the like. The "parenteral" includes administration such as intravenous, intramuscular, intrathecal, subcutaneous, intraorgan, intranasal, intradermal, instillation, intracerebral, intrarectal, vaginal, intraperitoneal, intratumor administrations, administration to the vicinity of tumor, and the like and direct administration to the lesion.

The dose of the conjugate of the present invention varies depending on the administration route, symptoms, and the like. For example, when administered parenterally (e.g., intravenously) to a patient with Duchenne muscular dystrophy (adult, weight 40 to 80 kg, for example, 60 kg), the amount of the nucleic acid complex of the present invention contained in one dose of the composition, i.e., the single dose of the nucleic acid complex of the present invention is, for example, not less than 0.001 mg/kg, not less than 0.005 mg/kg, not less than 0.01 mg/kg, not less than 00.25 mg/kg, not less than 0.5 mg/kg, not less than 1 mg/kg, not less than 2.5 mg/kg, not less than 5 mg/kg, not less than 10 mg/kg, not less than 20 mg/kg, not less than 30 mg/kg, not less than 40 mg/kg, not less than 50 mg/kg, not less than 75 mg/kg, not less than 100 mg/kg, not less than 150 mg/kg, not less than 200 mg/kg, not less than 300 mg/kg, not less than 400 mg/kg, or not less than 500 mg/kg. For example, any amount within the range of 0.001 mg/kg to 500 mg/kg (e.g., 0.001 mg/kg, 0.01 mg/kg, 0.1 mg/kg, 1 mg/kg, 5 mg/kg, 10 mg/kg, 50 mg/kg, 100 mg/kg, or 200 mg/kg) can be appropriately selected. The composition can be administered in a single dose or multiple doses. In the case of multiple administrations, composition can also be administered daily or at appropriate time intervals (for example, at intervals of 1 day, 2 days, 3 days, 1 week, 2 weeks, 1 month, or 2 months).

Medicaments containing the conjugate of the present invention can be used alone or as a pharmaceutical composition of a mixture of the conjugate of the present invention and a pharmaceutically acceptable carrier, according to a method known per se as a method for producing pharmaceutical preparations (e.g., the method described in the Japanese Pharmacopoeia, etc.). A medicaments containing the conjugate of the present invention can be safely administered orally or parenterally (e.g., intravenously, intramuscularly, subcutaneously, intraorgan, intranasally, intradermally, instillation, intracerebrally, intrarectally, vaginally, intraperitoneally, lesion, etc.) as, for example, tablet (including sugar-coated tablet, film-coated tablet, sublingual tablet, orally disintegrating tablet, buccal tablet, and the like), pill, powder, granule, capsule (including soft capsule, microcapsule), troche, syrup, liquid, emulsion, suspension, controlled-release preparation (e.g., immediate-release preparation, sustained-release preparation, sustained-release microcapsule), aerosol, films (e.g., orally disintegrable films, oral mucosal adhesive film), injection (e.g., subcutaneous injection, intravenous injection, intramuscular injection, intraperitoneal injection), drip transfusion, transdermal absorption type preparation, ointment, lotion, adhesive preparation, suppository (e.g., rectal suppository, vaginal suppository), pellet, nasal preparation, pulmonary preparation (inhalant), eye drop, or the like.

In certain embodiments, the conjugate of the present invention has excellent solubility in water, the Japanese Pharmacopoeia 2nd dissolution test fluid, or the Japanese Pharmacopoeia 2nd disintegration test fluid, and has excellent pharmacokinetics (e.g., blood drug half-life, intracerebral transfer, metabolic stability, CYP inhibition), low toxicity (e.g., excellent as a drug in terms of acute toxicity, chronic toxicity, genetic toxicity, reproductive toxicity, cardiotoxicity, drug interaction, carcinogenicity, phototoxicity, etc.),and excellent properties as a pharmaceutical product such as having fewer side effects.

The conjugates of the present invention may contain other active ingredients as long as their combination with the conjugate of the present invention does not result in undesirable interactions. In one embodiment, the conjugate of the present invention may contain other conjugates of the present invention as long as their combination with the conjugate of the invention does not result in undesirable interactions. The target exons of other conjugates of the present invention may be the same as or different from the exon of the conjugate of the present invention, and preferably the conjugate of the present invention and other conjugates of the present invention have the same exons that have the same target exons. In another embodiment, a conjugate of the invention and other conjugates of the present invention differ in exons that have the same target exon. As other active ingredients (sometimes to be abbreviated as concomitant drug), various compounds having therapeutic effects on diseases such as dystrophin disorders (e.g., asymptomatic hyperCKemia, muscle spasms with myoglobinuria, quadriceps myopathy, Becker muscular dystrophy, Duchenne muscular dystrophy and DMD gene-associated dilated cardiomyopathy (DCM), female dystrophin disorders) can be blended as appropriate. For example, as other active ingredients, therapeutic drugs for Duchenne muscular dystrophy such as Eteplirsen, SRP-5051, Viltolarsen, Golodirsen, Renadirsen, NS-089, and the like; steroid drugs such as steroid, corticosteroid, glucocorticoid, anabolic steroid, mineralocorticoid, dexamethasone, hexestrol, cortisone acetate, and the like; NF-κB inhibitors such as edasalonexent and the like; non-steroidal antiinflammatory agents such as meloxicam, tenoxicam, indomethacin, ibuprofen, celecoxib, rofecoxib, aspirin and the like; disease-modified anti-rheumatic drugs; anti-cytokine drugs such as TNF inhibitor, MAP kinase inhibitor, and the like are optionally contained. These concomitant drugs can be formulated with the conjugate of the present invention and administered as a single preparation, or they can be formulated separately from the conjugate of the present invention and administered by the same or different routes from the conjugate of the present invention, either simultaneously or at staggered intervals. The dose of these concomitant drugs may be the amount normally used when the drugs are administered alone, or may be reduced from the amount normally used. These concomitant drugs may be linked to the conjugate of the present invention directly or via a linker. As the linker, known linkers, for example, linkers described in WO2021/054370, WO 2020/209285, WO 2020/028832, WO 2017/221883, WO 2012/150960, J. Am. Chem. Soc., 2021, 143, 3416-3429, Antibodies 2020, 9, 2, and the like can be mentioned.

As the aforementioned "pharmaceutically acceptable carrier", various organic or inorganic carriers conventionally used as preparation materials (starting materials) can be used. For example, in solid preparations, excipient, lubricant, binder, disintegrant, and the like are used, and in liquid preparations, solvent, solubilizing agent, suspending agent, isotonizing agent, buffering agent, soothing agent, and the like are used. In addition, where necessary, preparation additives such as antiseptic, antioxidant, colorant, sweetening agent, and the like can also be used. Furthermore, in order to improve the stability of the oligonucleotide-containing compound or change the in vivo behavior of the drug, a cationic molecule having binding properties can be added to a compound containing oligonucleotide.

Examples of the excipient include lactose, sucrose, D-mannitol, starch, cornstarch, crystalline cellulose, light anhydrous silicic acid, and the like.

Examples of the lubricant include magnesium stearate, calcium stearate, talc, colloid silica, and the like.

Examples of the binder include crystalline cellulose, sucrose, D-mannitol, dextrin, hydroxypropylcellulose, hydroxypropylmethylcellulose, polyvinylpyrrolidone, starch, sucrose, gelatin, methylcellulose, sodium carboxymethylcellulose, and the like.

Examples of the disintegrant include starch, carboxymethylcellulose, carboxymethylcellulose calcium, sodium carboxymethyl starch, L-hydroxypropylcellulose, and the like.

Examples of the solvent include water for injection, alcohol, propylene glycol, macrogol, sesame oil, corn oil, olive oil, and the like.

Examples of the solubilizing agent include polyethylene glycol, propylene glycol, D-mannitol, benzoic acid benzyl, ethanol, tris aminomethane, cholesterol, triethanolamine, sodium carbonate, sodium citrate, and the like.

Examples of the suspending agent include surfactants such as stearyltriethanolamine, sodium lauryl sulfate, lauryl aminopropionic acid, lecithin, benzalkonium chloride, benzethonium chloride, glyceryl monostearate, and the like; hydrophilic polymers such as poly(vinyl alcohol), polyvinylpyrrolidone, sodium carboxymethylcellulose, methylcellulose, hydroxymethylcellulose, hydroxyethylcellulose, hydroxypropylcellulose, and the like, and the like.

Examples of the isotonizing agent include glucose, D-sorbitol, sodium chloride, glycerol, D-mannitol, and the like.

Examples of the buffering agent include buffers of phosphate, acetate, carbonate, citrate, and the like, and the like.

Examples of the soothing agent include benzyl alcohol and the like.

Examples of the antiseptic include p-hydroxybenzoic acid ester, chlorobutanol, benzyl alcohol, phenylethylalcohol, dehydroacetic acid, sorbic acid, and the like.

Examples of the antioxidant include sulfite, ascorbic acid, α-tocopherol, and the like.

As the cationic molecule, peptides and oligosaccharides can be mentioned. Examples of the cationic peptide include 2,4-diamino butyric acid octamer (Dab8) and the like.

The conjugate of the present invention can be subjected to various modifications in view of the fact that polypeptides are easily metabolized and excreted. For example, polyethylene glycol (PEG) or a sugar chain can be added to a polypeptide to increase residence time in the blood and reduce antigenicity. In addition, biodegradable polymer compounds such as polylactic acid glycol (PLGA), porous hydroxyapatite, liposomes, surface-modified liposomes, emulsions prepared with unsaturated fatty acids, nanoparticles, nanospheres, and the like are used as sustained release bases, and a polypeptide may be encapsulated therein. When administered transdermally, a weak electric current can also be applied to the skin surface to penetrate the stratum corneum (iontophoresis method).

While pharmaceutical compositions vary depending on the dosage form, administration method, carrier, and the like, the conjugate of the present invention is generally added at a proportion of 0.01 to 100% (w/w), preferably 0.1 to 95% (w/w), with respect to the total amount of the preparation and the compositions can be produced according to a conventional method.

The present specification provides the use of a conjugate for the manufacture of a medicament for the prophylaxis or treatment of neuromuscular diseases. The conjugate in this case may be any of those described above.

### [Example]

The present invention is explained in detail by referring to the following Reference Examples and Examples, which are not to be construed as limitative, and may be changed without departing from the scope of the present invention.

In the following Examples, the "room temperature" generally means about 10°C to about 35°C. The ratios indicated for mixed solvents are volume mixing ratios, unless otherwise specified. % means wt%, unless otherwise specified.

Mass spectra (MS) were measured using a mass spectrometer. While a molecular ion peak is observed, it may sometimes be observed as a fragment ion. Data shows actual measured values (found) or calculated values obtained by deconvolution processing using MaxEnt1 using Waters MassLynx and the like. In the case of salts, a free molecular ion peak or fragment ion peak is usually observed.

The meanings of the abbreviations used in the examples are shown below.
M: mol concentration
N: normality
Å: angstrom
Adod: 12-aminododecanoic acid
AEEA: 2-[2-(2-aminoethoxy)ethoxy]acetic acid
BCN-NHS: 1-[({[(1R,8S,9s)-bicycle[6.1.0]non-4-yn-9-yl]methoxy}carbonyl)oxy]pyrrolidine-2,5-dione
Boc₂O: di-tert-butyl dicarbonate
ClAc: chloroacetyl
ClAcOH: chloroacetic acid
DBU: 1,8-diazabicyclo[5.4.0]-7-undecene
DCM: dichloromethane
DIEA: N,N-diisopropylethylamine
DIPCDI: N,N'-diisopropylcarbodiimide
DIPEA: N,N-diisopropylethylamine
DMF: dimethylformamide
DMSO: dimethyl sulfoxide
DODT: 6-dioxa-1,8-octane-dithiol
Fmoc: 9-fluorenylmethyloxycarbonyl
HATU: O-(7-azabenzotriazol-1-yl)-N,N,N',N'-tetramethyluronium hexafluorophosphate
HCTU: O-(6-chlorobenzotriazol-1-yl)-N,N,N',N'-tetramethyluronium hexafluorophosphate
HFIP: hexafluoro-2-propanol
HOAt: 1-hydroxybenzotriazole
HOSu: N-hydroxysuccinimide
HPLC: high performance liquid chromatography
iVDde: 1-(4,4-dimethyl-2,6-dioxocyclohex-1-ylidene)isovaleryl
LC-MS or LC/MS: liquid chromatograph mass spectrometer
MeCN: acetonitrile
Mmt: monomethoxytrityl
Mtt: monomethyltrityl
NMP: N-methyl-2-pyrrolidone
ODS: octadecyl bonded silica gel
o-Ns: 2-nitrobenzenesulfonyl
OSu: succinimidyloxy group
Pd₂(dba)₃: tris(dibenzylideneacetone)dipalladium (0)
PEG: polyethylene glycol
PBS: phosphate buffered saline
Pbf: 2,2,4,6,7-pentamethyldihydrobenzofuran-5-sulfonyl
SPhos: 2-dicyclohexylphosphino-2',6'-dimethoxybiphenyl
TBTA: tris[(1-benzyl-1H-1,2,3-triazol-4-yl)methyl]amine
tBu: tertiary butyl
TEA: triethylamine
TFA: trifluoroacetic acid
TIS: triisopropyl silane
TMSOTf: trimethylsilyl trifluoromethanesulfonic acid
Trt or Tr: trityl group

The sequence structures of the oligonucleotides used in the following examples are summarized in Tables 1 to 4. The PMO of the first nucleic acid strand used in the Examples was synthesized using Wuxi STA or Gene Tools. The complementary strand oligonucleotide of the second nucleic acid strand was synthesized by the known phosphoramidite method. Some oligonucleotides were further synthesized by condensing a BCN linker with BCN-NHS. Some oligonucleotides were synthesized by Gene Design Co., Ltd. (Osaka, Japan), Hokkaido System Science Co., Ltd., or Wuxi.

**[Table 3] PMO of starting materials (human exon53)**

| compound No. | sequence (5' to 3') | target gene |
|---|---|---|
| A1 | [Y1] [MoG] [MoT] [MoT] [MoG] [MoC] [MoC] [MoT] [MoC] [MoC] [MoG] [MoG] [MoT] [MoT] [MoC] [MoT] [MoG] [MoA] [MoA] [MoG ] [MoG] [MoT] [MoG] [MoT] [MoT] [MoC] (SEQ ID NO: 774) | human DMD exon53 |
| A2 | [Y1] [MoC] [MoC] [MoT] [MoC] [MoC] [MoG] [MoG] [MoT] [MoT] [MoC] [MoT] [MoG] [MoA] [MoA] [MoG] [MoG] [MoT] [MoG] [MoT ][MoT][MoC](SEQ ID NO: 775) | human DMD exon53 |
| A3 | [Y1] [MoG] [MoT] [MoT] [MoG] [MoC] [MoC] [MoT] [MoC] [MoC] [MoG] [MoG] [MoT] MoT] [MoC] [MoT] [MoG] [MoA] [MoA] [MoG ] [MoG] [MoT] [MoG] [MoT] [MoT] [MoC] [Y2] (SEQ ID NO: 776) | human DMD exon53 |

**[Table 4] PMO of starting materials (mouse exon23)**

| compound No. | sequence (5' to 3') | target gene |
|---|---|---|
| A4 | [Y1] [MoG] [MoG] [MoC] [MoC] [MoA] [MoA] [MoA] [MoC] [MoC] [MoT] [MoC] [MoG] [MoG] [MoC] [MoT] [MoT] [MoA] [MoC] [MoC ] [MoT] [MoG] [MoA] [MoA] [MoA] [MoT] (SEQ ID NO: 777) | mouse DMD exon23 |
| A5 | [Y1] [MoG] [MoG] [MoC] [MoC] [MoA] [MoA] [MoA] [MoC] [MoC] [MoT] [MoC] [MoG] [MoG] [MoC] [MoT] [MoT] [MoA] [MoC] [MoC ] [MoT] [MoG] [MoA] [MoA] [MoA] [MoT] [Y2] (SEQ ID NO: 778) | mouse DMD exon23 |

**[Table 5-1] complementary strand starting materials human exon53)**

| compound No. | sequence (5' to 3') |
|---|---|
| B1 | [Y3] [mGs] [mAs] [mAs] [de] [A] [de] [de] [dt] [dt] [de] [A] [G] [A] [ A] [de] [de] [G] [G] [A] [G] [G] [des] [mAs] [mAs] [mC] (SEQ ID NO: 779) |
| B2 | [Y3] [mGs] [mAs] [mAs] [C] [A] [C] [C] [U] [U] [C] [A] [G] [A] [A] [C] [ C] [G] [G] [A] [G] [G] [Cs] [mAs] [mAs] [mC] (SEQ ID NO: 780) |
| B3 | [Y3] [dgs] [das] [das] [de] [da] [de] [de] [dt] [dt] [de] [da] [dg] [ da] [da] [de] [d5] [dg] [dg] [da] [dg] [dg] [des] [das] [das] [de] (SEQ ID NO: 781) |
| B4 | [Y3] [mGs] [mAs] [mAs] [C] [A] [C] [C] [U] [U] [C] [A] [G] [As] [mAs] [ mCs][mC] (SEQ ID NO: 782) |
| B5 | [Y3] [mGs] [mAs] [mAs] [de] [da] [de] [de] [dt] [dt] [de] [da] [dg] [ da] [da] [de] [d5] [dg] [dg] [da] [dg] [dg] [des] [mAs] [mAs] [mC] (SEQ ID NO: 783) |
| B6 | [Y3] [mGs] [mAs] [mAs] [mC] [A] [mC] [mC] [mU] [mU] [mC] [A] [G] [A] [ A] [mC] [mC] [G] [G] [A] [G] [G] [mCs] [mAs] [mAs] [mC] (SEQ ID NO: 784) |
| B7 | [Y4] [bGs] [bAs] [bAs] [de] [A] [de] [de] [dt] [dt] [de] [A] [G] [A] [ A] [de] [de] [G] [G] [A] [G] [G] [des] [bAs] [bAs] [b5] (SEQ ID NO: 785) |
| B8 | [Y4] [bG] [bA] [bA] [de] [A] [de] [de] [dt] [dt] [de] [A] [G] [A] [A] [ dc] [dc] [G] [G] [A] [G] [G] [dc] [bA] [bA] [b5] (SEQ ID NO: 786) |
| B9 | [Y4] [bGs] [bAs] [bAs] [C] [A] [C] [C] [U] [U] [C] [A] [G] [A] [A] [C] [ C] [G] [G] [A] [G] [G] [Cs] [bAs] [bAs] [b5] (SEQ ID NO: 787) |
| B10 | [Y3] [mGs] [mAs] [mAs] [de] [da] [de] [de] [dt] [dt] [de] [da] [dg] [ da] [da] [de] [d5] [dg] [dgs] [mAs] [mGs] [mG] (SEQ ID NO: 788) |
| B11 | [Y3] [mGs] [mAs] [mAs] [de] [A] [de] [de] [dt] [dt] [de] [A] [G] [A] [ A] [de] [de] [G] [Gs] [mAs] [mGs] [mG] (SEQ ID NO: 789) |
| B12 | [Y3] [mGs] [mAs] [mAs] [mC] [A] [mC] [mC] [mU] [mU] [mC] [A] [G] [A] [ A] [mC] [mC] [G] [Gs] [mAs] [mGs] [mG] (SEQ ID NO: 790) |
| B13 | [Y3] [mGs] [mAs] [mAs] [mC] [da] [mC] [mC] [mU] [mU] [mC] [da] [dg] [ da] [da] [mC] [mC] [dg] [dgs] [mAs] [mGs] [mG] (SEQ ID NO: 791) |
| B15 | [Y4] [bGs] [bAs] [bAs] [C] [A] [C] [C] [U] [U] [C] [A] [G] [A] [A] [C] [ C] [G] [Gs] [bAs] [bGs] [bG] (SEQ ID NO: 792) |
| B16 | [Y4] [bGs] [bAs] [bAs] [de] [da] [de] [de] [dt] [dt] [de] [da] [dg] [ da] [da] [de] [d5] [dg] [dgs] [bAs] [bGs] [bG] (SEQ ID NO: 793) |
| B17 | [Y3] [mGs] [mAs] [mAs] [de] [A] [de] [de] [dt] [dt] [de] [A] [G] [As] [mAs] [mCs] [mC] (SEQ ID NO: 794) |
| B18 | [Y3] [mCs] [mCs] [mUs] [dt] [de] [A] [G] [A] [A] [de] [de] [G] [Gs] [m As][mGs][mG] (SEQ ID NO: 795) |
| B19 | [Y3] [mGs] [mAs] [mAs] [mC] [A] [mC] [mC] [mU] [mU] [mC] [A] [G] [As] [mAs][mCs][mC] (SEQ ID NO: 796) |
| B20 | [Y3] [mCs] [mCs] [mUs] [mU] [mC] [A] [G] [A] [A] [mC] [mC] [G] [Gs] [m As][mGs][mG] (SEQ ID NO: 797) |
| B21 | [Y3] [mCs] [mAs] [mGs] [A] [A] [mC] [mC] [G] [G] [A] [G] [G] [mCs] [mA s] [mAs] [mC] (SEQ ID NO: 798) |
| B26 | [Y3] [mGs] [mAs] [mAs] [mC] [da] [mC] [mC] [mU] [mU] [mC] [da] [dg] [das][mAs][mCs][mC] (SEQ ID NO: 799) |

**[Table 5-2]**

| compound No. | sequence (5' to 3') |
|---|---|
| B27 | [Y3] [mGs] [mAs] [mAs] [de] [A] [de] [C] [dt] [U] [de] [da] [dg] [das ] [mAs] [mCs] [mC] (SEQ ID NO: 800) |
| B28 | [Y3] [mGs] [mAs] [mAs] [de] [da] [C] [mC] [dt] [U] [de] [da] [dg] [da s] [mAs] [mCs] [mC] (SEQ ID NO: 801) |
| B29 | [Y3] [bGs] [bAs] [bAs] [C] [A] [C] [C] [U] [U] [C] [A] [G] [As] [bAs] [ b5s] [b5] (SEQ ID NO: 802) |
| B30 | [Y3] [bGs] [bAs] [bAs] [de] [A] [de] [de] [dt] [dt] [de] [A] [G] [As] [bAs] [b5s] [b5] (SEQ ID NO: 803) |
| B31 | [Y3] [bGs] [bAs] [bAs] [de] [da] [C] [mC] [dt] [U] [de] [da] [dg] [da s] [bAs] [b5s] [b5] (SEQ ID NO: 804) |
| B32 | [Y3] [mGs] [mAs] [mAs] [mC] [mA] [mC] [mC] [mU] [da] da] [da] [mU] [ mC] [mA] [mG] [mAs] [mAs] [mCs] [mC] (SEQ ID NO: 805) |
| B33 | [Y3] [mGs] [mAs] [mAs] [mC] [mA] [mC] [da] [da] [da] [mU] [mU] [mC] [ mA] [mG] [mA] [mA] [da] [da] [da] [mC] [mG] [mGs] [mAs] [mGs] [mG] (SEQ ID NO: 806) |
| B42 | [Y5] [mGs] [mAs] [mAs] [de] [A] [de] [de] [dt] [dt] [de] [A] [G] [As] [mAs] [mCs] [mC] [Y3] (SEQ ID NO: 807) |
| B44 | [Y3] [dt] [de] [da] [G] [A] [A] [de] [A] [de] [de] [dt] [dt] [de] [A] [ G] [As] [mAs] [mCs] [mC] (SEQ ID NO: 808) |
| B45 | [Y3] [mGs] [A] [A] [de] [A] [de] [de] [dt] [dt] [de] [A] [G] [A] [A] [d c] [de] [G] [G] [A] [G] [G] [de] [A] [As] [mC] (SEQ ID NO: 809) |
| B47 | [Y3] [dg] [da] [da] [de] [da] [de] [de] [dt] [dt] [de] [da] [dg] [da] [da] [dc] [dc] (SEQ ID NO: 810) |
| B65 | [Y3] [dg] [da] [da] [de] [da] [de] [de] [dt] [dt] [de] [da] [dg] [da] [da] [de] [d5] [dg] [dg] [da] [dg] [dg] [de] [da] [da] [de] (SEQ ID NO: 811) |
| B66 | [Y3] [dg] [da] [da] [de] [da] [de] [de] [dt] [dt] [de] [da] [dg] [da] [da] [de] [d5] [dg] [dg] [da] [dg] [dg] (SEQ ID NO: 812) |
| B86 | [Y6] [mGs] [mAs] [mAs] [C] [A] [C] [C] [U] [U] [C] [A] [G] [A] [A] [C] [ C] [G] [Gs] [mAs] [mGs] [mG] (SEQ ID NO: 813) |

**[Table 6] complementary strand starting materials (mouse exon23)**

| compound No. | sequence (5' to 3') |
|---|---|
| B55 | [Y3] [mAs] [mUs] [mUs] [U] [C] [A] [G] [G] [U] [A] [A] [G] [C] [C] [G] [A] [G] [G] [U] [U] [U] [Gs] [mGs] [mCs] [mC] (SEQ ID NO: 814) |
| B56 | [Y3] [mAs] [mUs] [mUs] [U] [C] [A] [G] [G] [U] [A] [A] [G] [Cs] [mCs] [mGs] [mA] (SEQ ID NO: 815) |
| B77 | [Y3] [mAs] [mUs] [mUs] [dt] [de] [A] [G] [G] [dt] [A] [A] [G] [de] [dc] [G] [A] [G] [G] [dt] [dt] [dt] [Gs] [mGs] [mCs] [mC] (SEQ ID NO: 816) |
| B78 | [Y3] [mAs] [mUs] [mUs] [dt] [de] [A] [G] [G] [dt] [A] [A] [G] [des] [m Cs] [mGs] [mA] (SEQ ID NO: 817) |
| B79 | [Y3] [mAs] [mUs] [mUs] [mU] [mC] [mA] [da] [da] [da] [mG] [mU] [mA] [mA] [mG] [mC] [mC] [da] [da] [da] [mA] [mG] [mGs] [mUs] [mUs] [mU] (S EQ ID NO: 818) |
| B81 | [Y5] [mAs] [mUs] [mUs] [dt] [de] [A] [G] [G] [dt] [A] [A] [G] [des] [m Cs] [mGs] [mA] [Y3] (SEQ ID NO: 819) |
| B82 | [Y3] [bAs] [bTs] [bTs] [dt] [de] [A] [G] [G] [dt] [A] [A] [G] [des] [b 5s] [bGs] [bA] (SEQ ID NO: 820) |
| B84 | [Y3] [da] [dt] [dt] [dt] [de] [da] [dg] [dg] [dt] [da] [da] [dg] [de] [d5] [dg] [da] [dg] [dg] [dt] [dt] [dt] [dg] [dg] [de] [de] (SEQ ID NO: 821) |

In Tables 3 to 6, A, U, G, C show adenine, uracil, guanine, and cytosine of ribonucleoside, and da, dt, dg, dc, d5 show adenine, thymine, guanine, cytosine, and 5-methylcytosine of deoxyribonucleoside.
MoA, MoT, MoG, MoC show morpholinonucleic acids.
mA, mU, mG, mC show 2'-O-methylnucleosides.
bA, bT, bG, b5 show 2',4'-BNA^{NC}[N-Me]nucleosides.

The bond between each base is a phosphorodiamidate bond having a dimethylamino group in the case of a morpholino nucleic acid, and a phosphodiester bond in the case of other oligonucleic acid strands. However, when s is added after the base, it indicates a phosphorothioate bond. Furthermore, when the 3'-terminal is not conjugated, it is an unmodified form that is not provided with a phosphate group.

The following also has the same meaning.

The structure of Y1 in the Tables is shown below.

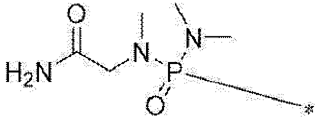

The structure of Y2(Pip9b2) in the Tables is shown below.

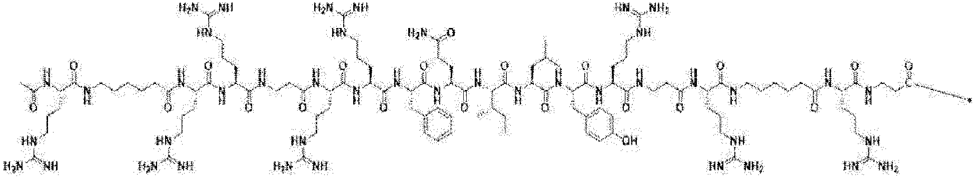

The structure of Y3 in the Tables is shown below.

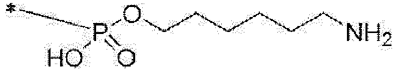

The structure of Y4 in the Tables is shown below.

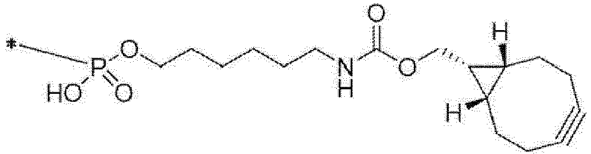

The structure of Y5 in the Tables is shown below.

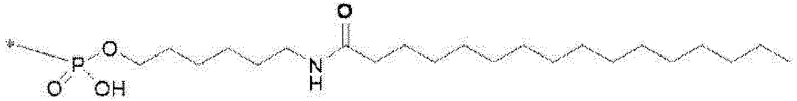

### Reference Example 1

### Synthesis of compound P0 (cyclic peptide having amino acid sequence of SEQ ID NO: 296)

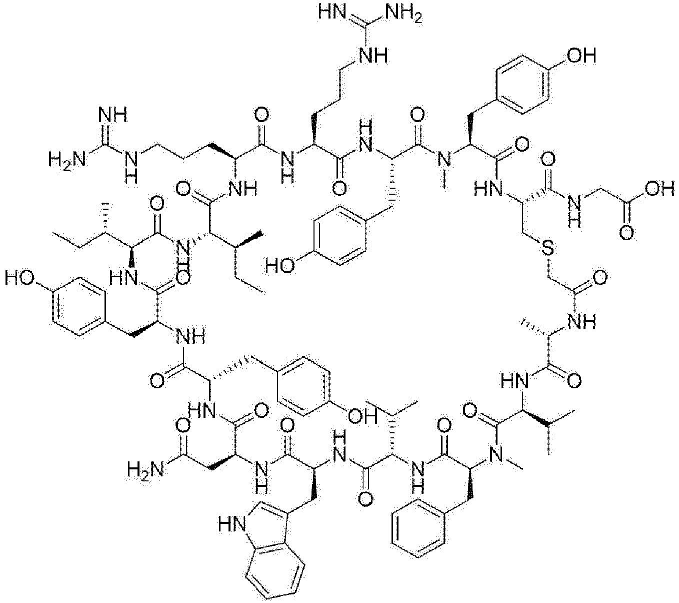

Using Fmoc-Gly-wang resin (Watanabe Chemical, 0.78 mmol/g, 1.31 g), the target peptide was synthesized by starting from the removal of the Fmoc group according to the general method described above. At that time, CEM's Liberty Blue was used as a solid phase synthesizer, and the synthesis was performed according to the manufacturer's manual. For the introduction of each residue, Fmoc-AA/HATU/DIEA (4.2 equivalents/4 equivalents/8 equivalents) was used for 1 equivalent of resin, and the reaction was repeated twice for 15 min in DMF at 60°C. However, when introducing the 15th residue, the reaction was performed once at 25°C for 30 min. When introducing the 11th residue and the 12th residue, the reaction was repeated twice at 25°C for 20 min. When introducing the 10th residue, the reaction was performed once at 60°C for 15 min. When introducing the 7th residue, the reaction was performed once at 60°C for 15 min. When introducing the fifth residue, the reaction was performed once at 60°C for 15 min. When introducing the third residue, the reaction was performed twice at 60°C for 45 min. The basic condition for Fmoc removal was to react with a 20% piperidine solution in DMF at 75°C for 3 min. For Fmoc removal at the 2nd residue and the 13th residue, the reaction was repeated twice at 25°C for 5 min. To introduce the chloroacetyl group, the Fmoc group of the α-amino group was removed by the aforementioned method from the solid phase resin carrying the Fmoc-protected peptide obtained in the previous step, chloroacetic acid (5 equivalents), 5 equivalents of DIPCDI, and 5 equivalents of HOSu were stirred in DCM, and the same amount of DMF as DCM was added to prepare a DCM/DMF solution (0.2 M) of ClAcOSu, the solution was added to the solid phase resin, and the mixture was shaken at room temperature for 240 min. For deprotection of side chains and cutting out from the solid-phase resin, first, the resin obtained after the chloroacetyl group introduction step was washed with DMF five times and methylene chloride three times, and then dried under reduced pressure. Then, reactant cocktail-A (a mixture of TFA/H₂O/TIS/DODT in a volume ratio of 92.5:2.5:2.5:2.5) was added to the reaction vessel containing the solid-phase resin, and the mixture was shaken at room temperature for 90 min. The reaction solution was collected by filtration through a frit. The solid-phase resin remaining in the reaction vessel was shaken again with the cutting cocktail, and the solution components were collected from the frit and mixed with the above-mentioned filtrate. When this filtrate was added to an excess diisopropyl ether cooled to 0°C, a cloudy white precipitate was formed. The mixture was centrifuged (9500 rpm, 1 min) and the solution was decanted. The obtained solid was washed again with a small amount of diethyl ether cooled to 0°C, and then dried under reduced pressure. The obtained solid was used in the next cyclization reaction. For the cyclization reaction of the peptide, the peptide was dissolved in DMSO so that the final concentration was 4 mM based on the number of moles of the solid phase resin, 10 equivalents of triethylamine was added, and the mixture was shaken at room temperature for about 12 hr. The obtained reaction solution was concentrated under reduced pressure using GenevaC EZ-2.

The obtained crude product was purified using the following conditions (column; Waters Xbridge (registered trademark) C18 5 um (registered trademark) 50x250 mm:mobile phase; A=0.1% TFA in H₂O, B=0.1% TFA in MeCN: temperature 60°C: gradient (%B); 0-0% over 5 min, then 0-4.2% over 2 min, then 4.2-28.6% over 3 min, then 28.6-33.7% over 15.5 min, then 33.7-60% over 1.5 min: flow 18 mL/min for 5 min, then 18 mL/min-118 mL/min over 2 min, then 118 mL/min).

The purity of the target product was calculated from the area ratio of LC/MS (UV wavelength 225 nm) chromatogram under analytical conditions and was 92.2%.

Analysis conditions: retention time=11.52 min: column; Kinetex EVO C18 2.6 µm 2.1x150 mm, 100Å:mobile phase; A=0.025% TFA in H₂O, B=0.025% TFA in MeCN: temperature 40°C: gradient (%B conc); 20-60% over 20 min, then 60-95% over 1 min, then 95% over 5 min

### ESI-MS(+) observed value m/z=1078.24(M+2H)²⁺

### Reference Example 2

### Synthesis of compound P1 (SEQ ID NO: 297 in Table 17)

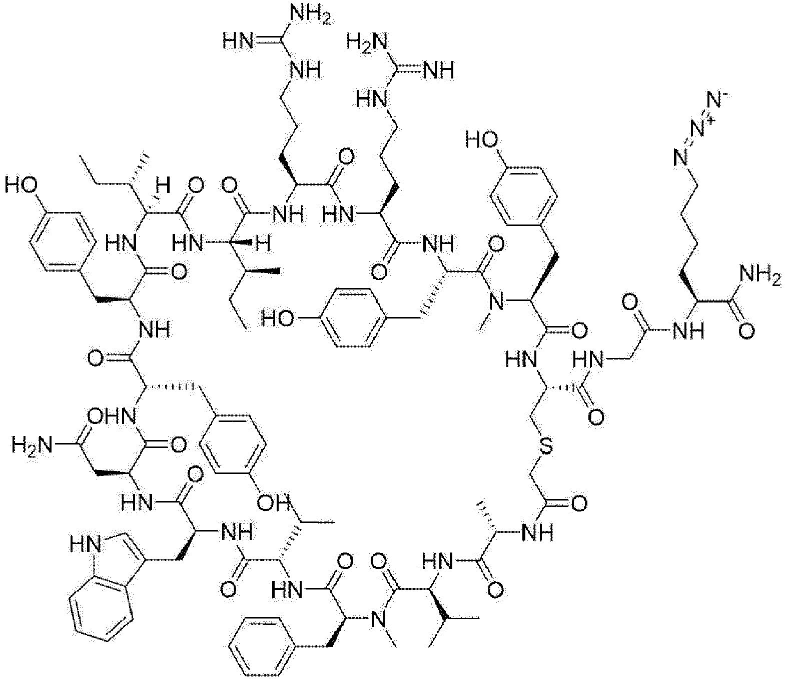

Separately synthesized compound P0 (3.3 g, 1.39 mmol), and 1.1 eq of H-KN₃-NH₂ (316 mg, 1.52 mmol) synthesized by a known method, 1.2 eq of HATU (632 mg, 1.66 mmol), and 5 eq of DIEA (1.21 mL, 6.93 mmol) were stirred in DMF at room temperature for 30 min.

The obtained mixture was purified using the following conditions (column; Waters Xbridge (registered trademark) C18 5 um (registered trademark) 50x150 mm:mobile phase; A=0.1% TFA in H₂O, B=0.1% TFA in MeCN: temperature 40°C: gradient (%B); 7-7% over 2 min, then 7-32% over 1 min, then 32-37% over 8 min, then 37-60% over 1 min: flow 20-20 mL/min over 1 min, then 20-120 mL/min over 1 min, then 120 mL/min).

The purity of the target product was calculated from the area ratio of LC/MS (UV wavelength 225 nm) chromatogram under analytical conditions and was 97.1%.

Analysis conditions: retention time =4.29 min: column; Kinetex EVO C18 2.6 µm 2.1x150 mm, 100Å:mobile phase; A=0.025% TFA in H₂O, B=0.025% TFA in MeCN: temperature 60°C: gradient (%B conc); 20-60% over 7.15 min, then 60-95% over 0.3 min, then 95-95% over 1.55 min: flow 0.5 mL/min.

ESI-MS(+) observed value m/z=1154.72(M+2H)²⁺

### Reference Example 3

### Synthesis of compound P2 (SEQ ID NO: 499 in Table 17)

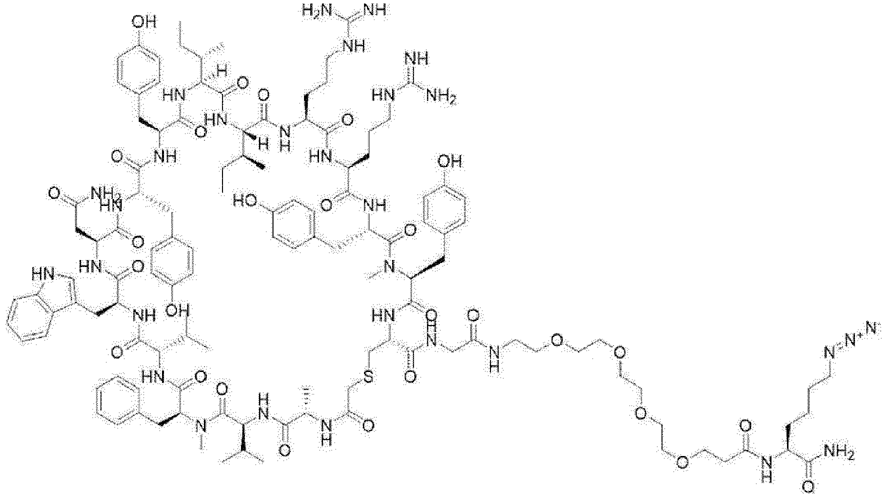

Using Sieber amide resin (Watanabe Chemical, 0.47 mmol/g, 0.16 g), H-PEG4cKN3-Sieber amide resin was synthesized by the general method described above, starting from the removal of the Fmoc group. At that time, CEM's Liberty Blue was used as a solid phase synthesizer, and the synthesis was performed according to the manufacturer's manual. 1 eq of the solid phase resin obtained above was swollen in DMF, and shaken with separately synthesized 3 eq of compound P0 (485 mg), 3 eq of HATU (86 mg), and 6 eq of DIEA (78 µL) at 25°C for 2 hr and 30 min. The resin was washed 3 times with DMF and 3 times with methylene chloride, and dried under reduced pressure. Then, reactant cocktail-A (a mixture of TFA/H₂O/TIS/DODT in a volume ratio of 92.5:2.5:2.5:2.5) was added to the reaction vessel containing the solid-phase resin, and the mixture was shaken at room temperature for 5 min. The reaction solution was collected by filtration through a frit. The solid-phase resin remaining in the reaction vessel was shaken again with the cutting cocktail, and the solution components were collected from the frit and mixed with the above-mentioned filtrate. When this filtrate was added to an excess diisopropyl ether cooled to 0°C, a cloudy white precipitate was formed. The mixture was centrifuged (9500 rpm, 1 min) and the solution was decanted and dried under reduced pressure.

The obtained crude product was purified using the following conditions (column: Waters Xbridge (registered trademark) C18 5 um 50x250 mm; mobile phase:A=0.1% TFA in H₂O, B=0.1% TFA in MeCN; temperature: 60°C; gradient (%B): 2.2-2.2% over 5 min, then 2.2-6.3% over 2 min, then 6.3-31.7% over 3 min, then 31.7-36.8% over 15.5 min, then 36.8-60% over 1.5 min; flow: 18-18 mL/min over 5 min, 18-118 mL/min over 2 min, then 118 mL/min).

The purity of the target product was calculated from the area ratio of LC/MS (UV wavelength 225 nm) chromatogram under analytical conditions and was 97.7%.

Analysis conditions: retention time =4.44 min; column: Kinetex EVO C18 2.6 µm 2.1x150 mm, 100Å; mobile phase: A=0.025% TFA in H₂O, B=0.025% TFA in MeCN; temperature: 40°C: gradient (%B conc): 20-60% over 7.15 min, then 60-95% over 0.3 min, then 95-95% over 1.55 min; flow: 0.5 mL/min

ESI-MS(+) observed value m/z=1278.34(M+2H)²⁺

### Reference Example 4

### Synthesis of compound P3 (SEQ ID NO: 501 in Table 17)

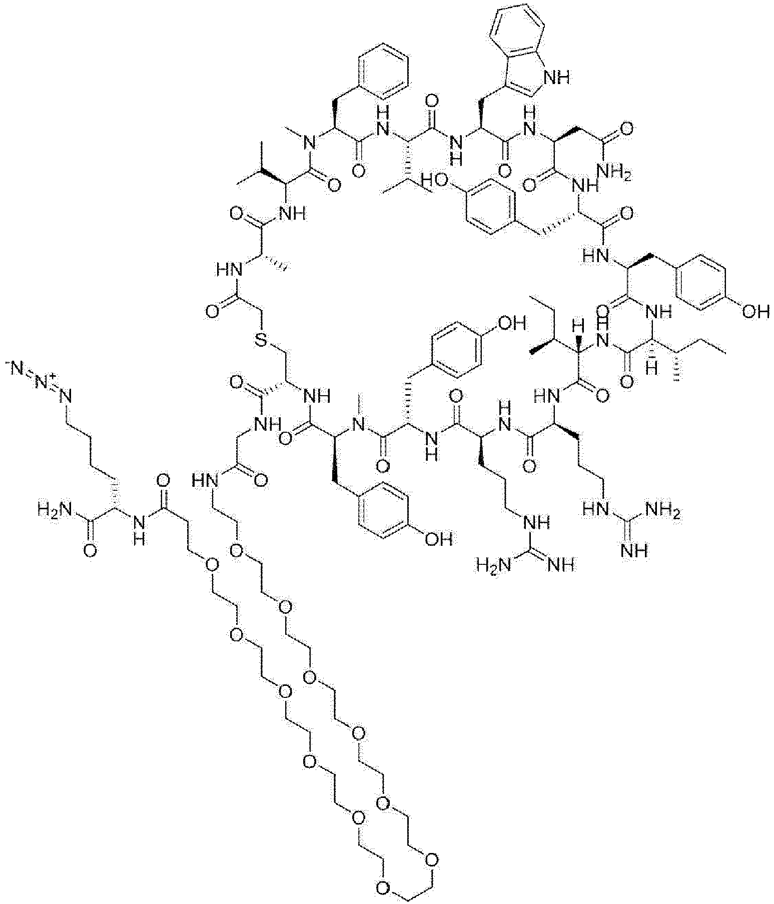

Using Sieber amide resin (Watanabe Chemical, 0.47 mmol/g, 0.11 g), H-PEG12cKN3-Sieber amide resin was synthesized by starting from the removal of the Fmoc group according to the general method described above. At that time, CEM's Liberty Blue was used as a solid phase synthesizer, and the synthesis was performed according to the manufacturer's manual. 1 eq of the solid phase resin obtained above was swollen in DMF, separately synthesized 3 eq of compound P0 (334 mg), 2.6 eq of HATU (57 mg), and 6 eq of DIEA (52 µL) were added and the mixture was shaken at 25°C for 2 hr 30 min. The resin was washed 3 times with DMF and 3 times with methylene chloride, and dried under reduced pressure. Then, reactant cocktail-A (a mixture of TFA/H₂O/TIS/DODT in a volume ratio of 92.5:2.5:2.5:2.5) was added to the reaction vessel containing the solid-phase resin, and the mixture was shaken at room temperature for 5 min. The reaction solution was collected by filtration through a frit. The solid-phase resin remaining in the reaction vessel was shaken again with the cutting cocktail, and the solution components were collected from the frit and mixed with the above-mentioned filtrate. When this filtrate was added to an excess diisopropyl ether cooled to 0°C, a cloudy white precipitate was formed. This mixture was centrifuged (9500 rpm, 1 min), and the solution was decanted and dried under reduced pressure.

The obtained crude product was purified using the following conditions (column: Waters Xbridge (registered trademark) C18 5 um 50x250 mm; mobile phase: A=0.1% TFA in H₂O, B=0.1% TFA in MeCN; temperature: 60°C; gradient (%B): 3.3-3.3% over 5 min, then 3.3-7.3% over 2 min, then 7.3-32.7% over 3 min, then 32.7-37.8% over 15.5 min, then 37.8-60% over 1.5 min; flow: 18-18 mL/min over 5 min, 18-118 mL/min over 2 min, then 118 mL/min).

The purity of the target product was calculated from the area ratio of LC/MS (UV wavelength 225 nm) chromatogram under analytical conditions and was 98.3%.

Analysis conditions: retention time =4.64 min; column: Kinetex EVO C18 2.6 µm 2.1x150 mm, 100Å; mobile phase: A=0.025% TFA in H₂O, B=0.025% TFA in MeCN; temperature: 40°C: gradient (%B conc): 20-60% over 7.15 min, then 60-95% over 0.3 min, then 95-95% over 1.55 min; flow: 0.5 mL/min
ESI-MS(+) observed value m/z=970.12(M+3H)³⁺

### Reference Example 5

### Synthesis of compound P4 (SEQ ID NO: 547 in Table 17)

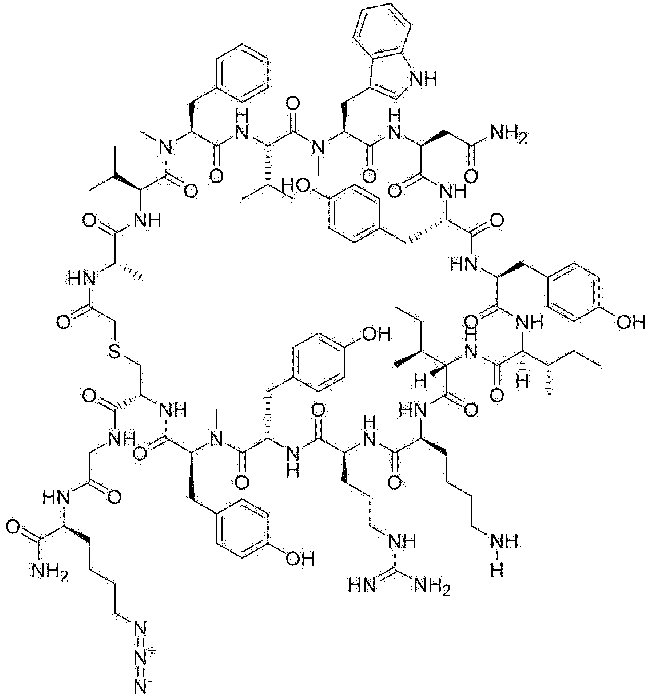

Using Sieber amide resin (Watanabe Chemical, 0.47 mmol/g, 0.42 g), the target peptide was synthesized by starting from the removal of the Fmoc group according to the general method described above. At that time, CEM's Liberty Blue was used as a solid phase synthesizer, and the synthesis was performed according to the manufacturer's manual. For the introduction of each residue, Fmoc-AA/DIPCDI/Oxyma Pure (5.3eq/10eq/5eq) was used for 1 eq of resin, and the reaction was performed once for 10 min in DMF at 75°C. However, for the 2nd residue and the 4th residue, the reaction was performed three times at 75°C for 30 min. For the 7th residue and the 13th residue, the reaction was performed twice at 75°C for 10 min. For the 12th residue, the reaction was performed twice at 50°C for 15 min. For the 15th residue, the reaction was performed once at 50°C for 15 min. In addition, Fmoc was removed by reacting with a 20% piperidine solution in DMF at 75°C for 3 min. For Fmoc removal at the 2nd residue, the 4th residue, and the 13th residue, the reaction was performed at 25°C for 5 min and then for 10 min. To introduce the chloroacetyl group, the Fmoc group of the α-amino group was removed from the solid-phase resin carrying the Fmoc-protected peptide obtained in the previous step by using the aforementioned method, a 0.1 M chloroacetic acid solution in DMF (about 5 eq), a 0.1 M HATU solution in DMF (about 5 eq), and a 0.2 M DIEA solution in DMF (about 10 eq) were added, and the mixture was shaken at 25°C for 30 min. For deprotection of side chains and cutting out from the solid-phase resin, first, the resin obtained after the chloroacetyl group introduction step was washed with DMF five times and methylene chloride three times, and then dried under reduced pressure. Next, reactant cocktail-A (7 mL, a mixture of TFA/H₂O/TIS/DODT in a volume ratio of 90:2.5:2.5:5) was added to the reaction vessel containing the solid phase resin, and the mixture was shaken at room temperature for 90 min. The reaction solution was collected by filtration through a frit. The solid-phase resin remaining in the reaction vessel was shaken again with the cutting cocktail, and the solution components were collected from the frit and mixed with the above-mentioned filtrate. When this filtrate was added to an excess diethyl ether/hexane (1/1) mixed solvent cooled to 0°C, a cloudy white precipitate was formed. This mixture was centrifuged (9000 rpm, 2 min), and the solution was decanted. The obtained solid was washed again with a small amount of diethyl ether cooled to 0°C, and then dried under reduced pressure. The obtained solid was used in the next cyclization reaction. For the cyclization reaction of the peptide, the peptide was dissolved in DMSO/water (9/1) so that the final concentration was 5 mM based on the number of moles of the solid phase resin, 10 equivalents of triethylamine was added, and the mixture was stirred at room temperature for 30 min. The reaction solution was quenched with 15 eq of acetic acid, and then concentrated under reduced pressure using Genevac EZ-2 Elite.

The obtained crude product was purified using the following conditions (column: Waters Xbridge (registered trademark) C18 5 um 50x150 mm; mobile phase: A=0.1% TFA in H₂O, B=0.1% TFA in MeCN; temperature: 40°C; gradient (%B): 7-32% over 3 min, then 32-37% over 8 min, then 37-60% over 1 min; flow: 120 mL/min).

The purity of the target product was calculated from the area ratio of LC/MS (UV wavelength 225 nm) chromatogram under analytical conditions and was 94.2%.

Analysis conditions: retention time =4.12 min; column: Kinetex EVO C18 2.6 µm 2.1x150 mm, 100Å; mobile phase: A=0.025% TFA in H₂O, B=0.025% TFA in MeCN; temperature: 60°C; gradient (%B conc): 20-60% over 7.15 min, then 60-95% over 0.3 min, then 95-95% over 1.55 min; flow: 0.5 mL/min
ESI-MS(+) observed value m/z=1147.70(M+2H)²⁺

### Reference Example 6

### Synthesis of compound P5 (SEQ ID NO: 645 in Table 17)

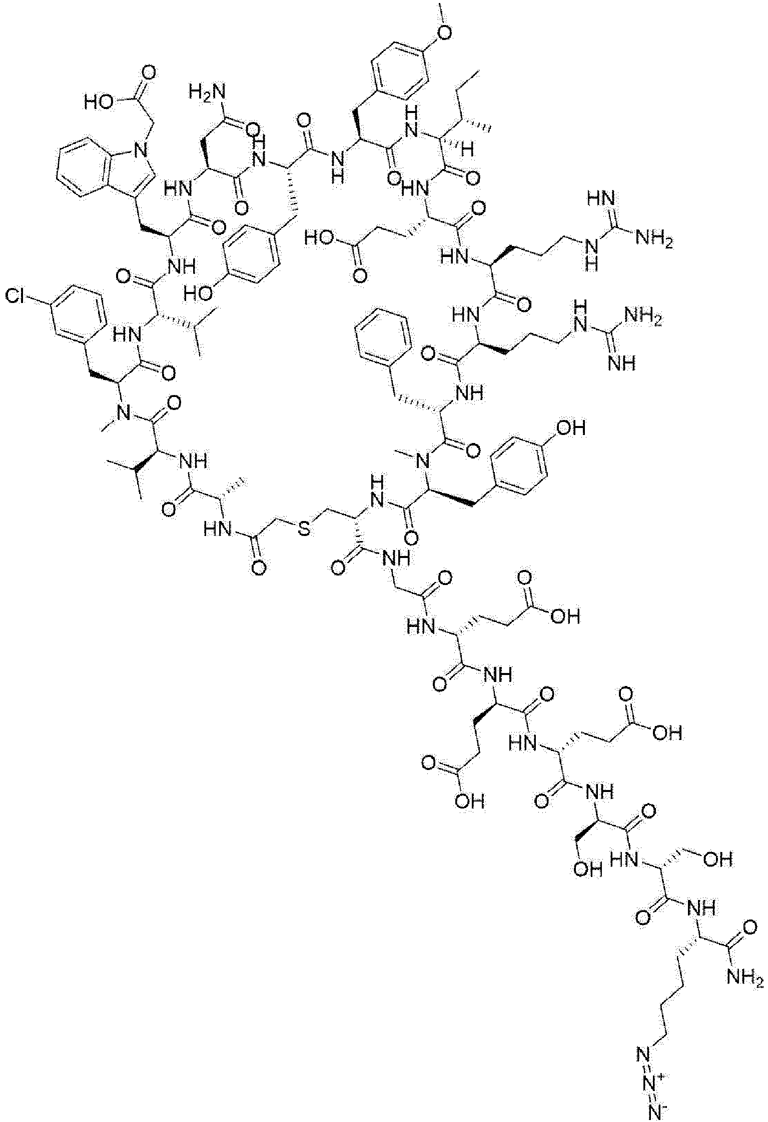

Using Sieber amide resin (Watanabe Chemical, 0.48 mmol/g, 0.52 g), the target peptide was synthesized by starting from the removal of the Fmoc group according to the general method described above. At that time, CEM's Liberty Blue was used as a solid phase synthesizer, and the synthesis was performed according to the manufacturer's manual. For the introduction of each residue, Fmoc-AA/DIPCDI/Oxyma Pure(4.2eq/8eq/4eq) was used for 1 eq of resin, and the reaction was performed once for 3 min in DMF at 90°C. However, for the 2nd residue, the reaction was performed twice at 75°C for 30 min. For the 11th residue and the 12th residue, the reaction was performed twice at 50°C for 15 min. For the 13th residue, the reaction was performed twice at 90°C for 3 min. For the 15th residue, the reaction was performed once at 50°C for 15 min. For the 16th residue, the 17th residue, the 18th residue, the 19th residue, and the 20th residue, the reaction was performed once at 90°C for 10 min. In addition, Fmoc was removed by reacting with a 10% pyrrolidine solution in DMF at 90°C for 1 min. Fmoc removal at the 2nd residue and the 13th residue was performed by reacting at 25°C for 1 min and then for 1 min. To introduce the chloroacetyl group, the Fmoc group of the α-amino group was removed from the solid-phase resin carrying the Fmoc-protected peptide obtained in the previous step by using the aforementioned method, chloroacetic acid (about 10eq), DIPCDI (about 10eq), and HOSu (about 10eq) were shaken in DCM for 60 min, and then ClAcOSu (0.25 M) prepared by adding the same amount of DMF as DCM was added and the mixture was shaken at 25°C for 60 min. For deprotection of side chains and cutting out from the solid-phase resin, first, the resin obtained after the chloroacetyl group introduction step was washed with DMF five times and methylene chloride three times, and then dried under reduced pressure. Next, reactant cocktail-A (15 mL, a mixture of TFA/H₂O/TIS/DODT in a volume ratio of 90:2.5:2.5:5) was added to the reaction vessel containing the solid phase resin, and the mixture was shaken at room temperature for 90 min. The reaction solution was collected by filtration through a frit. The solid-phase resin remaining in the reaction vessel was shaken again with the cutting cocktail, and the solution components were collected from the frit and mixed with the above-mentioned filtrate. When this filtrate was added to an excess diethyl ether/hexane (1/1) mixed solvent cooled to 0°C, a cloudy white precipitate was formed. This mixture was centrifuged (9000 rpm, 2 min), and the solution was decanted. The obtained solid was washed again with a small amount of diethyl ether cooled to 0°C, and then dried under reduced pressure. The obtained solid was used in the next cyclization reaction. For the cyclization reaction of the peptide, the peptide was dissolved in acetonitrile/water (1/1) so that the final concentration was 5 mM based on the number of moles of the solid phase resin, 10 equivalents of triethylamine was added, and the mixture was shaken at room temperature for 30 min. The reaction solution was concentrated under reduced pressure using Genevac EZ-2 Elite.

The obtained crude product was purified using the following conditions (column: Waters Xbridge (registered trademark) C18 5 um 50x150 mm; mobile phase: A=0.1% TFA in H₂O, B=0.1% TFA in MeCN; temperature: 40°C; gradient (%B): 8-8% over 2 min, then 8-33% over 1 min, then 33-38% over 8 min, then 38-60% over 1 min; flow: 20-20 mL/min over 1 min, then 20-120 mL/min over 1 min, then 120 mL/min).

The purity of the target product was calculated from the area ratio of LC/MS (UV wavelength 225 nm) chromatogram under analytical conditions and was 93.3%.

Analysis conditions: retention time =4.17 min; column: Kinetex EVO C18 2.6 µm 2.1x150 mm, 100Å; mobile phase: A=0.025% TFA in H₂O, B=0.025% TFA in MeCN; temperature: 60°C; gradient (%B conc): 20-60% over 7.15 min, then 60-95% over 0.3 min, then 95-95% over 1.55 min; flow: 0.5 mL/min
ESI-MS(+) observed value m/z=1488.62(M+2H)²⁺

### Example 1

### Synthesis of Y6-HDO conjugate

### A) Synthesis of Y4-B1 conjugate

Aqueous solution of compound B1 (2060 µL, 17637 nmol), distilled water (2166 µL), DMSO (8525 µL), 50 mM DMSO solution of BCN-NHS (1764 µL), and DIPEA (184 µL) were mixed in a conical tube, stirred, and precipitated, and the mixture was allowed to stand at room temperature for 1 hr. The reaction mixture was diluted with distilled water, filtered through a 0.45 um membrane filter, and low-molecular-weight compounds and DMSO were removed by gel filtration (Sephadex G-25, fine, distilled water). Fractions containing the target compound were collected and concentrated. The obtained solution was filtered through a 0.45 µm membrane filter, and purified by preparative HPLC using Triart Bio C18 5 µm (150×20 mm I.D.) using linear concentration gradient elution (15 min) at 40°C with SOLUTION A: 15 mM TEA-100 mM HFIP aqueous solution, SOLUTION B: methanol, flow 14 mL/min, A/B: 65/35-62/38. Fractions containing the target compound were collected, concentrated, and freeze-dried to give the title compound (6270 nmol).

### B) Synthesis of Y6-B1 conjugate (compound

C1:[Y6][mGs][mAs][mAs][dc][A][dc][dc][dt][dt][dc][A][G][A][A][d
c][dc][G][G][A][G][G][dcs][mAs][mAs][mC] (SEQ ID NO: 822))

Aqueous solution (1531 µL, 6269 nmol) of Y4-B1 conjugate obtained in the previous step A, DMSO (567 µL), and compound P1 (10 mM DMSO solution, 940 µL) were mixed in a conical tube, stirred, and precipitated, and the mixture was allowed to stand at room temperature for 1 hr. The reaction mixture was filtered through a 0.45 µm membrane filter, and purified by preparative HPLC using Triart Bio C18 5 µm (150×20 mm I.D.) using linear concentration gradient elution (15 min) at 40°C with SOLUTION A: 15 mM TEA-100 mM HFIP aqueous solution, SOLUTION B: methanol, flow 14 mL/min, A/B: 65/35-62/38. Fractions containing the target compound were collected and freeze-dried. The obtained powder was dissolved in distilled water (4 mL), saline (4 mL) was added and the mixture was stirred well, and left standing for 5 min to perform ion exchange. Thereafter, filtered through a 0.45 um membrane filter, and a desalting treatment was performed by gel filtration (Sephadex G-25, fine, distilled water). Fractions containing the target compound were collected, concentrated, and filtered through a 0.20 um membrane filter to give the title compound (4377 nmol).
MS(Calculated): 10748.1

The structure of Y6 is shown below. The BCN moiety shows relative configuration.

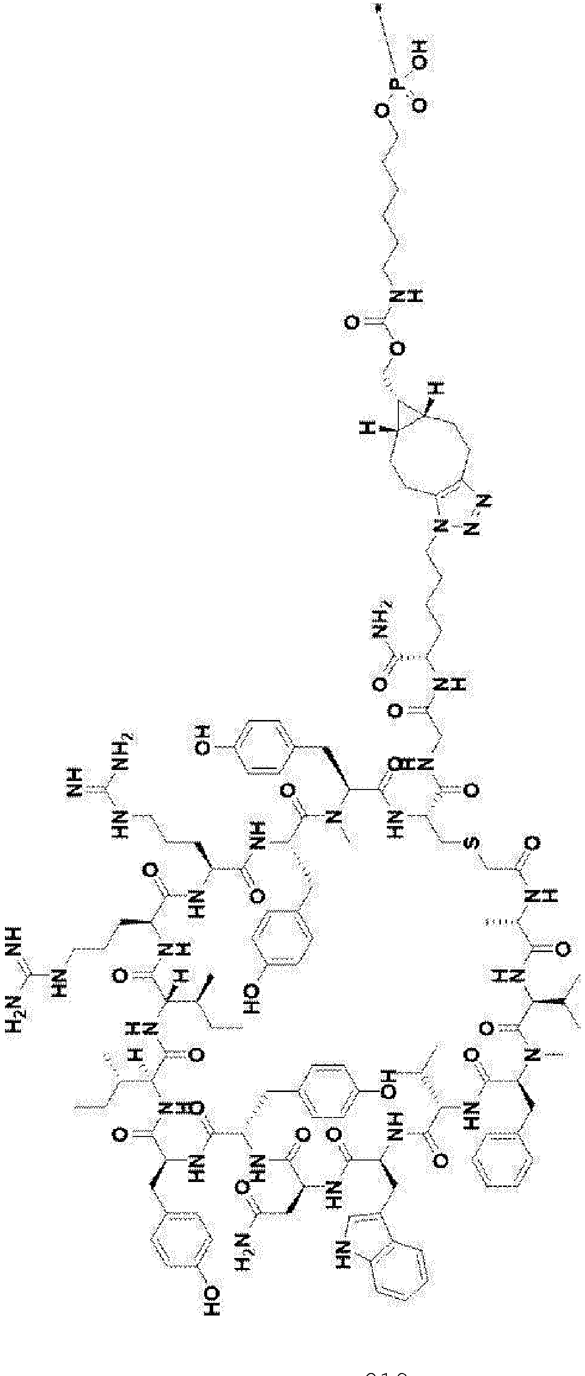

### C) Synthesis of Y6-HDO conjugate

Compound A1 (first nucleic acid strand) and compound C1 (second nucleic acid strand) obtained in the previous step B were mixed in equimolar amounts, and the mixed solution was heated at 90°C for 5 min. Thereafter, the mixture was slowly cooled to room temperature, whereby Y6-HDO conjugate was synthesized.

The peptide-complementary strand conjugates shown in the following Tables 7-1, 7-2 and Table 8 were synthesized in the same manner as in Example 1, step A and step B.

**[Table 7-1]**

| peptide-complementary strand conjugate (human exon53) | | | | |
|---|---|---|---|---|
| compound No. | complementary strand starting material | peptide starting material | Sequence (5' to 3') | MS (Calculated) |
| C2 | B2 | P1 | [Y6] [mGs] [mAs] [mAs] [C] [A] [C] [C] [U] [U] [C] [A] [G] [A] [A] [C] [C] [G] [G] [A] [G] [G] [Cs] [mAs] [mAs ] [mC] (SEQ ID NO: 823) | 10863.9 |
| C3 | B3 | P1 | [Y6] [dgs] [das] [das] [de] [da] [de] [de] [dt] [dt] [de] [da] [dg] [da] [da] [de] [d5] [dg] [dg] [da] [dg ] [dg] [des] [das] [das] [de] (SEQ ID NO: 824) | 10422.9 |
| C4 | B4 | P1 | [Y6] [mGs] [mAs] [mAs] [C] [A] [C] [C] [U] [U] [C] [A] [G] [As] [mAs] [mCs] [mC] (SEQ ID NO: 825) | 7886.4 |
| C5 | B5 | P1 | [Y6] [mGs] [mAs] [mAs] [de] [da] [de] [de] [dt] [dt] [de] [da] [dg] [da] [da] [de] [d5] [dg] [dg] [da] [dg ] [dg] [des] [mAs] [mAs] [mC] (SEQ ID NO: 826) | 10602.5 |
| C6 | B6 | P1 | [Y6] [mGs] [mAs] [mAs] [mC] [A] [mC] [mC] [mU] [mU] [ mC] [A] [G] [A] [A] [mC] [mC] [G] [G] [A] [G] [G] [mCs] [mAs] [mAs] [mC] (SEQ ID NO: 827) | 10990.0 |
| C7 | B7 | P1 | [Y6] [bGs] [bAs] [bAs] [de] [A] [de] [de] [dt] [dt] [ de] [A] [G] [A] [A] [de] [de] [G] [G] [A] [G] [G] [des] [bAs][bAs][b5] (SEQ ID NO: 828) | 10924.1 |
| C8 | B8 | P1 | [Y6] [bG] [bA] [bA] [de] [A] [de] [de] [dt] [dt] [de] [A] [G] [A] [A] [de] [de] [G] [G] [A] [G] [G] [de] [bA] [bA] [b5] (SEQ ID NO: 829) | 10828.2 |
| C9 | B9 | P1 | [Y6] [bGs] [bAs] [bAs] [C] [A] [C] [C] [U] [U] [C] [A] [G] [A] [A] [C] [C] [G] [G] [A] [G] [G] [Cs] [bAs] [bAs ] [b5] (SEQ ID NO: 830) | 11042.0 |
| C10 | B10 | P1 | [Y6] [mGs] [mAs] [mAs] [de] [da] [de] [de] [dt] [dt] [de] [da] [dg] [da] [da] [de] [d5] [dg] [dgs] [mAs] [ mGs] [mG] (SEQ ID NO: 831) | 9397.9 |
| C11 | B11 | P1 | [Y6] [mGs] [mAs] [mAs] [de] [A] [de] [de] [dt] [dt] [ de] [A] [G] [A] [A] [de] [de] [G] [Gs] [mAs] [mGs] [mG ] (SEQ ID NO: 832) | 9495.9 |
| C12 | B12 | P1 | [Y6] [mGs] [mAs] [mAs] [mC] [A] [mC] [mC] [mU] [mU] [ mC] [A] [G] [A] [A] [mC] [mC] [G] [Gs] [mAs] [mGs] [mG ] (SEQ ID NO: 833) | 9708.0 |
| C13 | B13 | P1 | [Y6] [mGs] [mAs] [mAs] [mC] [da] [mC] [mC] [mU] [mU] [mC] [da] [dg] [da] [da] [mC] [mC] [dg] [dgs] [mAs] [ mGs] [mG] (SEQ ID NO: 834) | 9596.0 |
| C15 | B15 | P1 | [Y6] [bGs] [bAs] [bAs] [C] [A] [C] [C] [U] [U] [C] [A] [G] [A] [A] [C] [C] [G] [Gs] [bAs] [bGs] [bG] (SEQ ID NO: 835) | 9759.9 |
| C16 | B16 | P1 | [Y6] [bGs] [bAs] [bAs] [dc] [da] [dc] [dc] [dt] [dt] [de] [da] [dg] [da] [da] [de] [d5] [dg] [dgs] [bAs] [bGs][bG] (SEQ ID NO: 836) | 9561.0 |
| C17 | B17 | P1 | [Y6] [mGs] [mAs] [mAs] [de] [A] [de] [de] [dt] [dt] [ de] [A] [G] [As] [mAs] [mCs] [mC] (SEQ ID NO: 837) | 7817.0 |
| C18 | B18 | P1 | [Y6] [mCs] [mCs] [mUs] [dt] [de] [A] [G] [A] [A] [de] [de] [G] [Gs] [mAs] [mGs] [mG] (SEQ ID NO: 838) | 7908.5 |
| C19 | B19 | P1 | [Y6] [mGs] [mAs] [mAs] [mC] [A] [mC] [mC] [mU] [mU] [ mC] [A] [G] [As] [mAs] [mCs] [mC] (SEQ ID NO: 839) | 7969.0 |
| C20 | B20 | P1 | [Y6] [mCs] [mCs] [mUs] [mU] [mC] [A] [G] [A] [A] [mC] [mC] [G] [Gs] [mAs] [mGs] [mG] (SEQ ID NO: 840) | 8014.6 |
| C21 | B21 | P1 | [Y6] [mCs] [mAs] [mGs] [A] [A] [mC] [mC] [G] [G] [A] [ G] [G] [mCs] [mAs] [mAs] [mC] (SEQ ID NO: 841) | 8046.6 |

**[Table 7-2]**

| compound No. | complementary strand starting material | peptide starting material | Sequence (5' to 3') | MS (Calculated) |
|---|---|---|---|---|
| C26 | B26 | P1 | [Y6] [mGs] [mAs] [mAs] [mC] [da] [mC] [mC] [mU] [mU] [mC] [da] [dg] [das] [mAs] [mCs] [mC] (SEQ ID NO: 842) | 7906.5 |
| C27 | B27 | P1 | [Y6] [mGs] [mAs] [mAs] [de] [A] [de] [C] [dt] [U] [dc ] [da] [dg] [das] [mAs] [mCs] [mC] (SEQ ID NO: 843) | 7787.4 |
| C28 | B28 | P1 | [Y6] [mGs] [mAs] [mAs] [de] [da] [C] [mC] [dt] [U] [d c] [da] [dg] [das] [mAs] [mCs] [mC] (SEQ ID NO: 844) | 7802.6 |
| C29 | B29 | P1 | [Y6] [bGs] [bAs] [bAs] [C] [A] [C] [C] [U] [U] [C] [A] [G][As][bAs][b5s][b5] (SEQ ID NO: 845) | 8075.4 |
| C30 | B30 | P1 | [Y6] [bGs] [bAs] [bAs] [de] [A] [de] [de] [dt] [dt] [ de] [A] [G] [As] [bAs] [b5s] [b5] (SEQ ID NO: 846) | 8007.0 |
| C31 | B31 | P1 | [Y6] [bGs] [bAs] [bAs] [de] [da] [C] [mC] [dt] [U] [d c] [da] [dg] [das] [bAs] [b5s] [b5] (SEQ ID NO: 847) | 7992.9 |
| C32 | B32 | P1 | [Y6] [mGs] [mAs] [mAs] [mC] [mA] [mC] [mC] [mU] [da] [da] [da] [mU] [mC] [mA] [mG] [mAs] [mAs] [mCs] [mC] (SEQ ID NO: 848) | 8965.8 |
| C33 | B33 | P1 | [Y6] [mGs] [mAs] [mAs] [mC] [mA] [mC] [da] [da] [da] [mU] [mU] [mC] [mA] [mG] [mA] [mA] [da] [da] [da] [mC ] [mG] [mGs] [mAs] [mGs] [mG] (SEQ ID NO: 849) | 11046.2 |
| C42 | B42 | P1 | [Y5] [mGs] [mAs] [mAs] [de] [A] [de] [de] [dt] [dt] [ de] [A] [G] [As] [mAs] [mCs] [mC] [Y6] (SEQ ID NO: 850) | 8235.8 |
| C44 | B44 | P1 | [Y6] [dt] [de] [da] [G] [A] [A] [de] [A] [de] [de] [dt ] [dt] [de] [A] [G] [As] [mAs] [mCs] [mC] (SEQ ID NO: 851) | 8634.8 |
| C45 | B45 | P1 | [Y6] [mGs] [A] [A] [de] [A] [de] [de] [dt] [dt] [dc] [ A] [G] [A] [A] [de] [de] [G] [G] [A] [G] [G] [de] [A] [A s] [mC] (SEQ ID NO: 852) | 10629.2 |
| C47 | B47 | P1 | [Y6] [dg] [da] [da] [de] [da] [de] [de] [dt] [dt] [dc ] [da] [dg] [da] [da] [de] [de] (SEQ ID NO: 853) | 7477.1 |
| C49 | B17 | P2 | [Y7] [mGs] [mAs] [mAs] [de] [A] [de] [de] [dt] [dt] [ de] [A] [G] [As] [mAs] [mCs] [mC] (SEQ ID NO: 854) | 8066.7 |
| C50 | B17 | P3 | [Y8] [mGs] [mAs] [mAs] [de] [A] [de] [de] [dt] [dt] [ de] [A] [G] [As] [mAs] [mCs] [mC] (SEQ ID NO: 855) | 8417.9 |
| C51 | B17 | P4 | [Y9] [mGs] [mAs] [mAs] [de] [A] [de] [de] [dt] [dt] [ de] [A] [G] [As] [mAs] [mCs] [mC] (SEQ ID NO: 856) | 7805.6 |
| C65 | B65 | P1 | [Y6] [dg] [da] [da] [de] [da] [de] [de] [dt] [dt] [dc ] [da] [dg] [da] [da] [de] [d5] [dg] [dg] [da] [dg] [d g] [de] [da] [da] [dc] (SEQ ID NO: 857) | 10327. 3 |
| C66 | B66 | P1 | [Y6] [dg] [da] [da] [de] [da] [de] [de] [dt] [dt] [dc ] [da] [dg] [da] [da] [de] [d5] [dg] [dg] [da] [dg] [d g] (SEQ ID NO: 858) | 9123.9 |
| C67 | B1 | P4 | [Y9] [mGs] [mAs] [mAs] [de] [A] [de] [de] [dt] [dt] [ de] [A] [G] [A] [A] [de] [de] [G] [G] [A] [G] [G] [des] [mAs] [mAs] [mC] (SEQ ID NO: 859) | 10735.1 |
| C68 | B1 | P4 | [Y9] [mGs] [mAs] [mAs] [de] [A] [de] [de] [dt] [dt] [ de] [A] [G] [A] [A] [de] [de] [G] [Gs] [mAs] [mGs] [mG ] (SEQ ID NO: 860) | 9481. 8 |
| C86 | B86 | P1 | [Y6] [mGs] [mAs] [mAs] [C] [A] [C] [C] [U] [U] [C] [A] [G] [A] [A] [C] [C] [G] [Gs] [mAs] [mGs] [mG] (SEQ ID NO: 861) | 9595.8 |

**[Table 8]**

| peptide-complementary strand conjugate (mouse exon23) | | | | |
|---|---|---|---|---|
| compound No. | complementary strand starting material | peptide starting material | Sequence (5' to 3') | MS (Calculated) |
| C55 | B55 | P1 | [Y6] [mAs] [mUs] [mUs] [U] [C] [A] [G] [G] [U] [A] [A] [G] [C] [C] [G] [A] [G] [G] [U] [U] [U] [Gs] [mGs] [mCs ][mC](SEQ ID NO: 862) | 10854.1 |
| C56 | B56 | P1 | [Y6] [mAs] [mUs] [mUs] [U] [C] [A] [G] [G] [U] [A] [A] [G] [Cs] [mCs] [mGs] [mA] (SEQ ID NO: 863) | 7942.8 |
| C77 | B77 | P1 | [Y6] [mAs] [mUs] [mUs] [dt] [de] [A] [G] [G] [dt] [A] [A] [G] [de] [de] [G] [A] [G] [G] [dt] [dt] [dt] [Gs] [ mGs][mCs][mC] (SEQ ID NO: 864) | 10797.2 |
| C78 | B78 | P1 | [Y6] [mAs] [mUs] [mUs] [dt] [de] [A] [G] [G] [dt] [A] [A] [G] [des] [mCs] [mGs] [mA] (SEQ ID NO: 865) | 7908.5 |
| C79 | B79 | P1 | [Y6] [mAs] [mUs] [mUs] [mU] [mC] [mA] [da] [da] [da] [mG] [mU] [mA] [mA] [mG] [mC] [mC] [da] [da] [da] [mA ] [mG] [mGs] [mUs] [mUs] [mU] (SEQ ID NO: 866) | 10925.3 |
| C80 | B77 | P4 | [Y9] [mAs] [mUs] [mUs] [dt] [de] [A] [G] [G] [dt] [A] [A] [G] [de] [de] [G] [A] [G] [G] [dt] [dt] [dt] [Gs] [ mGs] [mCs][mC] (SEQ ID NO: 867) | 10785.1 |
| C81 | B81 | P1 | [Y5] [mAs] [mUs] [mUs] [dt] [de] [A] [G] [G] [dt] [A] [A] [G] [des] [mCs] [mGs] [mA] [Y6] (SEQ ID NO: 868) | 8325.9 |
| C82 | B82 | P1 | [Y6] [bAs] [bTs] [bTs] [dt] [de] [A] [G] [G] [dt] [A] [A] [G] [des] [b5s] [bGs] [bA] (SEQ ID NO: 869) | 8112.6 |
| C84 | B84 | P1 | [Y6] [da] [dt] [dt] [dt] [de] [da] [dg] [dg] [dt] [da ] [da] [dg] [de] [d5] [dg] [da] [dg] [dg] [dt] [dt] [d t] [dg] [dg] [de] [dc] (SEQ ID NO: 870) | 10386.4 |

The structure of Y7 in the Table is shown below. The BCN moiety shows relative configuration.

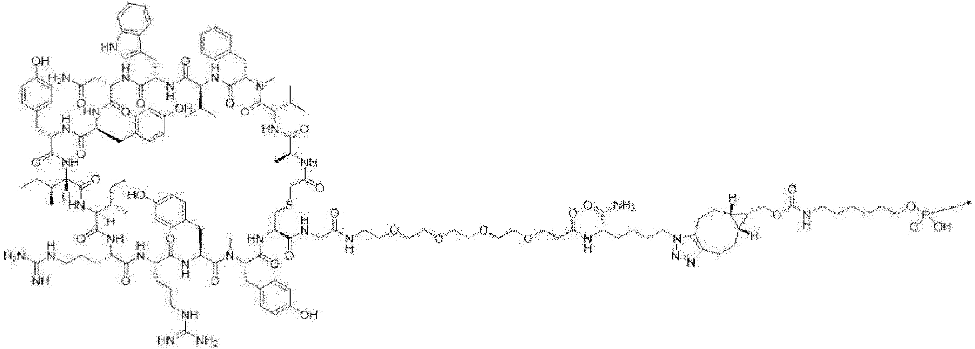

The structure of Y8 in the Table is shown below. The BCN moiety shows relative configuration.

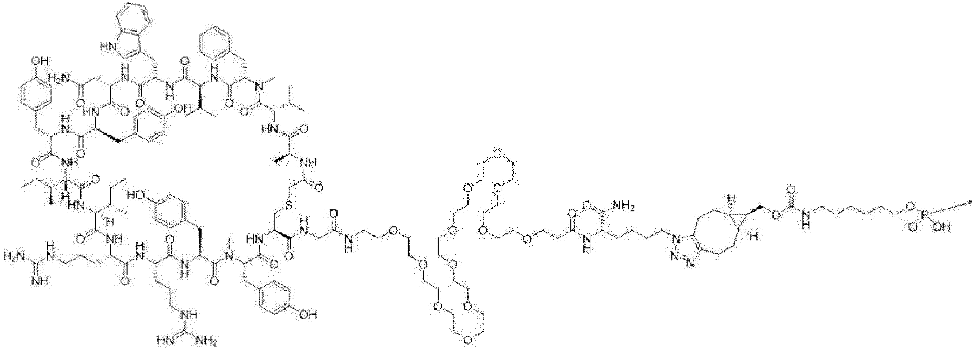

The structure of Y9 in the Table is shown below. The BCN moiety shows relative configuration.

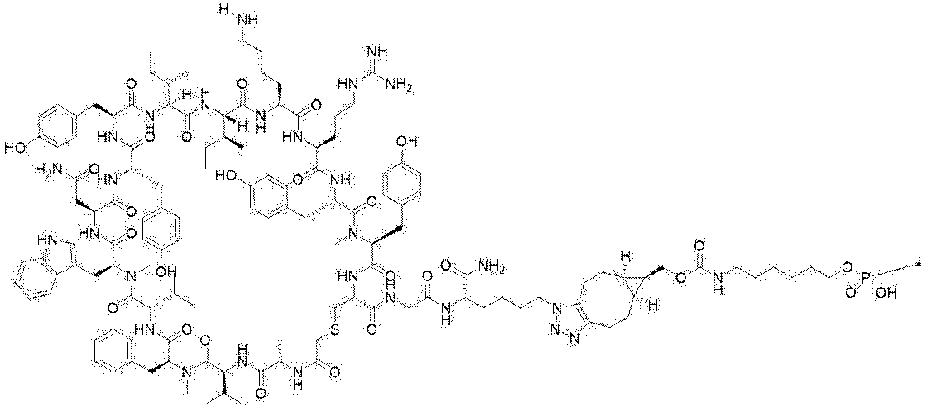

The Example compounds shown in the following Tables 9-1, 9-2 and Table 10 were synthesized in the same manner as in Example 1, step C.

**[Table 9-1] peptide-HDO conjugate (human exon53)**

| Example No. | first nucleic acid strand compound No. | second nucleic acid strand compound No. |
|---|---|---|
| 2 | A1 | C2 |
| 3 | A1 | C3 |
| 4 | A1 | C4 |
| 5 | A1 | C5 |
| 6 | A1 | C6 |
| 7 | A1 | C7 |
| 8 | A1 | C8 |
| 9 | A1 | C9 |
| 10 | A2 | C10 |
| 11 | A2 | C11 |
| 13 | A2 | C13 |
| 14 | A2 | C4 |
| 15 | A2 | C15 |
| 16 | A2 | C16 |
| 17 | A1 | C17 |
| 18 | A1 | C18 |
| 19 | A1 | C19 |
| 20 | A1 | C20 |
| 21 | A1 | C21 |
| 22 | A2 | C17 |
| 23 | A2 | C18 |
| 24 | A2 | C19 |
| 25 | A2 | C20 |
| 26 | A1 | C26 |
| 27 | A1 | C27 |
| 28 | A1 | C28 |
| 29 | A1 | C29 |
| 30 | A1 | C30 |
| 31 | A1 | C31 |
| 32 | A1 | C32 |

**[Table 9-2]**

| Example No. | first nucleic acid strand compound No. | second nucleic acid strand compound No. |
|---|---|---|
| 33 | A1 | C33 |
| 34 | A2 | C26 |
| 35 | A2 | C27 |
| 36 | A2 | C28 |
| 37 | A2 | C29 |
| 38 | A2 | C30 |
| 39 | A2 | C31 |
| 40 | A2 | C32 |
| 41 | A2 | C33 |
| 42 | A1 | C42 |
| 43 | A2 | C42 |
| 44 | A1 | C44 |
| 45 | A1 | C45 |
| 46 | A2 | C44 |
| 47 | A1 | C47 |
| 48 | A2 | C47 |
| 49 | A1 | C49 |
| 50 | A1 | C50 |
| 51 | A1 | C51 |
| 52 | A2 | C49 |
| 53 | A2 | C50 |
| 54 | A2 | C51 |

**[Table 10] peptide-HDO conjugate (mouse exon23)**

| Example No. | first nucleic acid strand compound No. | second nucleic acid strand compound No. |
|---|---|---|
| 55 | A4 | C55 |
| 56 | A4 | C56 |

### Example 57

### Synthesis of Y10-PMO conjugate

([Y1][MoG][MoT][MoT][MoG][MoC][MoC][MoT][MoC][MoC][MoG][MoG][Mo T][MoT][MoC][MoT][MoG][MoA][MoA][MoG][MoG][MoT][MoG][MoT][MoT][ MoC][Y10] (SEQ ID NO: 871)

### A) Synthesis of BCN-AEEA-Arg-Arg-Arg-βAla-OH (SEQ ID NO: 872)

Fmoc-βAla-Alko resin (0.62 meq/g, 80.65 mg, 0.05 mmol) was added to the reaction tube, it was set in a peptide synthesizer, and the amino acids were sequentially elongated by protocol using 20% piperidine/NMP to deprotect the Fmoc group [50°C, 5 min reaction] and using 5 equivalents of Fmoc-amino acid/DIPCDI/OxymaPure for Fmoc-amino acid condensation [50°C, 15 min reaction]. At this time, a double coupling method was used in which all condensation reactions were carried out at 50°C for 15 min, the solution was removed by filtration, and the same condensation reaction was carried out again. After the solid phase synthesis was completed, the resin was washed with MeOH and dried under reduced pressure to obtain the desired protected peptide resin H-AEEA-Arg(Pbf)-Arg(Pbf)-Arg(Pbf)-βAla-Alko resin. Subsequently, 1.5 mL of TFA:water:triisopropylsilane (95:2.5:2.5) was added to the entire amount of the obtained resin, and the mixture was stirred for 2.0 hr. Diethyl ether was added to the reaction solution to obtain a precipitate, and after centrifugation, the supernatant was removed. This operation was repeated twice to wash the precipitate. The residue was dissolved in a 50% acetonitrile aqueous solution, the resin was removed by filtration, and then freeze-dried. The obtained crude peptide H-AEEA-Arg-Arg-Arg-βAla-OH was dissolved in 50% acetonitrile aqueous solution (1.2 mL), BCN-NHS 130 mM 75% acetonitrile aqueous solution (0.5 mL), and DIPEA (34.8 µL) were added and stirred at room temperature for 2 hr. The reaction solution was diluted with 20% acetonitrile water, filtered through a 0.45 um membrane filter, and subjected to preparative HPLC using Phenomenex Kinetex 5 µ XB-C18 (250×21.2 mmI.D), and purified by linear concentration gradient elution (60 min) with SOLUTION A: 0.1% TFA-water, SOLUTION B: 0.1% TFA-containing acetonitrile at a flow of 8 mL/min to A/B: 76/24-66/34. The above-mentioned procedure was repeated once again using Fmoc-βAla-Alko resin (0.62 meq/g, 80.65 mg, 0.05 mmol). (At this time, preparative HPLC was performed using Phenomenex Kinetex 5 µ XB-C18 (250×21.2 mmI.D), linear concentration gradient elution (60 min) with SOLUTION A: 0.1% TFA-water, SOLUTION B: 0.1% TFA-containing acetonitrile at a flow of 8 mL/min to A/B: 79/21-69/31 for purification.) Fractions containing the target compound were collected and freeze-dried to obtain the title compound as a white powder (55.3 mg).
(M+H)⁺ 879.474 by mass spectrometry HPLC elution time: 4.86 min elution conditions:
column: Kinetex 1.7 µm XB-C18 100A, (100×2.1 mmID).
eluent: Using SOLUTION A: 0.1% TFA-water, SOLUTION B: 0.1% TFA-containing acetonitrile to A/B: 95/5 - 45/55. linear concentration gradient elution (10 min).
flow rate: 0.5 mL/min
temperature: 40°C

### B) Synthesis of Y10-PMO conjugate

([Y1][MoG][MoT][MoT][MoG][MoC][MoC][MoT][MoC][MoC][MoG][MoG][Mo T][MoT][MoC][MoT][MoG][MoA][MoA][MoG][MoG][MoT][MoG][MoT][MoT][ MoC][Y10])

BCN-AEEA-Arg-Arg-Arg-βAla-OH (100 mM DMSO solution, 24 µL) obtained in the previous step A, HATU (75 mM DMSO solution, 48 µL), DIPEA (500 mM DMSO solution, 30 µL), and compound A1 (5950 µM DMSO solution, 336 µL, 2000 nmol) were mixed in a microtube, stirred, and precipitated, and then reacted at room temperature for 3 hr. Subsequently, compound P1 (50 mM DMSO solution, 60 µL) was added to the reaction solution, and the mixture was stirred and precipitated, followed by reaction overnight at room temperature. The reaction solution was diluted with 0.1% TFA-water, filtered through a 0.45 um membrane filter, and subjected to preparative HPLC using Phenomenex Kinetex 5 µ XB-C18 (250×21.2 mmI.D), and purified in triplicat by linear concentration gradient elution (30 min) with SOLUTION A: 0.1% TFA-water, SOLUTION B: 0.1% TFA-containing acetonitrile at a flow of 8 mL/min to A/B: 75/25-65/35. Fractions containing the target compound were concentrated, and freeze-dried. 5 mM hydrochloric acid was added to the residue, and the mixture was stirred thoroughly, and desalted by ultrafiltration (Amicon Ultra 3kDa, manufactured by Merck Millipore, distilled water). The final product was filtered through a 0.20 um membrane filter, and freeze-dried to give the title compound (356 nmol). MS(Calculated): 11639.0

The structure of Y10 is shown below. The BCN moiety shows relative configuration.

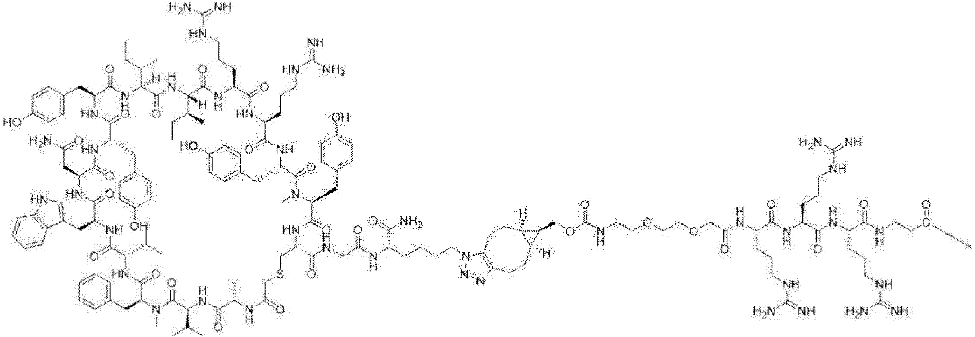

### Example 58

### Synthesis of Y11-PMO conjugate

([Y1][MoG][MoT][MoT][MoG][MoC][MoC][MoT][MoC][MoC][MoG][MoG][Mo T][MoT][MoC][MoT][MoG][MoA][MoA][MoG][MoG][MoT][MoG][MoT][MoT][ MoC][Y11]) (SEQ ID NO: 873)

### A) Synthesis of 3-maleimide propionylA1

3-Maleimidopropionic acid (50 mM NMP solution, 80 µL), HATU (50 mM NMP solution, 80 µL), and DIPEA (100 mM NMP solution, 80 µL) were mixed in a microtube, stirred, and precipitated, and left standing at room temperature for 5 min. Compound A1 (DMSO solution, 663 µL, 2000 nmol) was added, stirred, and precipitated, and the mixture was reacted at room temperature for 1 hr. The reaction mixture was diluted with acetonitrile/water (20/80) solution, filtered through a 0.45 um membrane filter, subjected to preparative HPLC using Phenomenex Kinetex 5 um XB-C18 (150×21.2 mm I.D.) and purified by linear concentration gradient elution (30 min) with SOLUTION A: 0.1% TFA-water, SOLUTION B: 0.1% TFA-containing acetonitrile at a flow of 8 mL/min to A/B: 79/21-69/31. Fractions containing the target compound were collected, concentrated, filtered through a 0.20 um membrane filter to give the title compound (1611 nmol).

### B) Synthesis of BCN-Gly-Gly-Gly-Glu-Glu-Glu-Ser-Ser-Cys(tBuS)-NH₂ (SEQ ID NO: 874)

Sieber amide resin (0.6 meq/g, 83.3 mg, 50 µmol) was added to the reaction tube, it was set in a peptide synthesizer, and the amino acids were sequentially elongated by protocol using 20% piperidine/NMP to deprotect the Fmoc group [50°C, 5 min reaction] and using 5 equivalents of Fmoc-amino acid/N,N'-diisopropyl carbodiimide/OxymaPure for Fmoc-amino acid condensation [50°C, 15 min reaction]. After elongation, the resulting resin was washed with MeOH and dried under reduced pressure to obtain the desired protected peptide resin H-Gly-Gly-Gly-Glu(OtBu)-Glu(OtBu)-Glu(OtBu)-Ser(tBu)-Ser(tBu)-Cys(tBuS) (SEQ ID NO: 875)-Sieber amide resin (133 mg). 1.4 mL of TFA:m-cresol:thioanisole:1,2-ethanedithiol:water:triisopropylsilane (80:5:5:5:2.5:2.5) was added to the entire amount of the obtained resin, and the mixture was stirred for 1.5 hr. Diethyl ether was added to the reaction solution to obtain a precipitate, and after centrifugation, the supernatant was removed. This operation was repeated twice to wash the precipitate. DMSO (1 mL), BCN-NHS (21.8 mg), and DIPEA (17.4 µL) were added to the residual crude peptide H-Gly-Gly-Gly-Glu-Glu-Glu-Ser-Ser-Cys(tBuS)-NH₂ (SEQ ID NO: 876), and the mixture was stirred at room temperature for 18 hr. The reaction solution was diluted with acetonitrile/water (20/80) solution, filtered through a 0.45 um membrane filter, and subjected to preparative HPLC using DAISOPAK SP-100-5-ODS-P (250×20 mm I.D.) and purified by linear concentration gradient elution (60 min) with SOLUTION A: 0.1% TFA-water, SOLUTION B: 0.1% TFA-containing acetonitrile at a flow of 8 mL/min to A/B: 74/26-64/36. Fractions containing the target compound were collected and freeze-dried to obtain 35.4 mg of a white powder. MS:[M+Na]⁺ 1139.304

### C) Synthesis of P1-BCN-Gly-Gly-Gly-Glu-Glu-Glu-Ser-Ser-Cys(tBuS)-NH₂

BCN-Gly-Gly-Gly-Glu-Glu-Glu-Ser-Ser-Cys(tBuS)-NH₂ obtained in the previous step B in an equal volume mixture (1041 µL, 16500 nmol) of acetonitrile and neutral phosphate pH standard solution (pH 6.86), DMSO (2112 µL), and 50 mM DMSO solution of compound P1 (198 µL) were mixed in a conical tube, stirred, precipitated, and then left overnight at room temperature. Thereafter, the temperature was raised to 60 degrees and the mixture was left for 1 hr. The reaction mixture was diluted with acetonitrile/water (20/80) solution, filtered through a 0.45 um membrane filter, subjected to preparative HPLC using DAISOPAK SP-100-5-ODS-P (250×20 mm I.D.), and purified by linear concentration gradient elution (60 min) with SOLUTION A: 0.1% TFA-water, SOLUTION B: 0.1% TFA-containing acetonitrile at a flow of 8 mL/min to A/B: 74/26-64/36. Fractions containing the target compound were collected and freeze-dried to give 22.0 mg of a white powder.

### D) Synthesis of P1-BCN-Gly-Gly-Gly-Glu-Glu-Glu-Ser-Ser-Cys-NH₂

The protected peptide P1-BCN-Gly-Gly-Gly-Glu-Glu-Glu-Ser-Ser-Cys(tBuS)-NH₂ (20.5 mg) obtained in previous step C was dissolved in DMF (1 mL), a 300 mM aqueous solution (200 µL) of tris (2-carboxyethyl)phosphine hydrochloride was added, and the mixture was stirred at 50°C overnight. The reaction mixture was diluted with acetonitrile/water (5/95) solution, filtered through a 0.45 um membrane filter, subjected to preparative HPLC using Phenomenex Kinetex 5 um XB-C18 (150×21.2 mm I.D.) and purified by linear concentration gradient elution (36 min) with SOLUTION A: 0.1% TFA-water, SOLUTION B: 0.1% TFA-containing acetonitrile at a flow of 8 mL/min to A/B: 79/21-69/31. Fractions containing the target compound were collected and freeze-dried to give 18.0 mg of a white powder.

### E) Synthesis of Y11-PMO conjugate

([Y1][MoG][MoT][MoT][MoG][MoC][MoC][MoT][MoC][MoC][MoG][MoG][Mo T][MoT][MoC][MoT][MoG][MoA][MoA][MoG][MoG][MoT][MoG][MoT][MoT][ MoC][Y11])

A DMSO solution (710 µL, 1611 nmol) of 3-maleimide propionyl A1 obtained in step A, neutral phosphate pH standard solution (pH 6.86) (316 gL), and 50 mM DMF solution (48 µL) of peptide P1-BCN-Gly-Gly-Gly-Glu-Glu-Glu-Ser-Ser-Cys-NH₂ obtained in the previous step D were mixed in a microtube, stirred, and precipitated, and reacted at 45°C for 4 hr. The reaction mixture was diluted with acetonitrile/water (5/95) solution, filtered through a 0.45 µm membrane filter, subjected to preparative HPLC using YMC-Actus Triart C8 S-5 um 12nm (250×20.0 mm I.D.), and purified by linear concentration gradient elution (60 min) with SOLUTION A: 0.1% TFA-water, SOLUTION B: 0.1% TFA-containing acetonitrile at a flow of 8 mL/min to A/B: 67/33-57/43. Fractions containing the target compound concentrated, and freeze-dried. 5 mM hydrochloric acid was added to the residue, and the mixture was stirred thoroughly, and desalted with distilled water by ultrafiltration (Amicon Ultra 3kDa, manufactured by Merck Millipore, distilled water). The final product was filtered through a 0.20 um membrane filter to give the title compound (362 nmol).
MS(Calculated): 11956.7

The structure of Y11 is shown below. The BCN moiety shows relative configuration.

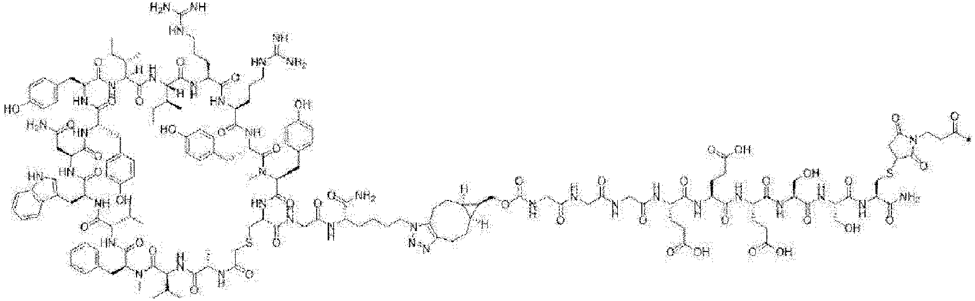

### Example 59

### Synthesis of Y12-PMO conjugate

([Y1][MoG][MoT][MoT][MoG][MoC][MoC][MoT][MoC][MoC][MoG][MoG][Mo T][MoT][MoC][MoT][MoG][MoA][MoA][MoG][MoG][MoT][MoG][MoT][MoT][ MoC][Y12] (SEQ ID NO: 877))

### A) Synthesis of BCN-A1

Compound A1 (2964 µM DMSO solution, 675 µL, 2000 nmol), DMSO (771 µL), BCN-NHS (50 mM DMSO solution, 200 µL), and DIPEA (20.8 µL) were mixed in a microtube, stirred, precipitated, and left standing at room temperature for 0.5 hr. Distilled water (10 mL) was added, and ultrafiltration (Amicon Ultra 3kDa, manufactured by Merck Millipore, distilled water) was performed to give the title compound as an aqueous solution (0.5 mL, 3014 µM, 1507 nmol).

### B) Synthesis of Y12-PMO conjugate

([Y1][MoG][MoT][MoT][MoG][MoC][MoC][MoT][MoC][MoC][MoG][MoG][Mo T][MoT][MoC][MoT][MoG][MoA][MoA][MoG][MoG][MoT][MoG][MoT][MoT][ MoC][Y12])

An aqueous solution (199 µL, 600 nmol) of BCN-A1 obtained in the previous step A, DMSO (710 µL), and compound P5 (10 mM DMSO solution, 90 µL) were mixed in a microtube, stirred, and precipitated, and the mixture was allowed to stand at room temperature for 1 hr. The reaction mixture was diluted 2 times with acetonitrile/distilled water =1/1, filtered through a 0.45 µm membrane filter, and purified by HPLC (Aeris PEPTIDE, XB-C18 column, 3.6 um, 150 × 4.6 mm, SOLUTION A: 0.1% TFA in DW, SOLUTION B: 0.1% TFA in acetonitrile). Fractions containing the target compound were collected and freeze-dried, the obtained powder was dissolved in distilled water (1 mL), 5 mM HCl aqueous solution (0.5 mL) was added, stirred well, and left for 5 min to perform ion exchange. Thereafter, ultrafiltration (Amicon Ultra 3kDa, manufactured by Merck Millipore, distilled water) was performed, and the solution containing the target compound was filtered through a 0.20 um membrane filter to give the title compound (88.2 nmol).
MS(Calculated): 11620.7

The structure of Y12 is shown. The BCN moiety shows relative configuration.

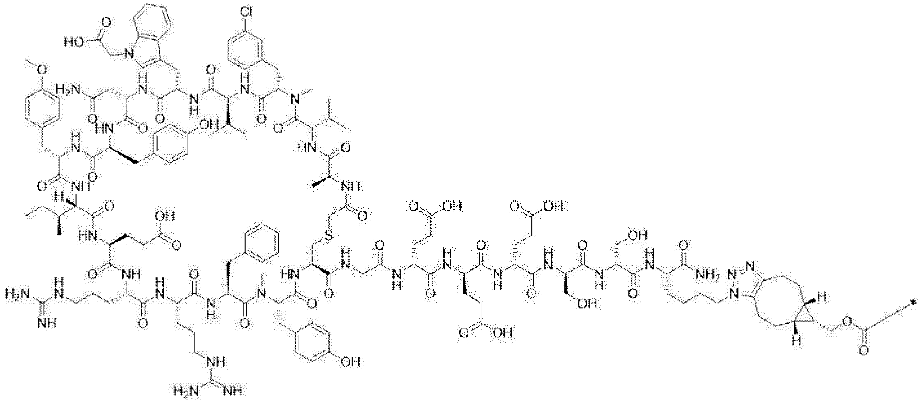

### Example 60

### Synthesis of Y13-PMO conjugate

([Y1] [MoG] [MoG] [MoC] [MoC] [MoA] [MoA] [MoA] [MoC] [MoC] [MoT] [MoC] [Mo G][MoG][MoC][MoT][MoT][MoA][MoC][MoC][MoT][MoG][MoA][MoA][MoA][ MoT][Y13] (SEQ ID NO: 878))

### A) Synthesis of 4-pentynoyl-AEEA-Arg-Arg-Arg-Gly-OH (SEQ ID NO: 879)

Fmoc-Gly-Alko resin (0.78 meq/g, 64.10 mg, 0.05 mmol) was added to the reaction tube, it was set in a peptide synthesizer, and the amino acids were sequentially elongated by protocol using 20% piperidine/NMP to deprotect the Fmoc group [50°C, 5 min reaction], 5 equivalents of Fmoc-amino acid/DIPCDI/OxymaPure for Fmoc-amino acid condensation [50°C, 15 min reaction]. At this time, a double coupling method was used in which all condensation reactions were performed at 50°C for 15 min, the solution was removed by filtration, and the same condensation reaction was performed again. After solid phase synthesis, the resin was washed with MeOH and dried under reduced pressure to obtain the desired protected peptide resin 4-pentynoyl-AEEA-Arg(Pbf)-Arg(Pbf)-Arg(Pbf)-Gly(SEQ ID NO: 880)-Alko resin. Subsequently, 1.5 mL of TFA:water:triisopropylsilane (95:2.5:2.5) was added to the entire amount of the obtained resin, and the mixture was stirred for 2.0 hr. Diethyl ether was added to the reaction solution to obtain a precipitate, and after centrifugation, the supernatant was removed. The obtained crude peptide 4-pentynoyl-AEEA-Arg-Arg-Arg-Gly-OH was dissolved in 50% acetonitrile aqueous solution, filtered through a 0.45 um membrane filter, and freeze-dried to give 34.0 mg of a white powder. (M+H)⁺ 769.284 by mass spectrometry HPLC elution time: 2.53 min elution conditions:
column: Kinetex 1.7 µm XB-C18 100A, (100×2.1 mmID).
Using eluent: SOLUTION A: 0.1% TFA-water, SOLUTION B: 0.1% TFA-containing acetonitrile to A/B: 95/5 - 45/55. linear concentration gradient elution (10 min).
flow rate: 0.5 mL/min
temperature: 40°C

### B) Synthesis of 4-pentynoyl-AEEA-Arg-Arg-Arg-Gly-A4 conjugate

A 50 mM DMSO solution (120 µL) of 4-pentynoyl-AEEA-Arg-Arg-Arg-Gly-OH obtained in the previous step A, a 75 mM DMSO solution (120 µL) of HATU, a 500 mM DMSO solution (60 µL) of DIPEA, and a 10110 µM DMSO solution (396.6 µL, 4000 nmol) of compound A4 were mixed in a microtube, stirred, precipitated, and reacted at room temperature for 1 hr. Subsequently, the reaction mixture was diluted with 0.1% TFA-water, filtered through a 0.45 µm membrane filter, subjected to preparative HPLC using Phenomenex Kinetex 5 pXB-C18 (150×21.2 mmI.D) and purified in triplicat by linear concentration gradient elution (30 min) with SOLUTION A: 0.1% TFA-water, SOLUTION B: 0.1% TFA-containing acetonitrile at a flow of 8 mL/min to A/B:75/25-65/35. Fractions containing the target compound were freeze-dried to give 29.8 mg of a white powder.

### C) Synthesis of Y13-PMO conjugate

([Y1][MoG][MoG][MoC][MoC][MoA][MoA][MoA][MoC][MoC][MoT][MoC][Mo G][MoG][MoC][MoT][MoT][MoA][MoC][MoC][MoT][MoG][MoA][MoA][MoA][ MoT][Y13])

Sodium ascorbate (200 mM aqueous solution, 120 µL), copper sulfate (80 mM aqueous solution, 120 µL), and TBTA (80 mM DMSO solution, 120 µL) were mixed in a microtube, stirred, and precipitated. The above-mentioned mixture, 4-pentynoyl-AEEA-Arg-Arg-Arg-Gly-A4 conjugate obtained in the previous step B (1356 mM DMSO solution, 600 µL), compound P1 (50 mM DMSO solution, 72 µL), DMSO (300 µL), and water (100 µL) were mixed in a microtube, stirred, precipitated, and reacted at room temperature for 3 hr. The reaction mixture was diluted with 50% acetonitrile aqueous solution, filtered through a 0.45 um membrane filter, subjected to preparative HPLC using Phenomenex Kinetex 5 µ XB-C18 (150×21.2 mmI.D), and purified in triplicat by linear concentration gradient elution (30 min) with SOLUTION A: 0.1% TFA-water, SOLUTION B: 0.1% TFA-containing acetonitrile at a flow of 8 mL/min to A/B: 69/31-59/41. Fractions containing the target compound were freeze-dried. 5 mM hydrochloric acid was added to the residue, stirred thoroughly, and desalted by ultrafiltration (Amicon Ultra 3kDa, manufactured by Merck Millipore, distilled water). The final product was filtered through a 0.20 um membrane filter, and freeze-dried to give the title compound (366 nmol). MS(Calculated): 11466.8

The structure of Y13 is shown below.

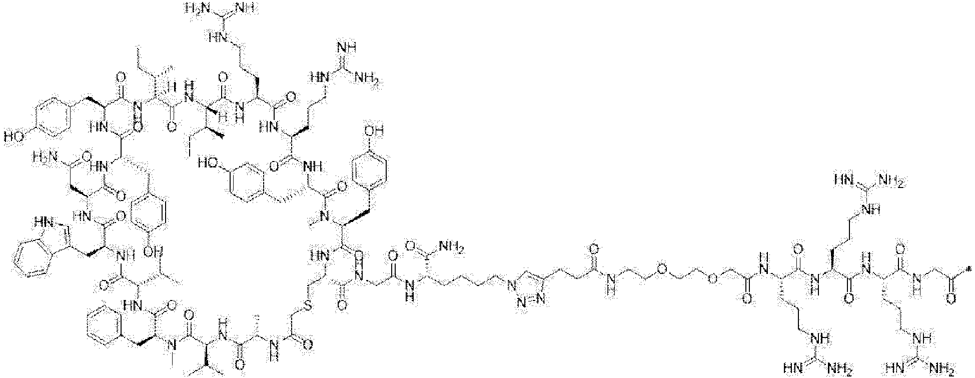

### Example 61

### Synthesis of Y14-PMO conjugate

### A) Synthesis of 4-pentynoyl-AEEA-Lys(ivDde)-Lys(ivDde)-Lys(ivDde)-Gly-OH (SEQ ID NO: 882)

### Synthesized in the same manner as in Example 60, step A. MS:[M+H]⁺ 1303.475

### B) Synthesis of 4-pentynoyl-AEEA-Lys(ivDde)-Lys(ivDde)-Lys(ivDde)-Gly-A4 conjugate

4-Pentynoyl-AEEA-Lys(ivDde)-Lys(ivDde)-Lys(ivDde)-Gly-OH obtained in the previous step A (100 mM DMSO solution, 90 µL) was mixed with HATU (75 mM DMSO solution, 240 µL), DIPEA (500 mM DMSO solution, 180 µL), and compound A4 (10110 µM, DMSO solution, 593.5 µL, 6000 nmol) in a microtube, stirred, precipitated, and reacted at room temperature for 24 hr. The reaction mixture was diluted with 0.1% TFA-water, filtered through a 0.45 µm membrane filter, subjected to preparative HPLC using Phenomenex Kinetex 5 µ XB-C18 (150×21.2 mmI.D), and purified in triplicat by linear concentration gradient elution (30 min) with SOLUTION A: 0.1% TFA-water, SOLUTION B: 0.1% TFA-containing acetonitrile at a flow of 8 mL/min to A/B: 66/34-49/51. Fractions containing the target compound were concentrated and freeze-dried to give the title compound (3436 nmol).

### C) Synthesis of Y14-PMO conjugate

([Y1][MoG][MoG][MoC][MoC][MoA][MoA][MoA][MoC][MoC][MoT][MoC][Mo
G][MoG][MoC][MoT][MoT][MoA][MoC][MoC][MoT][MoG][MoA][MoA][MoA][
MoT][Y14])

4-Pentynoyl-AEEA-Lys(ivDde)-Lys(ivDde)-Lys(ivDde)-Gly-A4 conjugate obtained in the previous step B (8590 µM DMSO solution, 200 µL, 1718 nmol), sodium ascorbate (400 mM aqueous solution, 85.9 µL), copper sulfate (80 mM aqueous solution, 172 µL), TBTA (80 mM DMSO solution, 172 µL), and compound P1 (20 mM NMP solution, 172 µL) were mixed in a microtube, stirred, precipitated, and reacted at room temperature for 16 hr. The reaction mixture was diluted with 0.1% TFA-water, filtered through a 0.45 um membrane filter, subjected to preparative HPLC using Phenomenex Kinetex 5 µ XB-C18 (150×21.2 mmI.D), and purified by linear concentration gradient elution (30 min) with SOLUTION A: 0.1% TFA-water, SOLUTION B: 0.1% TFA-containing acetonitrile at a flow of 8 mL/min to A/B: 64/36-51/49. Fractions containing the target compound were concentrated and freeze-dried.

A DMSO solution (236 µL) of the obtained powder, hydrazine (80 mM aqueous solution, 177 µL), and DMSO (400 µL,) were mixed in a microtube, stirred, precipitated, and reacted at room temperature for 2 hr. Thereafter, hydrazine hydrate (68.8 µL) was added, stirred, precipitated, and reacted at room temperature for 20 hr. The reaction mixture was diluted with 50% acetonitrile aqueous solution, filtered through a 0.45 µm membrane filter, subjected to preparative HPLC using Phenomenex Kinetex 5 µ XB-C18 (150×21.2 mmI.D), and purified in triplicat by linear concentration gradient elution (30 min) with SOLUTION A: 0.1% TFA-water, SOLUTION B: 0.1% TFA-containing acetonitrile at a flow of 8 mL/min to A/B: 69/31-59/41. Fractions containing the target compound were freeze-dried. 5 mM hydrochloric acid was added to the residue, stirred thoroughly, and desalted by ultrafiltration (Amicon Ultra 3kDa, manufactured by Merck Millipore, distilled water). The final product was filtered through a 0.20 µm membrane filter to give the title compound (111 nmol).
MS(Calculated): 11384.8

The structure of Y14 is shown below.

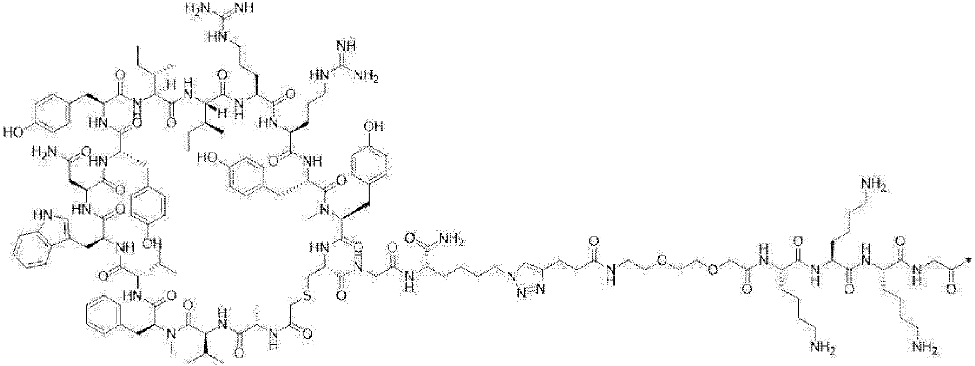

### Example 85

### Synthesis of Y12-PMO conjugate

([Y1][MoG][MoG][MoC][MoC][MoA][MoA][MoA][MoC][MoC][MoT][MoC][Mo
G][MoG][MoC][MoT][MoT][MoA][MoC][MoC][MoT][MoG][MoA][MoA][MoA][
MoT][Y12] (SEQ ID NO: 883))

Using compound A4 and compound P5, an operation similar to that in Example 59, step A and step B to give the title compound.
MS(Calculated): 11560.4

The peptide-HDO conjugates shown in the following Table 11 and Table 12 are synthesized in the same manner as in Example 1, step C.

**[Table 11]**

| peptide-HDO conjugate (human exon53) | | |
|---|---|---|
| Example No. | first nucleic acid strand compound No. | second nucleic acid strand compound No. |
| 12 | A2 | C12 |
| 65 | A1 | C65 |
| 66 | A2 | C66 |
| 67 | A1 | C67 |
| 68 | A2 | C68 |
| 69 | A1 | C10 |
| 70 | A1 | C11 |
| 71 | A1 | C12 |
| 72 | A1 | C13 |
| 73 | A1 | C15 |
| 74 | A1 | C16 |
| 75 | A1 | C66 |
| 86 | A2 | C86 |
| 88 | A3 | C17 |
| 89 | A1 | C86 |

**[Table 12]**

| peptide-HDO conjugate (mouse exon23) | | |
|---|---|---|
| Example | first nucleic acid | second nucleic acid strand |
| No. | strand compound No. | compound No. |
| 77 | A4 | C77 |
| 78 | A4 | C78 |
| 79 | A4 | C79 |
| 80 | A4 | C80 |
| 81 | A4 | C81 |
| 82 | A4 | C82 |
| 84 | A4 | C84 |
| 87 | A5 | C78 |

### Experimental Example

### Intermolecular interaction evaluation test between transferrin receptor (hTfR) and peptide using surface plasmon resonance (SPR)

The various synthesized peptide-nucleic acid conjugates were tested for intermolecular interaction by surface plasmon resonance (SPR) of the peptide-nucleic acid conjugate with transferrin receptor (hTfR) using the method shown below. The specific test method is shown below.

### [SPR measurement]

NTA sensor chip (Global Life Science Technologies Japan Co., Ltd.) was inserted into Biacore T200 (Global Life Science Technologies Japan Co., Ltd.), prime operation was performed three times with running buffer: 10 mM HEPES pH 8.0 (Nacalai Tesque Co., Ltd.), 150 mM NaCl (Nacalai Tesque Co., Ltd.), 0.05% Tween 20 (Nacalai Tesque Co., Ltd.), 0.1% BSA (SIGMA-ALDRICH), and 1.0% DMSO (FUJIFILM Wako Pure Chemical Corporation), and equilibrated at a flow rate of 30 µL/min. 350mM EDTA solution was reacted for 60 seconds at a flow rate of 10 µL/min, 0.5 mM NiCl₂ solution (Kishida Chemical) was reacted for 60 seconds at a flow rate of 10 µL/min, and the NTA sensor chip was washed with 3 mM EDTA solution (Nacalai Tesque Co., Ltd.) for 60 seconds at a flow rate of 10 µL/min. After mixing 50 µL each of 60 mM EDC solution (Global Life Science Technologies Japan Co., Ltd.) and 650 mM NHS solution (Global Life Science Technologies Japan Co., Ltd.), the mixture was reacted for 420 seconds at a flow rate of 10 µL/min. 150 µL of a 0.5 µM hTfR solution was prepared by diluting with a running buffer and reacted for 300 seconds at a flow rate of 10 µL/min to immobilize hTfR on the NTA sensor chip. After immobilization, capping was performed by reacting with a 1.0 M ethanolamine aqueous solution (Global Life Science Technologies Japan Co., Ltd.) at a flow rate of 10 µL/min for 420 seconds. A peptide-nucleic acid conjugate solution adjusted to 10 mM in DMSO solution was diluted with running buffer to a final concentration of 10 µM peptide-nucleic acid conjugate solution, and 100 nM, 50 nM, 25 nM, 10 nM, 5 nM peptide-nucleic acid conjugate solutions were prepared. Using the aforementioned samples, the kinetics of the peptide against hTfR was obtained by SPR measurement.

The kinetics evaluation model was Single Cycle Kinetics, and curve fitting was performed using Biacore T200 Evaluation Software Version 3.0 (Global Life Science Technologies Japan Co., Ltd.). The obtained sensorgram was subjected to curve fitting using the least squares method, and the binding of the peptide-nucleic acid conjugate to hTfR was evaluated by determining the KD value.

The following Tables 13-1 and 13-2 show the measurement results of the KD values of Example compounds to hTfR by SPR. When the KD value is less than 100 nM, it is expressed as A, when it is 100 nM or more and less than 1000 nM, it is expressed as B, and when it is 1000 nM or more, it is expressed as C.

**[Table 13-1]**

| Example No. | KD |
|---|---|
| 1 | A |
| 2 | A |
| 3 | B |
| 4 | A |
| 5 | B |
| 6 | A |
| 7 | A |
| 8 | B |
| 9 | A |
| 10 | A |
| 11 | A |
| 13 | A |
| 14 | A |
| 15 | A |
| 16 | A |
| 17 | B |
| 18 | A |
| 19 | A |
| 20 | A |
| 21 | A |
| 22 | A |
| 23 | A |
| 24 | A |
| 25 | A |
| 26 | A |
| 27 | A |
| 28 | A |
| 29 | A |
| 30 | A |
| 31 | A |
| 32 | A |
| 33 | A |
| 34 | A |
| 35 | A |
| 36 | A |
| 37 | A |
| 38 | A |

**[Table 13-2]**

| Example No. | KD |
|---|---|
| 39 | A |
| 40 | A |
| 41 | A |
| 42 | A |
| 43 | A |
| 44 | A |
| 45 | A |
| 46 | A |
| 47 | A |
| 48 | A |
| 49 | A |
| 50 | A |
| 51 | A |
| 52 | A |
| 53 | A |
| 54 | A |
| 55 | A |
| 56 | A |
| 57 | A |
| 58 | A |
| 60 | A |
| 61 | A |

Synthetic Examples of peptide or linker-added peptides are shown below.

### Synthetic Example 1

### Chemical synthesis

All starting materials, building blocks, reagents, acids, bases, solid-phase resins, and solvents used in the chemical synthesis in the following Examples were either commercially available as they were, or were synthesized using organic chemical techniques by those skilled in the art. In addition, unless otherwise specified, for amino acids containing protecting groups, commercially available products were used.

Elongation of peptide chain in the solid phase resin was performed using the resin described in each Example as a starting material and using commonly used peptide coupling reaction conditions and Fmoc removal reaction conditions. The reaction was performed using an automatic peptide synthesizer, Liberty Blue manufactured by CEM, according to the manufacturer's manual. General amino acids used are listed below, and side chain protecting groups are shown in parentheses.

Fmoc-Trp(Boc)-OH; Fmoc-Thr(tBu)-OH; Fmoc-N-Me-Gly-OH; Fmoc-Asp(OtBu)-OH; Fmoc-N-Me-Phe-OH; Fmoc-Ala-OH; Fmoc-N-Me-Ala-OH; Fmoc-His(Trt)-OH; FYnoc-Tyr (tBu) -OH; Fmoc-Val-OH; Fmoc-HydPro(tBu)-OH; Fmoc-Cys(Trt)-OH; Fmoc-Lys(Mtt)-OH; Fmoc-Ser(tBu)-OH; Fmoc-N-Me-Ser(tBu)-OH.

To introduce the chloroacetyl group, Fmoc group of α-amino group was removed by the aforementioned method from a solid phase resin carrying the Fmoc-protected peptide obtained in the previous step, chloroacetic acid (3 equivalents), 3 equivalents of N,N'-diilopropylcarbodiimide solution in DMF (0.5 M), 3 equivalents of HOAt solution in DMF (0.5 M) were added thereto, and the mixture was shaken at room temperature for 40 min.

For deprotection of side chains and cutting out from the solid-phase resin, first, the resin obtained after the chloroacetyl group introduction step was washed five times each with DMF and methylene chloride, and dried under reduced pressure. Then, reactant cocktail-A (a mixture of TFA/H₂O/TIS/DODT in a volume ratio of 92.5:2.5:2.5:2.5) was added to the reaction vessel containing the solid-phase resin, and the mixture was shaken at room temperature for 150 min. The reaction solution was collected by filtration through a frit. The solid-phase resin remaining in the reaction vessel was shaken again with the cutting cocktail, and the solution components were collected from the frit and mixed with the above-mentioned filtrate. When this filtrate was added to an excess diethyl ether cooled to 0°C, a cloudy white precipitate was formed. The mixture was centrifuged (9000 rpm, 3 min) and the solution was decanted. After washing the obtained solid with a small amount of diethyl ether cooled to 0°C again, the obtained solid was used in the next cyclization reaction.

For the cyclization reaction of the peptide, the peptide was dissolved in DMSO so that the final concentration was 5 mM based on the number of moles of the solid phase resin, 6 equivalents of triethylamine was added, and the mixture was stirred at room temperature for about 16 hr. The obtained reaction solution was acidified with acetic acid and concentrated under reduced pressure using Biotage (registered trademark) V-10 (Biotage Japan).

As a method for purifying the obtained crude purified peptide, reversed-phase preparative HPLC was used with a Waters AutoPurification System-SQD2 single quadruple mass spectrometer, and elution was performed while monitoring m/z ions derived from the target product. It was confirmed that the mass spectrum obtained in the ESI-positive scan mode and the mass spectrum containing multiply charged ions calculated from the molecular formula of the target product matched within the error range of the mass spectrometer used. The purification conditions including the column used are shown in each Example.

The purity of the peptide chemically synthesized in the present invention was determined by any of the following analytical methods.

### (Analysis conditions)

### Analysis conditions A

column; CORTECS (registered trademark) UPLC (registered trademark) C18 column (Japan Waters), 90Å, 1.6 um, 2.1x100 mm mobile phase: 0.025% TFA in MeCN/0.025% TFA in H₂O temperature: 40°C
gradient: 5-95% MeCN/0.025% TFA in H₂O in 5.56 min; linear gradient
flow 0.4 mL/min
detection method: UV 220nm

### Analysis conditions B

column; Kinetex EVO C18 2.6 µm, 2.1 IDx150 mm, 100Å
(Phenomenex)
column temperature: 60°C
mobile phase A: 0.025% TFA in H₂O
mobile phase B: 0.025% TFA in CH₃CN
gradient: described in each Example
flow rate: 0.25 mL/min
detection: PDA (225 nm)

The structure of the chemically synthesized peptide was determined by confirming the molecular weight calculated by considering the amino acids used according to the target sequence and the building blocks used as necessary, by ESI-MS(+) in mass spectrum analysis method. The "ESI-MS(+)" refers to electrospray ionization mass spectrometry performed in positive ion mode. Detected masses were reported in "m/z" units. The compounds with molecular weights approximately greater than 1000 were highly frequently detected as divalent ions or trivalent ions.

The peptides shown in the following Table 14 were synthesized.

**[Table 14]**

| SEQ ID NO: | name | Seq | | | | | | | | | | | | | | | | | | |
|---|---|---|---|---|---|---|---|---|---|---|---|---|---|---|---|---|---|---|---|---|
| | | 1 | 2 | 3 | 4 | 5 | 6 | 7 | 8 | 9 | 10 | 11 | 12 | 13 | 14 | 15 | 16 | 17 | ESI(m/z) | [M+XH)X+ |
| 2 | JCR_hTfR_00089 | Ala | Val | Phe | Val | Trp | Asn | Tyr | Tyr | lie | Ile | Arg | Arg | Tyr | MeTyr | Cys | | | 1042.0 | 2 |
| 3 | JCR_hTfR_100022 | Ala | Val | Phe | Val | Trp | Asn | Tyr | Tyr | Ile | Val | Asp | Arg | Phe | Gly | Thr | Gly | Cys | 1025.6 | 2 |
| 4 | JCR_hTfR_100025 | Ala | Val | Phe | Val | Trp | Asn | Tyr | Tyr | Ile | Val | Gly | Arg | Asn | Ual | Pro | Asn | Cys | 1027.9 | 2 |
| 5 | JCR_hTfR_000809 | Ala | Asn | Arg | lie | Val | Phe | Val | Trp | Asn | Tyr | Tyr | Ile | Ile | Pro | Arg | MeHis | Cys | 740.1 | 3 |
| 6 | JCR_hTfR_000837 | Ala | Val | Trp | Val | Trp | Asn | Tyr | Phe | Ile | Ile | Pro | Arg | His | Cys | | | | 922.4 | 2 |
| 7 | JCR_hTfR_00C895 | Ala | Val | Phe | Val | Trp | Asn | Tyr | Tyr | Ile | Ile | Asp | Arg | Phe | Arg | Ser | MeTyr | Cys | 1135.0 | 2 |
| 8 | JCR_hTfR_000812 | Ala | Val | Phe | Val | Trp | Asn | Tyr | Tyr | Ile | Ile | Asp | Arg | Phe | Arg | Ser | MeHis | Cys | 748. 5 | 3 |
| 9 | JCR_hTfR_000690 | Ala | Vai | Phe | Val | Trp | Asn | Tyr | Tyr | Ile | Ile | Asp | Arg | Phe | Arg | Cys | | | 1003.0 | 2 |
| 10 | JCR_hTfR_100014 | Ala | Ser | Ala | Val | Phe | Val | Trp | Asn | Tyr | Tyr | Ile | Ile | Asn | Arg | Trp | Ala | Cys | 1058.8 | 2 |
| 11 | JCR_hTfR_100019 | Ala | Val | Phe | Val | Trp | Asn | Tyr | Tyr | Ile | Val | Pro | Arg | Glu | Cys | | | | 899.9 | 2 |
| 12 | JCR_hTfR_000687 | Ala | Val | Phe | Val | Trp | Asn | Tyr | Tyr | lie | Val | Pro | Arg | Asn | Cys | | | | 892.4 | 2 |
| 13 | JCR_hTfR_100017 | Ala | Asn | Ser | Arg | Thr | Val | Trp | Val | Trp | Asn | Tyr | Tyr | Ile | Ual | Thr | Arg | Cys | 1086.0 | 2 |
| 14 | JCR_hTfR_000696 | Ala | Asn | Arg | Ile | Val | Phe | Val | Trp | Asn | Tyr | Tyr | Ile | Ile | Pro | Arg | MeTyr | Cys | 1122.6 | 2 |
| 15 | JCR_hTfR_000692 | Ala | Val | Phe | Val | Trp | Asn | Tyr | Tyr | Ile | Val | Pro | Arg | Asp | Cys | | | | 892.9 | 2 |
| 16 | JCR_hTfR_100003 | Ala | Val | Phe | Val | Trp | Asn | Tyr | Tyr | Ile | Val | His | Cys | | | | | | 777.3 | 2 |
| 17 | JCR_hTfR_100016 | Ala | Val | Phe | Val | Trp | Asn | Tyr | Tyr | Ile | Val | Pro | Arg | MeTrp | Cys | | | | 935.5 | 2 |
| 18 | JCR_hTfR_000554 | Ala | Val | Phe | Val | Trp | Asn | Tyr | Tyr | Ile | Val | Ser | Cys | | | | | | 752.2 | 2 |
| 19 | JCR_hTfR_000109 | Ala | Val | Phe | Val | Trp | Asn | Tyr | Tyr | Ile | Ile | Ser | Cys | | | | | | 759.6 | 2 |
| 20 | JCR_hTfR_000569 | Ala | Val | Trp | Val | Trp | Asn | Tyr | Tyr | Ile | Ile | Asn | Cys | | | | | | 792.2 | 2 |

Using Sieber amide resin (Watanabe Chemical, 0.6 mmol/g, 0.33 g), the target peptide was synthesized by starting from the removal of the Fmoc according to the general method described above. At that time, CEM's Liberty Blue HT was used as a solid phase synthesizer, and the synthesis was performed according to the manufacturer's manual. The basic condition for condensation reaction was to react at 75°C for 10 min. The basic condition for Fmoc removal was to react with a 20% piperidine solution in DMF at 75°C for 3 min. To introduce the chloroacetyl group, the Fmoc group of the α-amino group was removed by the aforementioned method from the solid phase resin carrying the Fmoc-protected peptide obtained in the previous step, 5 equivalents of chloroacetic acid solution in DMF (0.2 M), 5 equivalents of HATU solution in DMF (0.5 M), and 10 equivalents of DIPEA solution in DMF (1 M) were added and the mixture was shaken at room temperature for 30 min. For deprotection of side chains and cutting out from the solid-phase resin, first, the resin obtained after the chloroacetyl group introduction step was washed with DMF five times and methylene chloride three times, and then dried under reduced pressure. Then, reactant cocktail (a mixture of TFA/H₂O/TIS/DODT in a volume ratio of 92.5:2.5:2.5:2.5) was added to the reaction vessel containing the solid-phase resin, and the mixture was shaken at room temperature for 90 min. The reaction solution was collected by filtration through a frit. The solid-phase resin remaining in the reaction vessel was shaken again with the cutting cocktail, and the solution components were collected from the frit and mixed with the above-mentioned filtrate. When this filtrate was added to an excess diethylether/hexane (1/1) cooled to 0°C, a cloudy white precipitate was formed. The mixture was centrifuged (10000 rpm, 1 min) and the solution was decanted. After washing the obtained solid with a small amount of diethyl ether cooled to 0°C again, the obtained solid was dried and used in the next cyclization reaction. For the cyclization reaction of the peptide, the peptide was dissolved in DMSO so that the final concentration was 5 mM based on the number of moles of the solid phase resin, 6 equivalents of triethylamine was added, and the mixture was stirred at room temperature for about 16 hr. The obtained reaction solution was concentrated under reduced pressure using Savant Explorer SpeedVac.

The obtained crude product was purified using the following conditions (column; Waters Xbridge (registered trademark) C18 5 um (registered trademark) 50x150 mm: mobile phase; A=0.1% TFA in H₂O, B=0.1% TFA in MeCN: temperature 40°C: gradient (%B); 5-30% over 3 min, then 30-35% over 8 min, then 35-60% over 1 min: flow 120 mL/min).

The obtained target product was analyzed under the above-mentioned analysis conditions A or analysis conditions B, and the structure was confirmed by ESI-MS(+) in mass spectrum analysis method. Table 14 shows the obtained ESI-MS(+) observed value and the value of X when expressed as the number of proton addition (M+XH)X+ in that case.

### Synthetic Example 2

The following peptide or linker-added peptides were synthesized.

### [Synthetic Example 2-1]

### Synthesis of 894 v01 PEG12 (SEQ ID NO: 102)

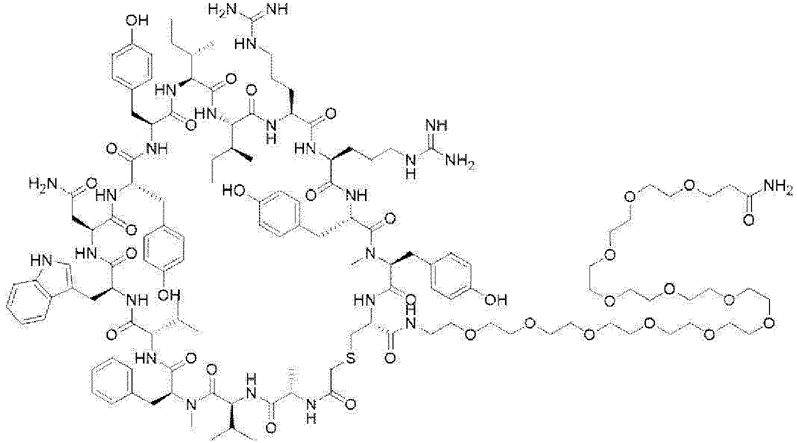

Using Sieber amide resin (Watanabe Chemical, 0.52 mmol/g, 0.19 g), the target peptide was synthesized by starting from the removal of the Fmoc group. At that time, Biotage's SyroI was used as an automatic synthesizer, and the synthesis was performed according to the manufacturer's manual. The basic condition for condensation reaction was to repeat twice at 75°C for 20 min. However, when introducing the 15th residue and the 16th residue, the reactions were performed at room temperature for 60 min and 15 min, respectively. However, when introducing PEG, the reaction was performed once, and when introducing 11th amino acid residue, the condensation reaction was performed three times. The basic conditions for Fmoc removal were to react in a DMF solution of piperazine (5%) and Oxima pure (0.2M) at 50°C for 5 min, and then to react again for 15 min. However, the Fmoc groups at the 15th and 16th residues were removed by heating at 25°C for 5 min and then reacting again for 15 min. To introduce the chloroacetyl group, the Fmoc group of the α-amino group was removed by the aforementioned method from the solid phase resin carrying the Fmoc-protected peptide obtained in the previous step, a DMF solution of 5 equivalents of ClAcOSu was added and the mixture was shaken at room temperature for 60 min. For deprotection of side chains and cutting out from the solid-phase resin, first, the resin obtained after the chloroacetyl group introduction step was washed with DMF five times and methylene chloride three times, and dried under reduced pressure. Then, reactant cocktail-A (a mixture of TFA/H₂O/TIS/DODT in a volume ratio of 92.5:2.5:2.5:2.5) was added to the reaction vessel containing the solid-phase resin, and the mixture was shaken at room temperature for 90 min. The reaction solution was collected by filtration through a frit. The solid-phase resin remaining in the reaction vessel was shaken again with the cutting cocktail, and the solution components were collected from the frit and mixed with the above-mentioned filtrate. When this filtrate was added to an excess diethylether/hexane (1/1) cooled to 0°C, a cloudy white precipitate was formed. This mixture was centrifuged (10000 rpm, 1 min), and the solution was decanted. The obtained solid was washed again with a small amount of diethyl ether cooled to 0°C, and the obtained solid was used in the next cyclization reaction. For the cyclization reaction of the peptide, the peptide was dissolved in DMSO so that the final concentration was 5 mM based on the number of moles of the solid phase resin, 10 equivalents of triethylamine was added, and the mixture was shaken at room temperature for about 1 hr. The obtained reaction solution was concentrated under reduced pressure using Savant Explorer SpeedVaC.

Successively, using the obtained solid phase resin and according to the aforementioned general method, deprotection of the side chain and cutting out from the solid-phase resin, and cyclization reaction were performed.

The obtained crude product was purified using the following conditions (column; Waters Xbridge (registered trademark) C18 5 um OBD (registered trademark) 50x150 mm: mobile phase; A=0.1% TFA in H₂O, B=0.1% TFA in MeCN: temperature 40°C: gradient (%B); 8-33% over 3 min, then 33-38% over 8 min, then 38-60% over 1 min: flow 120 mL/min).

The purity of the target product was calculated from the area ratio of the LC/MS (UV wavelength 225 nm) chromatogram under analysis condition B and was 91.5%.
analysis conditions A: retention time =3.57 min: ESI-MS(+) observed value m/z=Calculated (M+3H)³⁺
analysis conditions B: retention time =12.8 min: gradient (%B cone); 20-60% over 20 min, then 60-95% over 1 min, then 95% over 5 min.
ESI-MS(+) observed value m/z=899.6(M+3H)³⁺

### [Synthetic Example 2-2]

### Synthesis of 894 v05 PEG8 Me (SEQ ID NO: 43)

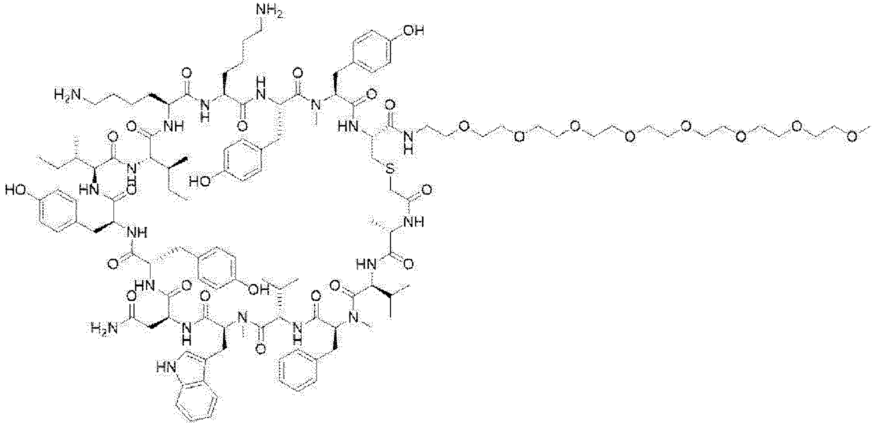

Cl-Trt(2-Cl) resin (1 g, 1.6 mmol, Watanabe Chemical Industry, 1.6 mmol/g) was swollen with DCM (dehydrated, 10 mL, Nacalai Tesque) for 10 min, and after filtration, washed twice with DCM (dehydrated, 10 mL). A DCM (dehydrated, 10 mL) solution of Fmoc-CyS(CH₂COOH)-PEG8Me (767 mg, 1 mmol) and DIEA (836 uL, 4.8 mmol, Watanabe Chemical Industries) were sequentially added to the swollen resin, and the mixture was shaken at room temperature for 35 min. After further adding MeOH (1 mL, Kishida Kagaku) and shaking for 10 min, the resin was washed three times with DMF (10 mL, Kishida Kagaku), three times with DCM (10 mL, Kishida Kagaku), and three times with diethyl ether (10 mL, Kishida Kagaku), it was dried under reduced pressure to obtain Fmoc-CyS[CH₂COO-Trt(2-Cl)-resin]-PEG8 Me (1.686 g, 94%). Using the obtained resin, the target peptide was synthesized by starting from the removal of the Fmoc group according to the general method described above. At that time, Biotage's SyroI was used as a solid phase synthesizer, and the synthesis was performed according to the manufacturer's manual. For the introduction of each residue, Fmoc-AA-OH/HATU/DIEA (3.2eq/3eq/6.3eq) was used for 1 eq of resin, and the reaction was repeated twice in DMF at room temperature for 30 min. When introducing the 1st residue, the 2nd residue, and the 4th residue, the reaction was performed three times at room temperature for 60 min. When introducing the 3rd residue, the reaction was repeated twice at 25°C for 60 min. When introducing the 9th residue, the reaction was repeated three times at room temperature for 30 min. In addition, Fmoc was removed by reacting with a 20% piperidine solution in DMF at room temperature for 5 min, and then reacting again for 15 min. To introduce the chloroacetyl group, the Fmoc group of the α-amino group was removed by the aforementioned method from the solid phase resin carrying the Fmoc-protected peptide obtained in the previous step, 3 equivalents of chloroacetic acid solution in DMF (0.45 M), 3 equivalents of HCTU solution in DMF (0.43 M), 3 equivalents of DIPEA solution in DMF (1.57 M) were added to the solid phase resin, and the mixture was shaken at room temperature for 30 min. For deprotection of side chains and cutting out from the solid-phase resin, first, the resin obtained after the chloroacetyl group introduction step was washed with DMF five times and methylene chloride three times, and then dried under reduced pressure. Subsequently, a reactant cocktail-HFIP/DCM (1/4) was added to the reaction vessel containing the solid-phase resin, and the mixture was shaken at room temperature for 90 min. The reaction solution was collected by filtration through a frit. The solid-phase resin remaining in the reaction vessel was shaken with a cutting cocktail, the solution components were collected from the frit, and the above-described operation of mixing with the filtrate was repeated three times. This filtrate was concentrated under reduced pressure using GenevaC EZ-2 Elite, and then added to an excess diisopropyl ether cooled to 0°C, resulting in a cloudy white precipitate. This mixture was centrifuged (9000 rpm, 5 min), and the supernatant was decanted. The obtained solid was washed again with a small amount of diethyl ether cooled to 0°C, and then dried under reduced pressure. The obtained solid was used in the next cyclization reaction. For the cyclization reaction of the peptide, the peptide was dissolved in DMF so that the final concentration was 2.5 mM based on the number of moles of the solid phase resin, and then 1.1 equivalents of HATU in DMF solution (0.43 M) and 1.5 equivalents of DIPEA were added, and the mixture was shaken at room temperature for about 1 hr. 3 equivalents of acetic acid was added to the obtained reaction solution at room temperature, and the mixture was concentrated under reduced pressure using GenevaC EZ-2. Ice-cooled diisopropyl ether was added to the resulting residue, resulting in a white precipitate. This mixture was centrifuged (9000 rpm, 10 min), and the supernatant was decanted. The obtained solid was washed again with diethyl ether cooled to 0°C and dried under reduced pressure. Reactant cocktail-A (a mixture of TFA/H₂O/TIS/DODT in a volume ratio of 92.5:2.5:2.5:2.5) was added to the obtained solid, and the mixture was shaken at room temperature for 60 min. An ice-cold excess of diisopropyl ether was added and a precipitate formed. The mixture was centrifuged (9000 rpm, 5 min), and the supernatant was decanted. The obtained solid was washed again with ice-cooled diethyl ether and then dried under reduced pressure.

The obtained crude product was purified using the following conditions (column; Waters Xbridge (registered trademark) C18 5 um (registered trademark) 30x150 mm: mobile phase; A=0.1% TFA in H₂O, B=0.1% TFA in MeCN: temperature 40°C: gradient (%B); 8-33% over 3 min, then 33-38% over 8 min, then 38-60% over 1 min: flow 45 mL/min).

The purity of the target product was calculated from the area ratio of the LC/MS (UV wavelength 225 nm) chromatogram under analysis condition B and was 97.4%.
analysis conditions B: retention time =11.93 min: gradient (%B cone); 20-60% over 20 min, then 60-95% over 1 min, then 95% over 5 min
ESI-MS(+) observed value m/z=1211.6(M+2H)²⁺

### [Synthetic Example 2-3]

### Synthesis of hTfR 894 E1 PEG12 (Hydrazide) (SEQ ID NO: 218)

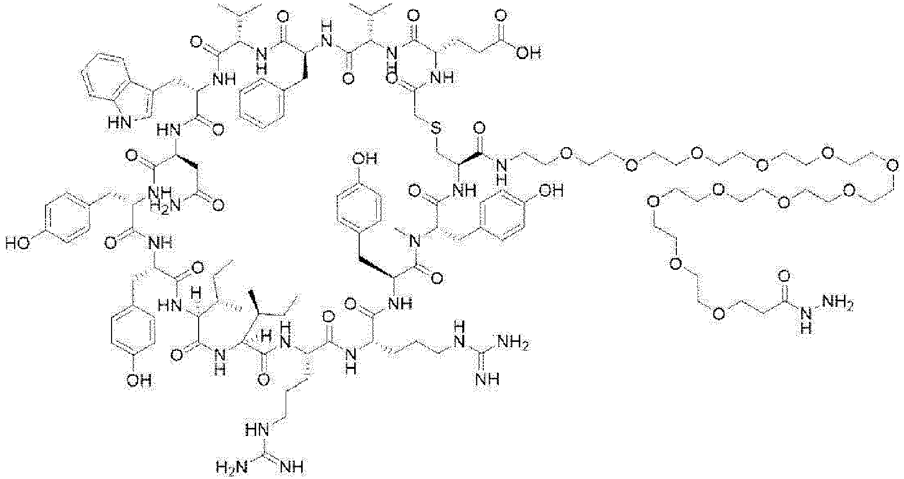

Using NH₂NH-Trt(2-Cl)-resin (Watanabe Chemical, 0.78 mmol/g, 0.13 g), the target peptide was synthesized by starting from the removal of the Fmoc group according to the general method described above. At that time, CEM's Liberty Blue HT was used as a solid phase synthesizer, and the synthesis was performed according to the manufacturer's manual. For the introduction of each residue, Fmoc-AA-OH/HATU/DIEA (5.3eq/5eq/10eq) was used for 1 eq of resin, and the basic condition was to perform the condensation reaction at 75°C for 10 min. When introducing the 11th and 12th amino acid residues, each condensation reaction was performed twice at 25°C for 30 min. For the 13th residue and the 14th residue, the reaction was performed twice at 75°C for 10 min. For the 15th residue, the reaction was performed once at 25°C for 30 min. For the 16th residue, the reaction was performed once using Fmoc-AA-OH/HATU/DIEA (3eq/3eq/6eq) at 25°C for 120 min. The basic condition for Fmoc removal was to react with a 20% piperidine in DMF at 75°C for 3 min. For Fmoc removal at the 13th and 15th residues, the reaction was performed at 25°C for 5 min and then for 10 min. To introduce the chloroacetyl group, the Fmoc group of the α-amino group was removed by the aforementioned method from the solid phase resin carrying the Fmoc-protected peptide obtained in the previous step, 10 eq of chloroacetic acid, 10 eq of DIPCI, and 10 eq of HOSu were stirred in DCM, and the same amount of NMP as DCM was added to prepare a DCM/NMP solution (0.2 M) of ClAcOSu, the solution was added to the solid phase resin, and the mixture was shaken at room temperature for 60 min. For deprotection of side chains and cutting out from the solid-phase resin, first, the resin obtained after the chloroacetyl group introduction step was washed with DMF five times and methylene chloride three times, and then dried under reduced pressure. Then, reactant cocktail (a mixture of TFA/H₂O/TIS/DODT in a volume ratio of 92.5:2.5:2.5:2.5) was added to the reaction vessel containing the solid-phase resin, and the mixture was shaken at room temperature for 60 min. The reaction solution was collected by filtration through a frit. The solid-phase resin remaining in the reaction vessel was shaken again with the cutting cocktail, and the solution components were collected from the frit and mixed with the above-mentioned filtrate. When this filtrate was added to an excess diethylether/hexane (1/1) cooled to 0°C, a cloudy white precipitate was formed. This mixture was centrifuged (9000 rpm, 2 min), and the solution was decanted. The obtained solid was washed again with a small amount of diethyl ether cooled to 0°C, and the obtained solid was dried and used in the next cyclization reaction. For the cyclization reaction of the peptide, the peptide was dissolved in DMSO so that the final concentration was 5 mM based on the number of moles of the solid phase resin, 5 equivalents of triethylamine was added, and the mixture was stirred at room temperature fo about 14 hr. The reaction solution was quenched with acetic acid and concentrated under reduced pressure using Biotage V-10.

The obtained crude product was purified using the following conditions (column; Waters Xbridge (registered trademark) C18 5 um (registered trademark) 30x150 mm: mobile phase; A=0.1% TFA in H₂O, B=0.1% TFA in MeCN: temperature 40°C: gradient (%B); 5-29% over 3 min, then 29-34% over 8 min, then 34-60% over 1 min: flow 45 mL/min.

The purity of the target product was calculated from the area ratio of the LC/MS (UV wavelength 225 nm) chromatogram under analysis condition B and was 77.0%.
analysis conditions B: retention time =10.57 min: column Kinetex EVO C18 2.6 µm, 2.1x150 mm, 100Å: mobile phase;
A=0.025% TFA in H₂O, B=0.025% TFA in MeCN: temperature 40°C: gradient (%B conc); 20-60% over 20 min, then 60-95% over 1 min, then 95-95% over 5 min: flow 0.25 mL/min.
ESI-MS(+) observed value m/z=1378.48(M+2H)²⁺

### [Synthetic Example 2-4]

### Synthesis of 894 3 m G Azi (SEQ ID NO: 297)

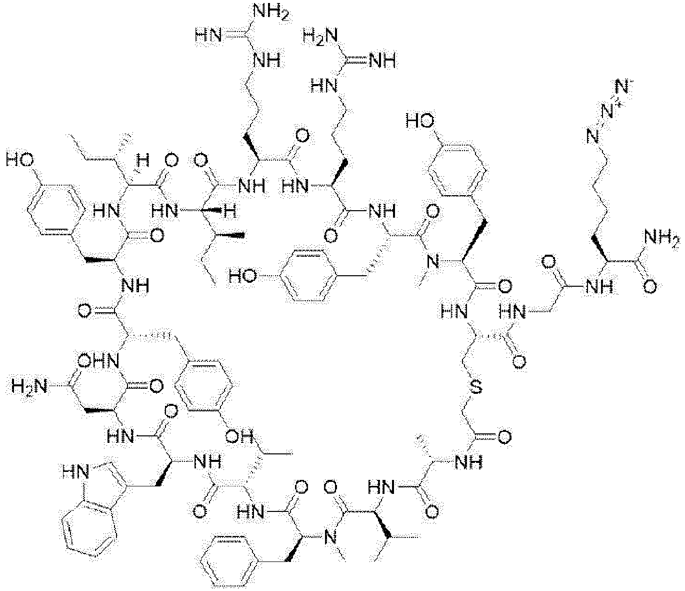

Separately synthesized 894_3 m_G (3.3 g, 1.39 mmol) was stirred with 1.1 eq of H-KN₃-NH₂ (316 mg, 1.52 mmol) synthesized by a known method, 1.2 eq of HATU (632 mg, 1.66 mmol), and 5 eq of DIEA (1.21 mL, 6.93 mmol) at room temperature for 30 min.

The obtained mixture was purified using the following conditions (column; Waters Xbridge (registered trademark) C18 5 um (registered trademark) 50x150 mm: mobile phase; A=0.1% TFA in H₂O, B=0.1% TFA in MeCN: temperature 40°C: gradient (%B); 7-7% over 2 min, then 7-32% over 1 min, then 32-37% over 8 min, then 37-60% over 1 min: flow 20-20 mL/min over 1 min, then 20-120 mL/min over 1 min, then 120 mL/min).

The purity of the target product was calculated from the area ratio of the LC/MS (UV wavelength 225 nm) chromatogram under analysis condition B and was 97.1%.
analysis conditions B: retention time =4.29 min: column;
Kinetex EVO C18 2.6 µm 2.1x150 mm, 100Å: mobile phase; A=0.025% TFA in H₂O, B=0.025% TFA in MeCN: temperature 60°C: gradient (%B cone); 20-60% over 7.2 min, then 60-95% over 0.3 min, then 95-95% over 1.6 min: flow 0.5 mL/min.
ESI-MS(+) observed value m/z=1154.72(M+2H)²⁺

### [Synthetic Example 2-5]

### Synthesis of 894 variant 39 (SEQ ID NO: 327)

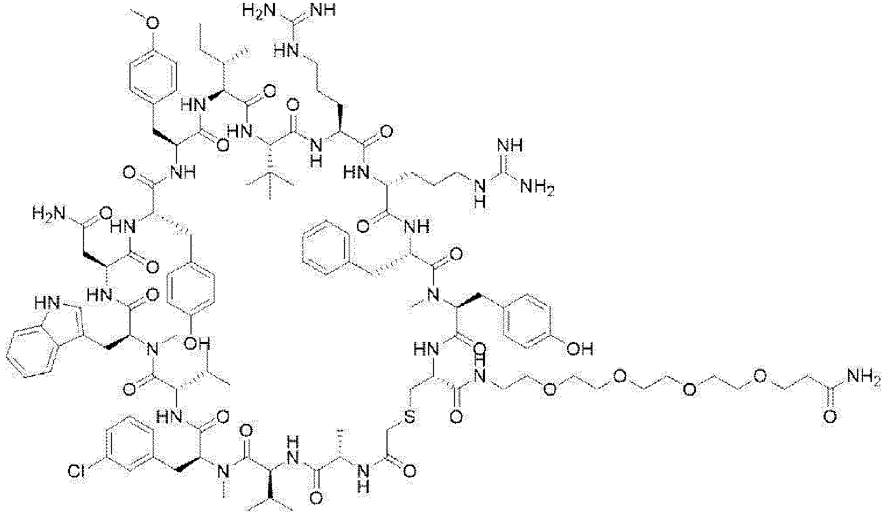

Using Sieber amide resin (Watanabe Chemical, 0.47 mmol/g, 0.21 g), the target peptide was synthesized by starting from the removal of the Fmoc group according to the general method described above. At that time, CEM's Liberty Blue HT was used as a solid phase synthesizer, and the synthesis was performed according to the manufacturer's manual. For the introduction of each residue, Fmoc-AA-OH/DIPCI/Oxima pure (5.3eq/10eq/5eq) was used for 1 eq of resin, and repeat reaction was performed once in DMF at 90°C for 3 min. When introducing the 2nd and 4th residues, the reaction was performed twice at 75°C for 30 min. For the 9th residue, the reaction was performed twice at 90°C for 10 min. For the 11th residue and the 12th residue, the reaction was performed twice at 50°C for 15 min. For the 13th residue, the reaction was performed twice at 90°C for 3 min. For the 15th residue, the reaction was performed once at 50°C for 15 min. The basic condition for Fmoc removal was to react with a 20% piperidine solution in DMF at 75°C for 3 min. For Fmoc removal at the 2nd residue, the 4th residue and the 13th residue, the reaction was repeated twice at 25°C for 5 min. To introduce the chloroacetyl group, the Fmoc group of the α-amino group was removed by the aforementioned method from the solid phase resin carrying the Fmoc-protected peptide obtained in the previous step, 5 eq of chloroacetic acid in DMF, 5 eq of HATU in DMF, and 10 eq of DIEA in DMF were added to the solid phase resin and the mixture was shaken at room temperature for 30 min. For deprotection of side chains and cutting out from the solid-phase resin, first, the resin obtained after the chloroacetyl group introduction step was washed with DMF five times and methylene chloride three times, and then dried under reduced pressure. Then, reactant cocktail-A (a mixture of TFA/H₂O/TIS/DODT in a volume ratio of 92.5:2.5:2.5:2.5) was added to the reaction vessel containing the solid-phase resin, and the mixture was shaken at room temperature for 60 min. The reaction solution was collected by filtration through a frit. The solid-phase resin remaining in the reaction vessel was shaken again with the cutting cocktail, and the solution components were collected from the frit and mixed with the above-mentioned filtrate. When this filtrate was added to an excess diethyl ether/hexane (1/1) mixed solvent cooled to 0°C, a cloudy white precipitate was formed. This mixture was centrifuged (9000 rpm, 1.5 min), and the solution was decanted. The obtained solid was washed again with a small amount of diethyl ether cooled to 0°C, and then dried under reduced pressure. The obtained solid was used in the next cyclization reaction. For the cyclization reaction of the peptide, the peptide was dissolved in DMSO so that the final concentration was 5 mM based on the number of moles of the solid phase resin, 10 equivalents of triethylamine was added, and the mixture was shaken at room temperature about for 24 hr. The obtained reaction solution was concentrated under reduced pressure using Savant Explorer SpeedVaC.

The obtained crude product was purified using the following conditions (column; Waters Xbridge (registered trademark) C18 5 um (registered trademark) 50x150 mm: mobile phase; A=0.1% TFA in H₂O, B=0.1% TFA in MeCN: temperature 40°C: gradient (%B); 11-36% over 3 min, then 36-41% over 8 min, then 41-60% over 1 min: flow 120 mL/min.

The purity of the target product was calculated from the area ratio of the LC/MS (UV wavelength 225 nm) chromatogram under analysis condition B and was 93.1%.
analysis conditions B: retention time =12.76 min: column;
Kinetex EVO C18 2.6 µm 2.1x150 mm, 100Å: mobile phase; A=0.025% TFA in H₂O, B=0.025% TFA in MeCN: temperature 60°C: gradient (%B cone); 20-60% over 20 min, then 60-95% over 1 min, then 95% over 5 min: flow 0.25 mL/min.
ESI-MS(+) observed value m/z=1196.12(M+2H)²⁺

### [Synthetic Example 2-6]

### Synthesis of 894 variant 120 (SEQ ID NO: 353)

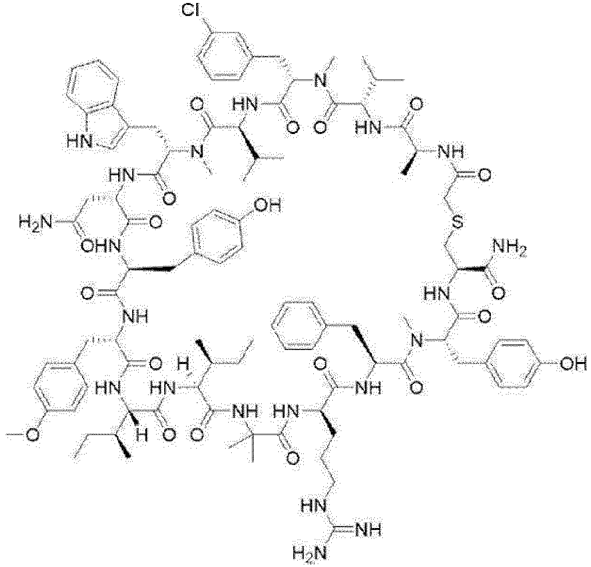

Using Sieber amide resin (Watanabe Chemical, 0.47 mmol/g, 0.43 g), the target peptide was synthesized by starting from the removal of the Fmoc group according to the general method described above. At that time, CEM's Liberty Blue HT was used as a solid phase synthesizer, and the synthesis was performed according to the manufacturer's manual. For the introduction of each residue, Fmoc-AA-OH/HATU/DIEA (5eq/5eq/10eq) was used for 1 eq of resin, and the reaction was performed once in DMF at 75°C for 10 min. For the 1st residue and the 3rd residue, the reaction was performed once at 75°C for 20 min. For the 2nd residue and the 4th residue, the reaction was performed twice at 75°C for 30 min. For the 9th residue, the 10th residue, and the 11th residue, the reaction was performed twice at 75°C for 20 min. For the 12th residue, the reaction was performed twice at 25°C for 30 min. For the 13th residue, the reaction was performed twice at 75°C for 10 min. For the 15th residue, the reaction was performed once at 25°C for 30 min. In addition, the basic condition for Fmoc removal was to react with a 20% piperidine solution in DMF at 75°C for 3 min. For Fmoc removal at the 2nd residue, the 4th residue and 13th residue, the reaction was repeated twice at 25°C for 5 min. To introduce the chloroacetyl group, the Fmoc group of the α-amino group was removed by the aforementioned method from the solid phase resin carrying the Fmoc-protected peptide obtained in the previous step, 5 eq of ClAcOH in DMF, 5 eq of HATU in DMF, and 10 eq of DIEA in DMF were added to the solid phase resin and the mixture was shaken at 25°C for 30 min. For deprotection of side chains and cutting out from the solid-phase resin, first, the resin obtained after the chloroacetyl group introduction step was washed with DMF five times and methylene chloride three times, and then dried under reduced pressure. Then, reactant cocktail-A (a mixture of TFA/H₂O/TIS/DODT in a volume ratio of 92.5:2.5:2.5:2.5) was added to the reaction vessel containing the solid-phase resin, and the mixture was shaken at room temperature for 60 min. The reaction solution was collected by filtration through a frit. The solid-phase resin remaining in the reaction vessel was shaken again with the cutting cocktail, and the solution components were collected from the frit and mixed with the above-mentioned filtrate. When this filtrate was added to an excess mixed solvent of diisopropyl ether/hexane (1/1) cooled to 0°C, a cloudy white precipitate was formed. This mixture was centrifuged (9000 rpm, 2 min), and the solution was decanted. The obtained solid was washed again with a small amount of diethyl ether cooled to 0°C, and then dried under reduced pressure. The obtained solid was used in the next cyclization reaction. For the cyclization reaction of the peptide, the peptide was dissolved in DMSO so that the final concentration was 5 mM based on the number of moles of the solid phase resin, 10 equivalents of triethylamine was added, and the mixture was shaken at room temperature for about 16 hr. The obtained reaction solution was concentrated under reduced pressure using Savant Explorer SpeedVaC.

The obtained crude product was purified using the following conditions (column; Waters Xbridge (registered trademark) C18 5 um (registered trademark) 50x150 mm: mobile phase; A=0.1% TFA in H₂O, B=0.1% TFA in MeCN: temperature 40°C: gradient (%B); 16-41% over 3 min, then 41-46% over 8 min, then 46-60% over 1 min: flow 120 mL/min).

The purity of the target product was calculated from the area ratio of the LC/MS (UV wavelength 225 nm) chromatogram under analysis condition B and was 97.5%.
analysis conditions B: retention time =6.07 min: column;
Kinetex EVO C18 2.6 µm 2.1x150 mm, 100Å: mobile phase; A=0.025% TFA in H₂O, B=0.025% TFA in MeCN: temperature 60°C: gradient (%B cone); 20-60% over 7.2 min, then 60-95% over 0.3 min, then 95-95% over 1.6 min: flow 0.5 mL/min.
ESI-MS(+) observed value m/z=1036.90(M+2H)²⁺

### [Synthetic Example 2-7]

### Synthesis of 894 variant 61 (SEQ ID NO: 354)

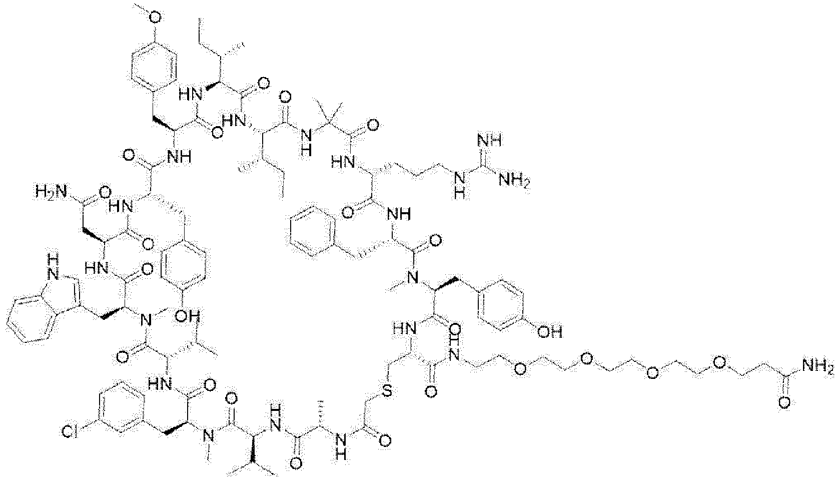

Using Sieber amide resin (Watanabe Chemical, 0.47 mmol/g, 0.21 g), the target peptide was synthesized by starting from the removal of the Fmoc group according to the general method described above. At that time, CEM's Liberty Blue HT was used as a solid phase synthesizer, and the synthesis was performed according to the manufacturer's manual. For the introduction of each residue, Fmoc-AA-OH/DIPCI/Oxima pure (5.3eq/10eq/5eq) was used for 1 eq of resin, and the reaction was performed once in DMF at 90°C for 3 min. For the 2nd residue and the 4th residue, the reaction was performed twice at 75°C for 30 min. For the 10th residue and the 11th residue, the reaction was performed twice at 90°C for 10 min. For the 12th residue, the reaction was performed twice at 50°C for 15 min. For the 13th residue, the reaction was performed twice at 90°C for 3 min. For the 15th residue, the reaction was performed once at 50°C for 15 min. The basic condition for Fmoc removal was to react with a 20% piperidine solution in DMF at 75°C for 3 min. For Fmoc removal at the 2nd residue, the 4th residue and the 13th residue, the reaction was repeated twice at 25°C for 5 min. To introduce the chloroacetyl group, the Fmoc group of the α-amino group was removed by the aforementioned method from the solid phase resin carrying the Fmoc-protected peptide obtained in the previous step, 10 eq of chloroacetic acid, 5 eq of DIPCI, and 5 eq of HOSu were stirred in DCM, and the same amount of DMF as DCM was added to prepare a DCM/DMF solution (0.2 M) of ClAcOSu, the solution was added to the solid phase resin, and the mixture was shaken at room temperature for 60 min. For deprotection of side chains and cutting out from the solid-phase resin, first, the resin obtained after the chloroacetyl group introduction step was washed with DMF five times and methylene chloride three times, and then dried under reduced pressure. Then, reactant cocktail-A (a mixture of TFA/H₂O/TIS/DODT in a volume ratio of 92.5:2.5:2.5:2.5) was added to the reaction vessel containing the solid-phase resin, and the mixture was shaken at room temperature for 90 min. The reaction solution was collected by filtration through a frit. The solid-phase resin remaining in the reaction vessel was shaken again with the cutting cocktail, and the solution components were collected from the frit and mixed with the above-mentioned filtrate. When this filtrate was added to an excess diethyl ether/hexane (1/1) mixed solvent cooled to 0°C, a cloudy white precipitate was formed. This mixture was centrifuged (9000 rpm, 2 min), and the solution was decanted. The obtained solid was washed again with a small amount of diethyl ether cooled to 0°C, and then dried under reduced pressure. The obtained solid was used in the next cyclization reaction. For the cyclization reaction of the peptide, the peptide was dissolved in DMSO so that the final concentration was 5 mM based on the number of moles of the solid phase resin, 5 eq of triethylamine was added, and the mixture was shaken at room temperature for about 14 hr. The obtained reaction solution was concentrated under reduced pressure using Savant Explorer SpeedVaC.

The obtained crude product was purified using the following conditions (column; Waters Xbridge (registered trademark) C18 5 um (registered trademark) 50x150 mm: mobile phase; A=0.1% TFA in H₂O, B=0.1% TFA in MeCN: temperature 40°C: gradient (%B); 15-40% over 3 min, then 40-45% over 8 min, then 45-60% over 1 min: flow 120 mL/min).

The purity of the target product was calculated from the area ratio of the LC/MS (UV wavelength 225 nm) chromatogram under analysis condition B and was 87.5%.
analysis conditions B: retention time = 16.50 min: column;
Kinetex EVO C18 2.6 µm 2.1x150 mm, 100Å: mobile phase; A=0.025% TFA in H₂O, B=0.025% TFA in MeCN: temperature 60°C: gradient (%B cone); 20-60% over 20 min, then 60-95% over 1 min, then 95% over 5 min: flow 0.25 mL/min
ESI-MS(+) observed value m/z=1160.58(M+2H)²⁺

### [Synthetic Example 2-8]

### Synthesis of 894 variant 14 (SEQ ID NO: 387)

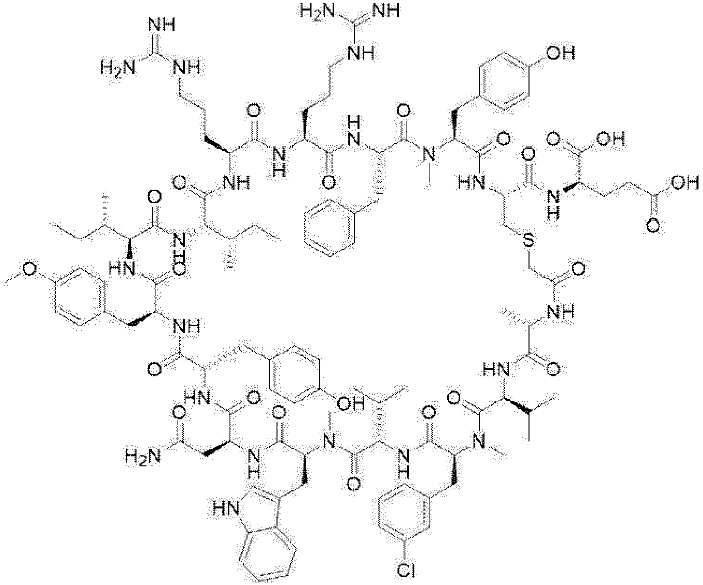

Using Fmoc-D-Glu(OtBu)-wang resin (Watanabe Chemical, 0.68 mmol/g, 184 mg), according to a general method, the target peptide was synthesized by starting from the removal of the Fmoc group. At that time, CEM's Liberty Blue HT was used as a solid phase synthesizer, according to the manufacturer's manual. The basic condition for the condensation reaction was to react twice at 75°C for 10 min using HATU as a condensing agent. For the 2nd residue, the reaction was performed twice at 75°C for 30 min. For the 4th residue, the reaction was performed twice at 75°C for 30 min. For the 6th residue, the reaction was performed once at 75°C for 10 min. For the 7th residue, the reaction was performed once at 75°C for 10 min. For the 8th residue, the reaction was performed once at 75°C for 10 min. For the 10th residue, the reaction was performed once at 75°C for 10 min. For the 11th residue, the reaction was performed twice at 30°C for 30 min. For the 12th residue, the reaction was performed once at 30°C for 30 min. For the 15th residue, the reaction was performed once at 30°C for 30 min. The basic condition for Fmoc removal was to react with a 20% piperidine solution in DMF at 75°C for 3 min. For the 2nd residue, the 4th residues, and the 13th residue, Fmoc was removed by reacting twice at room temperature for 5 min. To introduce the chloroacetyl group, chloroacetic acid (5 equivalents), DIPCI (5 equivalents), and HOSu (5 equivalents) were stirred in DCM, and the same amount of DMF as DCM were added to prepare a DCM/DMF solution of ClAcOSu, and the solution was added to the solid phase resin obtained in the previous step, and the mixture was shaken at room temperature for 60 min. For deprotection of side chains and cutting out from the solid-phase resin, first, the resin obtained after the chloroacetyl group introduction step was washed with DMF five times and methylene chloride three times, and dried under reduced pressure. Then, reactant cocktail-A (a mixture of TFA/H₂O/TIS/DODT in a volume ratio of 92.5:2.5:2.5:2.5) was added to the reaction vessel containing the solid-phase resin, and the mixture was shaken at room temperature for 90 min. The reaction solution was collected by filtration through a frit. The solid-phase resin remaining in the reaction vessel was shaken again with the cutting cocktail, and the solution components were collected from the frit and mixed with the above-mentioned filtrate. When this filtrate was added to an excess diethylether/hexane (3/1) cooled to 0°C, a cloudy white precipitate was formed. This mixture was centrifuged (9500 rpm, 1 min), the supernatant was decanted, washed with diethyl ether cooled to 0°C, and the obtained solid was used in the next cyclization reaction. For the cyclization reaction of the peptide, the peptide was dissolved in DMSO so that the final concentration was 4 mM based on the number of moles of the solid phase resin, 10 equivalents of triethylamine was added, and the mixture was shaken at room temperature for about 16 hr. The obtained reaction solution was concentrated under reduced pressure using GenevaC EZ-2 Elite.

The obtained reaction mixture was purified using the following conditions (column; Waters Xbridge (registered trademark) C18 5 um (registered trademark) 50x150 mm: mobile phase; A=0.1% TFA in H₂O, B=0.1% TFA in MeCN: temperature 40°C: gradient (%B); 11-36% over 3 min, then 36-41% over 8 min, then 41-60% over 1 min: flow 120 mL/min).

The purity of the target product was calculated from the area ratio of the LC/MS (UV wavelength 225 nm) chromatogram under analysis condition B and was 88.60%.
analysis conditions B: retention time =13.03 min: gradient (%B cone); 20-60% over 20 min, then 60-95% over 1 min, then 95% over 5 min.
ESI-MS(+) observed value m/z=1137.23(M+2H)²⁺

### [Synthetic Example 2-9]

### Synthesis of 894 variant 84 (SEQ ID NO: 390)

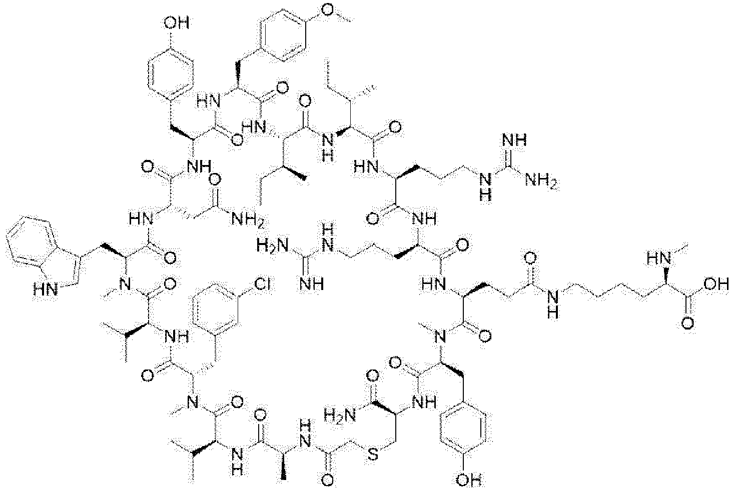

Using Sieber amide resin (Watanabe Chemical, 0.47 mmol/g, 0.21 g), the target peptide was synthesized by starting from the removal of the Fmoc group according to the general method described above. At that time, CEM's Liberty Blue HT was used as a solid phase synthesizer, and the synthesis was performed according to the manufacturer's manual. For the introduction of each residue, Fmoc-AA/DIPCI/Oxyma pure=5.3eq/10eq/5eq was used for 1 eq of resin, the reaction was performed once in DMF at 90°C for 3 min. For the 2nd residue and the 4th residue, the reaction was performed twice at 75°C for 45 min. For the 11th residue and the 12th residue, the reaction was repeated twice at 50°C for 15 min. When introducing the 13th residue, the reaction was performed twice at 90°C for 3 min. In addition, the basic condition for Fmoc removal was to react once with a 20% piperidine solution in DMF at 75°C for 3 min. For Fmoc removal at the 2nd residue, the 4th residue and the 13th residue, the reaction was repeated twice at 25°C for 5 min. After elongation to the 1st residue, the resin was suspended in DCM, Pd(PPh₃)₄/PhSiH₃(0.2eq/10eq) was added, and the mixture was shaken at room temperature for 1 hr. To introduce the chloroacetyl group, a solid phase resin carrying the Fmoc-protected peptide obtained in the previous step was washed three times with DMF and three times with DCM, Fmoc group of the α-amino group was removed by the aforementioned method, 5 eq of chloroacetic acid in DMF, 5 eq of HATU in DMF, and 10 eq of DIEA in DMF were added and the mixture was shaken at room temperature for 30 min. For deprotection of side chains and cutting out from the solid-phase resin, first, the resin obtained after the chloroacetyl group introduction step was washed with DMF five times and methylene chloride three times, and then dried under reduced pressure. Then, reactant cocktail-A (a mixture of TFA/H₂O/TIS/DODT in a volume ratio of 92.5:2.5:2.5:2.5) was added to the reaction vessel containing the solid-phase resin, and the mixture was shaken at room temperature for 90 min. The reaction solution was collected by filtration through a frit. The solid-phase resin remaining in the reaction vessel was shaken again with the cutting cocktail, and the solution components were collected from the frit and mixed with the above-mentioned filtrate. When this filtrate was added to an excess diethyl ether/hexane (1/1) mixed solvent cooled to 0°C, a cloudy white precipitate was formed. This mixture was centrifuged (10000 rpm, 1 min), and the solution was decanted. The obtained solid was washed again with a small amount of diethyl ether cooled to 0°C, and then dried under reduced pressure. The obtained solid was used in the next cyclization reaction. For the cyclization reaction of the peptide, the peptide was dissolved in DMSO so that the final concentration was 5 mM based on the number of moles of the solid phase resin, 10 equivalents of triethylamine was added, and the mixture was shaken at room temperature overnight. The reaction solution was concentrated under reduced pressure using Savant Explorer SpeedVaC. The obtained mixture was dissolved in DMSO (4 mL), HOSu (10eq) and EDC HCl (10eq) were added and the mixture was stirred at room temperature for 2 hr.

The obtained crude product was purified using the following conditions (column; Waters Xbridge (registered trademark) C18 5 um (registered trademark) 30x150 mm: mobile phase; A=0.1% TFA in H₂O, B=0.1% TFA in MeCN: temperature 40°C: gradient (%B); 11-36% over 3 min, then 36-41% over 8 min, then 41-60% over 1 min: flow 45 mL/min).

The obtained cyclic peptide-NHS ester (25 mg, 11.3 µmol) was dissolved in DMF (225 µL), Fmoc-MeK-OH hydrochloride (5 mg, 11.9 µmol) and DIEA (5.9 µL, 33.9 µmol) were added, and the mixture was stirred for 1 hr, Et₂NH (5.9 µL, 56.5 µmol) was added to the reaction mixture, and the mixture was stirred for 1 hr and quenched with acetic acid.

The obtained crude product was purified using the following conditions (column; Waters Xbridge (registered trademark) C18 5 µm (registered trademark) 19x150 mm: mobile phase; A=0.1% TFA in H₂O, B=0.1% TFA in MeCN: temperature 60°C: gradient (%B); 8-33% over 3 min, then 33-38% over 8 min, then 38-60% over 1 min: flow 17 mL/min.

The purity of the target product was calculated from the area ratio of the LC/MS (UV wavelength 225 nm) chromatogram under analysis condition B and was 97.9%.
analysis conditions B: retention time =12.33 min: column;
Kinetex EVO C18 2.6 µm 2.1x150 mm, 100Å; :mobile phase; A=0.025% TFA in H₂O, B=0.025% TFA in MeCN: temperature 60°C: gradient (%B conc); 20-60% over 20 min, then 60-95% over 1 min, then 95% over 5 min
ESI-MS(+) observed value m/z=1134.34(M+2H)²⁺

### [Synthetic Example 2-10]

### Synthesis of 894 variant 89 (SEQ ID NO: 391)

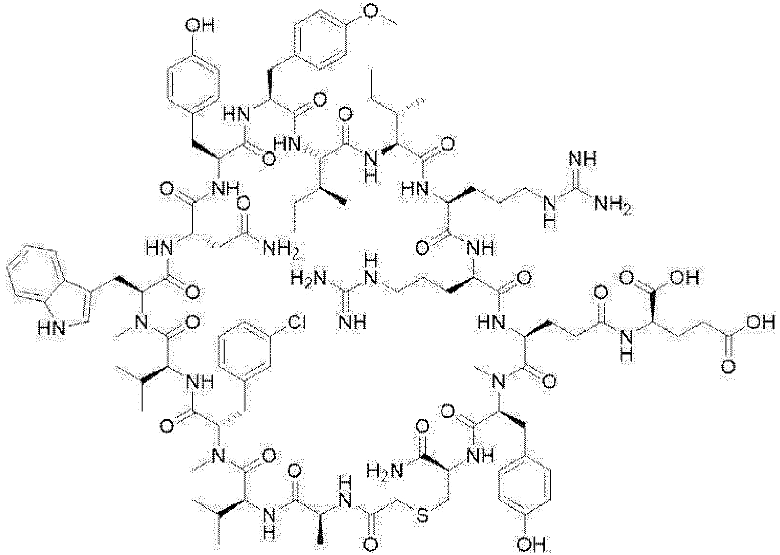

Using Sieber amide resin (Watanabe Chemical, 0.47 mmol/g, 0.21 g), the target peptide was synthesized by starting from the removal of the Fmoc group according to the general method described above. At that time, CEM's Liberty Blue HT was used as a solid phase synthesizer, and the synthesis was performed according to the manufacturer's manual. For the introduction of each residue, Fmoc-AA/DIPCI/Oxyma pure/DIEA (5.3eq/10eq/5eq) was used for 1 eq of resin, and the reaction was performed once in DMF at 90°C for 3 min. When introducing the 2nd residue and the 4th residue, the reaction was performed twice at 75°C for 45 min. When introducing the 11th residue and the 12th residue, the reaction was repeated twice at 50°C for 15 min. When introducing the 13th residue, the reaction was performed twice at 90°C for 3 min. In addition, the basic condition for Fmoc removal was to react once with a 20% piperidine solution in DMF at 75°C for 3 min. For Fmoc removal at the 2nd residue, the 4th residue and the 13th residue, the reaction was repeated twice at 25°C for 5 min. After elongation to the 1st residue, the resin was suspended in DCM, Pd(PPh₃)₄/PhSiH₃ (0.2eq/10eq) was added and the mixture was shaken at room temperature for 1 hr. The solid phase resin was washed three times with DMF and three times with DCM, and dried under reduced pressure. The resin (60 µmol) was suspended in DMF, H-D-Glu(OtBu)-OtBu hydrochloride (71 mg, 0.24 mmol), 0.5 M Oxyma pure in DMF (0.48 mL, 0.24 mmol), DIPCI (37 µL, 0.24 mmol), and DIEA (42 µL, 0.24 mmol) were added, and the mixture was reacted twice under microwave radiation at 75°C for 30 min. To introduce the chloroacetyl group, the Fmoc group of the α-amino group was removed from the solid-phase resin carrying the Fmoc-protected peptide obtained in the previous step by using the aforementioned method, 5 eq of chloroacetic acid in DMF, 5 eq of HATU in DMF, and 10 eq of DIEA in DMF were added, and the mixture was shaken at room temperature for 30 min. For deprotection of side chains and cutting out from the solid-phase resin, first, the resin obtained after the chloroacetyl group introduction step was washed with DMF five times and methylene chloride three times, and then dried under reduced pressure. Then, reactant cocktail-A (a mixture of TFA/H₂O/TIS/DODT in a volume ratio of 92.5:2.5:2.5:2.5) was added to the reaction vessel containing the solid-phase resin, and the mixture was shaken at room temperature for 90 min. The reaction solution was collected by filtration through a frit. The solid-phase resin remaining in the reaction vessel was shaken again with the cutting cocktail, and the solution components were collected from the frit and mixed with the above-mentioned filtrate. When this filtrate was added to an excess diethyl ether/hexane (1/1) mixed solvent cooled to 0°C, a cloudy white precipitate was formed. This mixture was centrifuged (10000 rpm, 1 min), and the solution was decanted. The obtained solid was washed again with a small amount of diethyl ether cooled to 0°C, and then dried under reduced pressure. The obtained solid was used in the next cyclization reaction. For the cyclization reaction of the peptide, the peptide was dissolved in DMSO so that the final concentration was 5 mM based on the number of moles of the solid phase resin, 10 eq of triethylamine was added, and the mixture was shaken at room temperature overnight. The reaction solution was concentrated under reduced pressure using Savant Explorer SpeedVaC.

The obtained crude product was purified using the following conditions (column; Waters Xbridge (registered trademark) C18 5 um (registered trademark) 50x150 mm: mobile phase; A=0.1% TFA in H₂O, B=0.1% TFA in MeCN: temperature 40°C: gradient (%B); 8-33% over 3 min, then 33-38% over 8 min, then 38-60% over 1 min: flow 120 mL/min).

The purity of the target product was calculated from the area ratio of the LC/MS (UV wavelength 225 nm) chromatogram under analysis condition B and was 94.9%.
analysis conditions B: retention time =11.81 min: column;
Kinetex EVO C18 2.6 µm 2.1x150 mm, 100Å: mobile phase; A=0.025% TFA in H₂O, B=0.025% TFA in MeCN: temperature 40°C: gradient (%B cone); 20-60% over 20 min, then 60-95% over 1 min, then 95% over 5 min: flow 0.25 mL/min.
ESI-MS(+) observed value m/z=1127.76(M+2H)²⁺

### [Synthetic Example 2-11]

### Synthesis of 894 variant 03 (SEQ ID NO: 397)

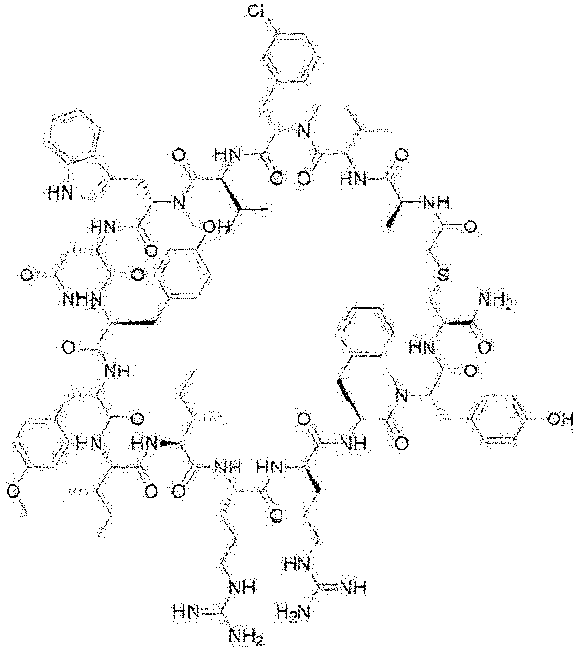

Using Sieber amide resin (Watanabe Chemical, 0.47 mmol/g, 0.21 g), the target peptide was synthesized by starting from the removal of the Fmoc group according to the general method described above. At that time, CEM's Liberty Blue HT was used as a solid phase synthesizer, and the synthesis was performed according to the manufacturer's manual. For the introduction of each residue, Fmoc-AA/DIPCI/Oxima pure (4.2eq/4eq/8eq) was used for 1 eq of resin, and the reaction was performed once in DMF at 75°C for 10 min. For the 2nd residue and the 4th residue, the reaction was performed twice at 75°C for 30 min. For the 11th residue and the 12th residue, the reaction was performed twice at 90°C for 10 min. For the 13th residue, the reaction was performed twice at 75°C for 10 min. For the 15th residue, the reaction was performed once at 25°C for 20 min. In addition, the basic condition for Fmoc removal was to react with a 20% piperidine solution in DMF at 75°C for 3 min. For Fmoc removal at the 2nd residue, the 4th residue and the 13th residue, the reaction was performed at 25°C for 5 min, and then at 25°C for 10 min. To introduce the chloroacetyl group, the Fmoc group of the α-amino group was removed by the aforementioned method from the solid phase resin carrying the Fmoc-protected peptide obtained in the previous step, chloroacetic acid (10 equivalents), 10 equivalents of DIPCI, and 10 equivalents of HOSu were stirred in DCM, and the same amount of DMF as DCM was added to prepare a DCM/DMF solution (0.2 M) of ClAcOSu, the solution was added to the solid phase resin, and the mixture was shaken at room temperature for 60 min. For deprotection of side chains and cutting out from the solid-phase resin, first, the resin obtained after the chloroacetyl group introduction step was washed with DMF five times and methylene chloride three times, and then dried under reduced pressure. Then, reactant cocktail-A (a mixture of TFA/H₂O/TIS/DODT in a volume ratio of 92.5:2.5:2.5:2.5) was added to the reaction vessel containing the solid-phase resin, and the mixture was shaken at room temperature for 90 min. The reaction solution was collected by filtration through a frit. The solid-phase resin remaining in the reaction vessel was shaken again with the cutting cocktail, and the solution components were collected from the frit and mixed with the above-mentioned filtrate. When this filtrate was added to an excess diethyl ether/hexane (1/1) mixed solvent cooled to 0°C, a cloudy white precipitate was formed. This mixture was centrifuged (9000 rpm, 2 min), and the solution was decanted. The obtained solid was washed again with a small amount of diethyl ether cooled to 0°C, and then dried under reduced pressure. The obtained solid was used in the next cyclization reaction. For the cyclization reaction of the peptide, the peptide was dissolved in 50% MeCN aqueous solution so that the final concentration was 5 mM based on the number of moles of the solid phase resin, 5 equivalents of triethylamine was added, and the mixture was shaken at room temperature for about 14 hr. The obtained reaction solution was concentrated under reduced pressure using Savant Explorer SpeedVaC.

The obtained crude product was purified using the following conditions (column; Waters Xbridge (registered trademark) C18 5 um (registered trademark) 50x150 mm: mobile phase; A=0.1% TFA in H₂O, B=0.1% TFA in MeCN: temperature 40°C: gradient (%B); 10-35% over 3 min, then 35-40% over 8 min, then 40-60% over 1 min: flow 120 mL/min.

The purity of the target product was calculated from the area ratio of the LC/MS (UV wavelength 225 nm) chromatogram under analysis condition B and was 98.2%.
analysis conditions B: retention time =13.18 min: column;
Kinetex EVO C18 2.6 µm 2.1x150 mm, 100Å: mobile phase; A=0.025% TFA in H₂O, B=0.025% TFA in MeCN: temperature 40°C: gradient (%B cone); 20-60% over 20 min, then 60-95% over 1 min, then 95-95% over 5 min: flow 0.25 mL/min.
ESI-MS(+) observed value m/z=1072.49(M+2H)²⁺

### [Synthetic Example 2-12]

### Synthesis of 894 variant 31 (SEQ ID NO: 400)

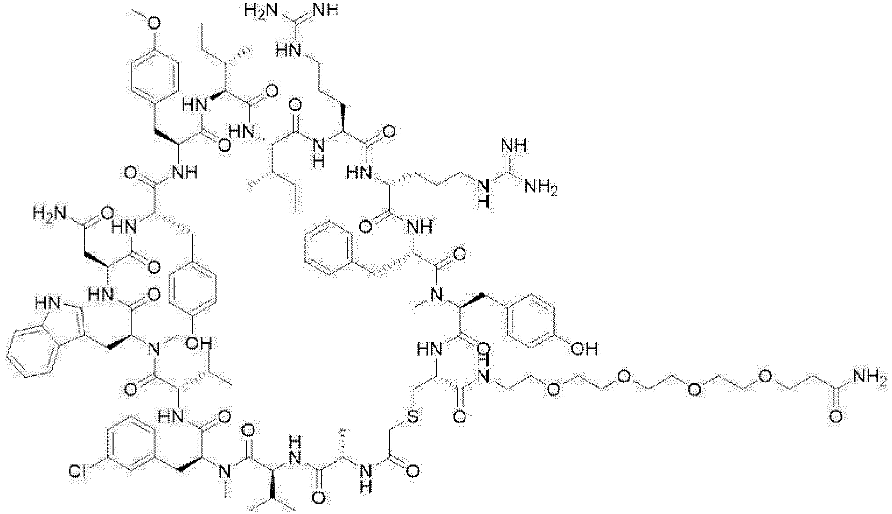

Using Sieber amide resin (Watanabe Chemical, 0.47 mmol/g, 0.21 g), the target peptide was synthesized by starting from the removal of the Fmoc group according to the general method described above. At that time, CEM's Liberty Blue was used as a solid phase synthesizer, and the synthesis was performed according to the manufacturer's manual. For the introduction of each residue, Fmoc-AA/DIPCI/Oxima pure (4.2eq/4eq/8eq) was used for 1 eq of resin, and the reaction was repeated twice in DMF at 90°C for 3 min. When introducing the 2nd residue and the 4th residue, the reaction was performed twice at 75°C for 45 min. For the 3rd residue, the reaction was performed twice at 75°C for 30 min. For the 7th residue and the 8th residue, the reaction was performed once at 90°C for 3 min. When introducing the 11th residue and the 12th residue, the reaction was performed twice at 50°C for 15 min. For the 15th residue and the 16th residue, the reaction was performed once at 50°C for 15 min. In addition, the basic condition for Fmoc removal was to react with a 20% piperidine solution in DMF at 75°C for 3 min. For Fmoc removal at the 2nd residue, the 4th residue and the 13th residue, the reaction was repeated twice at 25°C for 5 min. To introduce the chloroacetyl group, the Fmoc group of the α-amino group was removed by the aforementioned method from the solid phase resin carrying the Fmoc-protected peptide obtained in the previous step, chloroacetic acid (5 equivalents), 5 equivalents of DIPCI, and 5 equivalents of HOSu were stirred in DCM, and the same amount of DMF as DCM was added to prepare a DCM/DMF solution (0.2 M) of ClAcOSu, the solution was added to the solid phase resin, and the mixture was shaken at room temperature for 60 min. For deprotection of side chains and cutting out from the solid-phase resin, first, the resin obtained after the chloroacetyl group introduction step was washed with DMF five times and methylene chloride three times, and then dried under reduced pressure. Then, reactant cocktail-A (a mixture of TFA/H₂O/TIS/DODT in a volume ratio of 92.5:2.5:2.5:2.5) was added to the reaction vessel containing the solid-phase resin, and the mixture was shaken at room temperature for 90 min. The reaction solution was collected by filtration through a frit. The solid-phase resin remaining in the reaction vessel was shaken again with the cutting cocktail, and the solution components were collected from the frit and mixed with the above-mentioned filtrate. When this filtrate was added to an excess diisopropyl ether cooled to 0°C, a cloudy white precipitate was formed. The mixture was centrifuged (9500 rpm, 1 min) and the solution was decanted. The obtained solid was washed again with a small amount of diethyl ether cooled to 0°C, and then dried under reduced pressure. The obtained solid was used in the next cyclization reaction. For the cyclization reaction of the peptide, the peptide was dissolved in DMSO so that the final concentration was 4 mM based on the number of moles of the solid phase resin, 7 equivalents of triethylamine was added, and the mixture was shaken at room temperature for about 12 hr. The obtained reaction solution was concentrated under reduced pressure using GenevaC EZ-2 Elite.

The obtained crude product was purified using the following conditions (column; Waters Xbridge (registered trademark) C18 5 um (registered trademark) 50x150 mm: mobile phase; A=0.1% TFA in H₂O, B=0.1% TFA in MeCN: temperature 40°C: gradient (%B); 15-40% over 3 min, then 40-45% over 8 min, then 45-60% over 1 min: flow 120 mL/min).

The purity of the target product was calculated from the area ratio of the LC/MS (UV wavelength 225 nm) chromatogram under analysis condition B and was 95.4%.
analysis conditions B: retention time =12.64 min: column;
Kinetex EVO C18 2.6 µm 2.1x150 mm, 100Å; :mobile phase; A=0.025% TFA in H₂O, B=0.025% TFA in MeCN: temperature 60°C: gradient (%B conc); 20-60% over 20 min, then 60-95% over 1 min, then 95% over 5 min: flow 0.25 mL/min
ESI-MS(+) observed value m/z=1195.87(M+2H)²⁺

### [Synthetic Example 2-13]

### Synthesis of 894 variant 03 G4S2C (SEQ ID NO: 401)

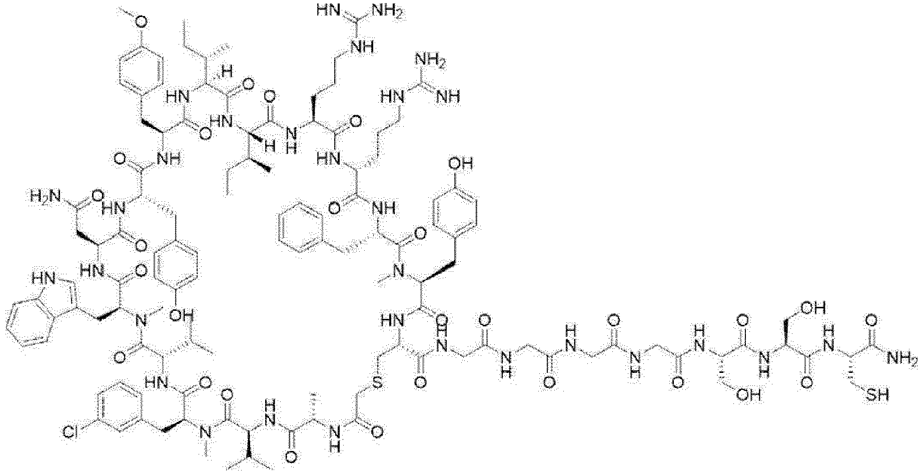

Using Sieber amide resin (Watanabe Chemical, 0.73 mmol/g, 1.37 g), the target peptide was synthesized by starting from the removal of the Fmoc group according to the general method described above. At that time, CEM's Liberty Blue was used as a solid phase synthesizer, and the synthesis was performed according to the manufacturer's manual. For the introduction of each residue, Fmoc-AA/HATU/DIEA (4.2eq/4eq/8eq) was used for 1 eq of resin, and the reaction was performed once in DMF at 75°C for 10 min. For the 2nd residue and the 4th residue, the reaction was performed twice at 75°C for 30 min. For the 11th residue and the 12th residue, the reaction was repeated twice at 25°C for 20 min. For the 13th residue, the reaction was performed twice at 75°C for 10 min. For the 15th residue and the 22nd residue, the reaction was performed once at 25°C for 30 min. The basic condition for Fmoc removal was to react with a 20% piperidine solution in DMF at 75°C for 3 min. For Fmoc removal at the 2nd residue, the 4th residue and 13th residue, reaction was performed at 25°C for 5 min and then at room temperature for 10 min. To introduce the chloroacetyl group, the Fmoc group of the α-amino group was removed by the aforementioned method from the solid phase resin carrying the Fmoc-protected peptide obtained in the previous step, 10 eq of chloroacetic acid, 10 eq of DIPCI, and 10 eq of HOSu were stirred in DCM, and the same amount of DMF as DCM was added to prepare a DCM/DMF solution (0.2 M) of ClAcOSu, the solution was added to the solid phase resin, and the mixture was shaken at room temperature for 60 min. For deprotection of side chains and cutting out from the solid-phase resin, first, the resin obtained after the chloroacetyl group introduction step was washed with DMF five times and methylene chloride three times, and then dried under reduced pressure. Successively, reactant cocktail-A (a mixture of TFA/H₂O/TIS/DODT in a volume ratio of 90:2.5:2.5:5) was added to the reaction vessel containing the solid-phase resin, and the mixture was shaken at room temperature for 90 min. The reaction solution was collected by filtration through a frit. The solid-phase resin remaining in the reaction vessel was shaken again with the cutting cocktail, and the solution components were collected from the frit and mixed with the above-mentioned filtrate. When this filtrate was added to an excess mixed solvent of diisopropyl ether/hexane (1/1) cooled to 0°C, a cloudy white precipitate was formed. This mixture was centrifuged (9500 rpm, 1 min), and the solution was decanted and dried under reduced pressure. The obtained solid was used in the next cyclization reaction. For the cyclization reaction of the peptide, the peptide was dissolved in DMSO so that the final concentration was 5 mM based on the number of moles of the solid phase resin, 10 eq of triethylamine was added, and the mixture was shaken at room temperature for about 15 hr. The obtained reaction solution was concentrated under reduced pressure using GenevaC HT-12. The obtained mixture was dissolved in DMSO, 3 eq of silver acetate was added, and the mixture was shaken for 3 hr. 2 M DTT aqueous solution (11 eq) was added, and the mixture was centrifuged, and the supernatant was recovered and concentrated under reduced pressure to give a crude product.

The obtained crude product was purified using the following conditions (column; Waters Xbridge (registered trademark) C18 5 um (registered trademark) 50x150 mm: mobile phase; A=0.1% TFA in H₂O, B=0.1% TFA in MeCN: temperature 40°C: gradient (%B); 11-11% over 3 min, then 11-36% over 3 min, then 36-41% over 8 min, then 41-60% over 1 min: flow 120 mL/min).

The purity of the target product was calculated from the area ratio of the LC/MS (UV wavelength 225 nm) chromatogram under analysis condition B and was 94.8%.
analysis conditions B: retention time =4.31 min: column;
Kinetex EVO C18 2.6 µm 2.1x150 mm, 100Å: mobile phase; A=0.025% TFA in H₂O, B=0.025% TFA in MeCN: temperature 60°C: gradient (%B cone); 20-60% over 7.2 min, then 60-95% over 0.3 min, then 95-95% over 1.6 min: flow 0.5 mL/min
ESI-MS(+) observed value m/z=1324.94(M+2H)²⁺

### [Synthetic Example 2-14]

### Synthesis of 894 A hgl CyCloamide (SEQ ID NO: 408)

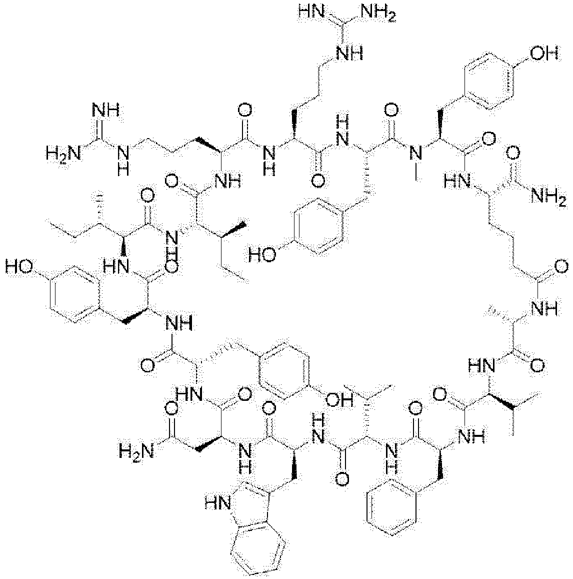

Using Sieber amide resin (Watanabe Chemical, 0.47 mmol/g), according to a general method, the target peptide was synthesized by starting from the removal of the Fmoc group. At that time, CEM's Liberty Blue HT was used as a solid phase synthesizer, and performed according to the manufacturer's manual. The basic condition for condensation reaction was to react once at 90°C for 3 min using DIPCI, Oxyma pure as the condensing agent. For the 11th residue and the 12th residue, the reaction was repeated twice at 50°C for 15 min. For the 13th residue, the reaction was repeated twice at 90°C for 3 min. The basic condition for Fmoc removal was to react with a 20% piperidine solution in DMF at 75°C for 3 min. For the 13th residue, Fmoc was removed by reacting twice at 25°C for 5 min. The obtained resin was washed with DMF five times and methylene chloride three times, and dried under reduced pressure. Then, reactant cocktail-A (a mixture of TFA/H₂O/TIS/DODT in a volume ratio of 92.5:2.5:2.5:2.5) was added to the reaction vessel containing the solid-phase resin, and the mixture was shaken at room temperature for 90 min. The reaction solution was collected by filtration through a frit. The solid-phase resin remaining in the reaction vessel was shaken again with the cutting cocktail, and the solution components were collected from the frit and mixed with the above-mentioned filtrate. When this filtrate was added to an excess diisopropyl ether-hexane mixed solvent cooled to 0°C, a cloudy white precipitate was formed. This mixture was centrifuged (9000 rpm, 2 min), and the supernatant was decanted. The obtained solid was washed with ice-cooled diethyl ether and dried. The obtained solid was used in the next cyclization reaction. For the cyclization reaction of the peptide, the peptide was dissolved in DMF so that the final concentration was 5 mM based on the number of moles of the solid phase resin, 1.5 equivalents of HATU, and 3 equivalents of triethylamine were added, and the mixture was shaken at room temperature for about 1 hr. The obtained reaction solution was concentrated under reduced pressure using Savant Explorer SpeedVaC.

The obtained crude product was purified using the following conditions (column; Waters Xbridge (registered trademark) C18 5 um (registered trademark) 30x150 mm: mobile phase; A=0.1% TFA in H₂O, B=0.1% TFA in MeCN: temperature 40°C: gradient (%B); 6-31% over 3 min, then 31-36% over 8 min, then 36-60% over 1 min: flow 45 mL/min).

The purity of the target product was calculated from the area ratio of the LC/MS (UV wavelength 225 nm) chromatogram under analysis condition B and was 96.8%.
analysis conditions B: retention time =10.03 min: gradient (%B cone); 20-60% over 20 min, then 60-95% over 1 min, then 95% over 5 min
ESI-MS(+) observed value m/z=1033.0(M+2H)²⁺

### [Synthetic Example 2-15]

### Synthesis of 894 BiCyCle 001 (SEQ ID NO: 409)

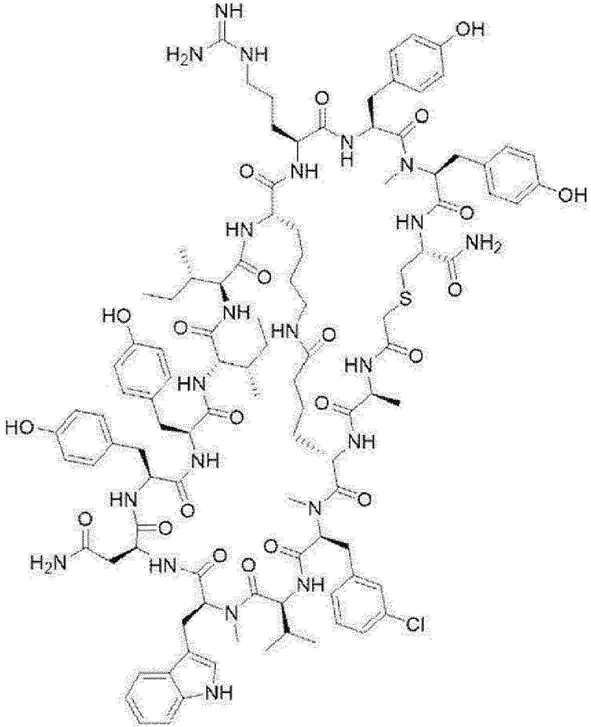

Using Sieber amide resin (Watanabe Chemical, 0.47 mmol/g, 0.53 mg), the target peptide was synthesized by starting from the removal of the Fmoc group according to the general method described above. At that time, CEM's Liberty Blue HT was used as a solid phase synthesizer, and the synthesis was performed according to the manufacturer's manual. For the introduction of each residue, Fmoc-AA/HATU/DIEA (4.2 equivalents/4 equivalents/8 equivalents) was used for 1 equivalent of the resin, and the reaction was performed twice in DMF at 75°C for 10 min. For the 1st residue, the 6th residue, and the 10th residue, the reaction was performed once at 75°C for 10 min. For the 2nd residue and the 3rd residue, the reaction was performed twice at 75°C for 20 min. For the 4th residue, the reaction was performed twice at 75°C for 30 min. For the 11th residue and the 14th residue, the reaction was performed once at 75°C for 20 min. For the 12th residue and the 15th residue, the reaction was performed once at 30°C for 30 min. In addition, the basic condition for Fmoc removal was to react once with a 20% piperidine solution in DMF at 75°C for 3 min. For Fmoc removal at the 2nd residue, the 4th residue, and the 13th residue, the reaction was repeated twice at 25°C for 5 min. To introduce the chloroacetyl group, the Fmoc group of the α-amino group was removed by the aforementioned method from the solid phase resin carrying the Fmoc-protected peptide obtained in the previous step, 5 equivalents of chloroacetic acid, 5 equivalents of DIPCI, and 5 equivalents of HOSu were stirred in DCM, and the same amount of DMF as DCM was added to prepare a DCM/DMF solution (0.2 M) of ClAcOSu, the solution was added to the solid phase resin, and the mixture was shaken at room temperature for 75 min. For deprotection of side chains and cutting out from the solid-phase resin, first, the resin obtained after the chloroacetyl group introduction step was washed with DMF five times and methylene chloride three times, and then dried under reduced pressure. Then, reactant cocktail-A (a mixture of TFA/H₂O/TIS/DODT in a volume ratio of 92.5:2.5:2.5:2.5) was added to the reaction vessel containing the solid-phase resin, and the mixture was shaken at room temperature for 90 min. The reaction solution was collected by filtration through a frit. The solid-phase resin remaining in the reaction vessel was shaken again with the cutting cocktail, and the solution components were collected from the frit and mixed with the above-mentioned filtrate. When this filtrate was added to an excess diisopropyl ether cooled to 0°C, a cloudy white precipitate was formed. The mixture was centrifuged (9500 rpm, 1 min) and the solution was decanted. The obtained solid was washed again with a small amount of diethyl ether cooled to 0°C, and then dried under reduced pressure. The obtained solid was used in the next cyclization reaction. For the cyclization reaction of the peptide, the peptide was dissolved in 5% water-containing DMSO so that the final concentration was 4 mM based on the number of moles of the solid phase resin, 10 equivalents of triethylamine was added, and the mixture was shaken at room temperature for about 12 hr. The obtained reaction solution was concentrated under reduced pressure using GenevaC EZ-2 Elite. The peptide was dissolved in DMF so that the final concentration was 25 mM with respect to the obtained mixture based on the number of moles of the solid phase resin, 1 equivalent of HATU and 3 equivalents of DIEA were added, and the mixture was shaken at room temperature for about 30 min.

The obtained crude product was purified using the following conditions (column; Waters Xbridge (registered trademark) C18 5 um (registered trademark) 50x150 mm: mobile phase; A=0.1% TFA in H₂O, B=0.1% TFA in MeCN: temperature 40°C: gradient (%B); 11-36% over 3 min, then 36-41% over 8 min, then 41-60% over 1 min: flow 120 mL/min)

The purity of the target product was calculated from the area ratio of the LC/MS (UV wavelength 225 nm) chromatogram under analysis condition B and was 94.8%.
analysis conditions B: retention time =13.78 min: column;
Kinetex EVO C18 2.6 µm 2.1x150 mm, 100Å; :mobile phase; A=0.025% TFA in H₂O, B=0.025% TFA in MeCN: temperature °C:60°C: gradient (%B conc); 20-60% over 20 min, then 60-95% over 1 min, then 95-95% over 5 min: flow 0.25%
ESI-MS(+) observed value m/z=1072.26(M+2H)²⁺

### [Synthetic Example 2-16]

### Synthesis of 894 BiCyCle 006 (SEQ ID NO: 414)

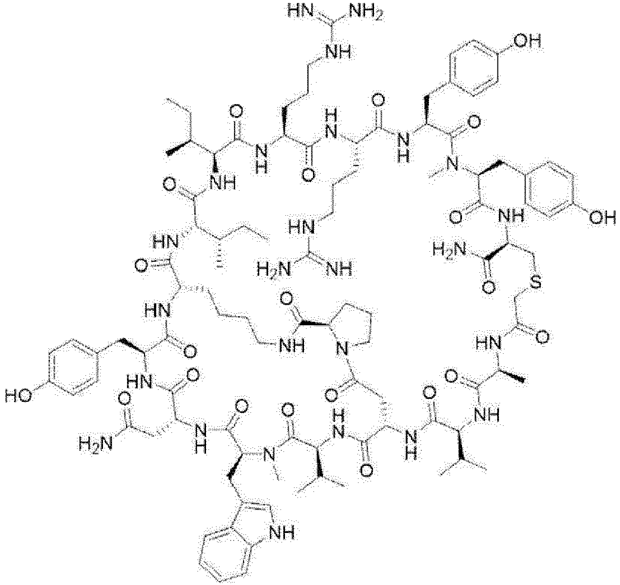

Using Sieber amide resin (Watanabe Chemical, 0. 47 mmol/g, 0.32 g), the target peptide was synthesized by starting from the removal of the Fmoc group according to the general method described above. At that time, CEM's Liberty Blue HT was used as a solid phase synthesizer, and the synthesis was performed according to the manufacturer's manual. For the introduction of each residue, Fmoc-AA/DIPCI/Oxyma pure/DIEA (5.3 equivalents/10 equivalents/5 equivalents) was used for 1 equivalent of resin, and the reaction was performed once in DMF at 90°C for 3 min. When introducing the 2nd residue, the 4th residue, the 11th residue, and the 13th residue, the reaction was performed twice at 90°C for 3 min. When introducing the 12th residue, the reaction was performed twice at 50°C for 15 min. When introducing the 15th residue, the reaction was performed once at 50°C for 15 min. In addition, the basic condition for Fmoc removal was to react once with a 20% piperidine solution in DMF at 75°C for 3 min. For Fmoc removal at the 2nd residue, the 4th residue, and the 13th residue, the reaction was repeated twice at 25°C for 5 min. The solid phase resin was washed successively with DMF, DCM, dried under reduced pressure, swollen with DCM, 10 equivalents of PhSiH₃ and 0.2 equivalents of Pd(PPh₃)₄ were added, and the mixture was shaken at room temperature for 1 hr. The solid phase resin was washed 5 times with DCM, 5 times with DMF, 3 times with diethyl ether, and dried. The solid phase resin was swollen with NMP, 5 equivalents of HATU and 5 equivalents of DIEA were added, and the mixture was shaken at room temperature for 30 min. The resin was washed, 5 equivalents of HATU and 5 equivalents of DIEA were added again, and the mixture was shaken at room temperature for 30 min. The solid phase resin was washed 5 times with DCM, 5 times with DMF, and 3 times with diethyl ether. To introduce the chloroacetyl group, the Fmoc group of the α-amino group was removed by the aforementioned method from the solid phase resin carrying the Fmoc-protected peptide obtained in the previous step, 5 equivalents of chloroacetic acid, 5 equivalents of DIPCI, and 5 equivalents of HOSu were stirred in DCM, and the same amount of DMF as DCM was added to prepare a DCM/DMF solution (0.2 M) of ClAcOSu, the solution was added to the solid phase resin, and the mixture was shaken at room temperature for 60 min. For deprotection of side chains and cutting out from the solid-phase resin, first, the resin obtained after the chloroacetyl group introduction step was washed with DMF five times and methylene chloride three times, and then dried under reduced pressure. Then, reactant cocktail-A (a mixture of TFA/H₂O/TIS/DODT in a volume ratio of 92.5:2.5:2.5:2.5) was added to the reaction vessel containing the solid-phase resin, and the mixture was shaken at room temperature for 90 min. The reaction solution was collected by filtration through a frit. The solid-phase resin remaining in the reaction vessel was shaken again with the cutting cocktail, and the solution components were collected from the frit and mixed with the above-mentioned filtrate. When this filtrate was added to an excess diisopropyl ether cooled to 0°C, a cloudy white precipitate was formed. The mixture was centrifuged (9500 rpm, 1 min) and the solution was decanted. The obtained solid was washed again with a small amount of diethyl ether cooled to 0°C, and then dried under reduced pressure. The obtained solid was used in the next cyclization reaction. For the cyclization reaction of the peptide, the peptide was dissolved in 5% water-containing DMSO so that the final concentration was 3.3 mM based on the number of moles of the solid phase resin, 7 equivalents of triethylamine was added, and the mixture was shaken at room temperature for about 12 hr. The obtained reaction solution was concentrated under reduced pressure using GenevaC EZ-2 Elite.

The obtained crude product was purified using the following conditions (column; Waters Xbridge (registered trademark) C18 5 um (registered trademark) 50x150 mm: mobile phase; A=0.1% TFA in H₂O, B=0.1% TFA in MeCN: temperature °C:40°C: gradient (%B); 7-32% over 3 min, then 32-37% over 8 min, then 37-60% over 1 min: flow rate 120 mL/min), freeze-dried, and purified again under the following conditions. (column; Waters Xbridge (registered trademark) C18 5 um (registered trademark) 50x150 mm: mobile phase; A=0.1% TFA in H₂O, B=0.1% TFA in MeCN: temperature 40°C: gradient (%B); 11-36% over 3 min, then 36-41% over 8 min, then 41-60% over 1 min: flow 120 mL/min)

The purity of the target product was calculated from the area ratio of the LC/MS (UV wavelength 225 nm) chromatogram under analysis condition B and was 89.0%.
analysis conditions B: retention time =3.67 min: column;
Kinetex EVO C18 2.6 µm. 2.1x150 mm, 100Å: mobile phase; A=0.025% TFA in H₂O, B=0.025% TFA in MeCN: temperature 60°C: gradient (%B conc); 20-60% over 7.2 min, then 60-95% over 0.3 min, then 95-95% over 1.6 min: flow 0.5 mL/min
ESI-MS(+) observed value m/z=1055.13(M+2H)²⁺

### [Synthetic Example 2-17]

### Synthesis of 894 BiCyCle 012 (SEQ ID NO: 419)

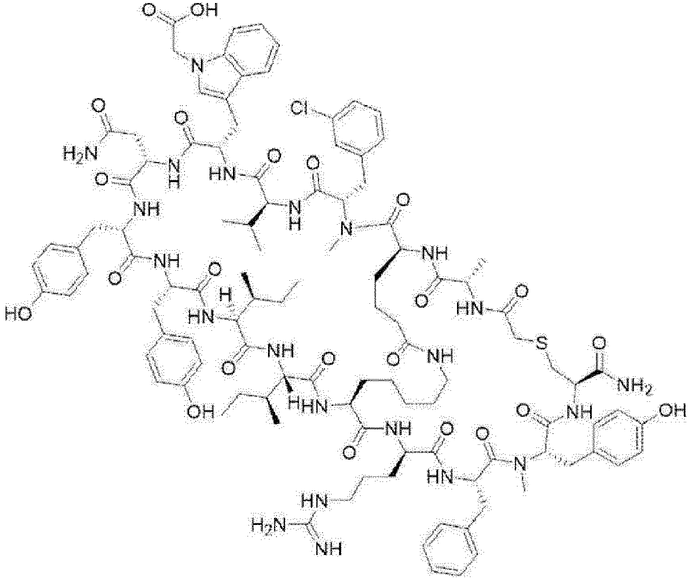

Using Sieber amide resin (Watanabe Chemical, 0.47 mmol/g, 213 mg), the target peptide was synthesized by starting from the removal of the Fmoc group according to the general method described above. At that time, CEM's Liberty Blue was used as a solid phase synthesizer, and the synthesis was performed according to the manufacturer's manual. By a method similar to the synthesis of Bicycle_01 and Bicycle_06 except that Fmoc-W1aa(Allyl)-OH, Fmoc-Hgl(tBu)-OH, Fmoc-Hly(Boc)-OH were used as amino acid starting materials, a crude product was obtained.

The obtained crude product was purified using the following conditions (column; Waters Xbridge (registered trademark) C18 5 um (registered trademark) 19x150 mm: mobile phase; A=0.1% TFA in H₂O, B=0.1% TFA in MeCN: temperature 60°C: gradient (%B); 11-36% over 3 min, then 36-41% over 8 min, then 41-60% over 1 min: flow rate 17 mL/min)

The purity of the target product was calculated from the area ratio of the LC/MS (UV wavelength 225 nm) chromatogram under analysis condition B and was 94.8%.
analysis conditions B: retention time =4.76 min: column;
Kinetex EVO C18 2.6 um 2.1x150 mm, 100Å; :mobile phase; A=0.025% TFA in H₂O, B=0.025% TFA in MeCN: temperature 60°C: gradient (%B conc); 20-60% over 7.2 min, then 60-95% over 0.3 min, then 95% over 1.6 min
ESI-MS(+) observed value m/z=1093.23(M+2H)²⁺

### [Synthetic Example 2-18]

### Synthesis of 894 3 m G (SEQ ID NO: 421)

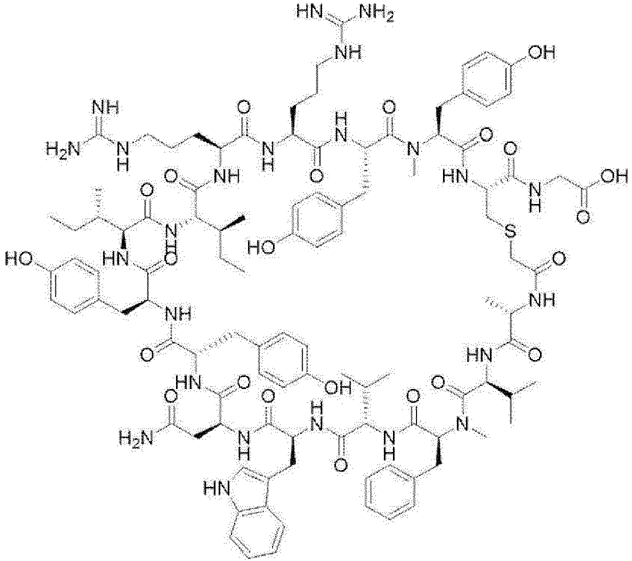

Using Fmoc-Gly-wang resin (Watanabe Chemical, 0.78 mmol/g, 1.31 g), the target peptide was synthesized by starting from the removal of the Fmoc group according to the general method described above. At that time, CEM's Liberty Blue was used as a solid phase synthesizer, and the synthesis was performed according to the manufacturer's manual. For the introduction of each residue, Fmoc-AA/HATU/DIEA (4.2 equivalents/4 equivalents/8 equivalents) was used for 1 equivalent of resin, and the reaction was repeated twice in DMF at 60°C for 15 min. However, when introducing the 15th residue, the reaction was performed once at 25°C for 30 min. When introducing the 11th residue and 12th residue, the reaction was repeated twice at 25°C for 20 min. When introducing the 10th residue, the reaction was performed once at 60°C for 15 min. When introducing the 7th residue, the reaction was performed once at 60°C for 15 min. When introducing the fifth residue, the reaction was performed once at 60°C for 15 min. When introducing the third residue, the reaction was performed twice at 60°C for 45 min. The basic condition for Fmoc removal was to react with a 20% piperidine solution in DMF at 75°C for 3 min. For Fmoc removal at the 2nd residue and the 13th residue, the reaction was repeated twice at 25°C for 5 min. To introduce the chloroacetyl group, the Fmoc group of the α-amino group was removed by the aforementioned method from the solid phase resin carrying the Fmoc-protected peptide obtained in the previous step, chloroacetic acid (5 equivalents), 5 equivalents of DIPCI, and 5 equivalents of HOSu were stirred in DCM, and the same amount of DMF as DCM was added to prepare a DCM/DMF solution (0.2 M) of ClAcOSu, the solution was added to the solid phase resin and the mixture was shaken at room temperature for 240 min. For deprotection of side chains and cutting out from the solid-phase resin, first, the resin obtained after the chloroacetyl group introduction step was washed with DMF five times and methylene chloride three times, and then dried under reduced pressure. Then, reactant cocktail-A (a mixture of TFA/H₂O/TIS/DODT in a volume ratio of 92.5:2.5:2.5:2.5) was added to the reaction vessel containing the solid-phase resin, and the mixture was shaken at room temperature for 90 min. The reaction solution was collected by filtration through a frit. The solid-phase resin remaining in the reaction vessel was shaken again with the cutting cocktail, and the solution components were collected from the frit and mixed with the above-mentioned filtrate. When this filtrate was added to an excess diisopropyl ether cooled to 0°C, a cloudy white precipitate was formed. The mixture was centrifuged (9500 rpm, 1 min) and the solution was decanted. The obtained solid was washed again with a small amount of diethyl ether cooled to 0°C, and then dried under reduced pressure. The obtained solid was used in the next cyclization reaction. For the cyclization reaction of the peptide, the peptide was dissolved in DMSO so that the final concentration was 4 mM based on the number of moles of the solid phase resin, 10 equivalents of triethylamine was added, and the mixture was shaken at room temperature for about 12 hr. The obtained reaction solution was concentrated under reduced pressure using GenevaC EZ-2.

The obtained crude product was purified using the following conditions (column; Waters Xbridge (registered trademark) C18 5 um (registered trademark) 50x250 mm: mobile phase; A=0.1% TFA in H₂O, B=0.1% TFA in MeCN: temperature 60°C: gradient (%B); 0-0% over 5 min, then 0-4.2% over 2 min, then 4.2-28.6% over 3 min, then 28.6-33.7% over 15.5 min, then 33.7-60% over 1.5 min: flow rate 18 mL/min for 5 min, then 18 mL/min-118 mL/min over 2 min, then 118 mL/min).

The purity of the target product was calculated from the area ratio of the LC/MS (UV wavelength 225 nm) chromatogram under analysis condition B and was 92.2%.
analysis conditions B: retention time =11.52 min: gradient (%B conc); 20-60% over 20 min, then 60-95% over 1 min, then 95% over 5 min
ESI-MS(+) observed value m/z=1078.24(M+2H)²⁺

### [Synthetic Example 2-19]

### Synthesis of 894 3 m G4S2C(ACNMe) (SEQ ID NO: 443)

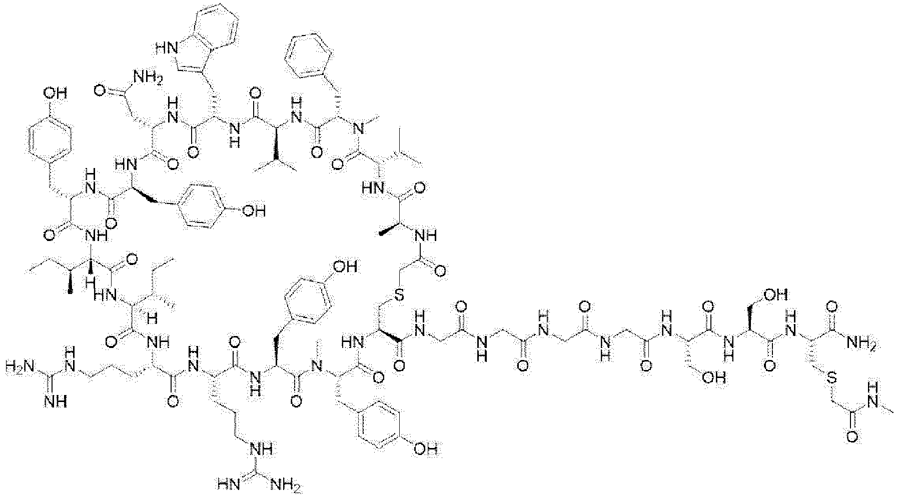

Separately synthesized 894_3 m_G4S2C (20 mg, 7.07 µmol) was dissolved in DMF (0.1 mL), 4 equivalents of TEA (3.94 µL, 28.3 µmol) in DMF (39 µL), 1.1 equivalents of 2-Iodo-N-Methylacetamide (39.1 mg, 0.25 mmol) in DMF (15 µL) were added and the mixture was stirred at room temperature for 1 hr.

The obtained mixture was purified using the following conditions (column; Waters Xbridge (registered trademark) C18 5 um (registered trademark) 50x150 mm: mobile phase; A=0.1% TFA in H₂O, B=0.1% TFA in MeCN: temperature 60°C: gradient (%B); 6-31% over 3 min, then 31-36% over 8 min, then 36-60% over 1 min: flow 17 mL/min).

The purity of the target product was calculated from the area ratio of the LC/MS (UV wavelength 225 nm) chromatogram under analysis condition B and was 97.3%.
analysis conditions B: retention time =3.53 min: column;
Kinetex EVO C18 2.6 um 2.1x150 mm, 100Å: mobile phase; A=0.025% TFA in H₂O, B=0.025% TFA in MeCN: temperature 60°C: gradient (%B conc); 20-60% over 7.2 min, then 60-95% over 0.3 min, then 95-95% over 1.6 min: flow 0.5 mL/min
ESI-MS(+) observed value m/z=1337.32(M+2H)²⁺

### [Synthetic Example 2-20]

### Synthesis of 894 0263 (SEQ ID NO: 487)

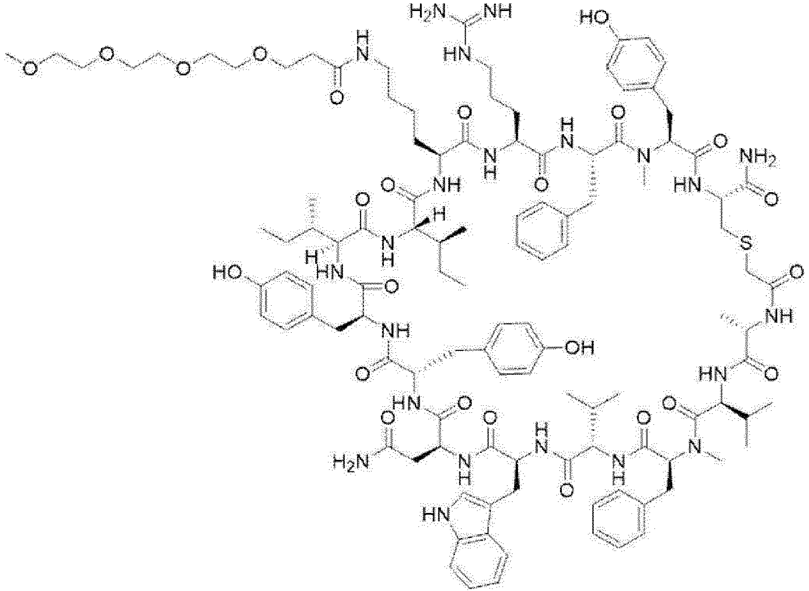

Using Sieber amide resin (Watanabe Chemical, 0.48 mmol/g, 0.26 g), the target peptide was synthesized by starting from the removal of the Fmoc group according to the general method described above. At that time, CEM's Liberty Blue HT was used as a solid phase synthesizer, and the synthesis was performed according to the manufacturer's manual. For the introduction of each residue, Fmoc-AA/DIPCI/Oxyma pure/DIEA (4.2 equivalents/8 equivalents/4 equivalents) was used for 1 equivalent of resin, and the reaction was performed. In addition, Fmoc was removed by reacting with a 20% piperidine solution in DMF. To introduce the chloroacetyl group, the Fmoc group of the α-amino group was removed from the solid-phase resin carrying the Fmoc-protected peptide obtained in the previous step by using the aforementioned method, 5 equivalents of chloroacetic acid in DMF, 5 equivalents of HATU in DMF, and 10 equivalents of DIEA in DMF were added and the mixture was shaken at 25°C for 30 min. For deprotection of side chains and cutting out from the solid-phase resin, first, the resin obtained after the chloroacetyl group introduction step was washed with DMF five times and methylene chloride three times, and then dried under reduced pressure. Then, reactant cocktail-A (a mixture of TFA/H₂O/TIS/DODT in a volume ratio of 92.5:2.5:2.5:2.5) was added to the reaction vessel containing the solid-phase resin, and the mixture was shaken at room temperature for 60 min. The reaction solution was collected by filtration through a frit. The solid-phase resin remaining in the reaction vessel was shaken again with the cutting cocktail, and the solution components were collected from the frit and mixed with the above-mentioned filtrate. When this filtrate was added to an excess mixed solvent of diisopropyl ether/hexane (1/1) cooled to 0°C, a cloudy white precipitate was formed. This mixture was centrifuged (9000 rpm, 2 min), and the solution was decanted. The obtained solid was washed again with a small amount of diethyl ether cooled to 0°C, and then dried under reduced pressure. The obtained solid was used in the next cyclization reaction. For the cyclization reaction of the peptide, the peptide was dissolved in DMSO so that the final concentration was 5 mM based on the number of moles of the solid phase resin, 10 equivalents of triethylamine was added, and the mixture was shaken at room temperature overnight. The reaction solution was concentrated under reduced pressure using Savant Explorer SpeedVac. The obtained solid was dissolved in DMSO so that the final concentration of the peptide was 5 mM based on the number of moles of the solid phase resin, MePEG4c (1.2 equivalents), HATU (1.1 equivalents), and DIEA (3 equivalents) were added and the mixture was shaken at room temperature for 1 hr.

The obtained crude product was purified using the following conditions (column; Waters Xbridge (registered trademark) C18 5 um (registered trademark) 50x150 mm: mobile phase; A=0.1% TFA in H₂O, B=0.1% TFA in MeCN: temperature 40°C: gradient (%B); 13-38% over 3 min, then 38-43% over 8 min, then 43-60% over 1 min: flow 120 mL/min.

The purity of the target product was calculated from the area ratio of the LC/MS (UV wavelength 225 nm) chromatogram under analysis condition B and was 92.3%.
analysis conditions B: retention time =5.28 min: column;
Kinetex EVO C18 2.6 um 2.1x150 mm, 100Å: mobile phase; A=0.025% TFA in H₂O, B=0.025% TFA in MeCN: temperature 60°C: gradient (%B conc); 20-60% over 7.2 min, then 60-95% over 0.3 min, then 95-95% over 1.6 min: flow 0.5 mL/min
ESI-MS(+) observed value m/z=1136.17(M+2H)²⁺

### [Synthetic Example 3]

### Synthesis of 894 variant 61 G4S2C (SEQ ID NO: 358)

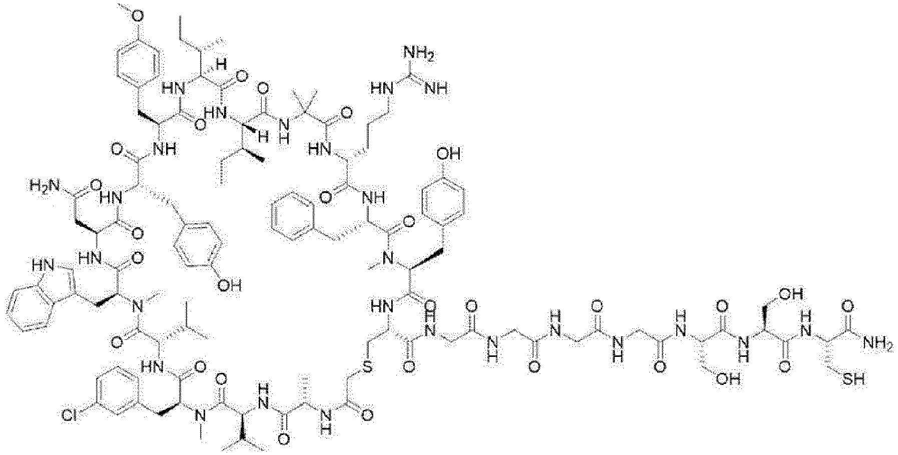

Using Sieber amide resin (Watanabe Chemical, 0.73 mmol/g, 1.37 g), the target peptide was synthesized by starting from the removal of the Fmoc group according to the general method described above. At that time, CEM's Liberty Blue was used as a solid phase synthesizer, and the synthesis was performed according to the manufacturer's manual. For the introduction of each residue, Fmoc-AA/HATU/DIEA (4.2 equivalents/4 equivalents/8 equivalents) was used for 1 equivalent of resin, and the reaction was performed once in DMF at 75°C for 10 min. For the 2nd residue and the 4th residue, the reaction was performed twice at 75°C for 30 min. For the 12th residue, the reaction was repeated twice at 25°C for 20 min. For the 13th residue, the reaction was performed twice at 75°C for 10 min. For the 15th residue and the 22nd residue, the reaction was performed once at 25°C for 30 min. In addition, the basic condition for Fmoc removal was to react with a 20% piperidine solution in DMF at 75°C for 3 min. For Fmoc removal at the 4th residue and the 13th residue, the reaction was performed at 25°C for 5 min and then at room temperature for 10 min. To introduce the chloroacetyl group, the Fmoc group of the α-amino group was removed by the aforementioned method from the solid phase resin carrying the Fmoc-protected peptide obtained in the previous step, 10 equivalents of chloroacetic acid, 10 equivalents of DIPCI, and 10 equivalents of HOSu were stirred in DCM, and the same amount of DMF as DCM was added to prepare a DCM/DMF solution (0.2 M) of ClAcOSu, the solution was added to the solid phase resin, and the mixture was shaken at room temperature for 60 min. For deprotection of side chains and cutting out from the solid-phase resin, first, the resin obtained after the chloroacetyl group introduction step was washed with DMF five times and methylene chloride three times, and then dried under reduced pressure. Successively, reactant cocktail-A (a mixture of TFA/H₂O/TIS/DODT in a volume ratio of 90:2.5:2.5:5) was added to the reaction vessel containing the solid-phase resin, and the mixture was shaken at room temperature for 90 min. The reaction solution was collected by filtration through a frit. The solid-phase resin remaining in the reaction vessel was shaken again with the cutting cocktail, and the solution components were collected from the frit and mixed with the above-mentioned filtrate. When this filtrate was added to an excess mixed solvent of diisopropyl ether/hexane (1/1) cooled to 0°C, a cloudy white precipitate was formed. This mixture was centrifuged (9500 rpm, 1 min), and the solution was decanted and dried under reduced pressure. The obtained solid was used in the next cyclization reaction. For the cyclization reaction of the peptide, the peptide was dissolved in DMSO so that the final concentration was 5 mM based on the number of moles of the solid phase resin, 10 equivalents of triethylamine was added, and the mixture was shaken at room temperature for about 15 hr. The reaction mixture was quenched with acetic acid, and concentrated under reduced pressure using GenevaC HT-12. The obtained mixture was dissolved in DMSO (20 mL), 3 equivalents of silver acetate was added and the mixture was shaken for 3 hr. 2 M DTT aqueous solution (11 equivalents) was added, and the mixture was centrifuged, and the supernatant was recovered and concentrated under reduced pressure to give a crude product.

The obtained crude product was purified using the following conditions (column; Waters Xbridge (registered trademark) C18 5 um (registered trademark) 50x250 mm: mobile phase; A=0.1% TFA in H₂O, B=0.1% TFA in MeCN: temperature 60°C: gradient (%B); 6.7-6.7% over 5 min, then 6.7-10.3% over 2 min, then 10.3-35.8% over 3 min, then 35.8-40.8% over 15.5 min, then 40.8-60% over 1.5 min: flow 18 mL/min-18 mL/min over 5 min, then 18 mL/min-118 mL/min over 2 min, then 118 mL/min.).

The purity of the target product was calculated from the area ratio of the LC/MS (UV wavelength 225 nm) chromatogram under analysis condition B and was 94.8%.
analysis conditions B: retention time =5.45 min: column;
Kinetex EVO C18 2.6 um 2.1x150 mm, 100Å: mobile phase; A=0.025% TFA in H₂O, B=0.025% TFA in MeCN: temperature 60°C: gradient (%B conc); 20-60% over 7.2 min, then 60-95% over 0.3 min, then 95-95% over 1.6 min: flow 0.5 mL/min
ESI-MS(+) observed value m/z=1289.65(M+2H)²⁺

### [Synthetic Example 4-1]

### Synthesis of 894 3142 (SEQ ID NO: 547)

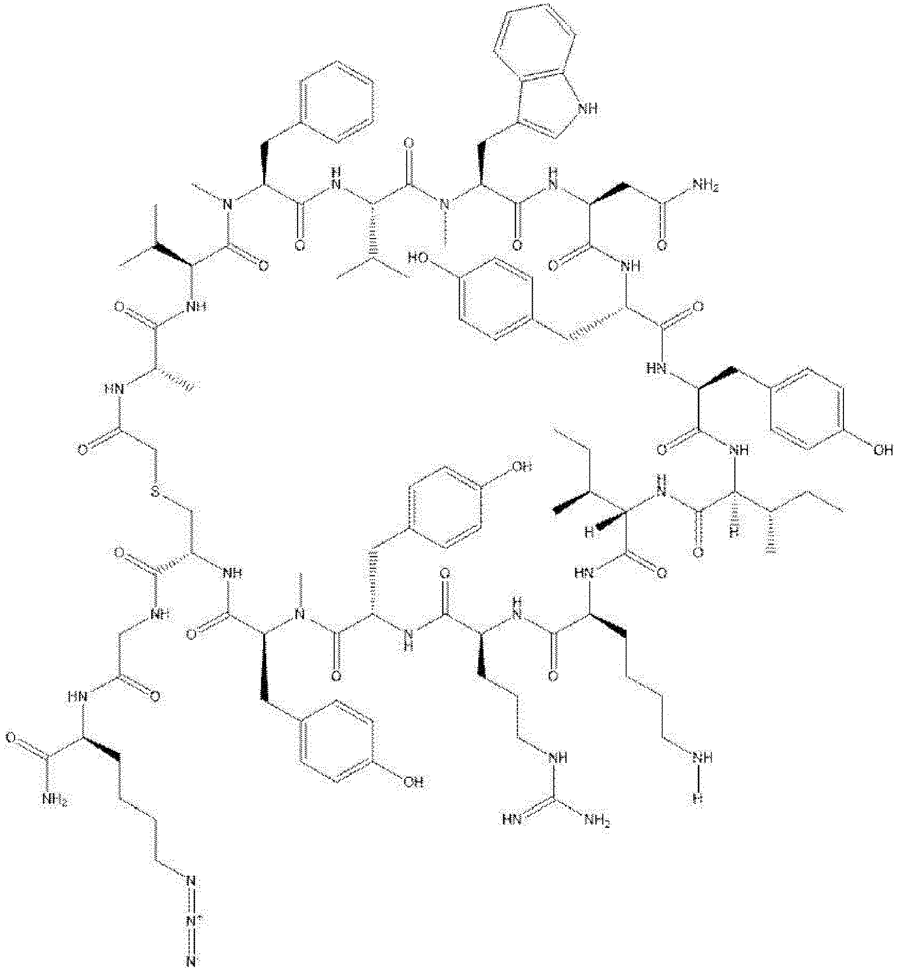

Using Sieber amide resin (Watanabe Chemical, 0.48 mmol/g, 0.52 g), the target peptide was synthesized by starting from the removal of the Fmoc group. At that time, CEM's Liberty Blue was used as an automatic synthesizer, and the synthesis was performed according to the manufacturer's manual. The synthesis was performed in the same manner as in Example 1.

The obtained crude product was purified using the following conditions (column: Waters Xbridge (registered trademark) C18 5 um (registered trademark) 50x150 mm; mobile phase: A=0.1% TFA in H₂O, B=0.1% TFA in MeCN; temperature: 40°C; gradient (%B): 7-32% over 3 min, then 32-37% over 8 min, then 37-60% over 1 min; flow; 120 mL/min).

The purity of the target product was calculated from the area ratio of LC/MS (UV wavelength 225 nm) chromatogram under the following analytical conditions and was 94.20%.
analysis conditions B: retention time =4.12 min; column Kinetex EVO C18 2.6 um, 2.1x150 mm, 100Å; mobile phase A=0.025% TFA in H₂O, B=0.025% TFA in MeCN; temperature 60°C; gradient (%B conc): 20-60% over 7.15 min, then 60-95% over 0.3 min, then 95-95% over 1.55 min; flow: 0.5 mL/min
ESI-MS(+) observed value m/z=1147.70(M+2H)²⁺

### [Synthetic Example 4-2]

### Synthesis of 894 3143 (SEQ ID NO: 548)

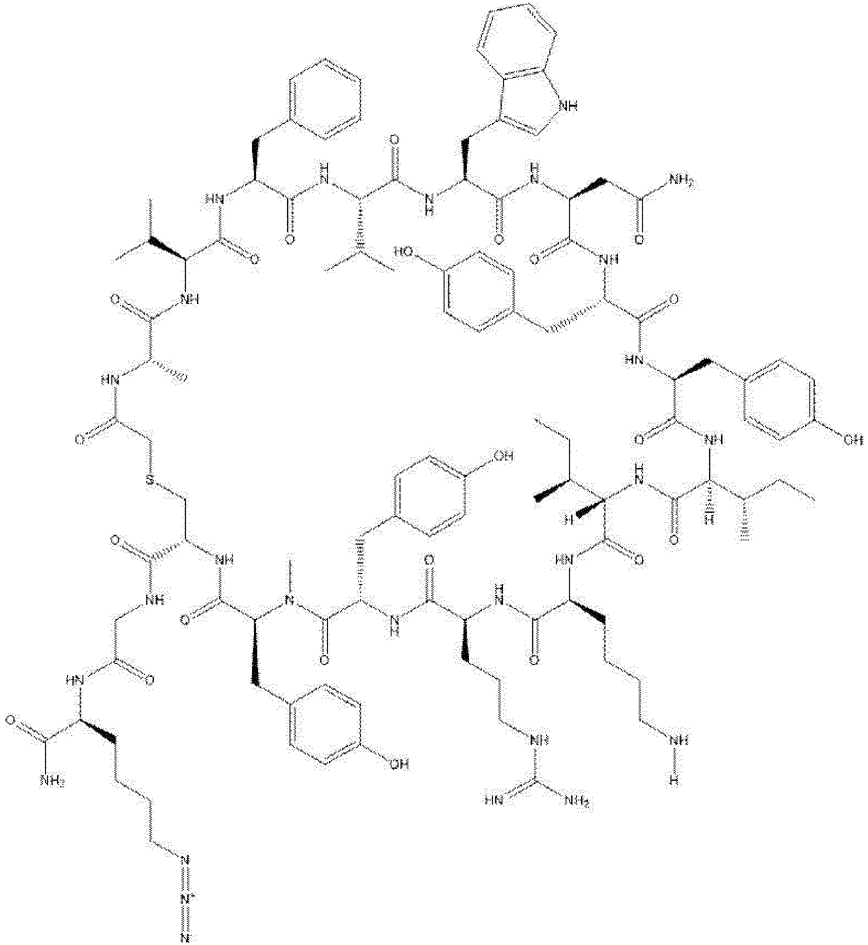

Using Sieber amide resin (Watanabe Chemical, 0.48 mmol/g, 0.52 g), the target peptide was synthesized by starting from the removal of the Fmoc group. At that time, CEM's Liberty Blue HT was used as an automatic synthesizer, and the synthesis was performed according to the manufacturer's manual. The synthesis was performed in the same manner as in Example 1.

The obtained crude product was purified using the following conditions (column: Waters Xbridge (registered trademark) C18 5 um (registered trademark) 50x150 mm; mobile phase: A=0.1% TFA in H₂O, B=0.1% TFA in MeCN; temperature: 40°C; gradient (%B): 6-6% over 2 min, then 6-31% over 1 min, then 31-36% over 8 min, then 36-60% over 1 min; flow; 20-20 mL/min over 1 min, then 20-120 mL/min over 1 min, then 120 mL/min).

The purity of the target product was calculated from the area ratio of LC/MS (UV wavelength 225 nm) chromatogram under the following analytical conditions and was 97.61%.
Analysis conditions: retention time =3.75 min; column Kinetex EVO C18 2.6 um, 2.1x150 mm, 100Å; mobile phase A=0.025% TFA in H₂O, B=0.025% TFA in MeCN; temperature 60°C; gradient (%B conc): 20-60% over 7.15 min, then 60-95% over 0.3 min, then 95-95% over 1.55 min; flow: 0.5 mL/min
ESI-MS(+) observed value m/z=1133.66(M+2H)²⁺

### [Synthetic Example 4-3]

### Synthesis of 894 3144 (SEQ ID NO: 549)

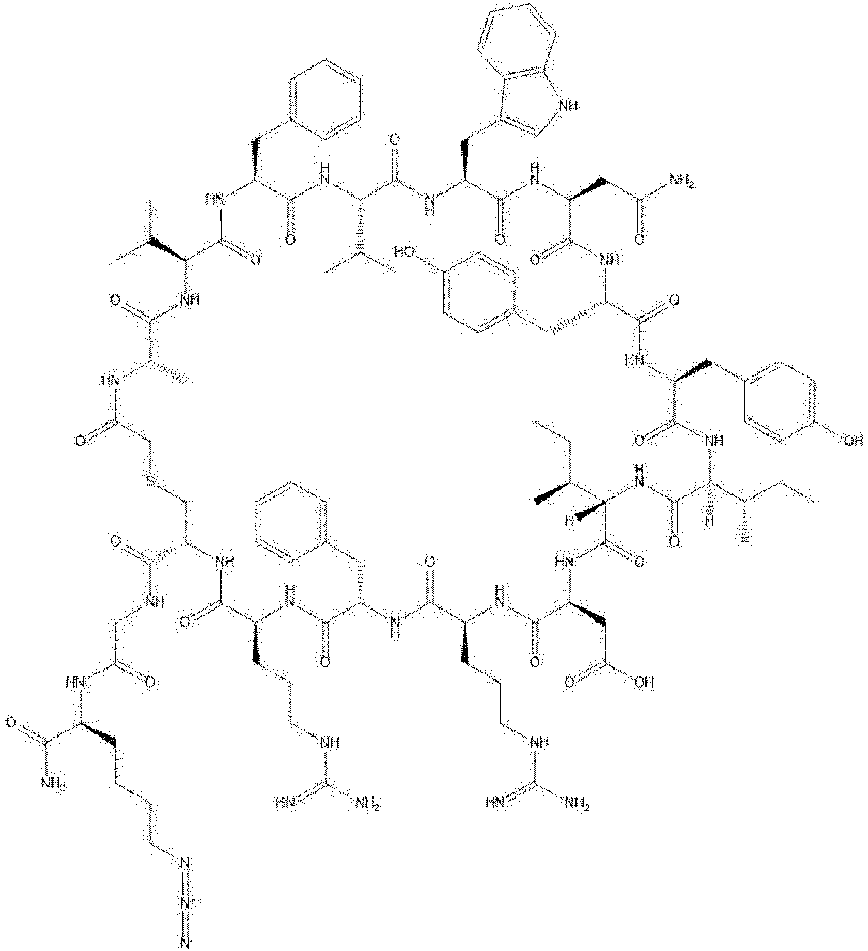

Using Sieber amide resin (Watanabe Chemical, 0.48 mmol/g, 0.52 g), the target peptide was synthesized by starting from the removal of the Fmoc group. At that time, CEM's Liberty Blue was used as an automatic synthesizer, and the synthesis was performed according to the manufacturer's manual. The synthesis was performed in the same manner as in Example 1.

The obtained crude product was purified using the following conditions (column: Waters Xbridge (registered trademark) C18 5 um (registered trademark) 50x150 mm; mobile phase: A=0.1% TFA in H₂O, B=0.1% TFA in MeCN; temperature: 40°C; gradient (%B): 8-8% over 2 min, then 8-33% over 1 min, then 33-38% over 8 min, then 38-60% over 1 min; flow; 20-20 mL/min over 1 min, then 20-120 mL/min over 1 min, then 120 mL/min).

The purity of the target product was calculated from the area ratio of LC/MS (UV wavelength 225 nm) chromatogram under the following analytical conditions and was 97.52%.
analysis conditions B: retention time =3.97 min; column Kinetex EVO C18 2.6 um, 2.1x150 mm, 100Å; mobile phase A=0.025% TFA in H₂O, B=0.025% TFA in MeCN; temperature 60°C; gradient (%B conc): 20-60% over 7.15 min, then 60-95% over 0.3 min, then 95-95% over 1.55 min; flow: 0.5 mL/min
ESI-MS(+) observed value m/z=1108.62(M+2H)²⁺

### [Synthetic Example 4-4]

### Synthesis of 894 3145 (SEQ ID NO: 550)

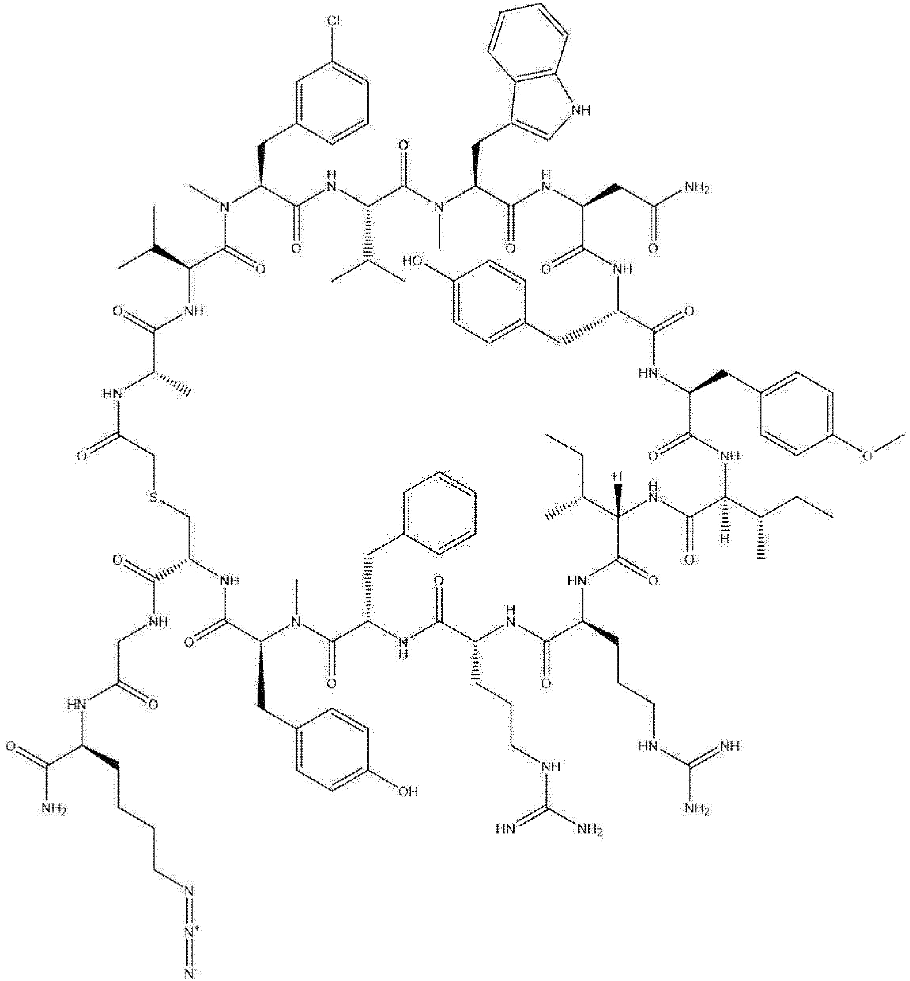

Using Sieber amide resin (Watanabe Chemical, 0.48 mmol/g, 0.52 g), the target peptide was synthesized by starting from the removal of the Fmoc group. At that time, CEM's Liberty Blue was used as an automatic synthesizer, and the synthesis was performed according to the manufacturer's manual. The synthesis was performed in the same manner as in Example 1.

The obtained crude product was purified using the following conditions (column: Waters Xbridge (registered trademark) C18 5 um (registered trademark) 50x150 mm; mobile phase: A=0.1% TFA in H₂O, B=0.1% TFA in MeCN; temperature: 40°C; gradient (%B): 13-38% over 3 min, then 38-43% over 8 min, then 43-60% over 1 min; flow; 120 mL/min).

The purity of the target product was calculated from the area ratio of LC/MS (UV wavelength 225 nm) chromatogram under the following analytical conditions and was 98.02%.
Analysis conditions: retention time =4.91 min; column Kinetex EVO C18 2.6 um, 2.1x150 mm, 100Å; mobile phase A=0.025% TFA in H₂O, B=0.025% TFA in MeCN; temperature 60°C; gradient (%B conc): 20-60% over 7.15 min, then 60-95% over 0.3 min, then 95-95% over 1.55 min; flow: 0.5 mL/min
ESI-MS(+) observed value m/z=1177.95(M+2H)²⁺

### [Synthetic Example 4-5]

### Synthesis of 894 3147 (SEQ ID NO: 551)

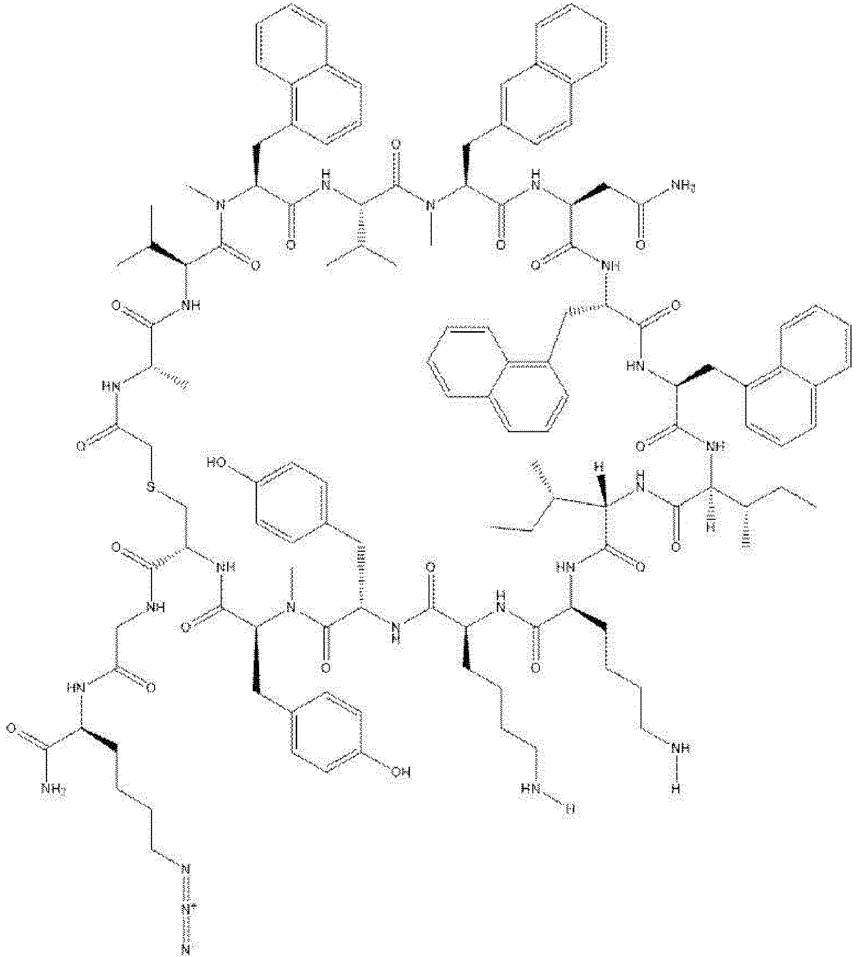

Using Sieber amide resin (Watanabe Chemical, 0.48 mmol/g, 0.52 g), the target peptide was synthesized by starting from the removal of the Fmoc group. At that time, CEM's Liberty Blue was used as an automatic synthesizer, and the synthesis was performed according to the manufacturer's manual. The synthesis was performed in the same manner as in Example 1.

The obtained crude product was purified using the following conditions (column: Waters Xbridge (registered trademark) C18 5 um (registered trademark) 50x150 mm; mobile phase: A=0.1% TFA in H₂O, B=0.1% TFA in MeCN; temperature: 40°C; gradient (%B): 21-46% over 3 min, then 46-51% over 8 min, then 51-60% over 1 min; flow; 120 mL/min).

The purity of the target product was calculated from the area ratio of LC/MS (UV wavelength 225 nm) chromatogram under the following analytical conditions and was 96.34%.
Analysis conditions: retention time =6.24 min; column Kinetex EVO C18 2.6 um, 2.1x150 mm, 100Å; mobile phase A=0.025% TFA in H₂O, B=0.025% TFA in MeCN; temperature 60°C; gradient (%B conc): 20-60% over 7.15 min, then 60-95% over 0.3 min, then 95-95% over 1.55 min; flow: 0.5 mL/min
ESI-MS(+) observed value m/z=1198.29(M+2H)²⁺

### [Synthetic Example 4-6]

### Synthesis of 894 3148 (SEQ ID NO: 552)

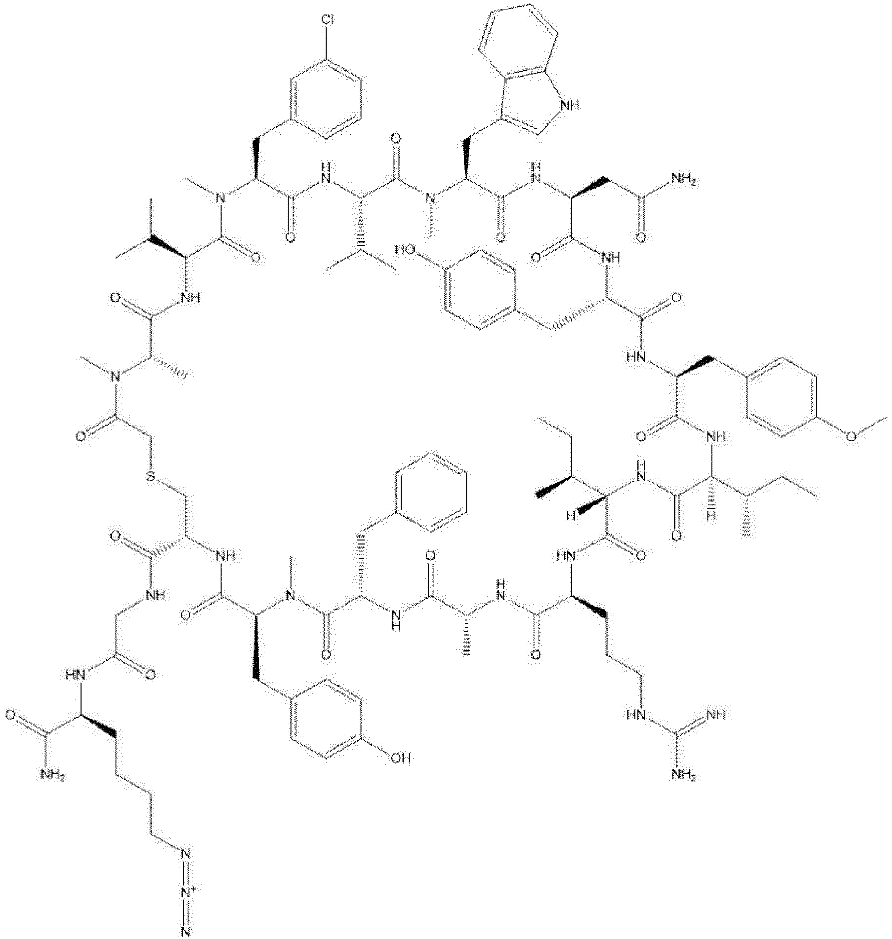

Using Sieber amide resin (Watanabe Chemical, 0.48 mmol/g, 0.52 g), the target peptide was synthesized by starting from the removal of the Fmoc group. At that time, CEM's Liberty Blue was used as an automatic synthesizer, and the synthesis was performed according to the manufacturer's manual. The synthesis was performed in the same manner as in Example 1.

The obtained crude product was purified using the following conditions (column: Waters Xbridge (registered trademark) C18 5 um (registered trademark) 50x150 mm; mobile phase: A=0.1% TFA in H₂O, B=0.1% TFA in MeCN; temperature: 40°C; gradient (%B): 18-43% over 3 min, then 43-48% over 8 min, then 48-60% over 1 min; flow; 120 mL/min).

The purity of the target product was calculated from the area ratio of LC/MS (UV wavelength 225 nm) chromatogram under the following analytical conditions and was 96.03%.
Analysis conditions: retention time =6.15 min; column Kinetex EVO C18 2.6 um, 2.1x150 mm, 100Å; mobile phase A=0.025% TFA in H₂O, B=0.025% TFA in MeCN; temperature 60°C; gradient (%B conc): 20-60% over 7.15 min, then 60-95% over 0.3 min, then 95-95% over 1.55 min; flow: 0.5 mL/min
ESI-MS(+) observed value m/z=1142.36(M+2H)²⁺

### [Synthetic Example 4-7]

### Synthesis of 894 variant 61 G Azi (SEQ ID NO: 543)

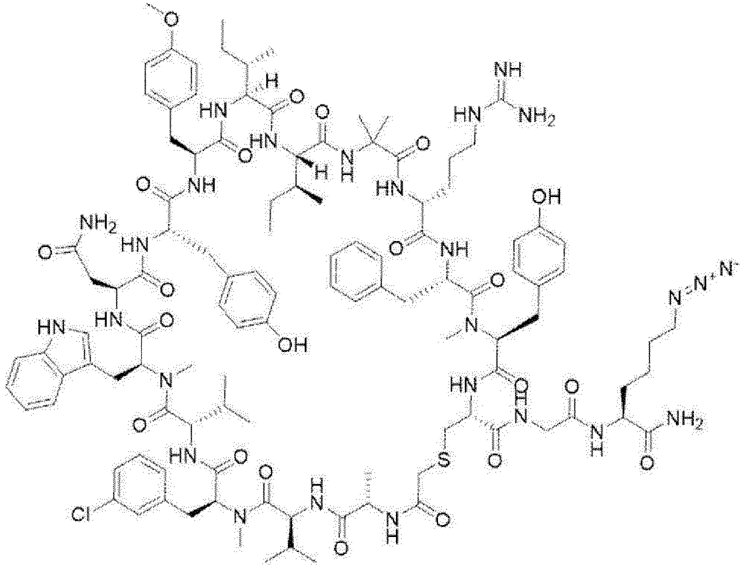

Using Sieber amide resin (Watanabe Chemical, 0.47 mmol/g, 0.21 g), the target peptide was synthesized by starting from the removal of the Fmoc group according to the general method described above. At that time, CEM's Liberty Blue HT was used as a solid phase synthesizer, and the synthesis was performed according to the manufacturer's manual. For the introduction of each residue, Fmoc-AA/HATU/DIEA (5.3 equivalents/5 equivalents/10 equivalents) was used for 1 equivalent of resin, and the reaction was performed once in DMF at 75°C for 10 min. For the 2nd residue and 4th residue, the reaction was performed twice at 75°C for 30 min. For the 10th residue, the 11th residue, and the 13th residue, the reaction was repeated twice at 75°C for 10 min. For the 12th residue, the reaction was performed twice at 25°C for 20 min. For the 15th residue, the reaction was performed once at 25°C for 20 min. In addition, the basic condition for Fmoc removal was to react with a 20% piperidine solution in DMF at 75°C for 3 min. For Fmoc removal at the 2nd residue, the 4th residue and 13th residue, the reaction was performed at 25°C for 5 min, and then at 25°C for 10 min. To introduce the chloroacetyl group, the Fmoc group of the α-amino group was removed by the aforementioned method from the solid phase resin carrying the Fmoc-protected peptide obtained in the previous step, 10 equivalents of chloroacetic acid, 10 equivalents of DIPCI, 10 equivalents of HOSu were stirred in DCM, and the same amount of DMF as DCM was added to prepare a DCM/DMF solution (0.2 M) of ClAcOSu, the solution was added to the solid phase resin, and the mixture was shaken at room temperature for 60 min. For deprotection of side chains and cutting out from the solid-phase resin, first, the resin obtained after the chloroacetyl group introduction step was washed with DMF five times and methylene chloride three times, and then dried under reduced pressure. Then, reactant cocktail-A (a mixture of TFA/H₂O/TIS/DODT in a volume ratio of 92.5:2.5:2.5:2.5) was added to the reaction vessel containing the solid-phase resin, and the mixture was shaken at room temperature for 90 min. The reaction solution was collected by filtration through a frit. The solid-phase resin remaining in the reaction vessel was shaken again with the cutting cocktail, and the solution components were collected from the frit and mixed with the above-mentioned filtrate. When this filtrate was added to an excess diethyl ether/hexane (1/1) mixed solvent cooled to 0°C, a cloudy white precipitate was formed. This mixture was centrifuged (9000 rpm, 2 min), and the solution was decanted and dried under reduced pressure. The obtained solid was used in the next cyclization reaction. For the cyclization reaction of the peptide, the peptide was dissolved in DMSO so that the final concentration was 5 mM based on the number of moles of the solid phase resin, 5 equivalents of triethylamine was added, and the mixture was shaken at room temperature for about 14 hr. The obtained reaction solution was concentrated under reduced pressure using Savant Explorer SpeedVaC.

The obtained crude product was purified using the following conditions (column; Waters Xbridge (registered trademark) C18 5 um (registered trademark) 50x150 mm: mobile phase; A=0.1% TFA in H₂O, B=0.1% TFA in MeCN: temperature 40°C: gradient (%B); 17-42% over 3 min, then 42-47% over 8 min, then 47-60% over 1 min: flow 120 mL/min).

The purity of the target product was calculated from the area ratio of the LC/MS (UV wavelength 225 nm) chromatogram under analysis condition B and was 92.8%.
analysis conditions B: retention time =6.22 min: column;
Kinetex EVO C18 2.6 um 2.1x150 mm, 100Å: mobile phase; A=0.025% TFA in H₂O, B=0.025% TFA in MeCN: temperature 60°C: gradient (%B conc); 20-60% over 7.2 min, then 60-95% over 0.3 min, then 95-95% over 1.6 min: flow 0.5 mL/min
ESI-MS(+) observed value m/z=1142.31(M+2H)²⁺

By a method similar to that in the above-mentioned Synthetic Examples, the peptides or linker-added peptides shown in the following Table 15-1 to Table 19 were synthesized.

**[Table 15-1-1]**

| SEQ ID NO: | name | Seq | | | | | | | | | | | | | | | | | |
|---|---|---|---|---|---|---|---|---|---|---|---|---|---|---|---|---|---|---|---|
| | | 1 | 2 | 3 | 4 | 5 | 8 | 7 | 8 | 9 | 10 | 11 | 12 | 13 | 14 | 15 | Linker | ESI (mlz) | M+XHIX+ |
| 21 | 894_V00_PEG12 | Ala | Val | Phe | Val | Trp | Asn | Tyr | Tyr | Ile | lie | Arg | Arg | Tyr | MeTyr | Cys | PEG12c | 1341.9 | 2 |
| 22 | 894_v01_PEG12 | Ala | Val | MePhe | Val | Trp | Asn | Tyr | Tyr | Ile | lie | Arg | Arg | Tyr | MeTyr | Cys | PEG12c | 899.6 | 3 |
| 23 | 894_v02_PEG12 | Ala | Val | MePhe | Val | Trp | Asn | Tyr | Tyr | Ile | lie | Lys | Arg | Tyr | MeTyr | Cys | PEG12c | 890.3 | 3 |
| 24 | 894_v03_PEG12 | Ala | Val | MePhe | Val | MeTrp | Asn | Tyr | Tyr | Ile | Ile | Arg | Arg | Tyr | MeTyr | Cys | PEG12c | 904.3 | 3 |
| 25 | 894_v04_PEG12 | Ala | Val | MePhe | Val | MeTrp | Asn | Tyr | Tyr | Ile | Ile | Lys | Arg | Tyr | MeTyr | Cys | PEG12c | 894.9 | 3 |
| 26 | 894_v05_PEG12 | Ala | Val | MePhe | Val | MeTrp | Asn | Tyr | Tyr | Ile | Ile | Lys | Lys | Tyr | MeTyr | Cys | PEG12c | 885.6 | 3 |
| 27 | 894_v00_PEG04 | Ala | Val | Phe | Val | Trp | Asn | Tyr | Tyr | Ile | Ile | Arg | Arg | Tyr | MeTyr | Cys | PEG4c | 1165.7 | 2 |
| 28 | 894_v01_PEG04 | Ala | Val | MePhe | Val | Trp | Asn | Tyr | Tyr | Ile | lie | Arg | Arg | Tyr | MeTyr | Cys | PEG4c | 1172.7 | 2 |
| 29 | 894_v02_PEG04 | Ala | Val | MePhe | Val | Trp | Asn | Tyr | Tyr | Ile | Ile | Lys | Arg | Tyr | MeTyr | Cys | PEG4c | 1158.8 | 2 |
| 30 | 894_A3_PEG04 | Ala | Val | MePhe | Val | MeTrp | Asn | Tyr | Tyr | Ile | Ile | Arg | Arg | Tyr | MeTyr | Cys | PEG4c | 1179.7 | 2 |
| 31 | 894_v04_PEG04 | Ala | Val | MePhe | Val | MeTrp | Asn | Tyr | Tyr | Ile | Ile | Lys | Arg | Tyr | MeTyr | Cys | PEG4c | 1165.7 | 2 |
| 32 | 894_v35_PEG04 | Ala | Val | MePhe | Val | MeTrp | Asn | Tyr | Tyr | Ile | Ile | Lys | Lys | Tyr | MeTyr | Cys | PEG4c | 1151.7 | 2 |
| 33 | 894_v04_PEG04 | Ala | Val | MePhe | Val | MeTrp | Asn | Tyr | Tyr | Ile | Ile | Lys | Arg | Tyr | MeTyr | Cys | PEG4c | 1165.7 | 2 |
| 34 | 894_v00_PEG04 | Ala | Val | Phe | Val | Trp | Asn | Tyr | Tyr | Ile | Ile | Arg | Arg | Tyr | MeTyr | Cys | PEG4c | 1165.7 | 2 |
| 35 | 894_v04_PEG04_10_V | Ala | Val | MePhe | Val | MeTrp | Asn | Tyr | Tyr | Ile | Val | Lys | Arg | Tyr | MeTyr | Cys | PEG4c | 1158.8 | 2 |
| 36 | 894_v04_PEG04_11Ame | Ala | Val | MePhe | Val | MeTrp | Asn | Tyr | Tyr | Ile | Ile | aMeK | Arg | Tyr | MeTyr | Cys | PEG4c | 1172.8 | 2 |
| 37 | 894_v04_PEG04_14_MeBph | Ala | Val | MePhe | Val | MeTrp | Asn | Tyr | Tyr | Ile | Ile | Lys | Arg | Tyr | MeBph | Cys | PEG4c | 1195,8 | 2 |
| 38 | 894_v1_PEG04_5_W1Et | Ala | Val | MePhe | Val | W1Et | Asn | Tyr | Tyr | Ile | Ile | Lys | Arg | Tyr | MeTyr | Cys | PEG4c | 1112.6 | 2 |
| 39 | 894_v1_PEG04_5_W1iP | Ala | Val | MePhe | Val | W1iPr | Asn | Tyr | Tyr | Ile | Ile | Lys | Arg | Tyr | MeTyr | Cys | PEG4c | 1179.6 | 2 |
| 40 | 894_v04_PEG04_11_Hly | Ala | Val | MePhe | Val | MeTrp | Asn | Tyr | Tyr | Ile | Ile | Hly | Arg | Tyr | MeTyr | Cys | PEG4c | 1172.7 | 2 |
| 41 | 894_v04_PEG04 | Ala | Val | MePhe | Val | MeTrp | Asn | Tyr | Tyr | Ile | Ile | Lys | Arg | Tyr | MeTyr | Cys | PEG4c | 1165.7 | 2 |

**[Table 15-1-2]**

| | | | | | | | | | | | | | | | | | | | |
|---|---|---|---|---|---|---|---|---|---|---|---|---|---|---|---|---|---|---|---|
| 42 | 894_v04_PEG04_14_MeNal2 | Ala | Val | MePhe | Val | MeTrp | Asn | Tyr | Tyr | Ile | Ile | Lys | Arg | Tyr | MeNal2 | Cys | PEG4c | 1182.8 | 2 |
| 43 | 894_v05_PEG8Me | Ala | Val | MePhe | Val | MeTrp | Asn | Tyr | Tyr | Ile | Ile | Lys | Lys | Tyr | MeTyr | Cys | PEGSMe | 1211.6 | 2 |
| 44 | 894_v04_PEG04_12_0rn | Ala | Val | MePhe | Val | MeTrp | Asn | Tyr | Tyr | Ile | Ile | Lys | Om | Tyr | MeTyr | Cys | PEG4c | 1144,8 | 2 |
| 45 | 894_v04_PEG04_10_L | Ala | Val | MePhe | Val | MeTrp | Asn | Tyr | Tyr | Ile | Leu | Lys | Arg | Tyr | MeTyr | Cys | PEG4c | 1165.8 | 2 |
| 46 | 894_v04_PEG04_14_MeF | Ala | Val | MePhe | Val | MeTrp | Asn | Tyr | Tyr | Ile | Ile | Lys | Arg | Tyr | MePhe | Cys | PEG4c | 1157.9 | 2 |
| 47 | 894_v04_PEG04_12_Hly | Ala | Val | MePhe | Val | MeTrp | Asn | Tyr | Tyr | lie | Ile | Lys | Hly | Tyr | MeTyr | Cys | PEG4c | 1158.6 | 2 |
| 48 | 894_v04_PEG04_14_MeW | Ala | Val | MePhe | Val | MeTrp | Asn | Tyr | Tyr | Ile | Ile | Lys | Arg | Tyr | MeTrp | Cys | PEG4c | 1177.4 | 2 |
| 49 | 894_v04_PEG04_14_MeoBph | Ala | Val | MePhe | Val | MeTrp | Asn | Tyr | Tyr | Ile | Ile | Lys | Arg | Tyr | MeoBph | Cys | PEG4c | 1195.9 | 2 |
| 50 | 894_v4_PEG04c_3_F2OMe | Ala | Val | F2OMe | Val | MeTrp | Asn | Tyr | Tyr | Ile | Ile | Lys | Arg | Tyr | MeTyr | Cys | PEG4c | 1173.6 | 2 |
| 51 | 894_v4_PEG04c_3_Cha | Ala | Val | Cha | Val | MeTrp | Asn | Tyr | Tyr | Ile | Ile | Lys | Arg | Tyr | MeTyr | Cys | PEG4c | 1161.7 | 2 |
| 52 | 894_v04_PEG04_14_MMeNal1 | Ala | Val | MePhe | Val | MeTrp | Asn | Tyr | Tyr | Ile | Ile | Lys | Arg | Tyr | MeNal1 | Cys | PEG4c | 1182.8 | 2 |
| 53 | 894_v04_PEG04_14_MemBph | Ala | Val | MePhe | Val | MeTrp | Asn | Tyr | Tyr | Ile | Ile | Lys | Arg | Tyr | MemBph | Cys | PEG4c | 1195.9 | 2 |
| 54 | 894_Nal25_PEG8Me | Ala | Val | MePhe | Val | Nal2 | Asn | Tyr | Tyr | lie | Ile | Lys | Lys | Tyr | MeTyr | Cys | PEG8Me | 1210.1 | 2 |
| 55 | 894_v4_PEG04c_14_pHPeG | Ala | Val | MePhe | Val | MeTrp | Asn | Tyr | Tyr | Ile | Ile | Lys | Arg | Tyr | pHPeG | Cys | PEG4c | 1165.7 | 2 |
| 56 | 894v1_PEG04_5_W1Et7Cl | Ala | Val | MePhe | Val | W1Et7CI | Asn | Tyr | Tyr | Ile | Ile | Lys | Arg | Tyr | MeTyr | Cys | PEG4c | 1189.9 | 2 |
| 57 | 894_MeNal13_PEG8Me | Ala | Val | MeNal1 | Ual | MeTrp | Asn | Tyr | Tyr | Ile | Ile | Lys | Lys | Tyr | MeTyr | Cys | PEG8Me | 1236.2 | 2 |
| 58 | 894_v04_PEG04_12_Cit | Ala | Val | MePhe | Val | MeTrp | Asn | Tyr | Tyr | Ile | Ile | Lys | Cit | Tyr | MeTyr | Cys | PEG4c | 1166.1 | 2 |
| 59 | 894_v04_PEG04_11_Dap | Ala | Val | MePhe | Val | MeTrp | Asn | Tyr | Tyr | Ile | Ile | Dap | Arg | Tyr | MeTyr | Cys | PEG4c | 1144.7 | 2 |
| 60 | 894_Nal25_F4COO114_PEG8Me | Ala | Val | MePhe | Val | Nal2 | Asn | Tyr | Tyr | Ile | Ile | Lys | F4G | Tyr | MeTyr | Cys | PEG8Me | 1247.7 | 2 |
| 61 | 894_v1_PEG04_5_Yph | Ala | Val | MePhe | Val | Yph | Asn | Tyr | Tyr | Ile | Ile | Lys | Arg | Tyr | MeTyr | Cys | PEG4c | 1185.2 | 2 |

**[Table 15-2-1]**

| | | | | | | | | | | | | | | | | | | | |
|---|---|---|---|---|---|---|---|---|---|---|---|---|---|---|---|---|---|---|---|
| 62 | 894_v04_PEG04_12_Dab | Ala | Val | MePhe | Val | MeTrp | Asn | Tyr | Tyr | Ile | Ile | Lys | Dab | Tyr | MeTyr | Cys | PEG4c | 1137.7 | 2 |
| 63 | 894_v1_PEG04_5_MeNal2 | Ala | Val | MePhe | Val | MeNal2 | Asn | Tyr | Tyr | Ile | Ile | Lys | Arg | Tyr | MeTyr | Cys | PEG4c | 1171.2 | 2 |
| 64 | 894_v1_PEG04_5_MemBph | Ala | Val | MePhe | Val | MemBph | Asn | Tyr | Tyr | Ile | Ile | Lys | Arg | Tyr | MeTyr | Cys | PEG4c | 1184.2 | 2 |
| 65 | 894_v04_PEG04_11_F4G | Ala | Val | MePhe | Val | MeTrp | Asn | Tyr | Tyr | Ile | Ile | F4G | Arg | Tyr | MeTyr | Cys | PEG4c | 803.0 | 3 |
| 66 | 894_v04_PEG04_12_F4G | Ala | Val | MePhe | Val | MeTrp | Asn | Tyr | Tyr | Ile | Ile | Lys | F4G | Tyr | MeTyr | Cys | PEG4c | 1189.8 | 2 |
| 67 | 894_v1_PEG04_5_MeNal1 | Ala | Val | MePhe | Val | MeNal1 | Asn | Tyr | Tyr | Ile | Ile | Lys | Arg | Tyr | MeTyr | Cys | PEG4c | 1171.2 | 2 |
| 68 | 894_MoNal13_F4G12_PEG8Me | Ala | Val | MeNal1 | Val | MeTrp | Asn | Tyr | Tyr | lie | Ile | Lys | F4G | Tyr | MeTyr | Cys | PEG8Me | 1274.2 | 2 |
| 69 | 894_v04_PEG04_10_S | Ala | Val | MePhe | Val | MeTrp | Asn | Tyr | Tyr | Ile | Ser | Lys | Arg | Tyr | MeTyr | Cys | PEG4c | 1152.7 | 2 |
| 70 | 894_v04_PEG04_15Ame | Ala | Va! | MePhe | Val | MeTrp | Asn | Tyr | Tyr | Ile | Ile | Lys | Arg | Tyr | MeTyr | aMeC | PEG4c | 1172.7 | 2 |
| 71 | B94_v4_PEG04c_3_F2C | Ala | Val | F2C | Val | MeTrp | Asn | Tyr | Tyr | Ile | Ile | Lys | Arg | Tyr | MeTyr | Cys | PEG4c | 1175.8 | 2 |
| 72 | 894_v04_PEG04_11_Orn | Ala | Val | MePhe | Val | MeTrp | Asn | Tyr | Tyr | Ile | Ile | Om | Arg | Tyr | MeTyr | Cys | PEG4c | 1158.7 | 2 |
| 73 | 894_vD4_PEG04_14Ame | Ala | Val | MePhe | Val | MeTrp | Asn | Tyr | Tyr | Ile | Ile | Lys | Arg | Tyr | aMeTyr | Cys | PEG4c | 1165.8 | 2 |
| 74 | 894_v04_PEG04_12_Dap | Ala | Val | MePhe | Val | MeTrp | Asn | Tyr | Tyr | Ile | Ile | Lys | Dap | Tyr | MeTyr | Cys | PEG4c | 1130.7 | 2 |
| 75 | 894 v4 PEG04c 13_pHPeG | Ala | Va! | MePhe | Val | MeTrp | Asn | Tyr | Tyr | lie | Ile | Lys | Arg | pHPeG | MeTyr | Cys | PEG4c | 1172.7 | 2 |
| 76 | 894_v04_PEG04_11_Dab | Ala | Val | MePhe | Val | MeTrp | Asn | Tyr | Tyr | Ile | Ile | Dab | Arg | Tyr | MeTyr | Cys | PEG4c | 1151.7 | 2 |
| 77 | 894_v04_PEG04_1Ame | Alaib | Val | MePhe | Val | MeTrp | Asn | Tyr | Tyr | Ile | Ile | Lys | Arg | Tyr | MeTyr | Cys | PEG4c | 1172.7 | 2 |
| 78 | 894_v94_PEG04_9_L | Ala | Val | MePhe | Val | MeTrp | Asn | Tyr | Tyr | Leu | Ile | Lys | Arg | Tyr | MeTyr | Cys | PEG4c | 1165.6 | 2 |
| 79 | 894_MeNal13_F4G11_PEG8Me | Ala | Val | MeNal1 | Val | MeTrp | Asn | Tyr | Tyr | lie | Ile | F4G | Lys | Tyr | MeTyr | Cys | PEG8Me | 1274.2 | 2 |
| 80 | 894_V04_PEG04_6_Q | Ala | Val | MePhe | Val | MeTrp | Gln | Tyr | Tyr | Ile | Ile | Lys | Arg | Tyr | MeTyr | Cys | PEG4c | 1172.7 | 2 |
| 81 | B94_v04_PEG04_7_Nel1 | Ala | Val | MePhe | Val | MeTrp | Asn | Nal1 | Tyr | lie | Ile | Lys | Arg | Tyr | MeTyr | Cys | PEG4c | 1182.7 | 2 |

**[Table 15-2-2]**

| | | | | | | | | | | | | | | | | | | | |
|---|---|---|---|---|---|---|---|---|---|---|---|---|---|---|---|---|---|---|---|
| 82 | 894_4xNa1_PEG8Me | Ala | Val | MeNal1 | Val | MeNal2 | Asn | Nall | Nal1 | Ile | Ile | Lys | Lys | Tyr | MeTyr | Cys | PEG8Me | 1275.8 | 2 |
| 83 | 894_v4_PEG04c_1_P | Pro | Val | MePhe | Val | MeTrp | Asn | Tyr | Tyr | Ile | Ile | Lys | Arg | Tyr | MeTyr | Cys | PEG4c | 1178.8 | 2 |
| 84 | 894_v4_PEG04c_3_F4C | Ala | Val | F4C | Val | MeTrp | Asn | Tyr | Tyr | Ile | Ile | Lys | Arg | Tyr | MeTyr | Cys | PEG4c | 1175.8 | 2 |
| 85 | 894_v40_PEG04_10_P | Ala | Val | MePhe | Val | MeTrp | Asn | Tyr | Tyr | Ile | Pro | Lys | Arg | Tyr | MeTyr | Cys | PEG4c | 1157.7 | 2 |
| 86 | 894_v04_PEG04_5Ame | Ala | Val | MePhe | Val | aMeTrp | Asn | Tyr | Tyr | lie | Ile | Lys | Arg | Tyr | MeTyr | Cys | PEG4c | 1165.7 | 2 |
| 87 | 894_v1_PEG04_5_MeoBph | Ala | Val | MePhe | Val | MeoBph | Asn | Tyr | Tyr | Ile | Ile | Lys | Arg | Tyr | MeTyr | Cys | PEG4c | 1184.3 | 2 |
| 88 | 894v04_120m_PEG12_Azi | Ala | Va! | MePhe | Val | MeTrp | Asn | Tyr | Tyr | Ile | Ile | Lys | Orn | Tyr | MeTyr | Cys | PEG12c | 932.3 | 3 |
| 89 | 894v04_11Hly_PEG12_Azi | Ala | Val | MePhe | Val | MeTrp | Asn | Tyr | Tyr | Ile | Ile | Hly | Arg | Tyr | MeTyr | Cys | PEG12c | 951.0 | 3 |
| 90 | 894v04_11Om_PEG12_Azi | Ala | Va! | MePhe | Val | MeTrp | Asn | Tyr | Tyr | Ile | Ile | Cm | Arg | Tyr | MeTyr | Cys | PEG12c | 941.7 | 3 |
| 91 | 894v04_11Dap_PEG12_Azi | Ala | Val | MePhe | Val | MeTrp | Asn | Tyr | Tyr | Ile | Ile | Dap | Arg | Tyr | MeTyr | Cys | PEG12C | 932.3 | 3 |
| 92 | 894v04_14MemBph_PEG12_Azi | Ala | Val | MePhe | Val | MeTrp | Asn | Tyr | Tyr | Ile | Ile | Lys | Arg | Tyr | MemBph | Cys | PEG12c | 966,4 | 3 |
| 93 | 894v04_7Nal1_PEG12_Azi | Ala | Val | MePhe | Val | MeTrp | Asn | Nall | Tyr | Ile | Ile | Lys | Arg | Tyr | MeTyr | Cys | PEG12c | 957.7 | 3 |
| 94 | 894v04_10P_PEG12_Azi | Ala | Val | McPhe | Val | MeTrp | Asn | Tyr | Tyr | Ile | Pro | Lys | Arg | Tyr | MeTyr | Cys | PEG12c | 941.0 | 3 |
| 95 | 894_v4_PEG08Me_5_dp | Ala | Va! | MePhe | Val | dp | Asn | Tyr | Tyr | Ile | Ile | Lys | Arg | Tyr | MeTyr | Cys | PEG8Me | 1173.7 | 2 |
| 96 | 894_v4_PEG08Me_9_Tbg | Ala | Val | MePhe | Val | MeTrp | Asn | Tyr | Tyr | Tbg | Ile | Lys | Arg | Tyr | MeTyr | Cys | PEG8Me | 1225.3 | 2 |
| 97 | 894_v4_PEG08Me_10_Tbg | Ala | Val | MePhe | Val | MeTrp | Asn | Tyr | Tyr | Ile | Tbg | Lys | Arg | Tyr | MeTyr | Cys | PEG8Me | 1225.3 | 2 |
| 98 | 894_3MeF4F | Ala | Val | MePhe4F | Val | Trp | Asn | Tyr | Tyr | lie | Ile | Arg | Arg | Tyr | MeTyr | Cys | PEG12c | 905.6 | 3 |
| 99 | 894_3MeF3C | Ala | Val | MePhe3C | Val | Trp | Asn | Tyr | Tyr | Ile | Ile | Arg | Arg | Tyr | MeTyr | Cys | PEG12c | 911.1 | 3 |
| 100 | 894_3m_8F4OMe | Ala | Val | MePhe | Val | Trp | Asn | Tyr | F4OMe | Ile | Ile | Arg | Arg | Tyr | MeTyr | Cys | PEG12C | 904.3 | 3 |
| 101 | 894_3MeF3F | Ala | Val | MePhe3F | Val | Trp | Asn | Tyr | Tyr | Ile | lie | Arg | Arg | Tyr | MeTyr | Cys | PEG12C | 905.6 | 3 |
| 102 | 894_3m_13F | Ala | Val | MePhe | Val | Trp | Asn | Tyr | Tyr | Ile | Ile | Arg | Arg | Phe | MeTyr | Cys | PEG12c | 1340.7 | 2 |
| 103 | 894_v01_PEG12 | Ala | Val | MePhe | Val | Trp | Asn | Tyr | Tyr | Ile | Ile | Arg | Arg | Tyr | MeTyr | Cys | PEG12c | 899.6 | 3 |

**[Table 15-3-1]**

| | | | | | | | | | | | | | | | | | | | |
|---|---|---|---|---|---|---|---|---|---|---|---|---|---|---|---|---|---|---|---|
| 104 | 894_3m_8W | Ala | Val | MePhe | Val | Trp | Asn | Tyr | Trp | Ile | lie | Arg | Arg | Tyr | MeTyr | Cys | PEG12c | 1360.3 | 2 |
| 105 | 894_3MeF3OMe | Ala | Val | MePhe3OMe | Val | Trp | Asn | Tyr | Tyr | Ile | Ile | Arg | Arg | Tyr | MeTyr | Cys | PEG12c | 909.6 | 3 |
| 106 | 894_3m_12Y | Ala | Val | MePhe | Val | Trp | Asn | Tyr | Tyr | Ile | Ile | Arg | Tyr | Tyr | MeTyr | Cys | PEG12c | 1352.2 | 2 |
| 107 | 894_3m_8F | Ala | Val | MePhe | Val | Trp | Asn | Tyr | Phe | Ile | Ile | Arg | Arg | Tyr | MeTyr | Cys | PEG12c | 894.5 | 3 |
| 108 | 894_1m_3m | MeAla | Val | MePhe | Val | Trp | Asn | Tyr | Tyr | Ile | Ile | Arg | Arg | Tyr | MeTyr | Cys | PEG12c | 904.3 | 3 |
| 109 | 894_3m_5F3C | Ala | Val | MePhe | Val | F3C | Asn | Tyr | Tyr | Ile | Ile | Arg | Arg | Tyr | MeTyr | Cys | PEG12c | 898.3 | 3 |
| 110 | 894_3m_2A | Ala | Ala | MePhe | Val | Trp | Asn | Tyr | Tyr | Ile | lie | Arg | Arg | Tyr | MeTyr | Cys | PEG12c | 890.3 | 3 |
| 111 | 894_3m_{_}1Abu | Alabu | Val | MePhe | Val | Trp | Asn | Tyr | Tyr | lie | Ile | Arg | Arg | Tyr | MeTyr | Cys | PEG12c | 904.3 | 3 |
| 112 | 894_3m_11m | Ala | Val | MePhe | Val | Trp | Asn | Tyr | Tyr | Ile | Ile | MeAlarg | Arg | Tyr | MeTyr | Cys | PEG12c | 1355.7 | 2 |
| 113 | 894_3m_5F3OMe | Ala | Val | MePhe | Val | F3OMe | Asn | Tyr | Tyr | Ile | lie | Arg | Arg | Tyr | MeTyr | Cys | PEG12C | 896.6 | 3 |
| 114 | 894_3m_5F4C | Ala | Val | MePhe | Val | F4C | Asn | Tyr | Tyr | lie | lie | Arg | Arg | Tyr | MeTyr | Cys | PEG12c | 898.1 | 3 |
| 115 | 894_3m_10Y | Ala | Val | MePhe | Val | Trp | Asn | Tyr | Tyr | Ile | Tyr | Arg | Arg | Tyr | MeTyr | Cys | PEG12c | 916.3 | 3 |
| 116 | 894_3m_12dr | Ala | Val | MePhe | Val | Trp | Asn | Tyr | Tyr | lie | lie | Arg | dr | Tyr | MeTyr | Cys | PEG12c | 899.8 | 3 |
| 117 | 894_3m_14A | Ala | Val | MePhe | Val | Trp | Asn | Tyr | Tyr | Ile | Ile | Arg | Arg | Tyr | Ala | Cys | PEG12c | 864.3 | 3 |
| 118 | 894_3m_5F4aMe | Ala | Val | MePhe | Val | F4OMe | Asn | Tyr | Tyr | Ile | Ile | Arg | Arg | Tyr | MeTyr | Cys | PEG12c | 896,9 | 3 |
| 119 | 894_3m_5F4F | Ala | Val | MePhe | Val | F4F | Asn | Tyr | Tyr | lie | Ile | Arg | Arg | Tyr | MeTyr | Cys | PEG12C | 892.7 | 3 |
| 120 | 894_3m_8m | Ala | Val | MePhe | Val | Trp | Asn | Tyr | MeTyr | Ile | Ile | Arg | Arg | Tyr | MeTyr | Cys | PEG12C | 1355.8 | 2 |
| 121 | 894_3m_11Y | Ala | Val | MePhe | Val | Trp | Asn | Tyr | Tyr | lie | Ile | Tyr | Arg | Tyr | MeTyr | Cys | PEG12c | 902.2 | 3 |
| 122 | 894_3m_5F3F | Ala | Vai | MePhe | Val | F3F | Asn | Tyr | Tyr | lie | Ile | Arg | Arg | Tyr | MeTyr | Cys | PEG12c | 1338.3 | 2 |
| 123 | 894_3m_13A | Ala | Val | MePhe | Val | Trp | Asn | Tyr | Tyr | Ile | lie | Arg | Arg | Ala | MeTyr | Cys | PEG12c | 1302.7 | 2 |
| 124 | 894_3m_14E | Ala | Val | MePhe | Val | Trp | Asn | Tyr | Tyr | Ile | lie | Arg | Arg | Tyr | Glu | Cys | PEG12c | 1324.7 | 2 |

**[Table 15-3-2]**

| | | | | | | | | | | | | | | | | | | | |
|---|---|---|---|---|---|---|---|---|---|---|---|---|---|---|---|---|---|---|---|
| 125 | 894_3m_7W | Ala | Val | MePhe | Val | Trp | Asn | Trp | Tyr | lie | Ile | Arg | Arg | Tyr | MeTyr | Cys | PEG12c | 907.6 | 3 |
| 126 | 894_3m_4G | Ala | Val | MePhe | Gly | Trp | Asn | Tyr | Tyr | Ile | Ile | Arg | Arg | Tyr | MeTyr | Cys | PEG12c | 885.8 | 3 |
| 127 | 894_3m_14R | Ala | Val | MePhe | Val | Trp | Asn | Tyr | Tyr | lie | Ile | Arg | Arg | Tyr | Arg | Cys | PEG12c | 1338.3 | 2 |
| 128 | 894_3m_5Hph | Ala | Val | MePhe | Val | Hph | Asn | Tyr | Tyr | lie | Ile | Arg | Arg | Tyr | MeTyr | Cys | PEG12c | 1336.2 | 2 |
| 129 | 894_3m_10A | Ala | Val | MePhe | Val | Trp | Asn | Tyr | Tyr | Ile | Ala | Arg | Arg | Tyr | MeTyr | Cys | PEG12c | 885.6 | 3 |
| 130 | 894_3m_2Ahp | Ala | Alahp | MePhe | Val | Trp | Asn | Tyr | Tyr | lie | Ile | Arg | Arg | Tyr | MeTyr | Cys | PEG12c | 909.0 | 3 |
| 131 | 894_3m_1G | Gly | Val | MePhe | Val | Trp | Asn | Tyr | Tyr | Ile | lie | Arg | Arg | Tyr | MeTyr | Cys | PEG12c | 894.9 | 3 |
| 132 | 894_3m_1S | Ser | Val | MePhe | Val | Trp | Asn | Tyr | Tyr | Ile | Ile | Arg | Arg | Tyr | MeTyr | Cys | PEG12c | 904.9 | 3 |
| 133 | 894_3m_2F | Ala | Phe | MePhe | Val | Trp | Asn | Tyr | Tyr | Ile | Ile | Arg | Arg | Tyr | MeTyr | Cys | PEG12c | 915.6 | 3 |
| 134 | 894_3m_6D | Ala | Val | MePhe | Val | Trp | Asp | Tyr | Tyr | Ile | Ile | Arg | Arg | Tyr | MeTyr | Cys | PEG12c | 1349.2 | 2 |
| 136 | 894_3m_7F | Ala | Val | MePhe | Val | Trp | Asn | Phe | Tyr | Ile | Ile | Arg | Arg | Tyr | MeTyr | Cys | PEG12C | 894.5 | 3 |
| 136 | 894_3MeF4OMe | Ala | Val | MePhe4OMe | Val | Trp | Asn | Tyr | Tyr | Ile | Ile | Arg | Arg | Tyr | MeTyr | Cys | PEG12c | 909.7 | 3 |
| 137 | 894_3m_6A | Ala | Val | MePhe | Val | Trp | Ala | Tyr | Tyr | Ile | lie | Arg | Arg | Tyr | MeTyr | Cys | PEG12c | 1327.2 | 2 |
| 138 | 894_3m_1F | Phe | Val | MePhe | Val | Trp | Asn | Tyr | Tyr | lie | Ile | Arg | Arg | Tyr | MeTyr | Cys | PEG12c | 925.0 | 3 |
| 139 | 894_3m_8Ahp | Ala | Val | MePhe | Val | Trp | Asn | Tyr | Alahp | Ile | Ile | Arg | Arg | Tyr | MeTyr | Cys | PEG12c | 887.6 | 3 |
| 140 | 894_3m_7A | Ala | Val | MePhe | Val | Trp | Asn | Ala | Tyr | lie | Ile | Arg | Arg | Tyr | MeTyr | Cys | PEG12c | 868.9 | 3 |
| 141 | 894_3m_4S | Ala | Val | MePhe | Ser | Trp | Asn | Tyr | Tyr | lie | Ile | Arg | Arg | Tyr | MeTyr | Cys | PEG12c | 895.6 | 3 |
| 142 | 894_3m_7F4C | Ala | Val | MePhe | Val | Trp | Asn | F4C | Tyr | lie | Ile | Arg | Arg | Tyr | MeTyr | Cys | PEG12c | 1358.0 | 2 |
| 143 | 894_3m_7Ahp | Ala | Val | MePhe | Val | Trp | Asn | Alahp | Tyr | Ile | Ile | Arg | Arg | Tyr | MeTyr | Cys | PEG12c | 1330.7 | 2 |
| 144 | 894_3m_4A | Ala | Val | MePhe | Ala | Trp | Asn | Tyr | Tyr | Ile | Ile | Arg | Arg | Tyr | MeTyr | Cys | PEG12c | 890.2 | 3 |
| 146 | 894_3m_7R | Ala | Val | MePhe | Val | Trp | Asn | Arg | Tyr | lie | Ile | Arg | Arg | Tyr | MeTyr | Cys | PEG12c | 1345.2 | 2 |

**[Table 16-1-1]**

| SEQ ID NO: | name | Seq | | | | | | | | | | | | | | | | |
|---|---|---|---|---|---|---|---|---|---|---|---|---|---|---|---|---|---|---|
| | | 1 | 2 | 3 | 4 | 5 | 6 | 7 | 8 | 9 | 10 | 11 | 12 | 13 | 14 | 15 | 16 | 17 |
| 146 | hTfR_000894_PEG11 | Ala | Val | Phe | Val | Trp | Asn | Tyr | Tyr | Ile | Ile | Arg | Arg | Tyr | MeTyr | Cys | | |
| 147 | JCR_hTfR_000894_PEG11 | Ala | Val | Phe | Val | Trp | Asn | Tyr | Tyr | Ile | Ile | Arg | Arg | Tyr | MeTyr | Cys | | |
| 148 | JCR_hTfR_000690_PEG11 | Ala | Val | Phe | Val | Trp | Asn | Tyr | Tyr | Ile | Ile | Asp | Arg | Phe | Arg | Cys | | |
| 149 | JCR_hTfR_000812_PEG11 | Ala | Val | Phe | Val | Trp | Asn | Tyr | Tyr | Ile | He | Asp | Arg | Phe | Arg | Ser | MeHis | Cys |
| 150 | JCR_hTfR_000895_PEG11 | Ala | Val | Phe | Val | Trp | Asn | Tyr | Tyr | Ile | Ile | Asp | Arg | Phe | Arg | Ser | MeTyr | Cys |
| 151 | JCR_hTfR_100022_PEG11 | Ala | Val | Phe | Val | Trp | Asn | Tyr | Tyr | Ile | Val | Asp | Arg | Phe | Gly | Thr | Gly | Cys |
| 152 | JCR_hTfR_100025_PEG11 | Ala | Val | Phe | Val | Trp | Asn | Tyr | Tyr | Ile | Val | Gly | Arg | Asn | Val | pro | Asn | Cys |
| 153 | JCR_hTfR_000696_PEG11 | Ala | Asn | Arg | Ile | Val | Phe | Val | Trp | Asn | Tyr | Tyr | Ile | Ile | pro | Arg | MeTyr | Cys |
| 154 | JCR_hTfR_000809_PEG11 | Ala | Asn | Arg | Ile | Val | Phe | Val | Trp | Asn | Tyr | Tyr | Ile | Ile | pro | Arg | MeHis | Cys |
| 155 | JCR_hTfR_100014_PEG11 | Ala | Ser | Ala | Val | Phe | Val | Trp | Asn | Tyr | Tyr | Ile | Ile | Asn | Arg | Trp | Ala | Cys |
| 156 | JCR_hTfR_100018_PEG11 | Ala | Val | Phe | Val | Trp | Asn | Tyr | Tyr | Ile | Val | PeG | Asn | Gly | Ser | Asn | Ser | Cys |
| 157 | JCR_hTfR_100017_PEG11 | Ala | Asn | Ser | Arg | Thr | Val | Trp | Val | Trp | Asn | Tyr | Tyr | Ile | Val | Thr | Arg | Cys |
| 158 | FLIP_000894_PEG11 | Ala | MeTyr | Tyr | Arg | Arg | Ile | Ile | Tyr | Tyr | Asn | Trp | Val | Phe | Val | Cys | | |
| 159 | SP01_hTfR_894 | Ala | Val | Phe | Val | Trp | Asn | Tyr | Tyr | Ile | Ile | Arg | Arg | Tyr | MeTyr | Cys | | |
| 160 | SP15_hTfR_894_15(d) | Ala | Val | Phe | Val | Trp | Asn | Tyr | Tyr | Ile | Ile | Arg | Arg | Tyr | MeTyr | dc | | |
| 161 | SP16_hTfR_894_01(me) | MeAla | Val | Phe | Val | Trp | Asn | Tyr | Tyr | Ile | Ile | Arg | Arg | Tyr | MeTyr | Cys | | |
| 162 | SP18_hTfR_894_03(me) | Ala | Val | MePhe | Val | Trp | Asn | Tyr | Tyr | Ile | Ile | Arg | Arg | Tyr | MeTyr | Cys | | |
| 163 | SP20_hTfR_894_05(me) | Ala | Val | Phe | Val | MeTrp | Asn | Tyr | Tyr | Ile | Ile | Arg | Arg | Tyr | MeTyr | Cys | | |
| 164 | SP26_hTfR_894_11(me) | Ala | Val | Phe | Val | Trp | Asn | Tyr | Tyr | Ile | Ile | MeArg | Arg | Tyr | MeTyr | Cys | | |
| 165 | 894_v00_PEG12_C | Ala | Val | Phe | Val | Trp | Asn | Tyr | Tyr | Ile | Ile | Arg | Arg | Tyr | MeTyr | Cys | | |
| 166 | 894_v01_PEG12_C | Ala | Val | MePhe | Val | Trp | Asn | Tyr | Tyr | Ile | Ile | Arg | Arg | Tyr | MeTyr | Cys | | |
| 167 | 894_v02_PEG12_C | Ala | Val | MePhe | Val | Trp | Asn | Tyr | Tyr | Ile | Ile | Lys | Arg | Tyr | MeTyr | Cys | | |
| 168 | 894_v03_PEG12_C | Ala | Val | MePhe | Val | MeTrp | Asn | Tyr | Tyr | Ile | Ile | Arg | Arg | Tyr | MeTyr | Cys | | |
| 169 | 894_v04_PEG12_C | Ala | Val | MePhe | Val | MeTrp | Asn | Tyr | Tyr | Ile | Ile | Lys | Arg | Tyr | MeTyr | Cys | | |
| 170 | 894_v04_PEG4_C | Ala | Val | MePhe | Val | MeTrp | Asn | Tyr | Tyr | Ile | Ile | Lys | Arg | Tyr | MeTyr | Cys | | |

**[Table 16-1-2]**

| | | | | | | | | | | | | | | | | |
|---|---|---|---|---|---|---|---|---|---|---|---|---|---|---|---|---|
| 171 | 894_vC6_PEG12_C | Ala | Val | Phe | Val | MeTrp | Asn | Tyr | Tyr | Ile | Ile | Arg | Arg | Tyr | MeTyr | Cys |
| 172 | 894_v07_PEG12_C | Ala | Val | Phe | Val | MeTrp | Asn | Tyr | Tyr | Ile | Ile | Lys | Arg | Tyr | MeTyr | Cys |
| 173 | 894_v07_PEG4_C | Ala | Val | Phe | Val | MeTrp | Asn | Tyr | Tyr | Ile | Ile | Lys | Arg | Tyr | MeTyr | Cys |
| 174 | 894v04_12Orn_PEG12 | Ala | Val | MePhe | Val | MeTrp | Asn | Tyr | Tyr | Ile | Ile | Lys | Orn | Tyr | MeTyr | Cys |
| 175 | 894v04_11Hly_PEG12 | Ala | Val | MePhe | Val | MeTrp | Asn | Tyr | Tyr | Ile | Ile | Hly | Arg | Tyr | MeTyr | Cys |
| 176 | 894v04_11Orn_PEG12 | Ala | Val | MePhe | Val | MeTrp | Asn | Tyr | Tyr | Ile | Ile | Orn | Arg | Tyr | MeTyr | Cys |
| 177 | 894v04_11Dap_PEG12 | Ala | Val | MePhe | Val | MeTrp | Asn | Tyr | Tyr | Ile | Ile | Dap | Arg | Tyr | MeTyr | Cys |
| 178 | 894v04_14MemBph_PEG12 | Ala | Val | MePhe | Val | MeTrp | Asn | Tyr | Tyr | Ile | Ile | Lys | Arg | Tyr | MemBph | Cys |
| 179 | 894v04_7Nal1_PEG12 | Ala | Val | MePhe | Val | MeTrp | Asn | Nel1 | Tyr | Ile | Ile | Lys | Arg | Tyr | MeTyr | Cys |
| 180 | 894v04_10P_PEG12 | Ala | Val | MePhe | Val | MeTrp | Asn | Tyr | Tyr | Ile | pro | Lys | Arg | Tyr | MeTyr | Cys |
| 181 | 894_3m_PEG4_PEG4_K(Mal) | Ala | Val | MePhe | Val | Trp | Asn | Tyr | Tyr | Ile | Ile | Arg | Arg | Tyr | MeTyr | Cys |
| 182 | 894_3m_PEG4_PEG4_PEG4_K(Mal) | Ala | Val | MePhe | Val | Trp | Asn | Tyr | Tyr | Ile | Ile | Arg | Arg | Tyr | MeTyr | Cys |
| 183 | 894_3_5m_PEG12_K(Mal) | Ala | Val | MePhe | Val | MeTrp | Asn | Tyr | Tyr | Ile | Ile | Arg | Arg | Tyr | MeTyr | Cys |
| 184 | 894_3_5m_PEG04_K(Mal) | Ala | Val | MePhe | Val | MeTrp | Asn | Tyr | Tyr | Ile | Ile | Arg | Arg | Tyr | MeTyr | Cys |
| 185 | 894_3_5m_PEG08_K(Mal) | Ala | Val | MePhe | Val | MeTrp | Asn | Tyr | Tyr | Ile | Ile | Arg | Arg | Tyr | MeTyr | Cys |
| 186 | 894_3m_K(Mal) | Ala | Val | MePhe | Val | Trp | Asn | Tyr | Tyr | Ile | Ile | Arg | Arg | Tyr | MeTyr | Cys |
| 187 | 894_3m_GGGSS_K(Mal) | Ala | Val | MePhe | Val | Trp | Asn | Tyr | Tyr | Ile | Ile | Arg | Arg | Tyr | MeTyr | Cys |
| 188 | 894_3m_G_K(Mal) | Ala | Val | MePhe | Val | Trp | Asn | Tyr | Tyr | Ile | Ile | Arg | Arg | Tyr | MeTyr | Cys |
| 189 | 894_3m_G2SGSGSS_K(Mal) | Ala | Val | MePhe | Val | Trp | Asn | Tyr | Tyr | Ile | Ile | Arg | Arg | Tyr | MeTyr | Cys |
| 190 | 894_3m_GGSGSS_K(Mal) | Ala | Val | MePhe | Val | Trp | Asn | Tyr | Tyr | Ile | lie | Arg | Arg | Tyr | MeTyr | Cys |
| 191 | 894_3m_GGSGES_K(Mal) | Ala | Val | MePhe | Val | Trp | Asn | Tyr | Tyr | Ile | Ile | Arg | Arg | Tyr | MeTyr | Cys |
| 192 | 894_3m_GGEGES_K(Mal) | Ala | Val | MePhe | Val | Trp | Asn | Tyr | Tyr | Ile | Ile | Arg | Arg | Tyr | MeTyr | Cys |
| 193 | 894_3m_GGSS_K(Mal) | Ala | Val | MePhe | Val | Trp | Asn | Tyr | Tyr | Ile | Ile | Arg | Arg | Tyr | MeTyr | Cys |
| 194 | 894_3m_GGS_K(Mal) | Ala | Val | MePhe | Val | Trp | Asn | Tyr | Tyr | Ile | Ile | Arg | Arg | Tyr | MeTyr | Cys |
| 195 | 894_3m_GG_K(Mal) | Ala | Val | MePhe | Val | Trp | Asn | Tyr | Tyr | Ile | Ile | Arg | Arg | Tyr | MeTyr | Cys |
| 196 | 894_3m_G2SGSG2SGS_K(Mal) | Ala | Val | MePhe | Val | Trp | Asn | Tyr | Tyr | Ile | Ile | Arg | Arg | Tyr | MeTyr | Cys |
| 197 | 894_5m_GSSGSS_K(Mal) | Ala | Val | Phe | Val | MeTrp | Asn | Tyr | Tyr | Ile | Ile | Arg | Arg | Tyr | MeTyr | Cys |
| 198 | 894_3m_5m_GGRGRS_K(Mal) | Ala | Val | MePhe | Val | MeTrp | Asn | Tyr | Tyr | Ile | Ile | Arg | Arg | Tyr | MeTyr | Cys |
| 199 | 894^{_}3m_5m_GGRSES_K(Mal) | Ala | Val | MePhe | Val | MeTrp | Asn | Tyr | Tyr | Ile | Ile | Arg | Arg | Tyr | MeTyr | Cys |
| 200 | 894_3_5m_G4S2_K(Mal) | Ala | Val | MePhe | Val | MeTrp | Asn | Tyr | Tyr | Ile | Ile | Arg | Arg | Tyr | MeTyr | Cys |

**[Table 16-2-1]**

| SEQ ID NO: | Linker | ESI (m/z) | [M+XH]X+ |
|---|---|---|---|
| 146 | PEG12 | | |
| 147 | PEG11 | | |
| 148 | PEG11 | | |
| 149 | PEG11 | | |
| 150 | PEG11 | | |
| 151 | PEG11 | | |
| 152 | PEG11 | | |
| 153 | PEG11 | | |
| 154 | PEG11 | | |
| 155 | PEG11 | | |
| 156 | PEG11 | | |
| 157 | PEG11 | | |
| 158 | PEG11 | | |
| 159 | PEG4c | | |
| 160 | PEG4c | | |
| 161 | PEG4c | | |
| 162 | PEG4c | | |
| 163 | PEG4c | | |
| 164 | PEG4c | | |
| 165 | PEG12c | | |
| 166 | PEG12c | | |
| 167 | PEG12c | | |
| 168 | PEG12c | | |
| 169 | PEG12c | | |
| 170 | PEG4c | | |

**[Table 16-2-2]**

| | | | | | | | | | | | | | | | |
|---|---|---|---|---|---|---|---|---|---|---|---|---|---|---|---|
| 171 | PEG12c | | | | | | | | | | | | | | |
| 172 | PEG12c | | | | | | | | | | | | | | |
| 173 | PEG4c | | | | | | | | | | | | | | |
| 174 | PEG12c | | | | | | | | | | | | | | |
| 175 | PEG12c | | | | | | | | | | | | | | |
| 176 | PEG12c | | | | | | | | | | | | | | |
| 177 | PEG12c | | | | | | | | | | | | | | |
| 178 | PEG12c | | | | | | | | | | | | | | |
| 179 | PEG12c | | | | | | | | | | | | | | |
| 180 | PEG12c | | | | | | | | | | | | | | |
| 181 | PEG4c | PEG4c | K(Maleimide) | | | | | | | | | | | 980.4 | 3 |
| 182 | PEG4c | PEG4c | PEG4c | K(Maleimide) | | | | | | | | | | 1062.8 | 3 |
| 183 | PEG12c | K(Maleimide) | | | | | | | | | | | | 1020.4 | 3 |
| 184 | PEG4c | K(Maleimide) | | | | | | | | | | | | 1353.4 | 2 |
| 185 | PEG8c | K(Maleimide) | | | | | | | | | | | | 961.4 | 3 |
| 186 | K(Maleimide) | | | | | | | | | | | | | 1222.8 | 2 |
| 187 | Gly | Gly | Gly | Ser | Ser | K(Maleimide) | | | | | | | | 1395.4 | 2 |
| 188 | Gly | K(Maleimide) | | | | | | | | | | | | 1251.3 | 2 |
| 189 | Gly | Gly | Ser | Gly | Ser | Ser | Gly | Ser | Ser | K(Maleimide) | | | | 1036.7 | 3 |
| 190 | Gly | Gly | Ser | Gly | Ser | Ser | K(Maleimide) | | | | | | | 1438.8 | 2 |
| 191 | Gly | Gly | Ser | Gly | Glu | Ser | K(Maleimide) | | | | | | | 1460.0 | 2 |
| 192 | Gly | Gly | Glu | Ser | Glu | Ser | K(Maleimide) | | | | | | | 1496.0 | 2 |
| 193 | Gly | Gly | Ser | Ser | K(Maleimide) | | | | | | | | | 911.9 | 3 |
| 194 | Gly | Gly | Ser | K(Maleimide) | | | | | | | | | | 882.8 | 3 |
| 195 | Gly | Gly | K(Maleimide) | | | | | | | | | | | 853.8 | 3 |
| 196 | Gly | Gly | Ser | Gly | Ser | Ser | Gly | Gly | Ser | Gly | Ser | Ser | K(Maleimide) | 1103.8 | 3 |
| 197 | Gly | Ser | Ser | Gly | Ser | Ser | K(Maleimide) | | | | | | | 1454.0 | 3 |
| 198 | Gly | Gly | Arg | Gly | Arg | Ser | K(Maleimide) | | | | | | | 1010.4 | 3 |
| 199 | Gly | Gly | Arg | Ser | Glu | Ser | K(Maleimide) | | | | | | | 1011.3 | 3 |
| 200 | Gly | Gly | Gly | Gly | Ser | Ser | K(Maleimide) | | | | | | | 1430.8 | 2 |

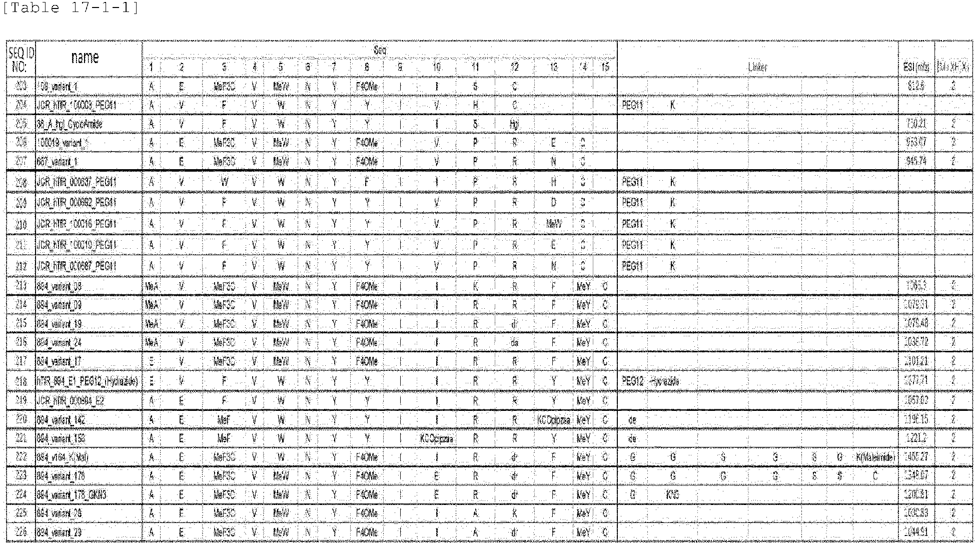

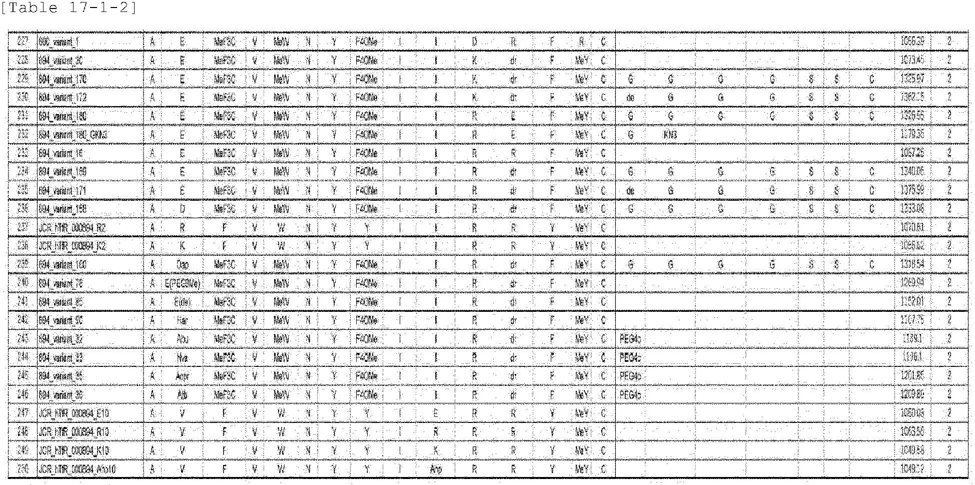

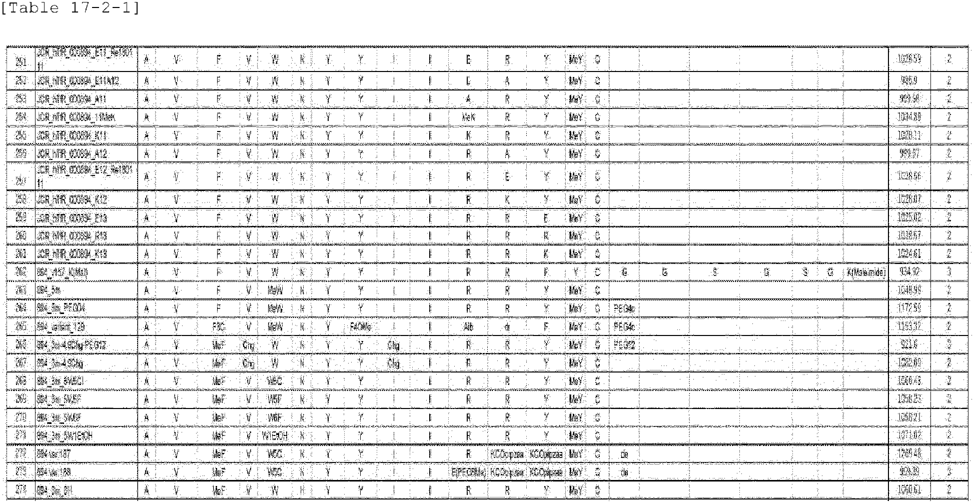

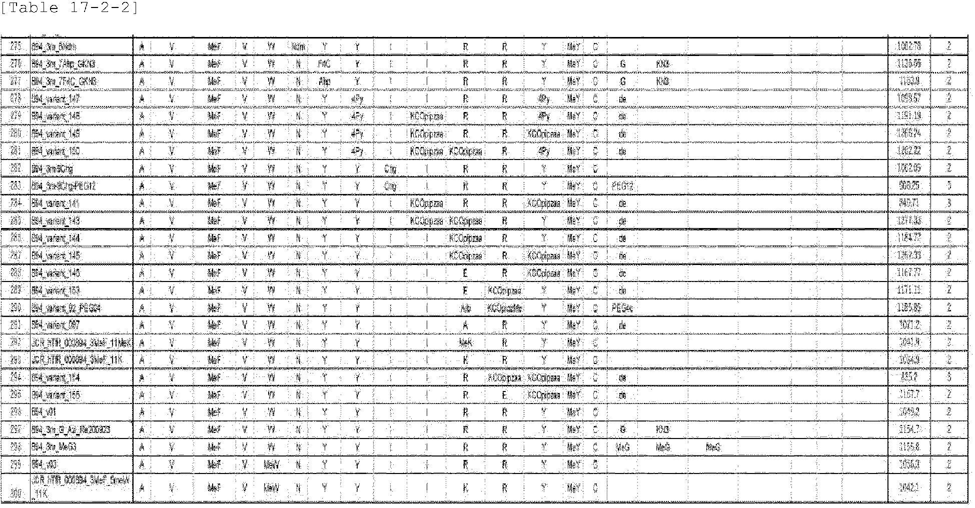

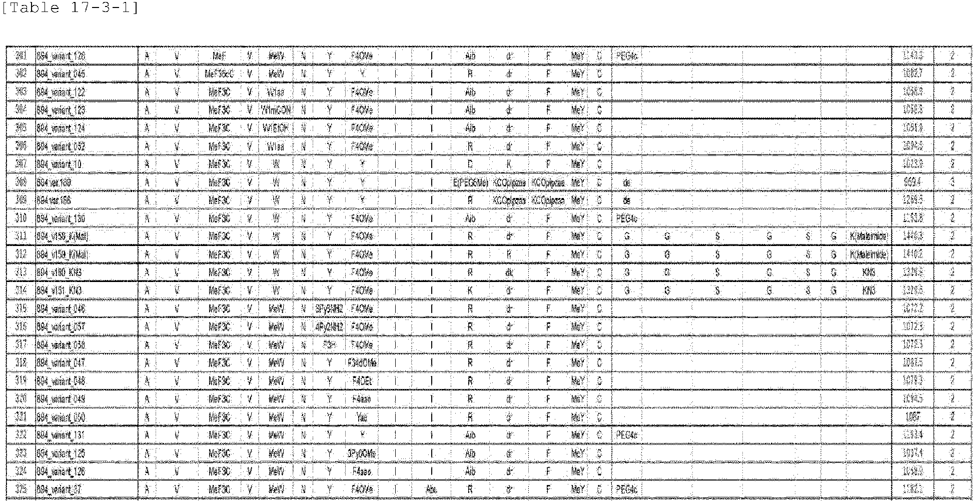

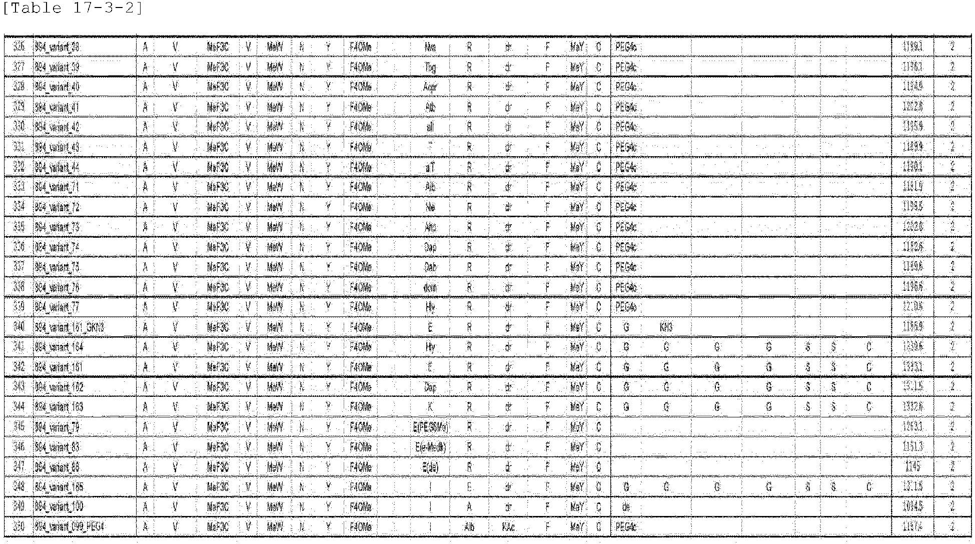

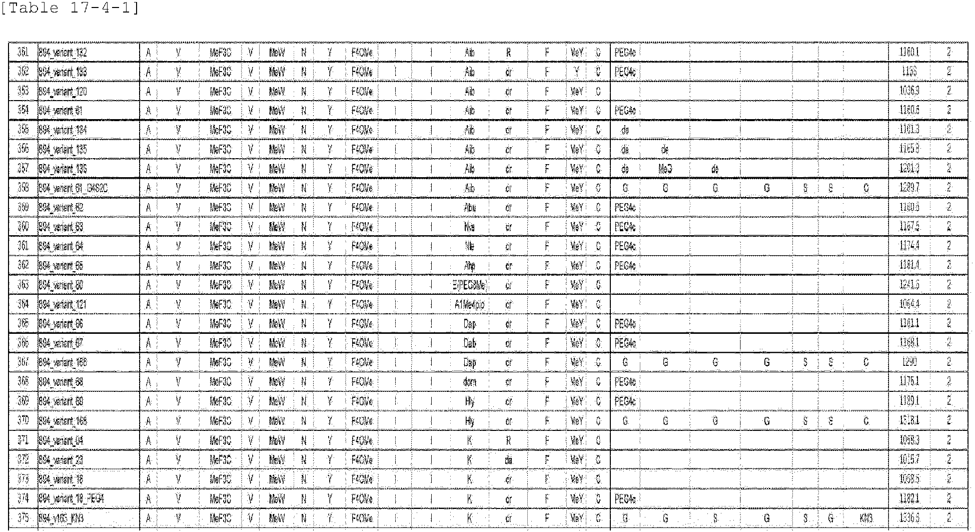

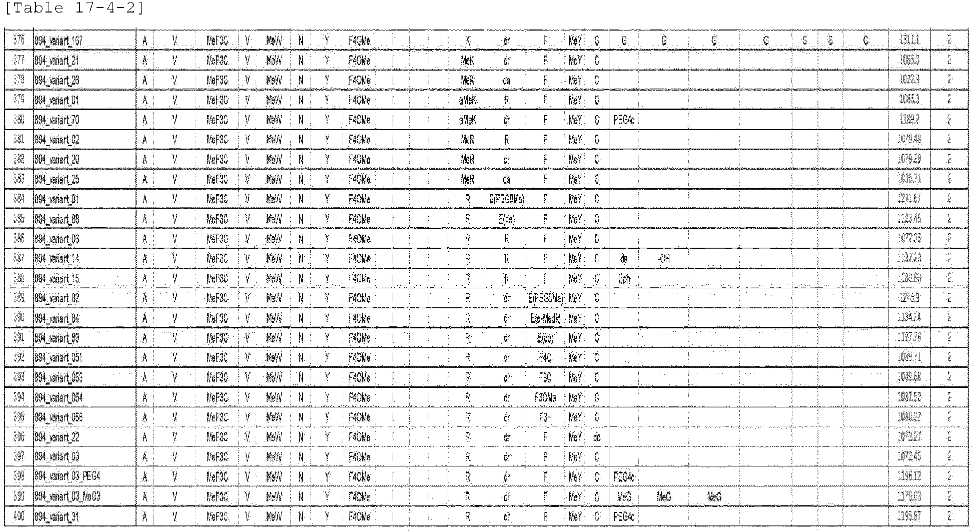

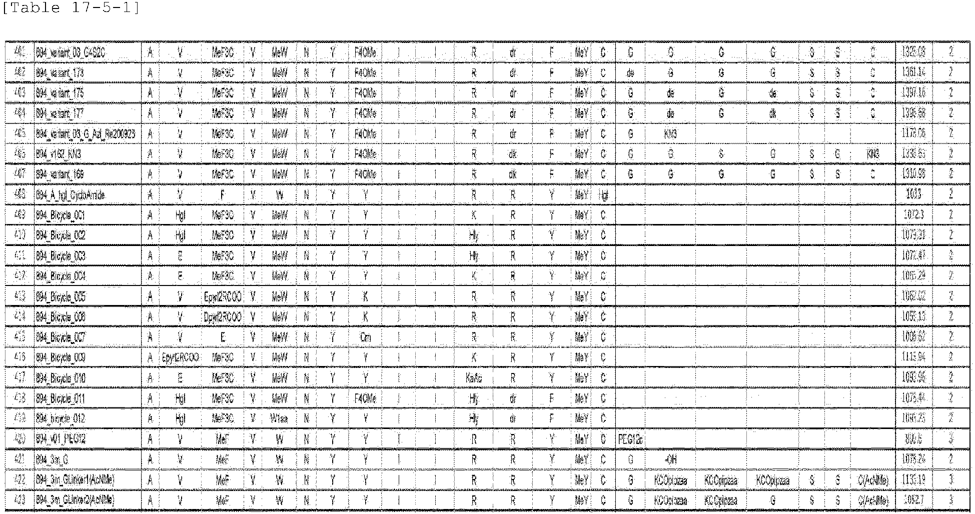

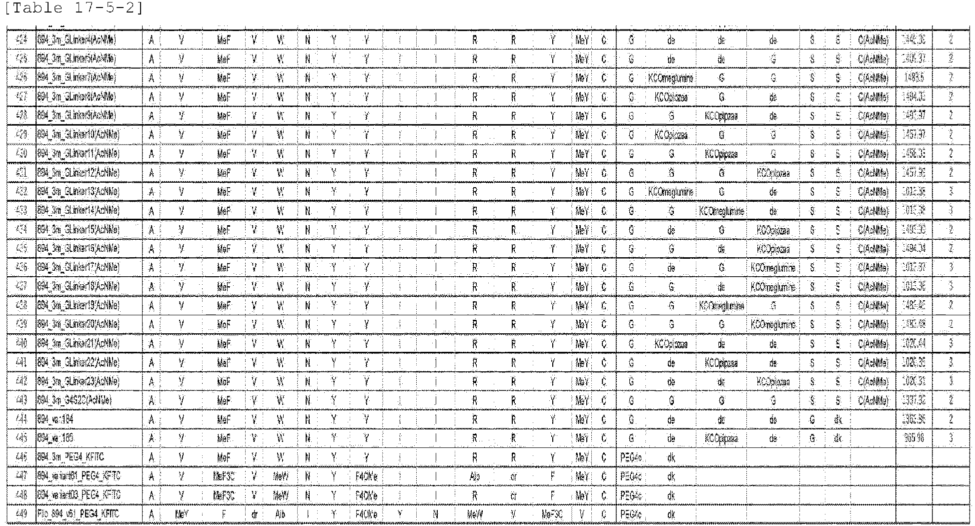

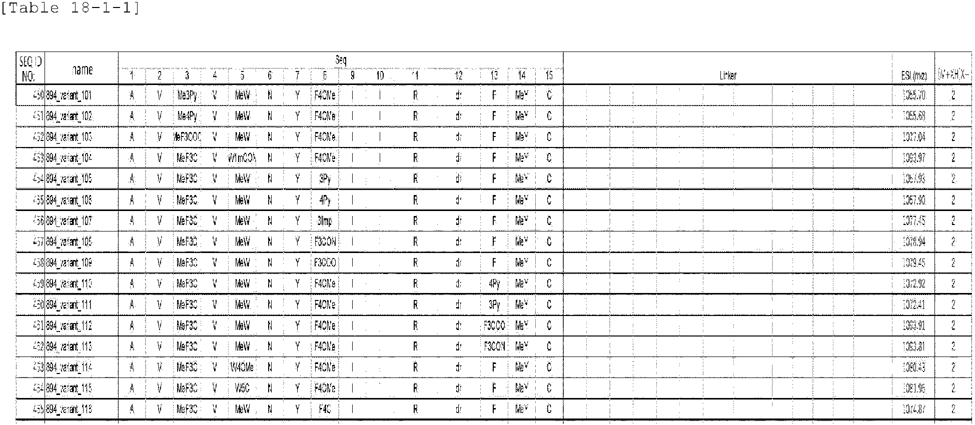

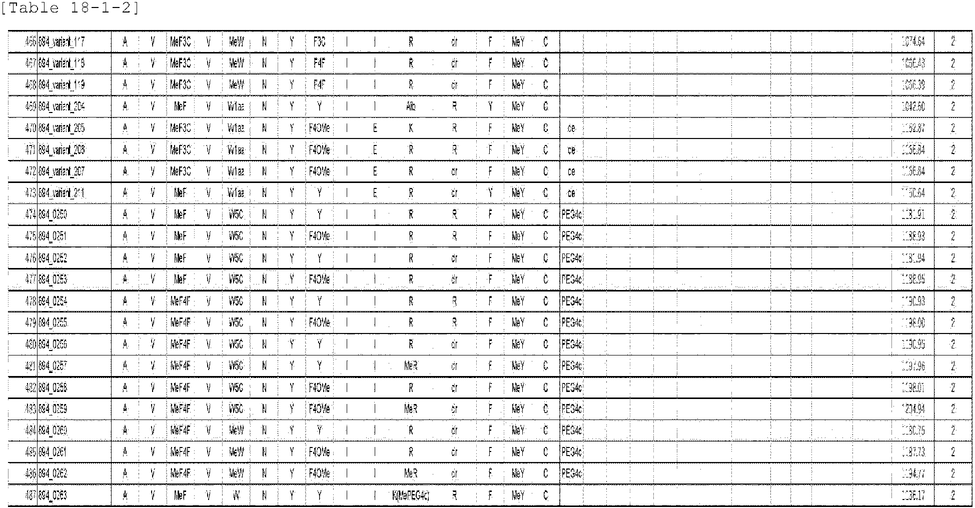

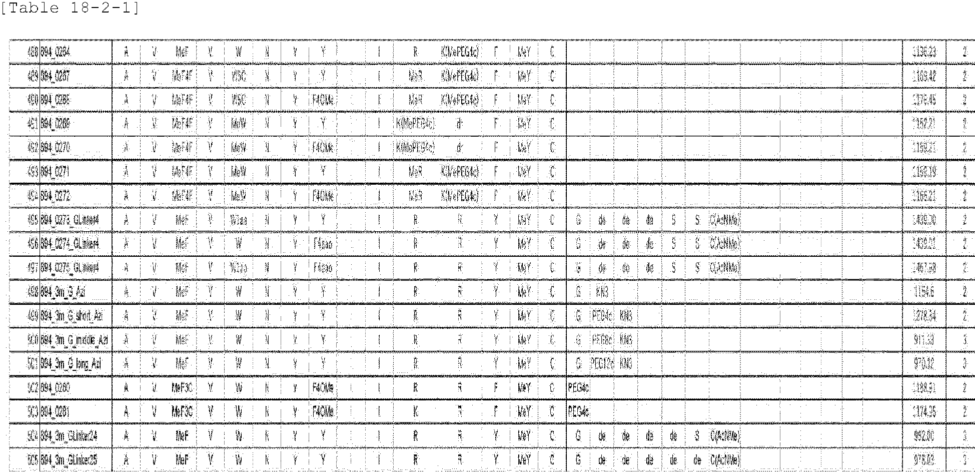

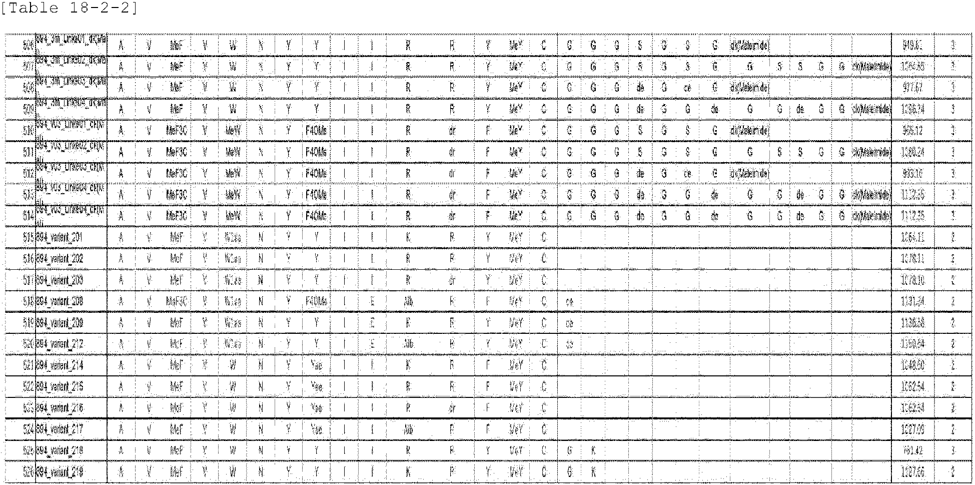

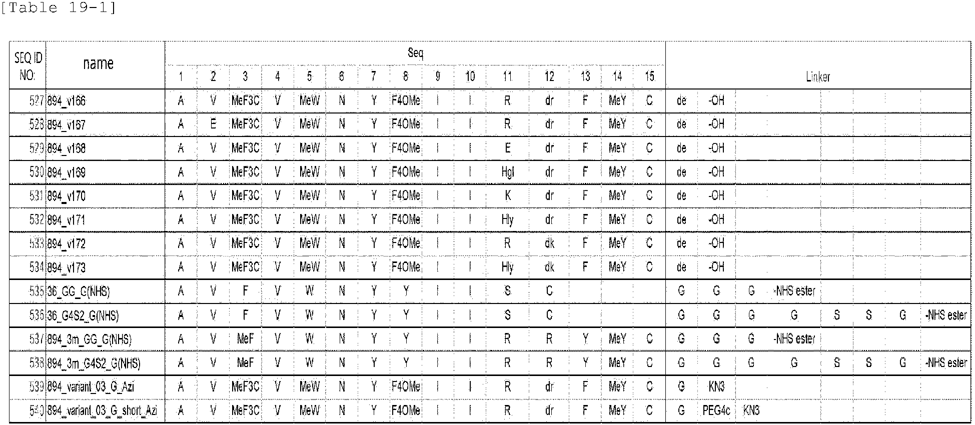

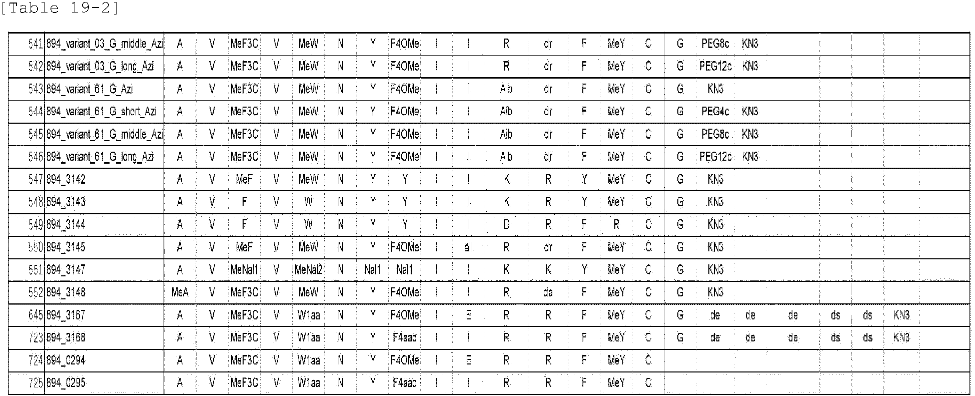

### [Synthetic Example 4-8]

### Synthesis of hTfR 894 3 m G PEG4 gAbu(NHS) (complex of linker described in SEQ ID NO: 635 and peptide described in SEQ ID NO: 296)

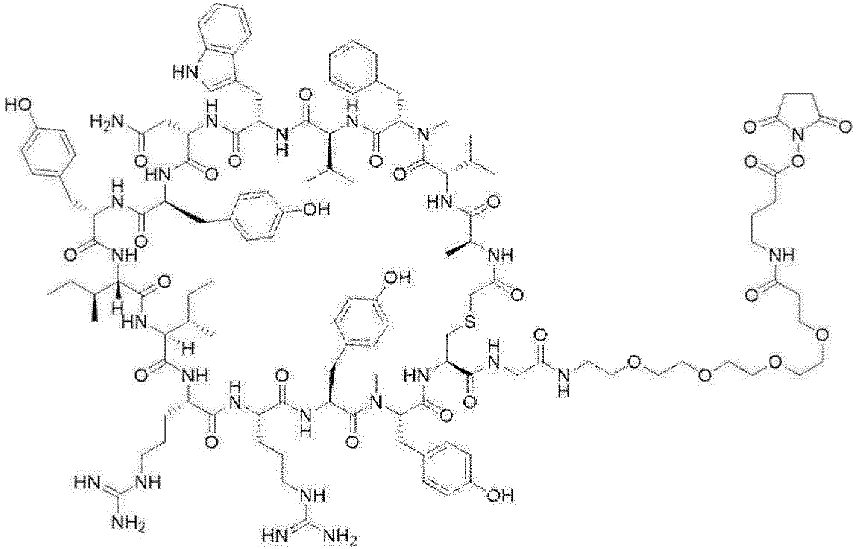

Using Fmoc-PEG4c-Abu-Alko resin prepared from Fmoc-Abu(4)-Alko resin (Watanabe Chemical, 1.00 mmol/g, 0.1 g) according to general solid phase synthesis, and starting from the removal of Fmoc according to the aforementioned general method, C-terminal carboxylic acid peptide was synthesized. At that time, CEM's Liberty Blue was used as a solid phase synthesizer, and the synthesis was performed according to the manufacturer's manual. To introduce the chloroacetyl group, the Fmoc group of the α-amino group was removed by the aforementioned method from the solid phase resin carrying the Fmoc-protected peptide obtained in the previous step, 0.2 M chloroacetic acid in DMF (5 equivalents), 0.5 M HATU in DMF (5 equivalents), and 1 M DIEA in DMF (10 equivalents) were added to the solid phase resin and the mixture was shaken at room temperature for 30 min. For deprotection of side chains and cutting out from the solid-phase resin, first, the resin obtained after the chloroacetyl group introduction step was washed with DMF five times and methylene chloride three times, and dried under reduced pressure. Successively, reactant cocktail (a mixture of TFA/H₂O/TIS/DODT in a volume ratio of 92.5:2.5:2.5:2.5) was added to the reaction vessel containing the solid-phase resin, and the mixture was shaken at room temperature for 90 min. The reaction solution was collected by filtration through a frit. The solid-phase resin remaining in the reaction vessel was shaken again with the cutting cocktail, and the solution components were collected from the frit and mixed with the above-mentioned filtrate. When this filtrate was added to an excess diethylether/hexane (1/1) cooled to 0°C, a cloudy white precipitate was formed. This mixture was centrifuged (10000 rpm, 1 min), and the solution was decanted. The obtained solid was washed again with a small amount of diethyl ether cooled to 0°C, The obtained solid was dried and used in the next cyclization reaction. For the cyclization reaction of the peptide, the peptide was dissolved in DMSO so that the final concentration was 5 mM based on the number of moles of the solid phase resin, 10 equivalents of triethylamine was added, and the mixture was stirred at room temperature for about 17 hr. The reaction solution was concentrated under reduced pressure using Savant Explorer SpeedVac. The mixture was dissolved in DMSO/H₂O(9/1, 4 mL) so that the final concentration of the obtained C-terminal carboxylic acid peptide was 5 mM based on the number of moles of the solid phase resin, HOSu (230 mg, 20 equivalents) and EDC HCl (383.4 mg, 20 equivalents) were added and the mixture was stirred.

The obtained crude product was purified using the following conditions (column; Waters Xbridge (registered trademark) C18 5 um (registered trademark) 30x150 mm: mobile phase; A=0.1% TFA in H₂O, B=0.1% TFA in MeCN: temperature 40°C: gradient (%B); 9-34% over 3 min, then 34-39% over 8 min, then 39-60% over 1 min: flow 45 mL/min.

The purity of the target product was calculated from the area ratio of the LC/MS (UV wavelength 225 nm) chromatogram under analysis condition B and was 87.4%.
analysis conditions B: retention time =4.25 min: column;
Kinetex EVO C18 2.6 um 2.1x150 mm, 100Å: mobile phase; A=0.025% TFA in H₂O, B=0.025% TFA in MeCN: temperature 40°C: gradient (%B conc); 20-60% over 7.2 min, then 60-95% over 0.3 min, then 95-95% over 1.6 min: flow 0.5 mL/min
ESI-MS(+) observed value m/z=1292.86(M+2H)²⁺

### [Synthetic Example 4-9]

### Synthesis of 894 C12NH2 (complex of fatty acid linker and SEQ ID NO: 1)

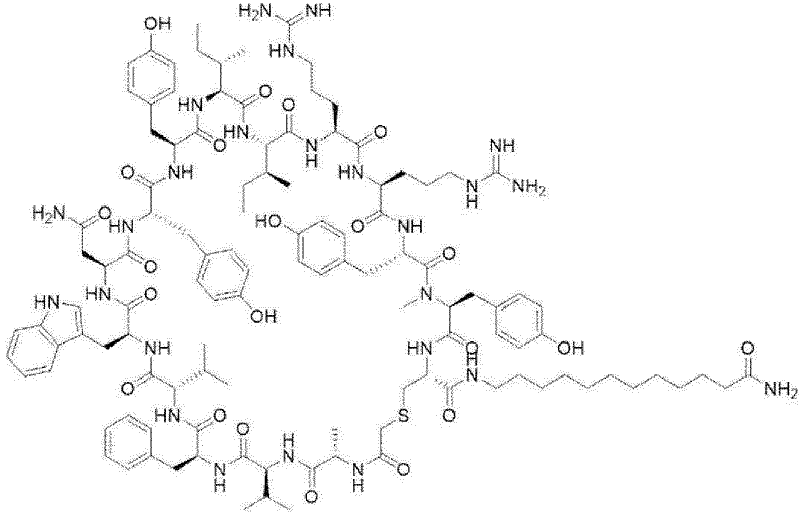

Using Sieber amide resin (Watanabe Chemical, 0.53 mmol/g, 0.21 g), the target peptide was synthesized by starting from the removal of the Fmoc group according to the general method described above. At that time, CEM's Liberty Blue was used as a solid phase synthesizer, and the synthesis was performed according to the manufacturer's manual. For the introduction of each residue, Fmoc-AA/HATU/DIEA (5.3 equivalents/5 equivalents/10 equivalents) was used for 1 equivalent of resin, and the reaction was performed in DMF at 75°C for 10 min. For the 1st residue, the 9th residue, the 10th residue, the 13th residue, and the 14th residue, the reaction was performed twice at 75°C for 10 min. For the 11th residue and the 12th residue, the reaction was performed twice at 30°C for 20 min. When introducing the 15th residue, the reaction was performed once at 30°C for 20 min. In addition, the basic condition for Fmoc removal was to react with a 20% piperidine solution in DMF at 75°C for 3 min. For Fmoc removal at the 11th residue, the 12th residue, the 14th residue, and the 15th residue, the reaction was performed at 25°C for 5 min, and then at 25°C for 10 min. To introduce the chloroacetyl group, the Fmoc group of the α-amino group was removed by the aforementioned method from the solid phase resin carrying the Fmoc-protected peptide obtained in the previous step, chloroacetic acid (5 equivalents), 5 equivalents of DIPCI, and 5 equivalents of HOSu were stirred in DCM, and the same amount of DMF as DCM was added to prepare a DCM/DMF solution (0.2 M) of ClAcOSu, the solution was added to the solid phase resin, and the mixture was shaken at room temperature for 60 min. For deprotection of side chains and cutting out from the solid-phase resin, first, the resin obtained after the chloroacetyl group introduction step was washed with DMF five times and methylene chloride three times, and then dried under reduced pressure. Then, reactant cocktail-A (a mixture of TFA/H₂O/TIS/DODT in a volume ratio of 92.5:2.5:2.5:2.5) was added to the reaction vessel containing the solid-phase resin, and the mixture was shaken at room temperature for 60 min shaken. The reaction solution was collected by filtration through a frit. The solid-phase resin remaining in the reaction vessel was shaken again with the cutting cocktail, and the solution components were collected from the frit and mixed with the above-mentioned filtrate. When this filtrate was added to an excess diisopropyl ether cooled to 0°C, a cloudy white precipitate was formed. This mixture was centrifuged (6000 rpm, 4 min), and the solution was decanted. The obtained solid was washed again with a small amount of diethyl ether cooled to 0°C, and then dried under reduced pressure. The obtained solid was used in the next cyclization reaction. For the cyclization reaction of the peptide, the peptide was dissolved in DMSO so that the final concentration was 5 mM based on the number of moles of the solid phase resin, 5 equivalents of triethylamine was added, and the mixture was shaken at room temperature for about 2 hr. The obtained reaction solution was concentrated under reduced pressure using Savant Explorer SpeedVaC.

The obtained crude product was purified using the following conditions (column; Waters Xbridge (registered trademark) C18 5 um (registered trademark) 30x150 mm: mobile phase; A=0.1% TFA in H₂O, B=0.1% TFA in MeCN: temperature 40°C: gradient (%B); 11-36% over 3 min, then 36-41% over 3 min, then 41-60% over 1 min: flow 45 mL/min).

The purity of the target product was calculated from the area ratio of the LC/MS (UV wavelength 225 nm) chromatogram under analysis condition B and was 96.3%.
analysis conditions B: retention time =12.61 min: column;
Kinetex EVO C18 2.6 um 2.1x150 mm, 100Å: mobile phase; A=0.025% TFA in H₂O, B=0.025% TFA in MeCN: temperature 60°C: gradient (%B conc); 20-60% over 20 min, then 60-95% over 1 min, then 95% over 5 min: flow 0.25 mL/min
ESI-MS(+) observed value m/z=1140.56(M+2H)²⁺

### [Synthetic Example 4-10]

### Synthesis of 894 3 m G4 (complex of linker described in SEQ ID NO: 643 and peptide of SEQ ID NO: 296)

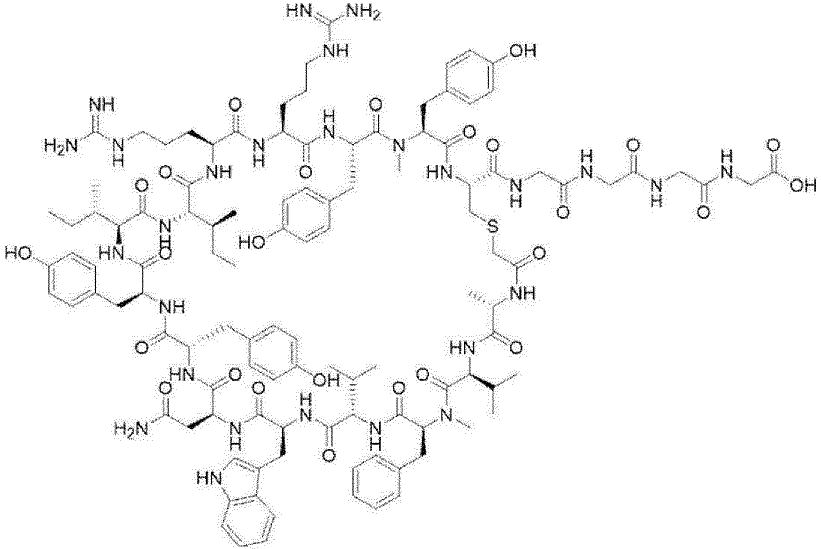

Using Fmoc-Gly-wang resin (Watanabe Chemical, 0.7 mmol/g, 1.43 g), the target peptide was synthesized by starting from the removal of the Fmoc group according to the general method described above. At that time, CEM's Liberty Blue was used as a solid phase synthesizer, and the synthesis was performed according to the manufacturer's manual. The basic condition for condensation reaction was to react once using HATU as a condensing agent at 75°C for 10 min. However, when introducing the 15th residue, the reaction was performed once at 25°C for 20 min. When introducing the 13th residue, the reaction was repeated twice at 75°C for 10 min. When introducing the 2nd residue, the reaction was repeated twice at 75°C for 45 min. In addition, the basic condition for Fmoc removal was to react with a 20% piperidine solution in DMF at 75°C for 3 min. For Fmoc removal at the 2nd residue and the 13th residue, the reaction was performed at 25°C for 5 min and then for 10 min. To introduce the chloroacetyl group, the Fmoc group of the α-amino group was removed by the aforementioned method from the solid phase resin carrying the Fmoc-protected peptide obtained in the previous step, 4 equivalents of chloroacetic acid solution in DMF (0.2 M), 4 equivalents of DIPCI solution in DMF (0.5 M), and 4 equivalents of HOSu solution in DMF (0.5 M) were added and the mixture was shaken at room temperature for 60 min. For deprotection of side chains and cutting out from the solid-phase resin, first, the resin obtained after the chloroacetyl group introduction step was washed with DMF five times and methylene chloride three times, and then dried under reduced pressure. Then, reactant cocktail-A (a mixture of TFA/H₂O/TIS/DODT in a volume ratio of 92.5:2.5:2.5:2.5) was added to the reaction vessel containing the solid-phase resin, and the mixture was shaken at room temperature for 90 min. The reaction solution was collected by filtration through a frit. The solid-phase resin remaining in the reaction vessel was shaken again with the cutting cocktail, and the solution components were collected from the frit and mixed with the above-mentioned filtrate. When this filtrate was added to an excess diisopropyl ether cooled to 0°C, a cloudy white precipitate was formed. This mixture was collected by filtration, washed with diethyl ether, and dried under reduced pressure. The obtained solid was used in the next cyclization reaction. For the cyclization reaction of the peptide, the peptide was dissolve in DMSO/IPA/H₂O(90/5/5) so that the final concentration was 5 mM based on the number of moles of the solid phase resin, 5 equivalents of triethylamine was added, and the mixture was stirred at room temperature for about 16 hr. The obtained reaction solution was concentrated under reduced pressure using GenevaC HT-12.

The obtained crude product was purified using the following conditions (column; Waters Xbridge (registered trademark) C18 5 um (registered trademark) 30x250 mm: mobile phase; A=0.1% TFA in H₂O, B=0.1% TFA in MeCN: temperature 40°C: gradient (%B); 50-7% over 0.1 min, then 7-7% over 1.9 min, then 7-32% over 3 min, then 32-37% over 11 min, then 37-60% over 1 min: flow 120 mL/min).

The purity of the target product was calculated from the area ratio of the LC/MS (UV wavelength 225 nm) chromatogram under analysis condition B and was 90.6%.
analysis conditions B: retention time =11.34 min: gradient (%B cone); 20-60% over 20 min, then 60-95% over 1 min, then 95% over 5 min
ESI-MS(+) observed value m/z=1163.59(M+2H)²⁺

### [Synthetic Example 4-11]

### Synthesis of 894 3 m G4S2 K(Mal) (complex of linker described in SEQ ID NO: 561 and peptide of SEQ ID NO: 296)

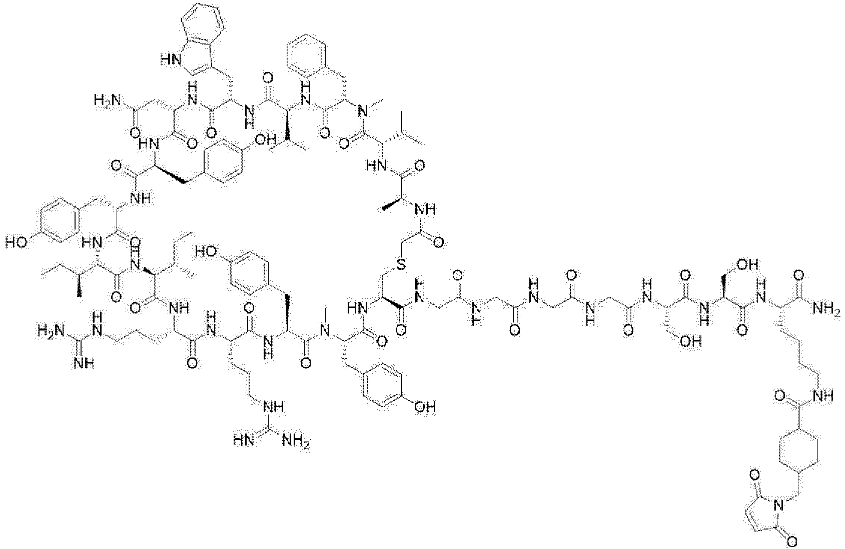

Using Sieber amide resin (Watanabe Chemical, 0.52 mmol/g, 2.4 g X 3), according to a general method, the target peptide was synthesized by starting from the removal of the Fmoc group. At that time, CEM's Liberty Blue was used as a solid phase synthesizer, and the synthesis was performed according to the manufacturer's manual. The basic condition for condensation reaction was to react once using DIPCI, Oxyma pure as a condensing agent at 75°C for 10 min. For the 15th residue, the reaction was performed at 50°C for 20 min. For the 3rd residue, the 4th residue, the 8th residue, the 10th residue, the 13th residue, and the 14th residue, the reaction was performed twice at 75°C for 10 min. For the 11th residue and the 12th residue, the reaction was performed twice at 50°C for 20 min. For the 2nd residue, the reaction was performed three times at 75°C for 60 min. For the 1st residue, the reaction was performed twice at 75°C for 20 min. In addition, the basic condition for Fmoc removal was to react with a 20% piperidine solution in DMF at 75°C for 3 min. For the 15th residue, the 16th residue, and the 17th residue, Fmoc was removed by reacting at room temperature for 5 min and then at 75°C for 3 min. To introduce the chloroacetyl group, first, the Fmoc group of the α-amino group was removed by the aforementioned method from the solid phase resin carrying the Fmoc-protected peptide obtained in the previous step. Thereafter, chloroacetic acid (5 equivalents), DIPCI (5 equivalents), and HOSu (5 equivalents) were stirred in DCM, and the same amount of DMF as DCM was added to prepare DCM/DMF solution (0.15 M) of ClAcOSu, the solution was added to the solid phase resin and the mixture was shaken at room temperature for 180 min. For deprotection of side chains and cutting out from the solid-phase resin, first, the resin obtained after the chloroacetyl group introduction step was washed with DMF five times and methylene chloride three times, and dried under reduced pressure. Then, reactant cocktail-A (a mixture of TFA/H₂O/TIS/DODT in a volume ratio of 92.5:2.5:2.5:2.5) was added to the reaction vessel containing the solid-phase resin, and the mixture was shaken at room temperature for 90 min. The reaction solution was collected by filtration through a frit. The solid-phase resin remaining in the reaction vessel was shaken again with the cutting cocktail, and the solution components were collected from the frit and mixed with the above-mentioned filtrate. When this filtrate was added to an excess diisopropyl ether cooled to 0°C, a cloudy white precipitate was formed. This mixture was collected by filtration, washed with diethyl ether cooled to 0°C, and the obtained solid was used in the next cyclization reaction. For the cyclization reaction of the peptide, the peptide was dissolved in DMSO so that the final concentration was 4.9 mM based on the number of moles of the solid phase resin, 7 equivalents of triethylamine was added, and the mixture was shaken at room temperature for about 1 hr. 1.05 equivalents of SMCC was added to the obtained reaction solution and the mixture was shaken at room temperature for 1.5 hr. The obtained reaction solution was concentrated under reduced pressure using GenevaC EZ-2 Elite.

The obtained crude product was purified using the following conditions (column; Waters Xbridge (registered trademark) C18 5 um (registered trademark) 50x250 mm: mobile phase; A=0.1% TFA in H₂O, B=0.1% TFA in MeCN: temperature 60°C: gradient (%B); 0-0% over 5.1 min, then 0-5% over 1.9 min, then 5-29% over 5 min, then 29-34% over 13.5 min, then 34-60% over 1.5 min: flow 1 mL/min 0-5.1 min, for 7.0 min from 5.1 min, 1-119 mL/min, then 119 mL/min).

The purity of the target product was calculated from the area ratio of the LC/MS (UV wavelength 225 nm) chromatogram under analysis condition B and was 94.1%.
analysis conditions B: retention time =11.33 min: gradient (%B cone); 20-60% over 20 min, then 60-95% over 1 min, then 95% over 5 min
ESI-MS(+) observed value m/z=1424.0(M+2H)²⁺

### [Synthetic Example 4-12]

### Synthesis of 894 3 m G4S2C (complex of linker described in SEQ ID NO: 644 and peptide of SEQ ID NO: 296)

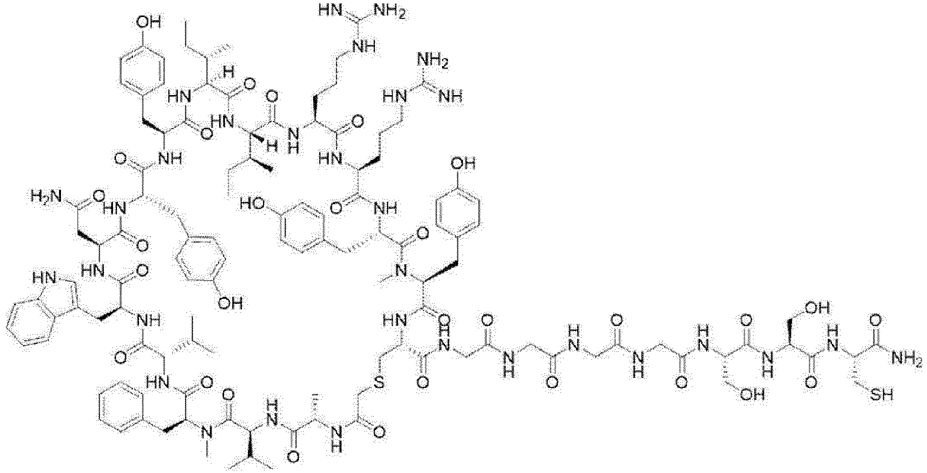

Separately synthesized 894_3 m_G (500 mg, 0.21 mmol) was dissolved in DMF (5 mL), and 1.2 equivalents of H-Gly-Gly-Gly-Ser(OtBu)-Ser(OtBu)-CyS(Trt)-NH2 (207 mg, 0.25 mmol) synthesized by a known method, 1.2 equivalents of EDC (39.1 mg, 0.25 mmol), and 1.2 equivalents of DIEA (35.8 mg, 0.25 mmol) were used and stirred at room temperature for 2.5 hr. 0.24 equivalents of H-Gly-Gly-Gly-Ser(OtBu)-Ser(OtBu)-CyS(Trt)-NH2 (41.4 mg, 0.05 mmol), 0.24 equivalents of 7.8 mg, 0.05 mmol), and 0.24 equivalents of DIEA (7.2 mg, 0.05 mmol) were added and the mixture was stirred at room temperature for 2 hr. After concentration using V-10 of Biotage, the obtained mixture was reacted in TFA/TIS/H₂O/DODT (92.5/2.5/2.5/2.5) at room temperature for 75 min. When the reaction mixture was added to an excess diisopropyl ether cooled to 0°C, a cloudy white precipitate was formed. This mixture was centrifuged (9000 rpm, 2 min), and the solution was decanted. The obtained solid was washed with diethyl ether cooled to 0°C, centrifuged (9000 rpm, 2 min), and the solution was decanted.

The obtained mixture was purified using the following conditions (column; Waters Xbridge (registered trademark) C18 5 um (registered trademark) 50x150 mm: mobile phase; A=0.1% TFA in H₂O, B=0.1% TFA in MeCN: temperature 40°C: gradient (%B); 5-30% over 3 min, then 30-35% over 8 min, then 35-60% over 1 min: flow 120 mL/min).

The purity of the target product was calculated from the area ratio of the LC/MS (UV wavelength 225 nm) chromatogram under analysis condition B and was 96.1%.
analysis conditions B: retention time =3.52 min: column;
Kinetex EVO C18 2.6 um 2.1x150 mm, 100Å: mobile phase; A=0.025% TFA in H₂O, B=0.025% TFA in MeCN: temperature 60°C: gradient (%B cone); 20-60% over 7.2 min, then 60-95% over 0.3 min, then 95-95% over 1.6 min: flow 0.5 mL/min
ESI-MS(+) observed value m/z=1301.89(M+2H)²⁺

### [Synthetic Example 4-13]

### Synthesis of 894 3 m GGRGRS K(Mal) (complex of linker described in SEQ ID NO: 568 and peptide of SEQ ID NO: 296)

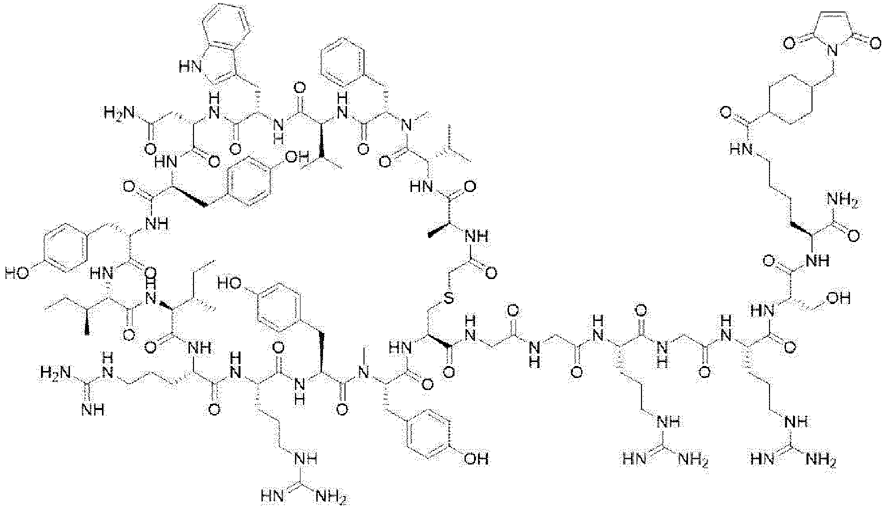

Using Sieber amide resin (Watanabe Chemical, 0.47 mmol/g, 532 mg), according to a general method, the target peptide was synthesized by starting from the removal of the Fmoc group. At that time, CEM's Liberty Blue HT was used as a solid phase synthesizer, and the synthesis was performed according to the manufacturer's manual. The basic condition for condensation reaction was to react twice using HATU as a condensing agent at 75°C for 10 min. For the 2nd residue, the reaction was performed twice at 75°C for 30 min. For the 5th residue, the 6th residue, the 7th residue, the 16th residue, the 17th residue, the 19th residue, the 21st residue, and the 22nd residue, the reaction was performed once at 75°C for 10 min. For the 11th residue, the reaction was performed twice at 50°C for 15 min. For the 12th residue, the 18th residue, and the 20th residue, the reaction was performed once at 50°C for 15 min. For the 15th residue, the reaction was performed once at 50°C for 15 min. In addition, the basic condition for Fmoc removal was to react with a 20% piperidine solution in DMF at 75°C for 3 min. For the 2nd residue and the 13th residue, Fmoc was removed by reacting twice at room temperature for 5 min. To introduce the chloroacetyl group, chloroacetic acid (5 equivalents), DIPCI (5 equivalents), and HOSu (5 equivalents) were stirred in DCM, and the same amount of DMF as DCM was added to prepare DCM/DMF solution (0.15 M) of ClAcOSu, which was added to the solid phase resin obtained in the previous step, and the mixture was shaken at room temperature for 60 min. For deprotection of side chains and cutting out from the solid-phase resin, first, the resin obtained after the chloroacetyl group introduction step was washed with DMF five times and methylene chloride three times, and dried under reduced pressure. Then, reactant cocktail-A (a mixture of TFA/H₂O/TIS/DODT in a volume ratio of 92.5:2.5:2.5:2.5) was added to the reaction vessel containing the solid-phase resin, and the mixture was shaken at room temperature for 150 min. The reaction solution was collected by filtration through a frit. The solid-phase resin remaining in the reaction vessel was shaken again with the cutting cocktail, and the solution components were collected from the frit and mixed with the above-mentioned filtrate. When this filtrate was added to an excess diethylether-hexane mixed solvent cooled to 0°C, a cloudy white precipitate was formed. This was centrifuged (9500 rpm, 1 min), the supernatant was decanted, washed with diethyl ether cooled to 0°C, and the obtained solid was used in the next cyclization reaction. For the cyclization reaction of the peptide, the peptide was dissolved in DMSO (containing 5% water) so that the final concentration was 5 mM based on the number of moles of the solid phase resin, 7 equivalents of triethylamine was added, and the mixture was shaken at room temperature for about 3 hr. 1.1 equivalents of SMCC was added to the obtained reaction solution and the mixture was shaken at room temperature for 3h. The obtained reaction solution was concentrated under reduced pressure using GenevaC EZ-2 Elite.

The obtained crude product was purified using the following conditions (column; Waters Xbridge (registered trademark) C18 5 um (registered trademark) 50x250 mm: mobile phase; A=0.1% TFA in H₂O, B=0.1% TFA in MeCN: temperature 40°C: gradient (%B); 5-29% over 3 min, then 29-34% over 8 min, then 34-60% over 1 min: flow 120 mL/min).

The purity of the target product was calculated from the area ratio of the LC/MS (UV wavelength 225 nm) chromatogram under analysis condition B and was 95.4%.
analysis conditions B: retention time =9.53 min:gradient (%B cone); 20-60% over 20 min, then 60-95% over 1 min, then 95% over 5 min
ESI-MS(+) observed value m/z=1005.7(M+3H)³⁺

### [Synthetic Example 4-14]

### Synthesis of 894 3 m PEG12dk(Biotin) (complex of linker described in SEQ ID NO: 613 and peptide of SEQ ID NO: 296)

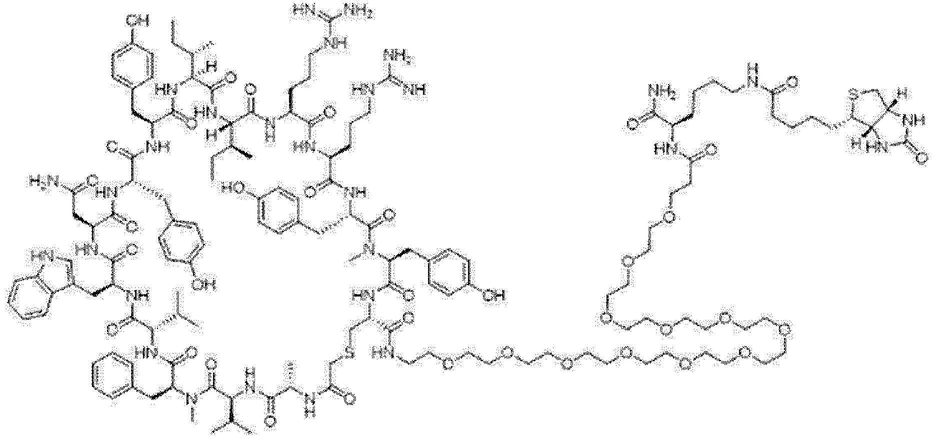

Using Sieber amide resin (Watanabe Chemical, 0.65 mmol/g, 0.54 g), the target peptide was synthesized by starting from the removal of the Fmoc group. At that time, CEM's Liberty Blue HT was used as an automatic synthesizer, and the synthesis was performed according to the manufacturer's manual. The basic condition for the introduction of each residue was to react using Fmoc-AA/DIC/Oxyma pure=5.3 equivalents/10 equivalents/5 equivalents was used for 1 equivalent of resin at 90°C for 3 min. For the 2nd residue, the reaction was performed twice at 75°C for 30 min. For the 11th residue and the 12th residue, the reaction was performed twice at 50°C for 15 min. For the 13th residue, the reaction was performed twice at 90°C for 3 min. For the 15th residue, the reaction was performed at 50°C for 15 min. The basic condition for de-Fmoc was to react with a 20% piperidine solution in DMF at 75°C for 3 min. For Fmoc removal at the 2nd residue and the 13th residue, the reaction was performed twice at 25°C for 5 min. To introduce the chloroacetyl group, the Fmoc group of the α-amino group was removed by the aforementioned method from the solid phase resin carrying the Fmoc-protected peptide obtained in the previous step, 5 equivalents of ClAcOH in DMF, 5 equivalents of HATU in DMF, and 10 equivalents of DIEA in DMF were added and the mixture was shaken at room temperature for 30 min. For deprotection of side chains and cutting out from the solid-phase resin, first, the resin obtained after the chloroacetyl group introduction step was washed with DMF five times and methylene chloride three times, and dried under reduced pressure. Then, reactant cocktail-A (a mixture of TFA/H₂O/TIS/DODT in a volume ratio of 92.5:2.5:2.5:2.5) was added to the reaction vessel containing the solid-phase resin, and the mixture was shaken at room temperature for 90 min. The reaction solution was collected by filtration through a frit. The solid-phase resin remaining in the reaction vessel was shaken again with the cutting cocktail, and the solution components were collected from the frit and mixed with the above-mentioned filtrate. When this filtrate was added to an excess diisopropyl ether/hexane (1/1) cooled to 0°C, a cloudy white precipitate was formed. This mixture was centrifuged (10000 rpm, 1 min), and the solution was decanted. The obtained solid was washed again with a small amount of diethyl ether cooled to 0°C, and the obtained solid was used in the next cyclization reaction. For the cyclization reaction of the peptide, the peptide was dissolved in 5% water-containing DMSO so that the final concentration was 5 mM based on the number of moles of the solid phase resin, 10 equivalents of triethylamine was added, and the mixture was shaken at room temperature for about 16 hr. The obtained reaction solution was concentrated under reduced pressure using Savant Explorer SpeedVaC.

The obtained crude intermediate peptide was purified using the following conditions (column; Waters Xbridge (registered trademark) C18 5 um OBD (registered trademark) 50x150 mm: mobile phase; A=0.1% TFA in H₂O, B=0.1% TFA in MeCN: temperature 40°C: gradient (%B); 5-30% over 3 min, then 30-35% over 8 min, then 35-60% over 1 min: flow 120 mL/min).

To DMSO solution (22 mM, 160 µL) of the obtained intermediate peptide were added 1.3 equivalents of Biotin-NHS and 5 equivalents of DIEA, and the mixture was stirred at room temperature for 2.5 hr, and the reaction mixture was quenched with acetic acid.

The obtained crude product was purified using the following conditions (column; Waters Xbridge (registered trademark) C18 5 um OBD (registered trademark) 19x150 mm: mobile phase; A=0.1% TFA in H₂O, B=0.1% TFA in MeCN: temperature 60°C: gradient (%B); 10-35% over 3 min, then 35-40% over 8 min, then 40-60% over 1 min: flow 17 mL/min).

The purity of the target product was calculated from the area ratio of the LC/MS (UV wavelength 225 nm) chromatogram under analysis condition B and was 98.7%.
analysis conditions B: retention time = 4.30 min : column;
Kinetex EVO C18 2.6 um 2.1x150 mm, 100Å: mobile phase; A=0.025% TFA in H₂O, B=0.025% TFA in MeCN: temperature 60°C: gradient (%B cone); 20-60% over 7.2 min, then 60-95% over 0.3 min, then 95% over 1.6 min: flow 0.5 mL/min
ESI-MS(+) observed value m/z=1017.76(M+3H)³⁺

### [Synthetic Example 4-15]

### Synthesis of 894 11K 3 Me PEG4C KTrzMal (complex of linker described in SEQ ID NO: 624 and peptide of SEQ ID NO: 293)

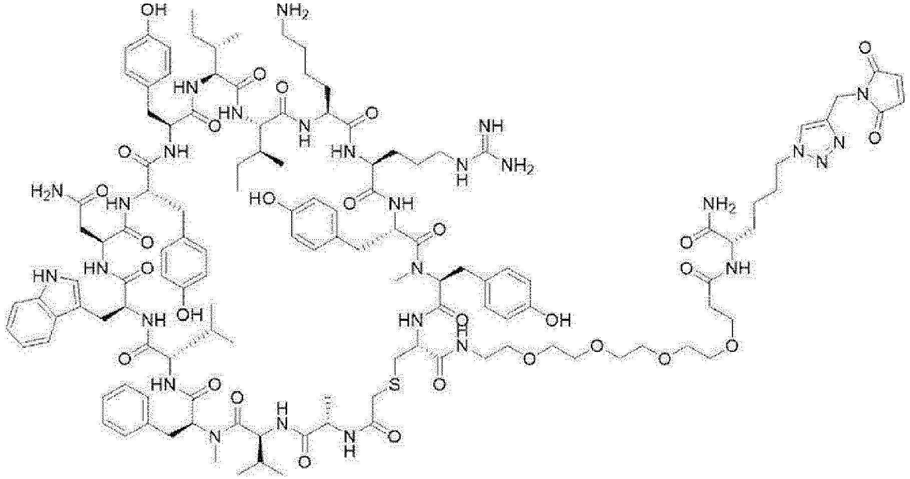

Using Sieber amide resin (Watanabe Chemical, 0.52 mmol/g, 0.19 g), the target peptide was synthesized by starting from the removal of the Fmoc group. At that time, Biotage's SyroI was used as an automatic synthesizer, and the synthesis was performed according to the manufacturer's manual. The basic condition for introduction of each residue was to react twice using Fmoc-AA/DIC/Oxyma pure=3.2 equivalents/3 equivalents/6.3 equivalents for 1 equivalent of resin at 75°C for 20 min. For the 2nd residue, the 13th residue, and the 17th residue, the reaction was performed three times at 75°C for 10 min. For the 15th residue, the reaction was performed twice at 25°C for 15 min. For the 16th residue, the reaction was performed at 25°C for 60 min. The de-Fmoc was performed by reacting with a 20% piperidine solution in DMF at room temperature for 5 min and then for 15 min. To introduce the chloroacetyl group, the Fmoc group of the α-amino group was removed by the aforementioned method from the solid phase resin carrying the Fmoc-protected peptide obtained in the previous step, 5 equivalents of chloroacetic acid, 5 equivalents of DIPCI, and 5 equivalents of HOSu were stirred in DCM, the same amount of NMP as DCM was added to prepare DCM/NMP solution (0.2 M) of ClAcOSu, added to the solid phase resin and the mixture was shaken at room temperature for 90 min. For deprotection of side chains and cutting out from the solid-phase resin, first, the resin obtained after the chloroacetyl group introduction step was washed with DMF five times and methylene chloride three times, and dried under reduced pressure. Then, reactant cocktail-A (a mixture of TFA/H₂O/TIS/DODT in a volume ratio of 92.5:2.5:2.5:2.5) was added to the reaction vessel containing the solid-phase resin, and the mixture was shaken at room temperature for 60 min. The reaction solution was collected by filtration through a frit. The solid-phase resin remaining in the reaction vessel was shaken again with the cutting cocktail, and the solution components were collected from the frit and mixed with the above-mentioned filtrate. When this filtrate was added to an excess diisopropyl ether/hexane(1/1) cooled to 0°C, a cloudy white precipitate was formed. This mixture was centrifuged (9000 rpm, 3 min), and the solution was decanted. The obtained solid was cooled to 0°C again, washed with a small amount of diethyl ether and hexane, and the obtained solid was used in the next cyclization reaction. For the cyclization reaction of the peptide, the peptide was dissolved in 9% water-containing DMSO so that the final concentration was 5 mM based on the number of moles of the solid phase resin, 10 equivalents of triethylamine was added, and the mixture was shaken at room temperature for about 1.5 hr. The obtained reaction solution was concentrated under reduced pressure using Savant Explorer SpeedVaC.

The obtained crude intermediate peptide was purified using the following conditions (column; Waters Xbridge (registered trademark) C18 5 um OBD (registered trademark) 50x150 mm: mobile phase; A=0.1% TFA in H₂O, B=0.1% TFA in MeCN: temperature 40°C: gradient (%B); 9-34% over 3 min, then 34-39% over 8 min, then 39-60% over 1 min: flow 120 mL/min).

After freeze-drying, 2 equivalents of CuSO₄·5H₂O aqueous solution (100 mM) and 10 equivalents of ascorbic acid aqueous solution (500 mM) were added to DMF solution (15 mM) of the obtained intermediate peptide, and 2 equivalents of N-propargyl maleimide solution in DMF (100 mM) was added and the mixture was stirred at room temperature.

The obtained crude product was purified using the following conditions (column; COSMOSIL PBr 10x150 mm: mobile phase; A=0.1% TFA in H₂O, B=0.1% TFA in MeCN: temperature 40°C: gradient (%B); 17-42% over 3 min, then 42-47% over 8 min, then 47-60% over 1 min: flow 5 mL/min).

The purity of the target product was calculated from the area ratio of the LC/MS (UV wavelength 225 nm) chromatogram under analysis condition B and was 82.7%.
analysis conditions B: retention time =11.37 min: column;
Kinetex EVO C18 2.6 um 2.1x150 mm, 100Å: mobile phase; A=0.025% TFA in H₂O, B=0.025% TFA in MeCN: temperature 40°C: gradient (%B cone); 20-60% over 7.2 min, then 60-95% over 0.3 min, then 95% over 1.6 min: flow 0.25 mL/min
ESI-MS(+) observed value m/z=1303.33(M+3H)³⁺

### [Synthetic Example 4-16]

### Synthesis of 894 variant 03 GKN3 (complex of linker described in SEQ ID NO: 554 and peptide of SEQ ID NO: 397)

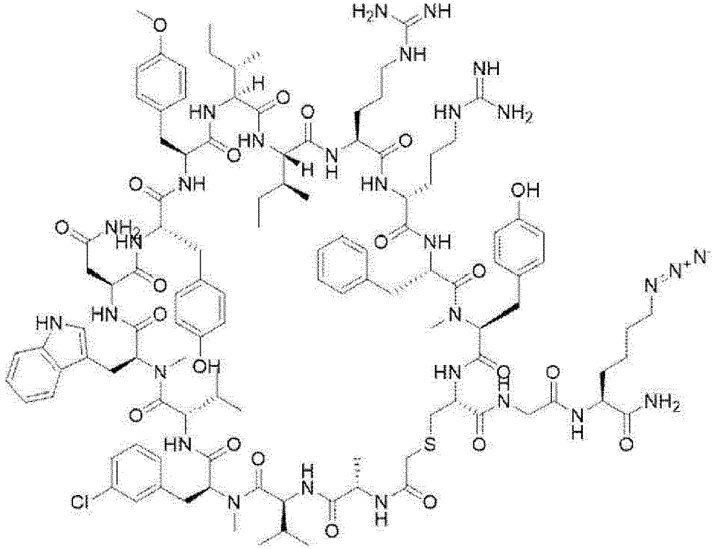

Using Sieber amide resin (Watanabe Chemical, 0.47 mmol/g, 0.21 g), the target peptide was synthesized by starting from the removal of the Fmoc group according to the general method described above. At that time, CEM's Liberty Blue HT was used as a solid phase synthesizer, and the synthesis was performed according to the manufacturer's manual. For the introduction of each residue, Fmoc-AA/HATU/DIEA(4.2eq/4eq/8eq) was used for 1 eq of resin, and the reaction was performed once in DMF at 75°C for 10 min. For the 2nd residue and the 4th residue, the reaction was performed twice at 75°C for 30 min. For the 11th residue and the 12th residue, the reaction was repeated twice at 25°C for 20 min. For the 13th residue, the reaction was performed twice at 75°C for 10 min. For the 15th residue and the 22nd residue, the reaction was performed once at 25°C for 30 min. In addition, the basic condition for Fmoc removal was to react with a 20% piperidine solution in DMF at 75°C for 3 min. Fmoc removal at the 2nd residue, and the 4th residue and 13th residue was performed by reacting at 25°C for 5 min, and then at room temperature for 10 min. To introduce the chloroacetyl group, the Fmoc group of the α-amino group was removed by the aforementioned method from the solid phase resin carrying the Fmoc-protected peptide obtained in the previous step, 5 eq of chloroacetic acid, 5 eq of DIPCI, 10 eq of HOSu were stirred in DCM, and the same amount of DMF as DCM was added to prepare a DCM/DMF solution (0.2 M) of ClAcOSu, the solution was added to the solid phase resin and the mixture was shaken at room temperature for 30 min. For deprotection of side chains and cutting out from the solid-phase resin, first, the resin obtained after the chloroacetyl group introduction step was washed with DMF five times and methylene chloride three times, and then dried under reduced pressure. Then, reactant cocktail-A (a mixture of TFA/H₂O/TIS/DODT in a volume ratio of 92.5:2.5:2.5:2.5) was added to the reaction vessel containing the solid-phase resin, and the mixture was shaken at room temperature for 20 min. The reaction solution was collected by filtration through a frit. The solid-phase resin remaining in the reaction vessel was shaken again with the cutting cocktail, and the solution components were collected from the frit and mixed with the above-mentioned filtrate. When this filtrate was added to an excess diethyl ether/hexane (1/1) mixed solvent cooled to 0°C, a cloudy white precipitate was formed. This mixture was centrifuged (10000 rpm, 1 min), and the solution was decanted and dried under reduced pressure. The obtained solid was used in the next cyclization reaction. For the cyclization reaction of the peptide, the peptide was dissolved in 5% water-containing DMSO so that the final concentration was 5 mM based on the number of moles of the solid phase resin, 6 equivalents of triethylamine was added, and the mixture was shaken at room temperature for about 16 hr. The obtained reaction solution was concentrated under reduced pressure using Savant Explorer SpeedVaC.

The obtained crude product was purified using the following conditions (column; Waters Xbridge (registered trademark) C18 5 um (registered trademark) 50x150 mm: mobile phase; A=0.1% TFA in H₂O, B=0.1% TFA in MeCN: temperature 40°C: gradient (%B); 11-36% over 3 min, then 36-41% over 8 min, then 41-60% over 1 min: flow 120 mL/min).

The purity of the target product was calculated from the area ratio of the LC/MS (UV wavelength 225 nm) chromatogram under analysis condition B and was 95.5%.
analysis conditions B: retention time =4.84 min: column;
Kinetex EVO C18 2.6 um 2.1x150 mm, 100Å: mobile phase; A=0.025% TFA in H₂O, B=0.025% TFA in MeCN: temperature 60°C: gradient (%B cone); 20-60% over 7.2 min, then 60-95% over 0.3 min, then 95% over 1.6 min: flow 0.5 mL/min.
ESI-MS(+) observed value m/z=1178.06(M+2H)²⁺

Using the linkers shown in the following Tables 20-1 and 20-2 and the peptides in the above-mentioned Tables, linker-added peptides can be synthesized by methods similar to those in the above-mentioned Synthetic Examples.

**[Table 20-1-1]**

| SEQ ID NO: | Seq | | | | | | | | | | | | |
|---|---|---|---|---|---|---|---|---|---|---|---|---|---|
| | 1 | 2 | 3 | 4 | 5 | 6 | 7 | 8 | 9 | 10 | 11 | 12 | 13 |
| 553 | G | | | | | | | | | | | | |
| 554 | G | KN3 | | | | | | | | | | | |
| 555 | G | CH | | | | | | | | | | | |
| 556 | G | K(Maleimide) | | | | | | | | | | | |
| 557 | G | G | K(Maleimide) | | | | | | | | | | |
| 558 | G | G | S | K(Maleimide) | | | | | | | | | |
| 559 | G | G | S | S | K(Maleimide) | | | | | | | | |
| 560 | G | G | G | S | S | K(Maleimide) | | | | | | | |
| 561 | G | G | G | G | S | S | | | | | | | |
| 562 | G | G | S | G | S | G | | | | | | | |
| 563 | de | G | G | G | S | S | | | | | | | |
| 564 | G | de | G | de | S | S | | | | | | | |
| 565 | G | dk | G | dk | S | S | | | | | | | |
| 566 | G | de | G | dk | S | 5 | | | | | | | |
| 567 | G | G | G | G | S | S | | | | | | | |
| 568 | G | G | R | G | R | S | K(Maleimide) | | | | | | |
| 569 | G | G | G | G | S | S | K(Maleimide) | | | | | | |
| 570 | G | MeG | S | MeG | | S | K(Maleimide) | | | | | | |
| 571 | G | G | G | G | S | S | KTrzMal | | | | | | |
| 572 | G | MeG | S | MeG | S | S | K(Maleimide) | | | | | | |
| 573 | G | G | G | G | S | S | K(Maleimide) | | | | | | |

**[Table 20-1-2]**

| | | | | | | | | | | | | | |
|---|---|---|---|---|---|---|---|---|---|---|---|---|---|
| 574 | G | G | S | G | S | S | K(Maleimide) | | | | | | |
| 575 | G | G | S | G | E | S | K(Maleimide) | | | | | | |
| 576 | G | G | E | S | E | S | K(Maleimide) | | | | | | |
| 577 | G | S | S | G | S | S | K(Maleimide) | | | | | | |
| 578 | G | G | R | G | R | S | K(Maleimide) | | | | | | |
| 579 | G | G | R | S | E | S | K(Maleimide) | | | | | | |
| 580 | G | G | G | G | S | S | K(Maleimide) | | | | | | |
| 581 | G | G | G | G | S | S | C(AcNMe) | | | | | | |
| 582 | G | KCOpipzaa | KCOpipzaa | KCOpipzaa | S | S | C(AcNMe) | | | | | | |
| 583 | G | KCOpipzaa | KCOpipzaa | G | S | S | C(AcNMe) | | | | | | |
| 584 | G | de | de | de | S | S | C(AcNMe) | | | | | | |
| 585 | G | de | de | G | S | 3 | C(AcNMe) | | | | | | |
| 586 | G | KCOmeglumine | G | G | S | S | C(AcNMe) | | | | | | |
| 587 | G | KCOpipzaa | G | de | S | S | C(AcNMe) | | | | | | |
| 588 | G | G | KCOpipzaa | de | S | S | C(AcNMe) | | | | | | |
| 589 | G | KCOpipzaa | G | G | S | 3 | C(AcNMe) | | | | | | |
| 590 | G | G | KCOpipzaa | G | S | S | C(AcNMc) | | | | | | |
| 591 | G | G | G | KCOpipzaa | 5 | S | C(AcNMe) | | | | | | |
| 592 | G | KCOmeglumine | G | de | S | S | C(AcNMe) | | | | | | |
| 593 | G | G | KCOmeglumine | de | S | S | C(AcNMe) | | | | | | |
| 594 | G | de | G | KCOpipzaa | S | 3 | C(AcNMe) | | | | | | |
| 595 | G | G | de | KCOpipzaa | S | S | C(AcNMe) | | | | | | |
| 596 | G | de | G | KCOmeglumine | S | S | C(AcNMe) | | | | | | |

**[Table 20-2-1]**

| | | | | | | | | | | | | | |
|---|---|---|---|---|---|---|---|---|---|---|---|---|---|
| 597 | G | G | de | KCOmeglumine | S | S | C(AcNMe) | | | | | | |
| 598 | G | G | KCOmeglumine | G | S | S | C(AcNMe) | | | | | | |
| 599 | G | G | G | KCOmeglumine | S | S | C(AcNMe) | | | | | | |
| 600 | G | KCOpipzaa | de | de | S | S | C(AcNMe) | | | | | | |
| 601 | G | de | KCOpipzaa | de | S | S | C(AcNMe) | | | | | | |
| 602 | G | de | de | KCOpipzaa | S | 5 | C(AcNMe) | | | | | | |
| 603 | G | de | de | de | S | S | C(AcNMe) | | | | | | |
| 604 | G | G | S | G | S | S | G | G | S | G | | | |
| 605 | G | G | S | G | S | S | G | S | S | K(Maleimide) | | | |
| 606 | G | G | R | G | R | S | G | G | R | G | R | S | |
| 607 | G | G | Q | G | Q | S | G | G | Q | G | Q | 5 | |
| 608 | de | | | | | | | | | | | | |
| 609 | de | de | | | | | | | | | | | |
| 610 | de | MeG | de | | | | | | | | | | |
| 611 | MeG | MeG | MeG | | | | | | | | | | |
| 612 | PEG4c | dk | | | | | | | | | | | |
| 613 | PEG12c | dk | | | | | | | | | | | |
| 614 | PEG4c | de | PEG4c | dk | | | | | | | | | |
| 615 | PEG4c | dr | PEG4c | dk | | | | | | | | | |
| 616 | PEG4c | de | PEG4c | de | PEG4c | dk | | | | | | | |
| 617 | PEG4c | dr | PEG4c | dr | PEG4c | dk | | | | | | | |
| 618 | PEG4c | dk | | | | | | | | | | | |
| 610 | PEG12c | dk | | | | | | | | | | | |

**[Table 20-2-2]**

| | | | | | | | | | | | | | |
|---|---|---|---|---|---|---|---|---|---|---|---|---|---|
| 620 | PEG4c | de | PEG4c | dk | | | | | | | | | |
| 621 | PEG4c | dr | PEG4c | dk | | | | | | | | | |
| 622 | PEG4c | de | PEG4c | de | PEG4c | dk | | | | | | | |
| 623 | PEG4c | dr | PEG4c | dr | PEG4c | dk | | | | | | | |
| 624 | PEG4c | KTrzMal | | | | | | | | | | | |
| 625 | PEG8c | KTrzMal | | | | | | | | | | | |
| 626 | PEG12c | KTrzMal | | | | | | | | | | | |
| 627 | PEG4c | K(Maleimide) | | | | | | | | | | | |
| 628 | PEG8c | K(Maleimide) | | | | | | | | | | | |
| 629 | PEG12c | K(Maleimide) | | | | | | | | | | | |
| 630 | KN3 | | | | | | | | | | | | |
| 631 | PEG4c | KN3 | | | | | | | | | | | |
| 632 | PEG8c | KN3 | | | | | | | | | | | |
| 633 | PEG12c | KN3 | | | | | | | | | | | |
| 634 | G | PEG12c | gAbu | | | | | | | | | | |
| 635 | G | PEG4c | gAbu | | | | | | | | | | |
| 636 | G | PEG4c | PEG4c | PEG4c | gAbu | | | | | | | | |
| 637 | G | G | R | G | R | S | gAbu | | | | | | |
| 638 | G | PEG4c | R | PEG4c | R | PEG4c | gAbu | | | | | | |
| 639 | G | G | Q | G | Q | 5 | gAbu | | | | | | |
| 640 | G | G | G | G | S | S | G | G | G | G | 5 | S | gAbu |
| 641 | G | G | G | G | S | S | dk(CHO4PhCO) | NH2 | | | | | |
| 642 | PEG12c | dk(CHO4PhCO) | NH2 | | | | | | | | | | |
| 648 | G | G | G | G | | | | | | | | | |
| 644 | G | G | G | G | S | S | C | | | | | | |

Novel amino acids were synthesized as follows.

### [Synthetic Example 5-1]

### Synthesis of (S)-2-[[(9H-fluoren-9-ylmethoxy)carbonyl]amino] (7-chloro-1-ethyl-1H-indol-3-yl)propanoic acid (N-protected form of W1Et7Cl)

NaH (60wt%, 8.7 g, 361 mmol) was added in portions to a DMF solution (500 mL) of 7-chloro-3-iodo-1H-indole (18 g, 64.9 mmol) at 0°C. Successively, ethyl iodide (10.3 mL, 130.4 mmol) was added in portions at 0°C, and the mixture was stirred at room temperature for about 16 hr. The reaction solution was diluted with water and extracted with ethyl acetate (3x200 mL). The combined organic layer was washed three times with water (100 mL), dried over anhydrous sodium sulfate, and filtered. The organic layer was concentrated, and the residue was purified by silica gel column chromatography (ethyl acetate:petroleum ether=1:5).

Under a nitrogen atmosphere, iodine (1.6 g, 12.6 mmol) was added to a DMF suspension (50 mL) of zinc (8 g, 122 mmol), and then methyl (2R)-2-[[(9H-fluoren-9-yl methoxy)carbonyl]amino]-3-iodopropanoate (18.5 g, 41 mmol) was added and the mixture was stirred at room temperature for 30 min. A solution of a part of the product obtained above (15 g, 49 mmol) in DMF (300 mL) was added to this reaction mixture. Successively, SPhos (0.84 g, 2.0 mmol) and Pd₂(dba)₃ (1.1 g, 1.2 mmol) were added, and the mixture was stirred at 50°C for 4 hr. The reaction was discontinued with water, the solid was filtered off, and the filtrate was extracted four times with ethyl acetate (400 mL). The combined organic layers were washed four times with water (200 mL). The organic layer was dried over anhydrous sodium sulfate, filtered, and concentrated. The obtained residue was purified by silica gel column chromatography (ethyl acetate:petroleum ether=2:8).

Hydrogen chloride (60 g, 1.7 mol) was bubbled into a mixture of a part of the obtained resultant product (18 g, 35.8 mmol) and 1,4-dioxane (200 mL), and the mixture was stirred at 100°C for 4 hr. The reaction mixture was concentrated, and the residue was purified by reversed-phase silica gel column chromatography (water:acetonitrile=100:0-0:100) to obtain the title compound.
ESI-MS(+) observed value m/z=489.10(M+H)⁺

### [Synthetic Example 5-2]

### Synthesis of (S)-2-[[(9H-fluoren-9-ylmethoxy)carbonyl]amino]-3-(2-((tertiary butyloxycarbonyl)amino)pyridin-4-yl)propanoic acid (N-protected form of 4Py2NH2)

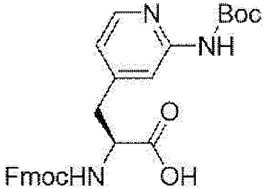

Under a nitrogen atmosphere, iodine (4.5 g, 17.7 mmol) was added to a suspension of zinc (5.8 g, 88.6 mmol) in DMF (200 mL), and a DMF solution (50 mL) of methyl (2R)-2-[[(9H-flouren-9-ylmethoxy)carbonyl]amino]-3-iodopropanate (20 g, 44.32 mmol, 1.0 equivalent) was added dropwise at room temperature. The mixture was stirred at room temperature for 1 hr. Successively, 4-bromopyridin-2-amine (7.7 g, 44.3 mmol, 1 equivalent), Pd₂(dba)₃ (2.0 g, 2.2 mmol, 0.05 equivalents), SPhos (1.8 g, 4.4 mmol, 0.1 equivalent) were added, and the mixture was stirred at 50°C for about 16 hr. Water (500 mL) was added to discontinue the reaction, and the solid was filtered off. The filtrate was extracted three times with ethyl acetate (300 mL). The combined organic extracts were washed three times with saturated brine (300 mL), dried over anhydrous sodium sulfate, filtered, and the filtrate was concentrated. The obtained residue was purified by silica gel column chromatography (ethyl acetate:petroleum ether=25:75).

Boc₂O (6.3 g, 28.7 mmol) and NaI (4.3 g, 28.7 mmol) were added to a part of the resultant product (10 g, 24.0 mmol) obtained above, and the mixture was stirred in tertiary butyl alcohol (110 mL) at room temperature for about 16 hr. The reaction mixture was concentrated, and the obtained residue was dissolved in ethyl acetate (80 mL), and washed three times with saturated brine (100 mL). The organic layer was dried over anhydrous sodium sulfate, filtered, and concentrated.

To a solution of a part (3 g, 5.8 mmol) of the obtained resultant product in 2-propanol (30 mL) was added CaCl₂ (6.4 g, 58.0 mmol), and then an aqueous solution (5 mL) of LiOH-H₂O (0.28 g, 11.6 mmol) was added at 0°C. The mixture was stirred at room temperature for about 16 hr and diluted with water (60 mL), and the solid was filtered off. The filtrate was adjusted to pH about 6 with an aqueous citric acid solution, and extracted three times with ethyl acetate (40 mL). The combined organic extract was washed three times with saturated brine (50 mL), dried over anhydrous sodium sulfate, filtered, and concentrated. The residue was purified by silica gel column chromatography (dichloromethane:methanol=92:8) to obtain the title compound.
ESI-MS(+) observed value m/z=504.15(M+H)⁺

### [Synthetic Example 5-3]

### Synthesis of (S)-2-[[(9H-fluoren-9-ylmethoxy)carbonyl]amino]-3-(6-((tertiary butyloxycarbonyl)amino)pyridin-3-yl) propanoic acid (N-protected form of 3Py6NH2)

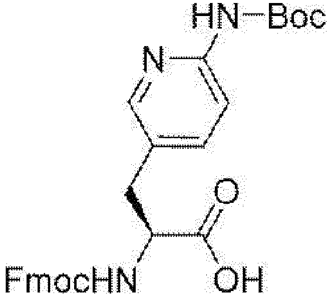

The title compound was obtained by a similar method according to [Synthetic Example 5-2] and using 5-bromopyridin-2-amine instead of 4-bromopyridin-2-amine. ESI-MS(+) observed value m/z=504.15(M+H)⁺

### [Synthetic Example 5-4]

### Synthesis of N2-[[(9H-fluoren-9-ylmethoxy)carbonyl]-N6-(4-methylpiperazine-1-carbonyl)-L-lysine (N-protected form of KCOpipzMe)

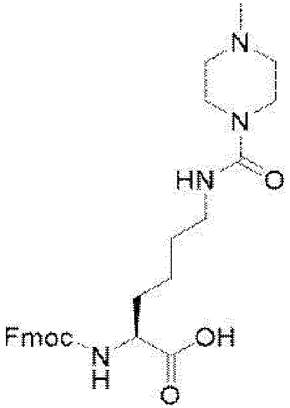

DIPEA (2.3 mL, 13.0 mmol) and triphosgene (1.23 g, 4.2 mmol) were added at 0°C to 1-methylpiperazine (1.4 mL, 12.7 mmol) dissolved in dichloromethane (20 mL). After stirring at the same temperature for 1 hr, the mixture was concentrated. To the obtained residue was added a solution (20 mL) of [(9H-fluoren-9-ylmethoxy)carbonyl]-L-lysine and DIPEA (2.8 mL, 16.3 mmol) in dichloromethane at 0°C. The mixture was stirred at 0°C for about 16 hr. The reaction was discontinued with saturated aqueous sodium hydrogen carbonate solution, the mixture was diluted with dichloromethane and washed once with water, twice with 5% acetic acid aqueous solution, once with saturated aqueous sodium hydrogen carbonate solution, and finally once with saturated brine. The organic layer was dried over anhydrous sodium sulfate and concentrated. The residue was purified by silica gel column chromatography (dichloromethane:methanol=100:0-80:20) to obtain the title compound.
ESI-MS(+) observed value m/z=495.40(M+H)⁺

### [Synthetic Example 5-5]

### Synthesis of N-α-[(9H-fluoren-9-ylmethoxy)carbonyl]-1-(2-amino-2-oxoethyl)-L-tryptophan (N-protected form of W1mCON)

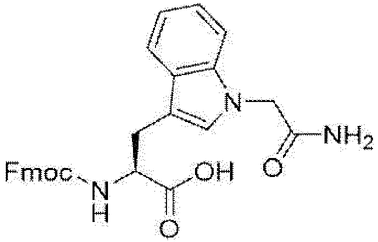

Under a nitrogen atmosphere, potassium-tertiary butoxide (3.7 g, 32.9 mmol) was added in several portions to a mixed solution of N-Boc-tryptophan (5 g, 16.4 mmol) in DMF (30 mL) and THF (30 mL) at 0°C. After stirring at the same temperature for 0.5 hr, 2-chloroacetamide (1.5 g, 16.4 mmol) was added in several portions at 0°C, and the mixture was stirred at room temperature for 3 hr. Water (100 mL) was added to discontinue the reaction, and the mixture was extracted three times with ethyl acetate (50 mL). The combined organic layer was washed three times with saturated brine (50 mL), dried over anhydrous magnesium sulfate, filtered, and the filtrate was concentrated. The obtained residue was purified by silica gel column chromatography (ethyl acetate:petroleum ether=90:10).

To a solution (30 mL) of a part of the resultant product (6 g, 16.6 mmol) in ethyl acetate was added an ethyl acetate solution (2 M, 30 mL) of hydrogen chloride, and the mixture was stirred at room temperature for about 16 hr. The resulting solid was collected by filtration and washed with ethyl acetate and diethyl ether. The obtained solid was dissolved in 1,4-dioxane (50 mL) and water (10 mL), and then 2,5-dioxypyrrolidin-1-yl-9H-fluoren-9-yl-methylcarbonate (5.9 g, 17.5 mmol) and sodium hydrogen carbonate (5.9 g, 69.9 mmol) were added at 0°C. The mixture was stirred at room temperature for 3 hr and diluted with water (200 mL). Citric acid was added to adjust to pH 5-6 and the mixture was extracted three times with ethyl acetate (60 mL). The combined organic extract was washed three times with saturated brine (100 mL). The organic layer was dried over anhydrous sodium sulfate, filtered, and concentrated. The residue was purified by silica gel column chromatography (dichloromethane/methanol=92/8) to obtain the title compound.
ESI-MS(+) observed value m/z=484.25(M+H)⁺

### [Synthetic Example 5-6]

### Synthesis of Fmoc-Glu(d-Pro-O-allyl)-OH (N-protected form of Epyrl2RCOO)

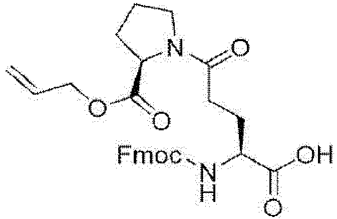

To a solution (10 mL) of 4-((((9H-fluoren-9-yl)methoxy)carbonyl)amino)-5-(tertiary butoxy)-5-oxopentanoic acid (2.5 g, 5.8 mmol) in DMF were added HATU (2.7 g, 7.0 mmol), DIPEA (1.3 mL, 7.5 mmol), and allyl-D-prolinate (0.9 g, 5.80 mmol), and the mixture was stirred at 0°C for 1 hr. The reaction solution was diluted with water, and extracted with a mixed solvent of ethyl acetate and hexane. The organic layer was washed four times with water, dried over anhydrous sodium sulfate, filtered, and concentrated under reduced pressure. The residue was purified by silica gel column chromatography.

A part of the resultant product (2.6 g, 4.5 mmol) obtained by the aforementioned reaction and dichloromethane/TFA (1:1, 30 mL) were stirred at room temperature for 3 hr. The reaction solution was concentrated, and the residue was dissolved in dichloromethane, and adjusted to pH 8 or above by adding saturated aqueous sodium hydrogen carbonate solution. Then, hydrochloric acid was added to adjust to pH 3-4, and the mixture was extracted with ethyl acetate. The organic layer was dried over anhydrous sodium sulfate, filtered, and concentration under reduced pressure to obtain the title compound.
ESI-MS(+) observed value m/z=507.40(M+H)⁺

### [Synthetic Example 5-7]

### Synthesis of Fmoc-Asp(d-Pro-O-allyl)-OH (N-protected form of Dpyrl2RCOO)

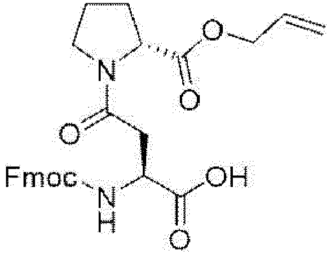

The title compound was obtained by a similar method according to [Synthetic Example 5-6] and using 3-((((9H-fluoren-9-yl)methoxy)carbonyl)amino)-4-(tertiary butoxy)-4-oxobutanoic acid instead of 4-((((9H-fluoren-9-yl)methoxy)carbonyl)amino)-5-(tertiary butoxy)-5-oxopentanoic acid.
ESI-MS(+) observed value m/z=493.30(M+H)⁺

### [Synthetic Example 5-8]

### Synthesis of Fmoc-Lys(Gly-O-allyl)-OH (N-protected form of KaAC)

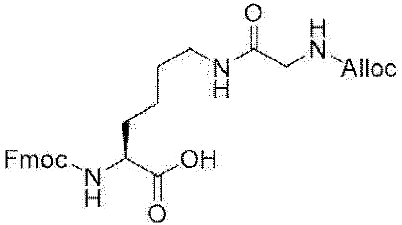

To a DMF solution of tertiary butyl(((9H-fluoren-9-yl)methoxy)carbonyl)-L-lysinemethyl-hydrochloride (1.2 g, 2.6 mmol), ((allyloxy)carbonyl)glycine (0.46 g, 2.9 mmol), and DIPEA (0.59 mL, 3.4 mmol) was added a DMF solution (20 mL) of HATU (1.22 g, 3.1 mmol), and the mixture was stirred at 0°C for 1 hr. It was diluted with a mixed solvent of ethyl acetate and hexane, and the organic layer was washed four times with water. The organic layer was dried over anhydrous sodium sulfate, concentrated, and the residue was purified by silica gel column chromatography (ethyl acetate:hexane=0:100-80:20).

To a solution (10 mL) of a part of the resultant product (1.5 g, 2.6 mmol) synthesized in the above in dichloromethane was added TFA (10 mL), and the mixture was stirred at room temperature for about 16 hr. The reaction solution was concentrated, and azeotropically distilled three times with toluene. The obtained residue was washed with diisopropyl ether and dried under reduced pressure at 55°C to give the title compound.
ESI-MS(+) observed value m/z=510.40(M+H)⁺

### [Synthetic Example 5-9]

### Synthesis of N^{α}-(((9H-fluoren-9-yl)methoxy)carbonyl)-1-(2-((tertiary butyldimethylsilyl)oxy)ethyl)-L-tryptophan (N- and O-protected form of W1EtOH)

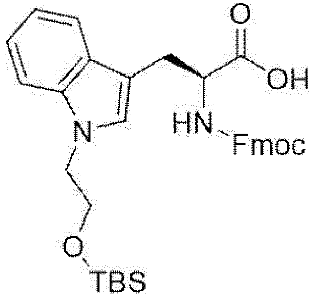

To a mixture of N-Boc-tryptophan (20 g, 64.9 mmol) in THF (100 mL) and DMF (100 mL) was added potassium-tertiary butoxide (14.8 g, 69.0 mmol) at 0°C. After stirring for 15 min, 2-bromoethoxy(tertiary butyl)dimethylsilane (16.5 g, 69.0 mmol) was added and the mixture was stirred at room temperature for about 16 hr. To the reaction solution was added aqueous citric acid solution at 0°C to adjust the pH to 6, and the mixture was diluted with 200 mL of water. The reaction solution was extracted three times with ethyl acetate (100 mL), and the combined organic extract was washed three times with saturated brine (100 mL). The organic layer was dried over sodium sulfate, filtered, and concentrated. The residue was purified by silica gel column chromatography (ethyl acetate:hexane=1:5).

Under a nitrogen atmosphere, to a solution of a part (16 g, 34.6 mmol) of the resultant product in dichloromethane (150 mL) was added 2,6-lutidine (20 mL, 172.9 mmol), and TMSOTf (25 mL, 112 mmol) was added at 0°C. The mixture was warmed to room temperature and stirred for 24 hr. The reaction solution was concentrated. To the obtained mixture were added dioxane (300 mL) and water (150 mL), and then sodium hydrogen carbonate (9.3 g, 110.35 mmol, 3.19 equivalents) and 9-fluorenylmethyl-N-succinimidylcarbonate (14.0 g, 41.4 mmol) were added and the mixture was stirred at room temperature for about 16 hr. The solid was filtered, and the filtrate was adjusted pH to 7 by adding citric acid. The mixture was diluted with water (200 mL) and extracted three times with ethyl acetate (200 mL). The combined organic extract was washed three times with saturated brine (200 mL). The organic layer was dried over sodium sulfate, filtered, and concentrated. The residue was purified by silica gel column chromatography (ethyl acetate:hexane (1:15) to obtain the title compound.
ESI-MS(+) observed value m/z=585.15(M+H)⁺

### [Industrial Applicability]

According to the present invention, a novel drug delivery system (DDS) technique capable of selectively delivering a drug (compound containing oligonucleotides for producing at least partially functional dystrophin protein) to muscle tissues such as cardiac muscle, skeletal muscle and the like and efficiently introducing the drug into the muscle cells can be provided.

This application is based on a patent application No. 2021-135254 filed in Japan (filing date: August 21, 2021), the contents of which are incorporated in full herein.

## Claims

1. A conjugate or a salt thereof comprising the following:
(1) a peptide that binds to a transferrin receptor, and comprises
the amino acid sequence shown in SEQ ID NO: 1 (Ala-Val-Phe-Val-Trp-Asn-Tyr-Tyr-Ile-Ile-Ser-Cys); or
an amino acid sequence resulting from substitution, deletion, addition, and/or insertion of not less than one and not more than 10 amino acid residues in the amino acid sequence shown in SEQ ID NO: 1, and
(2) a compound comprising an oligonucleotide for producing an at least partially functional dystrophin protein.

2. The conjugate or a salt thereof according to claim 1, wherein
the peptide that binds to a transferrin receptor is a peptide comprising
the amino acid sequence shown in SEQ ID NO: 296 (Ala-Val-MeF-Val-Trp-Asn-Tyr-Tyr-Ile-Ile-Arg-Arg-Tyr-MeY-Cys); or an amino acid sequence resulting from substitution, deletion, addition, and/or insertion of not less than one and not more than 10 amino acid residues in the amino acid sequence shown in SEQ ID NO: 296.

3. The conjugate or a salt thereof according to claim 1 or 2, wherein
the peptide that binds to a transferrin receptor is a peptide comprising
the amino acid sequence shown in SEQ ID NO: 296; or an amino acid sequence resulting from substitution, deletion, addition, and/or insertion of not less than one and not more than 5 amino acid residues in the amino acid sequence shown in SEQ ID NO: 296.

4. The conjugate or a salt thereof according to any one of claims 1 to 3, wherein the peptide that binds to a transferrin receptor is a peptide comprising
the amino acid sequence shown in SEQ ID NO: 296; or an amino acid sequence resulting from substitution of any of the 3rd, 5th, 7th, 8th, 11th, 12th, and 13th amino acid residues of the SEQ ID NO: 296.

5. The conjugate or a salt thereof according to any one of claims 1 to 4, wherein the peptide that binds to a transferrin receptor is any of peptides in which
the 3rd amino acid residue of SEQ ID NO: 296 is an optionally modified phenylalanine (Phe),
the 5th amino acid residue of SEQ ID NO: 296 is an optionally modified tryptophan (Trp),
the 7th amino acid residue of SEQ ID NO: 296 is an optionally modified tyrosine (Tyr),
the 8th amino acid residue of SEQ ID NO: 296 is an optionally modified tyrosine (Tyr),
the 11th amino acid residue of SEQ ID NO: 296 is an optionally modified arginine (Arg) or an optionally modified lysine (Lys),
the 12th amino acid residue of SEQ ID NO: 296 is an optionally modified arginine (Arg) or an optionally modified lysine (Lys), or
the 13th amino acid residue of SEQ ID NO: 296 is an optionally modified tyrosine (Tyr) or an optionally modified phenylalanine (Phe).

6. The conjugate or a salt thereof according to any one of claims 1 to 5, wherein the peptide that binds to a transferrin receptor is a peptide in which
the 3rd amino acid residue of SEQ ID NO: 296 is phenylalanine (Phe), methylated phenylalanine (MeF), or N-methyl-3-chloro-L-phenylalanine (MeF3C),
the 5th amino acid residue of SEQ ID NO: 296 is tryptophan (Trp) or methylated tryptophan (MeW),
the 7th amino acid residue of SEQ ID NO: 296 is tyrosine (Tyr),
the 8th amino acid residue of SEQ ID NO: 296 is tyrosine (Tyr) or (S)-2-amino-3-(4-methoxyphenyl)propanoic acid (F4OMe),
the 11th amino acid residue of SEQ ID NO: 296 is arginine (Arg) or lysine (Lys),
the 12th amino acid residue of SEQ ID NO: 296 is arginine (Arg) or D-type arginine (dr), and
the 13th amino acid residue of SEQ ID NO: 296 is tyrosine (Tyr) or phenylalanine (Phe).

7. The conjugate or a salt thereof according to any one of claims 1 to 6, wherein the peptide that binds to a transferrin receptor is a cyclic peptide.

8. The conjugate or a salt thereof according to any one of claims 1 to 7, wherein the peptide that binds to a transferrin receptor consists of 15 amino acid residues.

9. The conjugate or a salt thereof according to any one of claims 2 to 8, wherein the peptide that binds to a transferrin receptor is a peptide consisting of the 1st to the 15th amino acid sequences, and the amino acid sequence site has a cyclic structure.

10. The conjugate or a salt thereof according to any one of claims 1 to 9, wherein the oligonucleotide is an oligonucleotide that induces exon skipping of the dystrophin gene.

11. The conjugate or a salt thereof according to claim 10, wherein the exon is selected from exon 7, exon 8, exon 9, exon 19, exon 23, exon 44, exon 45, exon 46, exon 50, exon 51, exon 52, exon 53, exon 55, or any combination of these exons.

12. The conjugate or a salt thereof according to claim 11, wherein the exon is exon 44.

13. The conjugate or a salt thereof according to claim 11, wherein the exon is exon 53.

14. The conjugate or a salt thereof according to any one of claims 1 to 13, wherein a sugar moiety and/or a phosphate binding site of at least one nucleotide constituting the compound comprising an oligonucleotide is modified.

15. The conjugate or a salt thereof according to any one of claims 1 to 14, wherein the compound comprising an oligonucleotide comprises an antisense oligonucleotide of 10 to 35 bases in length.

16. The conjugate or a salt thereof according to any one of claims 1 to 15, wherein the compound comprising an oligonucleotide comprises an antisense oligonucleotide of 16 to 30 bases in length.

17. The conjugate or a salt thereof according to any one of claims 1 to 16, wherein the compound comprising an oligonucleotide comprises an antisense oligonucleotide of 18 to 25 bases in length.

18. The conjugate or a salt thereof according to any one of claims 15 to 17, wherein the antisense oligonucleotide comprises a sequence complementary to 12 or more continuous nucleotides in the target region of dystrophin mRNA.

19. The conjugate or a salt thereof according to claim 18, wherein the target region of the dystrophin mRNA is TACAAGAACACCTTCAGAACCGGAGGCAACAGTTG (SEQ ID NO: 647).

20. The conjugate or a salt thereof according to any one of claims 15 to 19, wherein the antisense oligonucleotide consists of a sequence complementary to GAACACCTTCAGAACCGGAGGCAAC (SEQ ID NO: 648).

21. The conjugate or a salt thereof according to any one of claims 15 to 19, wherein the antisense oligonucleotide consists of a sequence complementary to GAACACCTTCAGAACCGGAGG (SEQ ID NO: 649).

22. The conjugate or a salt thereof according to any one of claims 18 to 21, wherein the complementary sequence is not less than 90% complementary.

23. The conjugate or a salt thereof according to any one of claims 18 to 21, wherein the complementary sequence is 100% complementary.

24. The conjugate or a salt thereof according to any one of claims 15 to 23, wherein the antisense oligonucleotide is a phosphorodiamidatemorpholino oligomer.

25. The conjugate or a salt thereof according to any one of claims 1 to 24, wherein the compound comprising an oligonucleotide is a compound composed of an antisense oligonucleotide.

26. The conjugate or a salt thereof according to any one of claims 1 to 24, wherein the compound comprising an oligonucleotide consists of a nucleic acid complex comprising an antisense oligonucleotide and a second nucleic acid strand complementary to all or part of the antisense oligonucleotide, wherein the antisense oligonucleotide anneals to the second nucleic acid strand.

27. The conjugate or a salt thereof according to any one of claims 1 to 26, wherein the peptide and the compound comprising an oligonucleotide are bound via a linker.

28. A composition comprising the conjugate or a salt thereof according to any one of claims 1 to 27, for delivering the conjugate or a salt thereof to at least one selected from cardiac muscle and skeletal muscle.

29. A medicament comprising the conjugate or a salt thereof according to any one of claims 1 to 27 as an active ingredient.

30. The medicament according to claim 29, which is a prophylactic or therapeutic agent for Duchenne muscular dystrophy.

31. The conjugate or a salt thereof according to any one of claims 1 to 27, for use in the prophylaxis or treatment of Duchenne muscular dystrophy.

32. A method for the prophylaxis or treatment of Duchenne muscular dystrophy in a mammal, comprising administering the conjugate or a salt thereof according to any one of claims 1 to 27 to the mammal.
